# EUROPEAN PATENT APPLICATION

(11) **EP 4 471 099 A1**
(43) Date of publication of application: **04.12.2024**
(21) Application number: 23305850.2
(22) Date of filing: 30.05.2023
(51) Int. Cl.: C09D 4/06, C07C 261/02, C08F 290/06, C08F 2/48

(54) **SELF-CROSSLINKABLE URETHANE (METH)ACRYLATES**

(71) Applicant: ARKEMA FRANCE, 92700 Colombes (FR)
(72) Inventor: HERR, Donald, EXTON, 19341 (US); HE, Yuhong, EXTON, 19341 (US); LI, Ling, EXTON, 19341 (US); PLENDERLEITH, Richard, WETHERBY LS22 7NS (GB); SQUIRES, Kelly, WETHERBY LS22 7NS (GB); SEHNAL, Petr, WETHERBY LS22 7NS (GB)
(74) Representative: Arkema Patent

(57) **Abstract**

The invention relates to urethane (meth)acrylates which comprise a chromophore moiety into their backbone. These urethane (meth)acrylates are self-crosslinkable (or self-reactive), i.e. they are capable of polymerizing after exposure to actinic radiation without any added photoinitiator.

## Description

### FIELD OF THE INVENTION

The invention relates to urethane (meth)acrylates which comprise a chromophore moiety into their backbone. These urethane (meth)acrylates are self-crosslinkable (or self-reactive), i.e. they are capable of polymerizing after exposure to actinic radiation without any added radical initiator or initiating system.

### BACKGROUND

Urethane (meth)acrylates (also referred to as (meth)acrylate-functional polyurethane oligomers and polymers) are widely used resin components in various applications including ultraviolet and electron beam (UV/EB) curable materials. This utility stems from the wide range of physical and mechanical properties which can be obtained from such resins. In turn, this range of possible properties is derived from the wide variety of backbone functionality available to make urethane (meth)acrylates, combined with the high light and thermal reactivity of pendant or telechelic (meth)acrylate functionality.

In conventional actinic radiation curable systems, including those that contain urethane (meth)acrylates, the radicals which initiate cure are created through the photodecomposition of a photoinitiator. A wide variety of low-molecular weight photoinitiators are commercially available and well known to those skilled in the art. A drawback of using low-molecular weight photoinitiators is that low-molecular weight photofragments inevitably remain in the cured composition. This is true whether one uses fragmentation photoinitiators (Norrish Type I) or hydrogen abstraction bimolecular photoinitiators (Norrish Type II). Low-molecular weight photofragments are often the source of undesirable odor, volatile organic content, and extractable/leachable molecules. Oligomeric/polymeric photoinitiators are available to a much more limited extent and can sometimes be used to reduce extractable components and odor associated with the photoinitiator package. The use of oligomeric/polymeric photoinitiators is not an ideal solution, as trade-offs typically include reduced cure speed, limited resin compatibility, and increased formulation cost due to reduced active content of polymeric photoinitiation systems. In some cases, polymeric photoinitiators will also unfavorably affect cured material properties due to the unoptimized nature of the initiator polymer backbone for any given application.

US 2014/0316060 A1 discloses polymers including both dye and photoinitiator moieties.

US 7,148,265 discloses a polymer comprising α,β-unsaturation and a photoinitiator joined to the polymer. The photoinitiator is chosen from an imidazole dimer, an anthraquinone, a naphthaquinone, a ketal, a triazine compound, or combinations thereof. While it is possible that the oligomer-bound photoinitiator can participate in crosslinking when irradiated with UV light via a radical coupling type mechanism, this polymer-radical based coupling cure mechanism is inefficient, especially in systems which target high crosslink densities and rapid cure speed (as is common for many UV curable applications). It is thus beneficial to incorporate (meth)acrylate functional groups onto said inherently photosensitive urethanes in order to create oligomers which exhibit high cure speeds and can produce relatively high crosslink density.

### SUMMARY

In order to overcome the above-mentioned deficiencies, the photoinitiator may be built into the backbone of a urethane (meth)acrylate. In such a case, the photoinitiator is still part of a polymer/oligomer, but it has been incorporated into an optimized, functional part of the final cured product. As such, no portion of the formulation is wasted on unoptimized polymer content associated with a discrete, separately added polymeric photoinitiator. When the photoinitiator is incorporated in a high molecular weight oligomer/polymer component in this way, desirable effects noted above such as low odor, low or zero photoinitiator extractables, and ease of formulation processing can be realized while eliminating the limitations associated with traditional polymeric photoinitiators and low molecular weight photoinitiators. In terms of compatibility, introducing the photoinitiator into the urethane (meth)acrylate backbone ensures solubility or compatibility of the photoinitiator, unlike the case of added polymeric photoinitiator. This is because the polymeric photoinitiators may not be compatible with the components of the curable composition they are intended to initiate. Both Norrish Type I and Type II photoinitiators can be incorporated into the urethane (meth)acrylate in this way, but in order to truly access zero extractable photofragments, Norrish Type II photoinitiators may be preferred.

The inventors have found that it is beneficial to include (meth)acrylate groups on a polymer/oligomer that contains bound photoinitiator groups. Preferred for this invention are pendant or telechelic (meth)acrylate functionalities. Oligomers/polymers designed in this manner provide many desirable features. As noted above, the bound Norrish Type II photoinitiators are especially desirable since they provide systems with low odor, zero extractable photofragments, and optimized mechanical properties. By incorporating (meth)acrylate groups onto the same polymer/oligomer as the initiating chromophores, the resulting product rapidly reacts upon exposure to actinic radiation to form a cured product via its highly reactive (meth)acrylate groups and the incorporated photoinitiator moieties.

The invention thus relates to a self-crosslinkable urethane (meth)acrylate comprising:
- at least one (meth)acrylate-functionalized moiety ("ACR");
- at least two polyurethane moieties ("UU");
- at least one chromophore moiety ("Q");
- optionally at least one chain-extending moiety ("EXT").

The invention also relates to a process for preparing a self-crosslinkable urethane (meth)acrylate wherein the process comprises reacting:
a) a hydroxyl-functionalized (meth)acrylate component;
b) a polyisocyanate component;
c) a hydroxyl-functionalized photoinitiator component; and
d) optionally, a polyol component.

The invention further relates to a curable composition comprising:
A) a self-crosslinkable urethane (meth)acrylate according to the invention; and
B) optionally a polymerizable component other than A).

The invention further relates to a process for the preparation of a cured product, comprising curing the curable composition according to the invention, preferably by exposing the curable composition to actinic radiation such as UV, near-UV, visible, infrared, near-infrared and/or electron beam radiation, more particularly by exposing the polymerizable composition to a LED light source.

The invention further relates to a substrate on which the curable composition according to the invention has been applied and cured, in particular the substrate is a food and beverage packaging, a pharmaceutical packaging, a textile, a nail, a tooth, a medical device, a food and beverage processing equipment, a water pipe or a toy.

The invention further relates to a use of the self-crosslinkable urethane (meth)acrylate according to the invention, as a photoinitiating system in a curable composition, in particular in a UV or LED-curable composition.

The invention further relates to a use of the self-crosslinkable urethane (meth)acrylate according to the invention, to obtain a cured product having a reduced amount of extractables.

### BRIEF DESCRIPTION OF THE FIGURES

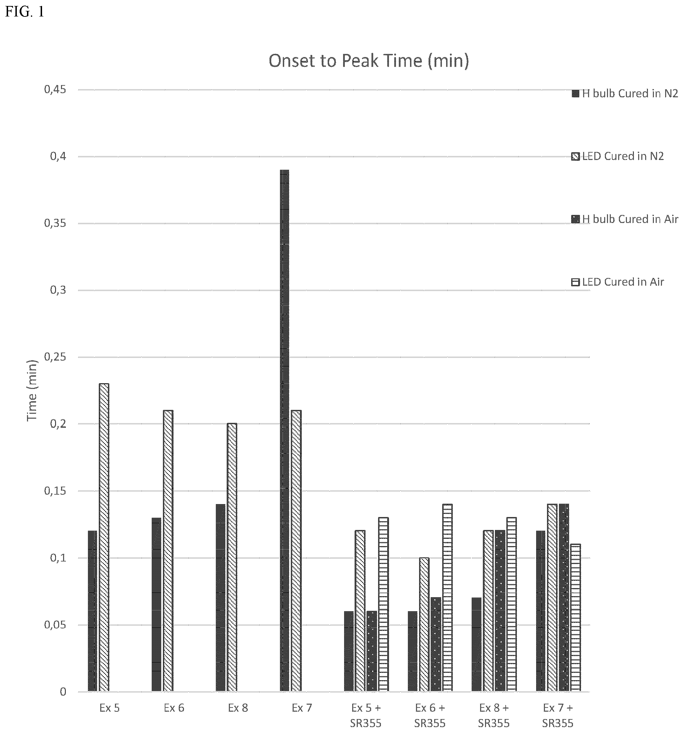

FIG.1: Plot of photo DSC analysis of self-crosslinkable urethane (meth)acrylates of Examples 5-8 neat, and as blends with di(trimethylolpropane) tetraacrylate in a 1:1 weight ratio.

### DETAILED DESCRIPTION

### Definitions

In the present application, the term "comprise(s) alan" means "comprise(s) one or more".

Unless mentioned otherwise, the % by weight in a compound or a composition are expressed based on the weight of the compound, respectively of the composition.

Molecular weights herein are number averaged molecular weights measured using gel permeation chromatography with polystyrene standards, unless stated otherwise.

As used herein, the term, "independently selected" with respect to choices of groups in a structure, means that if there are more than one of the group present in the structure, they need not all be the same, as long as they are selected from the listed choices. For example, the recitation, "R³ may be independently selected from a direct bond or a linker" means that one R³ in a structure may be a direct bond and another may be a linker for example.

As used herein, the term "urethane (meth)acrylate" means an oligomer comprising one or ore urethane bonds and one or more (meth)acrylate groups.

As used herein, the term "self-crosslinkable urethane (meth)acrylate" means a urethane (meth)acrylate which comprises at least one chromophore moiety into their backbone. Such urethane (meth)acrylates may be capable of polymerizing after exposure to actinic radiation (other than electron beam) without any added radical initiator or initiating system (such as for example a photoinitiator, a peroxide, an azo compound or a redox system).

The term "(meth)acrylate group", is indifferently used herein to refer to an acrylate group or a methacrylate group. An acrylate group corresponds to an acryloyl group of formula -C(=O)-CH=CH₂. A methacrylate group corresponds to a methacryloyl group of formula -C(=O)-C(CH₃)=CH₂.

The term, "residue" as used herein means the group of atoms left in a product after removal of the functional groups that react with another compound to form linkages. For example, the residue of a diol having structure HO-R₃-OH would be understood to be R₃.

The term « C1-C6 » refers to the number of carbon atoms comprised in a specific group or linker. For example, a C1-C6 alkylene is an alkylene comprising from 1 to 6 carbon atoms.

The term « linker » means a plurivalent group comprising at least one carbon atom and/or at least one heteroatom such as O, N or S. A linker may connect at least two moieties of a compound together, in particular 2 to 6 moieties of a compound together. For example, a linker that connects two moieties of a compound together is referred to as a divalent linker, a linker that connects three moieties of a compound together is referred to as a trivalent linker, etc....

The term « aliphatic compound, group or linker » means a non-aromatic compound, group or linker. Compounds, groups or linkers comprising a non-aromatic ring (i.e. a cycloaliphatic ring) are encompassed by the term aliphatic compound, group or linker. It may be linear or branched, saturated or unsaturated, cyclic or acyclic. It may be substituted by one or more groups, for example selected from alkyl, hydroxyl, halogen (Br, Cl, I), isocyanate, carbonyl (=O), amine, carboxylic acid, -C(=O)-OR', -C(=O)-O-C(=O)-R', each R' being independently a C1-C6 alkyl. It may comprise one or more bonds selected from ether, ester, amide, urethane, urea, carbonate, organosiloxane, and mixtures thereof.

The term « aromatic compound, group or linker » means a compound, group or linker comprising at least one aromatic ring (i.e. a ring respecting Hückel's aromaticity rule, such as a phenyl), in particular one, two or three, preferably one or two, aromatic rings. Araliphatic compounds, groups or linkers, i.e. comprising both an aromatic moiety and a non-aromatic moiety, are encompassed by the term aromatic compound, group or linker. It may be substituted by one or more groups as defined for the term « aliphatic compound, group or linker ». It may comprise one or more bonds as defined for the term « aliphatic compound, group or linker ».

The term « hydrocarbon linker » means a linker comprising carbon and hydrogen atoms. Unless mentioned otherwise, a hydrocarbon linker may not comprise atoms other than carbon and hydrogen.

The term « acyclic compound, group or linker » means a compound, group or linker that does not comprise any rings.

The term « cyclic compound, group or linker » means a compound, group or linker that comprises at least one aromatic or non-aromatic ring.

The term « saturated compound, group or linker » means a compound, group or linker that does not comprise any double or triple carbon-carbon bonds.

The term « unsaturated compound, group or linker » means a compound, group or linker that comprises one or more double or triple carbon-carbon bonds, in particular one or more double carbon-carbon bonds.

The term « polyether linker» means a linker comprising one or more ether bonds.

The term « polyester linker» means a linker comprising one or more ester bonds.

The term « polycarbonate linker» means a linker comprising one or more carbonate bonds.

The term « polyorganosiloxane linker» means a linker comprising one or more organosiloxane bonds.

The term « polycaprolactone linker» means a linker comprising one or more units derived from the ring-opening of ε-caprolactone, i.e. one or more units of formula -O-(C=O)-(CH₂)₅-. A polycaprolactone linker is a specific example of a polyester linker.

The term « polydiene linker» means a linker derived from the polymerization of a polydiene, such as butadiene or isoprene. A polydiene linker also encompasses fully or partially hydrogenated polydiene linkers obtained by the hydrogenation of a polydiene linker.

The term « isocyanurate linker» means a linker comprising an isocyanurate moiety, i.e. a moiety according to the following formula:

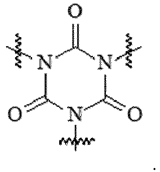

The term « alkyl » means a monovalent saturated acyclic hydrocarbon group of formula -CₙH₂ₙ₊₁ wherein n is 1 to 20. An alkyl may be linear or branched. Examples of alkyl groups include methyl, ethyl, propyl, isopropyl, butyl, isobutyl, tert-butyl, pentyl, hexyl, 2-methylbutyl, 2,2-dimethylpropyl, n-hexyl, 2-methylpentyl, 2,2-dimethylbutyl, n-heptyl, 2-ethylhexyl, and the like.

The term « heteroatom-containing alkyl » means an alkyl comprising one or more heteroatoms independently selected from O, N or S.

The term « cycloalkyl » means a monovalent saturated hydrocarbon group comprising a cycle. Examples of cycloalkyl groups include cyclopentyl, cyclohexyl and isobornyl.

The term « heterocycloalkyl » means a cycloalkyl having at least one ring atom that is a heteroatom selected from O, N or S.

The term « alkenyl » means a monovalent acyclic hydrocarbon group comprising at least one carbon-carbon double bond. An alkenyl may be linear or branched.

The term « alkynyl » means a monovalent acyclic hydrocarbon group comprising at least one carbon-carbon triple bond. An alkynyl may be linear or branched.

The term « aryl » means an optionally substituted polyunsaturated aromatic group. The aryl may contain a single ring (i.e. phenyl) or more than one ring wherein at least one ring is aromatic. When the aryl comprises more than one ring, the rings may be fused, linked via a direct bond (for example biphenyl). The aromatic ring may optionally comprise one to two additional fused rings (i.e. cycloalkyl, heterocycloalkyl or heteroaryl). The term "aryl" also encompasses partially hydrogenated derivatives of the carbocyclic system is described above. Examples include phenyl, naphtyl, biphenyl, phenanthrenyl and naphthacenyl.

The term « heteroaryl » means an aryl having at least one ring atom that is a heteroatom selected from O, N or S.

The term « aralkyl » means an aryl substituted by an alkyl group. An example of an aralkyl group is tolyl.

The term « alkaryl » means an alkyl substituted by an aryl group. An example of an alkaryl group is benzyl (-CH₂-Phenyl).

The term « halogen » means an atom selected from Cl, Br, F and I.

The term « alkoxy » means a group of formula -O-alkyl, wherein the alkyl is as defined above.

The term « aryloxy » means a group of formula -O-aryl, wherein the aryl is as defined above.

The term « thioalkyl » means a group of formula -S-alkyl, wherein the alkyl is as defined above.

The term « thioaryl » means a group of formula -S-aryl, wherein the aryl is as defined above.

The term « aralkyloxy » means a group of formula -O-aralkyl, wherein the aralkyl is as defined above.

The term « alkaryloxy » means a group of formula -O-alkaryl, wherein the alkaryl is as defined above.

The term « alkylene » means a plurivalent linker derived from an alkane of formula CₘH₂ₘ₊₂, wherein m is 1 to 50, by removing one hydrogen atom at each point of attachment of the linker. An alkylene may be divalent, trivalent, tetravalent or have even higher valencies.

The term « alkenylene » means a plurivalent aliphatic linker comprising at least one carbon-carbon double bond.

The term « heteroatom-containing alkylene » means an alkylene comprising one or more heteroatoms independently selected from O, N or S.

The term « oxyalkylene » means a group of formula -O-alkylene-.

The term « alkoxylated alkylene » means a group of formula -(alkylene-O)ₙ₃₉-alkylene-(O-alkylene)ₙ₄₀ wherein n₃₉ and n₄₀ are independently 0 to 100 with the proviso that at least one of n₃₉ and n₄₀ is not 0.

The term « thioalkylene » means a group of formula -S-alkylene-.

The term « ketoalkylene » means a group of formula -C(=O)-alkylene-.

The term « aminoalkylene » means a group of formula -N(R₄₀)-alkylene- wherein R₄₀ is H or an organic group.

The term « carboxyalkylene » means a group of formula -C(=O)-O-alkylene- or -O-C(=O)-alkylene-.

The term « amidoalkylene » means a group of formula -C(=O)-N(R₄₁)-alkylene- or -N(R₄₁)-C(=O)-alkylene- wherein R₄₁ is H or an organic group.

The term « heteroatom-containing alkenylene » means an alkenylene comprising one or more heteroatoms independently selected from O, N or S.

The term « cycloalkylene » means a plurivalent linker comprising a non-aromatic ring. Examples of cycloalkylene groups include cyclopentylene, cyclohexylene and cyclohexylenedimethylene (i.e -CH₂-Cy-CH₂, wherein Cy is a cyclohexylene).

The term « heterocycloalkylene » means a plurivalent linker comprising a non-aromatic ring having at least one ring atom that is a heteroatom selected from O, N or S.

The term « arylene » means a plurivalent linker comprising at least one aromatic ring.

The term « heteroarylene » means a plurivalent linker comprising an aromatic ring having at least one ring atom that is a heteroatom selected from O, N or S.

The term « alkylamino » means an alkyl substituted by at least one amino group.

The term « alkylthiol » means an alkyl substituted by at least one thiol group.

The term « hydroxyalkyl » means an alkyl substituted by at least one hydroxy group.

The term « haloalkyl » means an alkyl substituted by at least one halogen.

The term « perfluoroalkyl » means an alkyl wherein all of the hydrogen atoms are replaced with fluorine atoms.

The term « polyol » means a compound comprising at least two hydroxyl groups.

As used herein, the term "residue of a polyol" means the moiety that is obtained by removing the hydroxy groups of a polyol.

As used herein, the term "polyacid" means a compound bearing at least two carboxylic acid groups.

As used herein, the term "residue of a polyacid" means the moiety that is obtained by removing the carboxylic acid groups of a polyacid.

As used herein, the term "polyamine" means a compound bearing at least two primary and/or secondary amino groups.

As used herein, the term "residue of a polyamine" means the moiety that is obtained by removing the primary and/or secondary amino groups of a polyamine.

As used herein, the term "hydroxy-acid" means a compound bearing at least one hydroxyl group and at least one carboxylic acid group.

As used herein, the term "residue of a hydroxy-acid" means the moiety that is obtained by removing the hydroxyl and carboxylic acid groups of a hydroxyacid.

As used herein, the term "hydroxy-amine" means a compound bearing at least one hydroxy group and at least one primary and/or secondary amino group.

As used herein, the term "residue of a hydroxy-amine" means the moiety that is obtained by removing the hydroxyl and primary and/or secondary amino groups of a hydroxyamine.

The term « hydroxyl group » means a -OH group.

The term « amino group » means a -NRₐ₁R_{b1} group, wherein Rₐ₁ and R_{b1} are independently H or an optionally substituted alkyl. A « primary amino group » means a -NRₐ₁R_{b1} group, wherein Rₐ₁ and R_{b1} are H. A « secondary amino group » means a -NRₐ₁R_{b1} group, wherein Rₐ₁ is H and R_{b1} is an optionally substituted alkyl.

The term « carboxylic acid group » means a -COOH group.

The term « isocyanate group » means a -N=C=O group.

The term « direct bond » means a covalent bond.

The term « ester bond » means a -C(=O)-O- or -O-C(=O)- bond.

The term « ether bond » means a -O- bond.

The term « organosiloxane bond » means a -Si(R_{c1})₂-O- bond, wherein R_{c1} is an organic group, in particular an organic group selected from alkyl, alkoxy and aryl.

The term « carbonate bond » means a -O-C(=O)-O- bond.

The term « urethane or carbamate bond » means a -NH-C(=O)-O- or -O-C(=O)-NH- bond.

The term « polyisocyanate » means a compound comprising at least two isocyanate groups.

The term « optionally substituted compound, group or linker » means a compound, group or linker optionally substituted by one or more groups selected from halogen, alkyl, cycloalkyl, heterocycloalkyl, aryl, heteroaryl, alkoxy, aryloxy, aralkyl, alkaryl, aralkyloxy, alkaryloxy, haloalkyl, -OH, -SH, hydroxyalkyl, thioalkyl, thioaryl, alkylthiol, amino, alkylamino, isocyanate, nitrile, oxo (=O), -C(=O)-R', -O-C(=O)-R', -C(=O)-OR', -C(=O)-N(R')₂, -NR'-C(=O)-R', -C(=O)-O-C(=O)-R' and -SO₂-N(R')₂, each R' being independently H or an optionally substituted group selected from alkyl, aryl and alkylaryl.

### Self-crosslinkable urethane (meth)acrylate

The self-crosslinkable urethane (meth)acrylate of the invention may be described as an oligomer comprising (i.e. made up of) distinct moieties (or subunits) connected to one another to form a backbone. As is often the case with oligomers, the self-crosslinkable urethane (meth)acrylate of the invention may in fact correspond to a mixture of compounds with a distribution of molecular weights or degrees of polymerization (DP).

The self-crosslinkable urethane (meth)acrylate of the invention may comprise at least three distinct types of moieties: a (meth)acrylate-functionalized moiety ACR, a polyurethane moiety UU, a chromophore moiety Q and optionally a chain-extending moiety EXT as detailed below. Such moieties may be bound to one another in any order and at least part of said moieties may be repeated any number of times throughout the backbone of the oligomer.

### (Meth)acrylate-functionalized moiety ACR

The self-crosslinkable urethane (meth)acrylate of the invention comprises at least one (meth)acrylate-functionalized moiety ACR. The self-crosslinkable urethane (meth)acrylate of the invention may comprise more than one (meth)acrylate-functionalized moiety ACR. When this is the case, the (meth)acrylate-functionalized moieties may be identical or distinct from one another.

As used herein, a (meth)acrylate-functionalized moiety means a moiety functionalized with at least one (meth)acrylate group.

Each (meth)acrylate-functionalized moiety ACR may independently bear 1 to 5 (meth)acrylate groups, in particular 1 to 3 (meth)acrylate groups, more particularly 1 (meth)acrylate group.

Each (meth)acrylate-functionalized moiety ACR may correspond to the residue of a hydroxyl-functionalized (meth)acrylate compound (without the OH group(s)). Examples of suitable hydroxyl-functionalized (meth)acrylate compounds are detailed in component a) of the preparation process of the self-crosslinkable urethane (meth)acrylate of the invention.

In particular, each (meth)acrylate-functionalized moiety ACR may independently be according to the following formula (I): 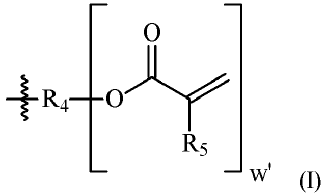 wherein
R₄ is a (w'+1)valent linker;
R₅ is H or methyl;
w' is 1 to 5, in particular 1 to 3, more particularly 1.

In formula (I), R₄ may be a (w'+1)valent linker selected from an aliphatic or aromatic hydrocarbon linker, a polyether linker, a polyester linker, a polycarbonate linker, a polyorganosiloxane linker, a polydiene linker, an isocyanurate linker, and combinations thereof.

In particular, R₄ may be a (w'+1)valent linker selected from an aliphatic or aromatic hydrocarbon linker, a polyether linker, a polyester linker and combinations thereof.

More particularly, R₄ may be a (w'+1)valent linker selected from an alkylene, an alkoxylated alkylene, a polycaprolactone linker and combinations thereof.

In one embodiment, R₄ may be a divalent linker selected from one of formula (IIa) to (VIa):

-(CR₂₂R'₂₂)ₘ- (IIa)

-[(CR₂₃R'₂₃)ₙ-O]ₒ-(CR₂₃R'₂₃)ₙ- (IIIa)

-[(CR₂₄R'₂₄)ₚ-O]_{q}-(CR₂₅R'₂₅)ᵣ-[O-(CR₂₆R'₂₆)_{p'}]_{q'}- (IVa)

-[(CR₂₇R'₂₇)ₛ-C(=O)O]ₜ-(CR₂₈R'₂₈)ᵤ-^{∗} (Va)

-[(CR₂₉R'₂₉)ᵥ-O-C(=O)-(CR₃₀R'₃₀)_{w}-C(=O)-O]ₓ-(CR₂₉R'₂₉)ᵥ- (VIa)

wherein
R₂₂, R'₂₂, R₂₅, R'₂₅, R₂₉, R'₂₉, R₃₀ and R'₃₀ are independently H or alkyl;
R₂₃, R'₂₃, R₂₄, R'₂₄, R₂₆, R'₂₆, R₂₇, R'₂₇, R₂₈ and R'₂₈ are independently H or methyl;
m is 2 to 50;
n, p and p' are independently 2 to 4;
o is 1 to 20;
q and q' are independently 0 to 20 with the proviso that at least one of q and q' is not 0 ;
r is 2 to 20;
s is 3 to 12;
t is 1 to 20;
u is 2 to 8;
v is 2 to 20;
w is 2 to 30;
x is 1 to 20;
the symbol * represents the point of attachment to the (meth)acrylate group.

In particularly, R₄ may be a divalent linker selected from an alkylene such as 1,2-ethylene, 1,2- or 1,3-propylene, 1,2-, 1,3- or 1,4-butylene, 1,5-pentylene, 1,6-hexylene, 1,8-octylene, 1,9-nonylene, 1,10-decylene, 1,12-dodecylene, 1,18-octadecylene, 2-methyl-1,3-propanediyl, 2,2-diethyl-1,3-propanediyl, 3-methyl-1,5-pentanediyl, 3,3-dimethyl-1,5-pentanediyl, 2,2-dimethyl-1,3-propanediyl, 2,4-diethyl-1,5-pentanediyl; an alkoxylated derivative of the aforementioned alkylenes; an esterified derivative of the aforementioned alkylenes; a residue of a di-, tri-, tetra- or polyoxyalkylene (without the OH groups) such as a residue of di-, tri- or tetraethylene glycol, di-, tri- or tetrapropylene glycol, di-, tri- or tetrabutylene glycol, polyethylene glycol, polypropylene glycol, polybutylene glycol, polyethylene glycol-co-propylene glycol); and a residue of a polyester polyol (without the OH groups).

In another embodiment, R₄ may be a trivalent linker according to one of the following formulae (12), (13), (19) and (20), or a tetravalent linker according to one of the following formulae (14) and (15): 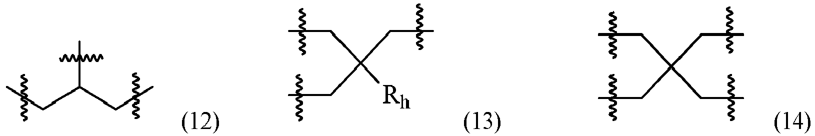 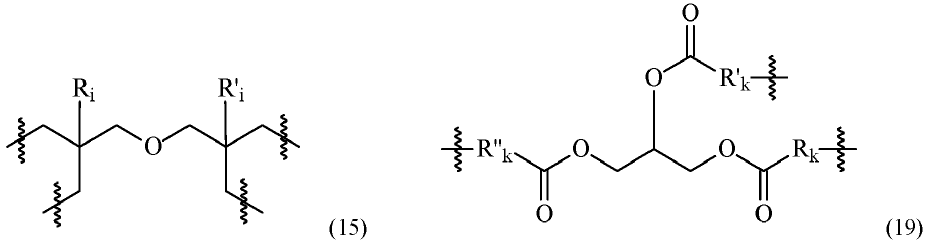 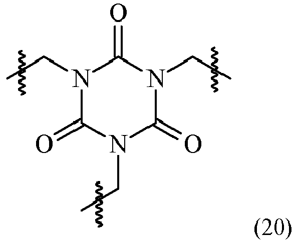 wherein
each Rₕ, Rᵢ and R'ᵢ is independently H or alkyl;
Rₖ, R'ₖ and R"ₖ are independently alkylene or alkenylene.

The total amount of (meth)acrylate-functionalized moieties ACR in the self-crosslinkable urethane (meth)acrylate of the invention may represent from 5 to 85%, in particular from 10 to 80%, more particularly from 15 to 75%, of the total weight of the self-crosslinkable urethane (meth)acrylate.

In one embodiment, the total amount of (meth)acrylate-functionalized moieties ACR in the self-crosslinkable urethane (meth)acrylate of the invention may represent from 50 to 85%, in particular from 55 to 80%, more particularly from 60 to 75%, of the total weight of the self-crosslinkable urethane (meth)acrylate. Such amounts may for example be obtained when the (meth)acrylate-functionalized moiety is derived from a (poly)caprolactone (meth)acrylate.

In another embodiment, the total amount of (meth)acrylate-functionalized moieties ACR in the self-crosslinkable urethane (meth)acrylate of the invention may represent from 5 to 40%, in particular from 10 to 35%, more particularly from 15 to 30%, of the total weight of the self-crosslinkable urethane (meth)acrylate. Such amounts may for example be obtained when the (meth)acrylate-functionalized moiety is derived from a hydroxyalkyl (meth)acrylate.

### Polyurethane moiety UU

The self-crosslinkable urethane (meth)acrylate of the invention comprises at least two polyurethane moieties UU. The polyurethane moieties may be identical or distinct from one another.

As used herein, a polyurethane moiety means a moiety bearing at least two urethane bonds (-NH-C(=O)-O-) connected to one another by a linker.

Each polyurethane moiety UU may independently bear 2 to 3 urethane bonds.

Each polyurethane moiety UU may be derived from a polyisocyanate compound. As used herein, the term "derived from a polyisocyanate compound" means that the polyurethane moiety UU corresponds to the moiety obtained when the polyisocyanate is reacted with a hydroxyl-functionalized compound, i.e. the -NCO groups of the polyisocyanate compound are transformed into urethane bonds (-NH-C(=O)-O-).

Examples of suitable polyisocyanate compounds are detailed in component b) of the preparation process of the self-crosslinkable urethane (meth)acrylate of the invention.

In particular, each polyurethane moiety UU may independently be according to one of the following formula (VIIa) or (VIIb): 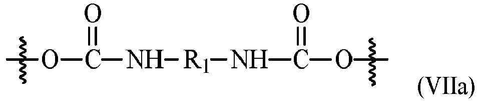 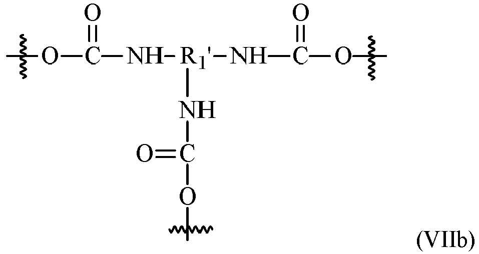 wherein
R₁ and R₁' are independently an aliphatic linker or an aromatic linker.

The self-crosslinkable urethane (meth)acrylate of the invention may comprise distinct types of polyurethane moieties, for example one or more polyurethane moieties of formula (VIIa) and one or polyurethane moieties of formula (VIIb).

In formula (VIIa), R₁ may correspond to the residue of a diisocyanate (without the -NCO groups). In particular, R₁ may be selected from one of the following formulae: 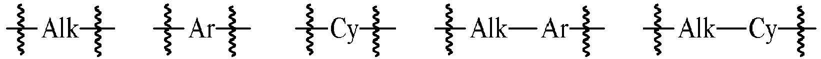 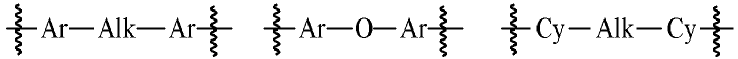 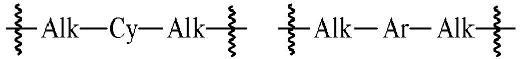 wherein :
Alk is a linear or branched alkylene, in particular methylene, 1,2-ethylene, 1,2- or 1,3-propylene, 1,2-, 1,3- or 1,4-butylene, 1,5-pentylene, 1,6-hexylene, 2,2,4- or 2,4-,4-trimethylhexylene, 1,8-octylene, 1,9-nonylene, 1,10-decylene, 1,12-dodecylene, 1,18-octadecylene;
Ar is an optionally substituted arylene, in particular an optionally substituted arylene selected from phenylene, tolylene, biphenylene, naphthylene, anthracenylene;
Cy is an optionally substituted cycloalkylene, in particular an optionally substituted cyclohexylene.

In formula (VIIb), R₁' may correspond to the residue of a triisocyanate (without the -NCO groups). In particular, R₁' may be selected from one of the following formulae: 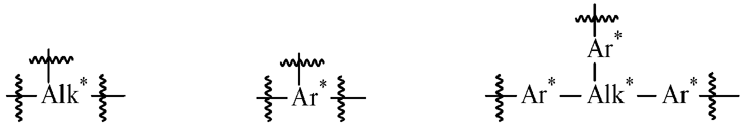 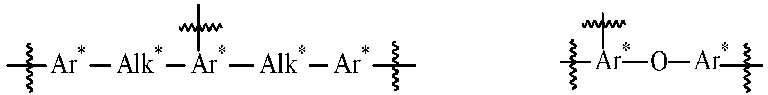 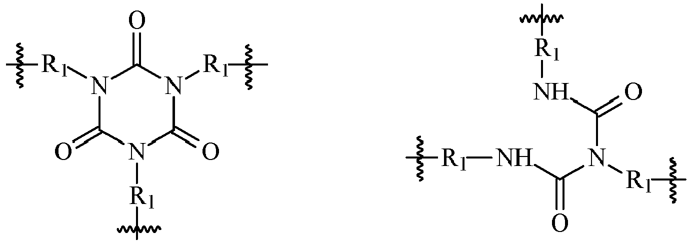 wherein
Alk* is a linear or branched alkylene, in particular methylene, methanetriyl, undecane-1,6,1 1-triyl;
Ar* is an optionally substituted arylene, in particular an optionally substituted arylene selected from phenylene, tolylene and biphenylene;
R₁ is as defined above for formula (VIIa), in particular 1,6-hexamethylene.

In a preferred embodiment, each polyurethane moiety UU may independently be selected from:
- a moiety of formula (VIIa) wherein R₁ is selected from one of the following formulae: 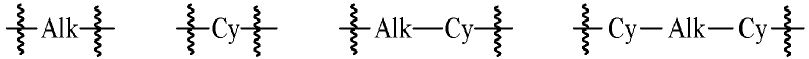 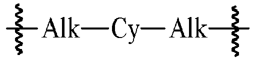 wherein
   Alk is a linear or branched alkylene, in particular methylene, 1,2-ethylene, 1,2- or 1,3-propylene, 1,2-, 1,3- or 1,4-butylene, 1,5-pentylene, 1,6-hexylene, 2,2,4- or 2,4,4-trimethylhexylene, 1,8-octylene, 1,9-nonylene, 1,10-decylene, 1,12-dodecylene, 1,18-octadecylene;
   Cy is an optionally substituted cycloalkylene, in particular an optionally substituted cyclohexylene;
- a moiety of (VIIb) wherein R'₁ corresponds to the following formula: 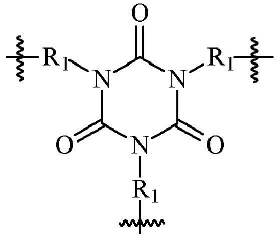 and R₁ is a linear or branched alkylene, in particular 1,6-hexylene;
and mixtures thereof.

The total amount of polyurethane moieties UU in the self-crosslinkable urethane (meth)acrylate of the invention may represent from 10 to 65%, in particular from 15 to 60%, more particularly from 20 to 55%, of the total weight of the self-crosslinkable urethane (meth)acrylate.

### Chromophore moiety Q

The self-crosslinkable urethane (meth)acrylate of the invention comprises at least one chromophore moiety Q. The self-crosslinkable urethane (meth)acrylate of the invention may comprise more than one chromophore moiety Q. When this is the case, the chromophore moieties may be identical or distinct from one another.

As used herein, a chromophore moiety means a moiety comprising a group capable of absorbing light and generating a reactive species useful to initiate a polymerisation reaction. In particular, a chromophore moiety may be a Norrish Type II chromophore moiety, i.e. a chromophore moiety that does not fragment upon exposure to radiation and so will not typically initiate radical-chain polymerisation unless a co-initiator such as an amine synergist is present. Upon exposure to radiation, interaction between the Type II chromophore moiety and the co-initiator leads to the generation of radical species which can initiate the polymerisation of UV-curable resins.

Each chromophore moiety Q may correspond to the residue of a hydroxyl-functionalized photoinitiator compound (without the OH group(s)). Examples of suitable hydroxyl-functionalized photoinitiator compounds are detailed in component c) of the preparation process of the self-crosslinkable urethane (meth)acrylate of the invention.

In particular, each chromophore moiety Q may independently comprise at least one monovalent or divalent photoinitiator moiety PI selected from a benzophenone moiety, a thioxanthone moiety, a xanthone moiety, an acridone moiety, a camphorquinone moiety, a benzil moiety, a coumarin moiety, a ketocoumarin moiety, derivatives thereof and combinations thereof, preferably at least one monovalent or divalent photoinitiator moiety PI selected from a benzophenone moiety or a thioxanthone moiety.

More particularly, each chromophore moiety Q may independently comprise at least one monovalent photoinitiator moiety PI corresponding to one of the following formulae (VIII), (XI), (XIV), (XVII), (XVIII) or (XIX), or at least one divalent photoinitiator moiety PI corresponding to one of the following formulae (IX), (X), (XII), (XIII), (XV) or (XVI): 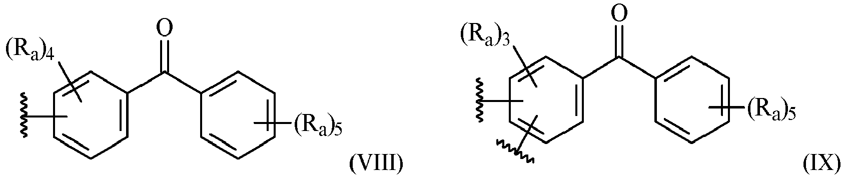 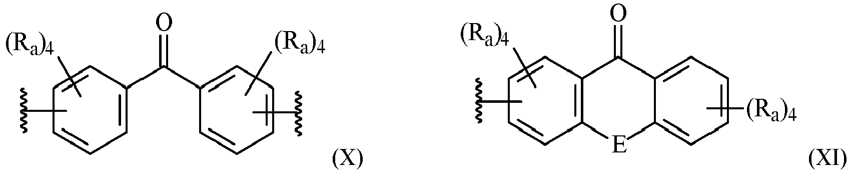 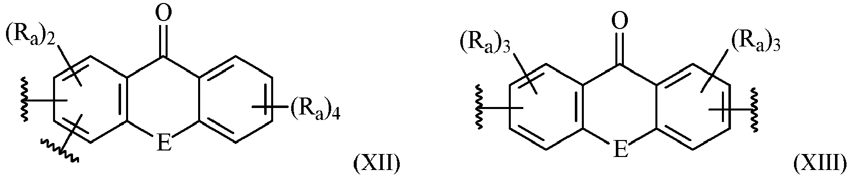 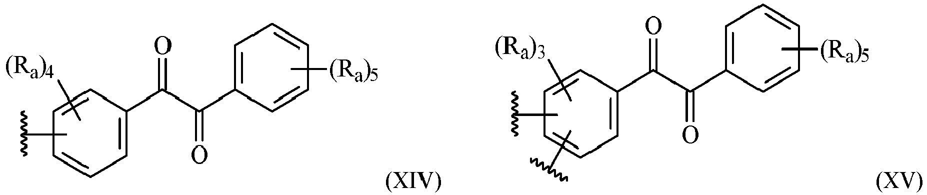 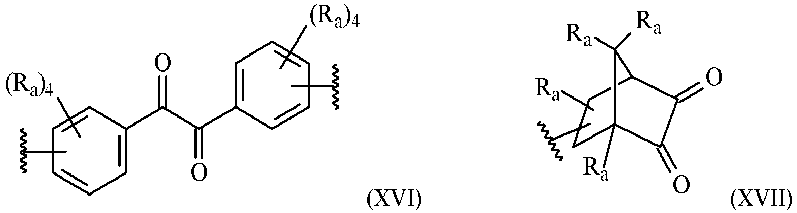 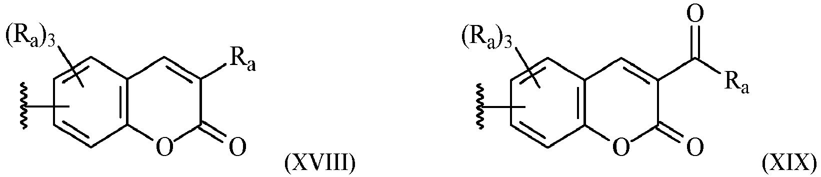 wherein
each E is independently S, O or NR, in particular S;
R is H, an optionally substituted alkyl or an optionally substituted aryl;
each Rₐ is independently H, F, Cl, Br, I, -OR_{b}, -SR_{b}, -N(R_{b})₂, -NO₂, -CN, -C(=O)R_{b},
-O-C(=O)R_{b}, -C(=O)OR_{b}, -C(=O)N(R_{b})₂, -NR_{b}-C(=O)-R_{b}, -SO₂-N(R_{b})₂, or an optionally substituted group selected from the group consisting of alkyl, heteroatom-containing alkyl, cycloalkyl, heterocycloalkyl, alkenyl, alkynyl, aryl, aralkyl, alkaryl and heteroaryl; or two adjacent Rₐ groups may form, with the carbon atoms to which they are attached, a 5 to 8 membered ring; each R_{b} is independently H or an optionally substituted group selected from alkyl, heteroatom-containing alkyl, cycloalkyl, heterocycloalkyl, alkenyl, alkynyl, aryl, aralkyl, alkaryl and heteroaryl.

More particularly still, each chromophore moiety Q may independently comprise at least one monovalent photoinitiator moiety PI corresponding to one of formulae (VIII) or (XI) wherein E is S, or at least one divalent photoinitiator moiety PI corresponding to one of formulae (IX), (X), (XII) or (XIII) wherein E is S.

More particularly yet, each chromophore moiety Q may independently comprise at least one monovalent photoinitiator moiety PI corresponding to one of the following formulae (Villa) or (XIa), or at least one divalent photoinitiator moiety PI corresponding to one of the following formulae (IXa), (Xa), (XIIa) or (XIIIa): 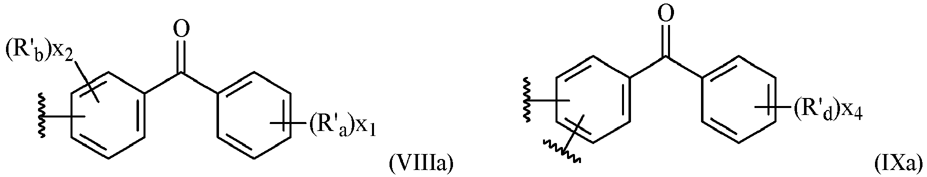 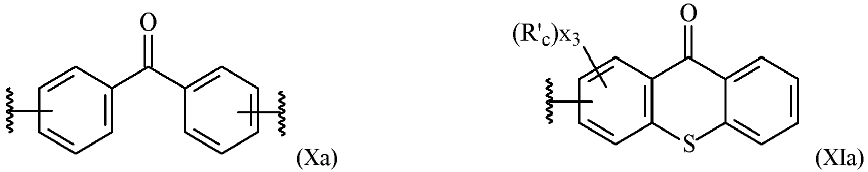 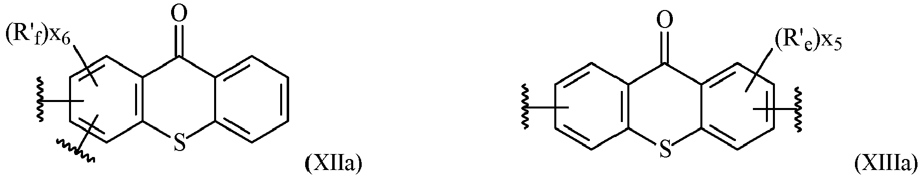 wherein
R'ₐ and R'_{d} are independently alkyl, Ar, -S-Ar or -O-Ar wherein Ar is aryl, in particular methyl, Ph, -S-Ph or -O-Ph wherein Ph is phenyl;
R'_{b} is alkyl, in particular methyl;
R'_{c}, R'ₑ and R'_{f} are independently is halogen, alkoxy, alkyl or -C(=O)-Ar wherein Ar is aryl, in particular F, Cl, methyl, ethyl, isopropyl or -C(=O)-Ph wherein Ph is phenyl;
x₁ and x₄ are independently 0, 1, 2 or 3, in particular 0 or 1;
x₂, x₃ and x₅ are independently 0, 1 or 2, in particular 0 or 1;
x₆ is 0 or 1.

Even more particularly, each chromophore moiety Q may independently comprise at least one monovalent photoinitiator moiety PI corresponding to formula (VIII) or (XI) wherein E is S or at least one divalent photoinitiator moiety PI corresponding to formula (X) or (XIII) wherein E is S.

Yet even more particularly, each chromophore moiety Q may independently comprise at least one monovalent photoinitiator moiety PI corresponding to formula (VIIIa) or (XIa) as defined above or at least one divalent photoinitiator moiety PI corresponding to formula (Xa) or (XIIIa) as defined above.

Each photoinitiator moiety PI may independently be connected to one or more linkers, thus forming a chromophore moiety Q.

In particular, each chromophore moiety Q may independently be according to one of the following formulae (XXa), (XXb) or (XXc): 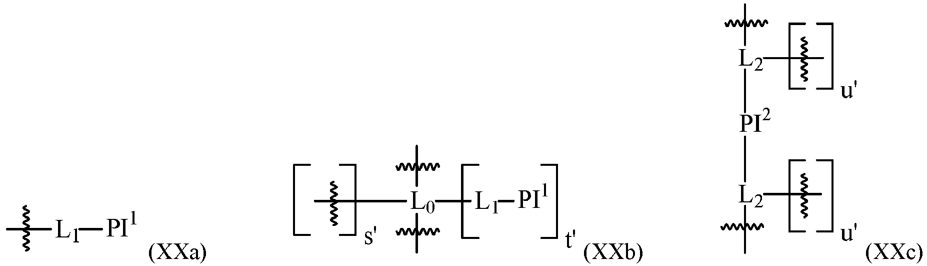 wherein
L₀ is a (s'+t'+2)valent linker;
each L₁ is independently a divalent linker;
each L₂ is independently a (u'+2)valent linker;
each PI¹ is independently a monovalent photoinitiator moiety PI as defined above;
each PI² is independently a divalent photoinitiator moiety PI as defined above;
s' is 0, 1 or 2; in particular 0 or 1;
t' is 1, 2, 3 or 4; in particular 2 or 3;
each u' is independently 0 or 1, in particular 0.

In formulae (XXa) and (XXc), each L₁ and L₂ may independently be selected from a direct bond or a linker comprising 1 to 20, preferably 1 to 10, carbon atoms, said linker optionally comprising one or more heteroatoms, such as O, N or S. In particular, each L₁ and L₂ may independently be selected from a direct bond or a hydrocarbon linker optionally comprising one or more bonds selected from -O-, -S-, -NR₁-, -C(=O)-, -O-C(=O)-, -C(=O)-O-, -NR₁-C(=O)-, -C(=O)-NR₁-, -O-C(=O)-O-, and combinations thereof, wherein R₁ is H, alkyl or aryl.

In formula (XXb), L₀ may be a linker comprising 1 to 20, preferably 1 to 10, carbon atoms, said linker optionally comprising one or more heteroatoms, such as O, N or S. In particular, L₀ may be a hydrocarbon linker optionally comprising one or more bonds selected from -O-, -S-, -NR₁-, -C(=O)-, -O-C(=O)-, -C(=O)-O-, -NR₁-C(=O)-, -C(=O)-NR₁-, -O-C(=O)-O-, and combinations thereof, wherein R₁ is H, alkyl or aryl.

The self-crosslinkable urethane (meth)acrylate of the invention may comprise at least one chromophore moiety Q according to formula (XXa) as defined above. A chromophore moiety Q according to formula (XXa) may have a single photoinitiator moiety PI and a single point of attachment to the backbone of the self-crosslinkable urethane (meth)acrylate.

In formula (XXa), L₁ is preferably selected from direct bond, C1-C6 alkylene, C1-C6 oxyalkylene, C1-C6 thioalkylene, C1-C6 ketoalkylene, C1-C6 aminoalkylene, C1-C6 carboxyalkylene, C1-C6 amidoalkylene, or a combination thereof. In particular, L₁ may be selected from direct bond, alkylene of formula (C-1), oxyalkylene of formula (C-2), thioalkylene of formula (C-3), ketoalkylene of formula (C-4), aminoalkylene of formula (C-5), carboxyalkylene of formula (C-6), amidoalkylene of formula (C-7), alkylene-aminoalkylene of formula (C-8), oxyalkylene-aminoalkylene of formula (C-9), oxyalkylene-amidoalkylene of formula (C-10): 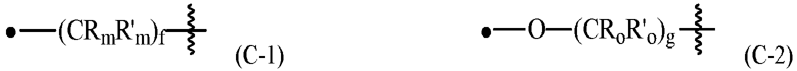 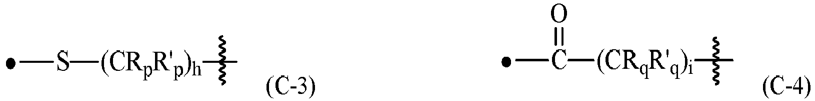 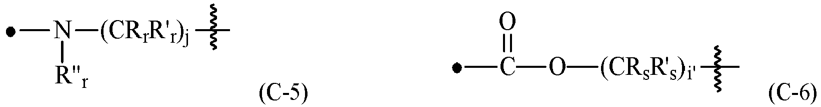 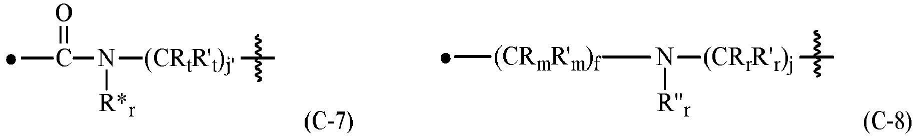 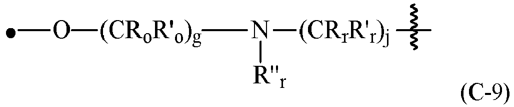 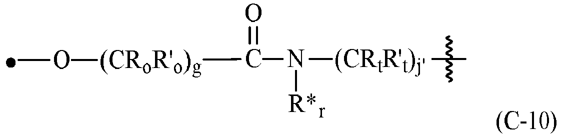 wherein
each Rₘ, R'ₘ, Rₒ, R'ₒ, Rₚ, R'ₚ, R_{q}, R'_{q}, Rᵣ, R'ᵣ, Rₛ, R'ₛ, Rₜ and R'ₜ is independently H or an optionally substituted alkyl, in particular H;
R"r and R^{∗}ᵣ are independently H or an optionally substituted alkyl;
f, g, h, j and j' are independently 1, 2, 3, 4, 5 or 6, in particular 1 or 2;
i and i' are independently 1, 2, 3, 4 or 5, in particular 1;
the symbol ● represents a point of attachment to the PI moiety.

In formula (XXa), L₁ is preferably selected from direct bond, alkylene of formula (C-1), oxyalkylene of formula (C-2), aminoalkylene of formula (C-5), amidoalkylene of formula (C-7), alkylene-aminoalkylene of formula (C-8), oxyalkylene-aminoalkylene of formula (C-9) or oxyalkylene-amidoalkylene of formula (C-10).

In formula (XXa), PI¹ is preferably according to formula (VIII) or (XI) as defined above and E in formula (XI) is preferably S. In formula (XXa), PI¹ is more preferably according to formula (VIIIa) or (XIa) as defined above.

In particular, the self-crosslinkable urethane (meth)acrylate of the invention may comprise at least one chromophore moiety Q corresponding to the following formulae (XXa1) or (XXa2) 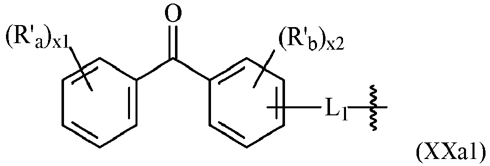 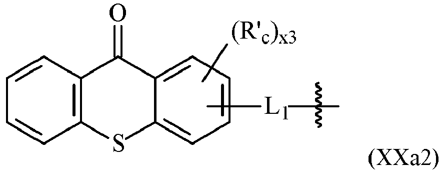 wherein
R'ₐ is alkyl, Ar, -S-Ar or -O-Ar wherein Ar is aryl, in particular methyl, Ph, -S-Ph or -O-Ph wherein Ph is phenyl;
R'_{b} is alkyl, in particular methyl;
R'_{c} is halogen, alkoxy, alkyl or -C(=O)-Ar wherein Ar is aryl, in particular F, Cl, methyl, ethyl, isopropyl or -C(=O)-Ph wherein Ph is phenyl;
x1 is 0, 1, 2 or 3, in particular 0 or 1;
x2 is 0, 1 or 2, in particular 0 or 1;
x3 is 0, 1, or 2, in particular 0 or 1;
L₁ is selected from direct bond, alkylene of formula (C-1), oxyalkylene of formula (C-2), aminoalkylene of formula (C-5), amidoalkylene of formula (C-7), alkylene-aminoalkylene of formula (C-8), oxyalkylene-aminoalkylene of formula (C-9) or oxyalkylene-amidoalkylene of formula (C-10).

More particularly, the self-crosslinkable urethane (meth)acrylate of the invention may comprise at least one chromophore moiety Q corresponding to one of the following formulae (XXa3)-(XXa17):

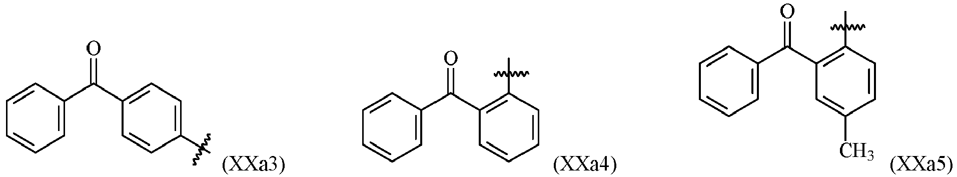

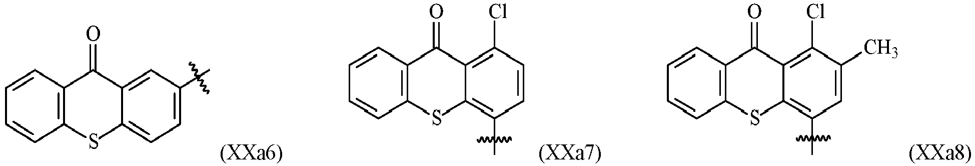

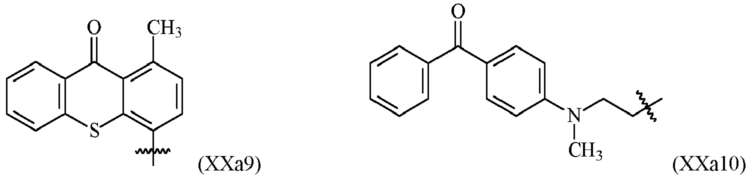

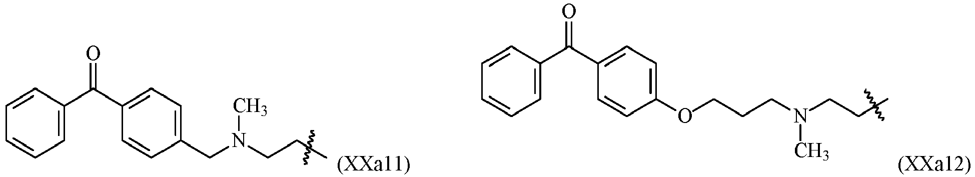

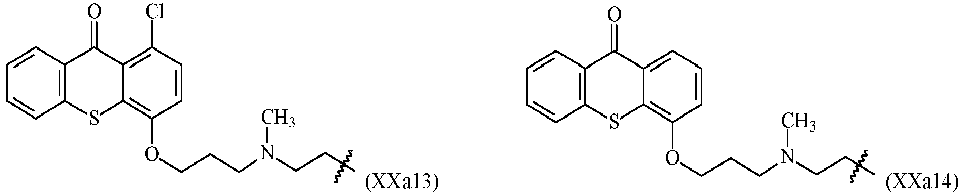

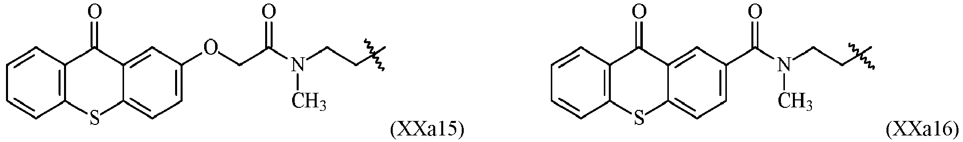

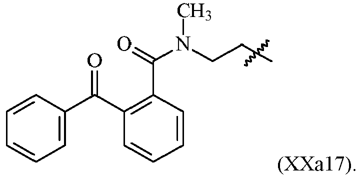

The self-crosslinkable urethane (meth)acrylate of the invention may comprise at least one chromophore moiety Q according to formula (XXc) as defined above. A chromophore moiety Q according to formula (XXc) may have a single photoinitiator moiety PI and at least two points of attachment, preferably two points of attachment, to the backbone of the self-crosslinkable urethane (meth)acrylate.

In formula (XXc), each L₂ is preferably independently selected from direct bond, C1-C6 alkylene, C1-C6 oxyalkylene, C1-C6 thioalkylene, C1-C6 ketoalkylene, C1-C6 aminoalkylene, C1-C6 carboxyalkylene, C1-C6 amidoalkylene, or a combination thereof. In particular, each L₂ may be selected from direct bond, alkylene of formula (C-1) as defined above, oxyalkylene of formula (C-2) as defined above, thioalkylene of formula (C-3) as defined above, ketoalkylene of formula (C-4) as defined above, aminoalkylene of formula (C-5) as defined above, carboxyalkylene of formula (C-6) as defined above, amidoalkylene of formula (C-7) as defined above, alkylene-aminoalkylene of formula (C-8) as defined above, oxyalkylene-aminoalkylene of formula (C-9) as defined above, or oxyalkylene-amidoalkylene of formula (C-10) as defined above. More particularly, each L₂ is independently selected from direct bond, amidoalkylene of formula (C-7) as defined above, or oxyalkylene-amidoalkylene of formula (C-10) as defined above.

In formula (XXc), each u' is preferably 0.

In formula (XXc), PI² is preferably according to formula (IX), (X), (XII) or (XIII) as defined above and E in formula (XII) and (XIII) is preferably S. In formula (XXc), PI² is more preferably according to formula (IXa), (Xa), (XIIa) or (XIIIa) as defined above.

In particular, the self-crosslinkable urethane (meth)acrylate of the invention may comprise at least one chromophore moiety Q according to one of the following formulae (XXc1)-(XXc4): 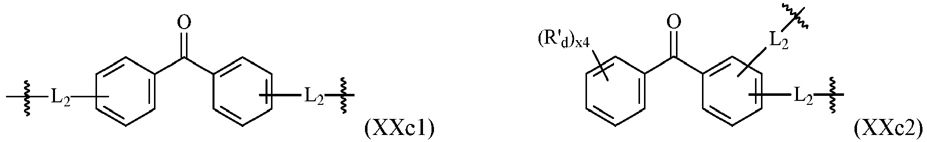 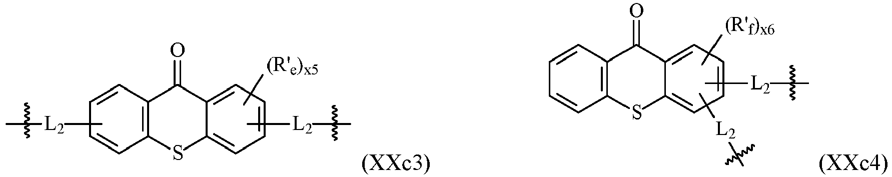 wherein
R'_{d} is alkyl, Ar, -S-Ar or -O-Ar wherein Ar is aryl, in particular methyl, Ph, -S-Ph or -O-Ph wherein Ph is phenyl;
R'ₑ and R'_{f} are independently halogen, alkoxy, alkyl or -C(=O)-Ar wherein Ar is aryl, in particular F, Cl, methyl, ethyl, isopropyl or -C(=O)-Ph wherein Ph is phenyl;
x4 is 0, 1, 2 or 3, in particular 0 or 1;
x5 is 0, 1 or 2, in particular 0 or 1;
x6 is 0 or 1;
each L₂ is independently selected from direct bond, amidoalkylene of formula (C-7) as defined above, or oxyalkylene-amidoalkylene of formula (C-10) as defined above.

More particularly, the self-crosslinkable urethane (meth)acrylate of the invention may comprise at least one chromophore moiety Q according to one of the following formulae (XXc5)-(XXc13):

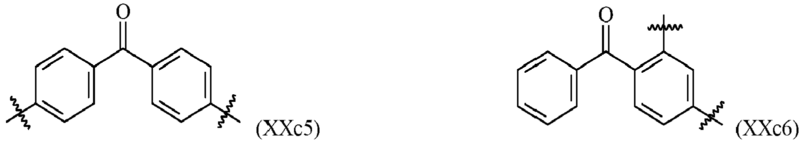

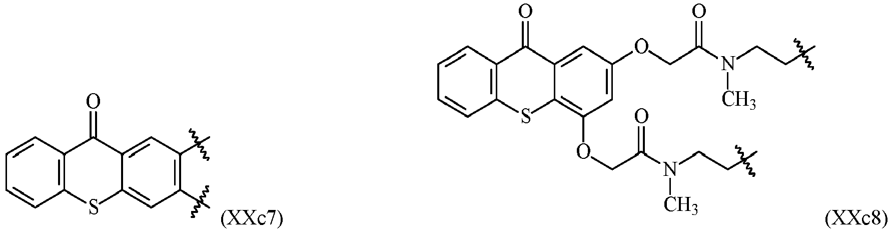

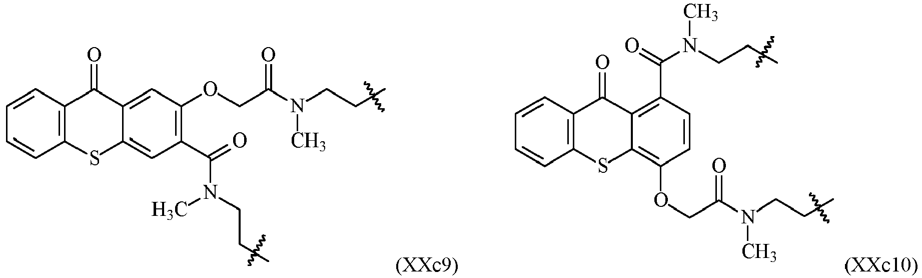

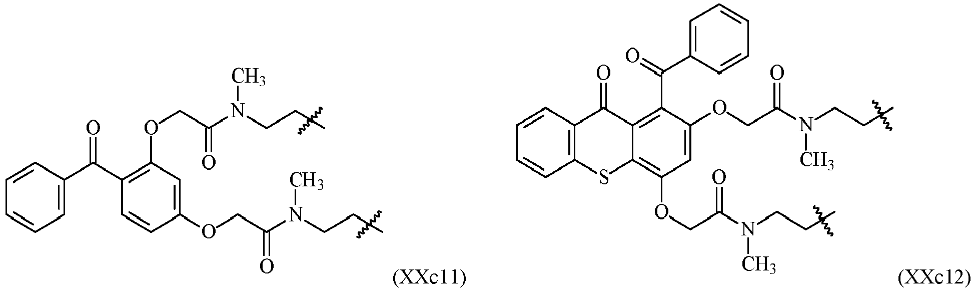

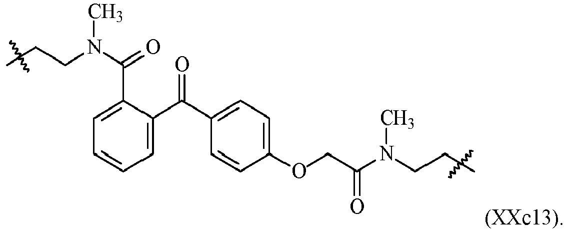

The self-crosslinkable urethane (meth)acrylate of the invention may comprise at least one chromophore moiety Q according to formula (XXb) as defined above. A chromophore moiety Q according to formula (XXb) as defined above may have at least one photoinitiator moiety PI and at least two points of attachment to the backbone of the self-crosslinkable urethane (meth)acrylate.

In one embodiment, the self-crosslinkable urethane (meth)acrylate of the invention may comprise at least one chromophore moiety Q according to formula (XXb) wherein said chromophore moiety Q has at least two photoinitiator moieties PI and at least two points of attachment to the backbone of the self-crosslinkable urethane (meth)acrylate.

In particular, the self-crosslinkable urethane (meth)acrylate of the invention may comprise at least one chromophore moiety Q according to formula (XXb) wherein said chromophore moiety Q has a number of photoinitiator moieties PI that is equal to the number of points of attachment to the backbone of the self-crosslinkable urethane (meth)acrylate.

More particularly, the self-crosslinkable urethane (meth)acrylate of the invention may comprise at least one chromophore moiety Q according to formula (XXI): 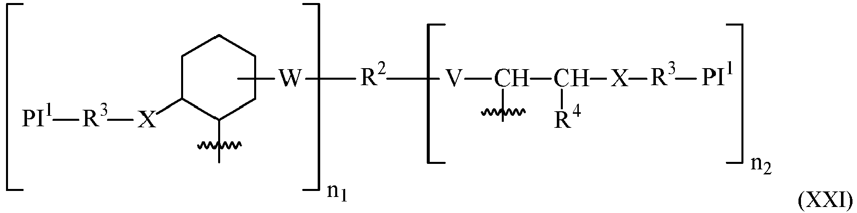 wherein
n₁ is 0, 1, 2, 3 or 4;
n₂ is 0, 1, 2, 3 or 4;
the sum n₁ + n₂ is equal to 1, 2, 3 or 4;
each PI¹ is independently a monovalent photoinitiator moiety PI as defined above; each X is independently -NR¹-, -O-, -S-, *-C(=O)-O-, or ^{∗}-C(=O)-NR¹-;
each V is independently direct bond, #-O-CH₂-, #-C(=O)-O-CH₂-, #-NR⁵-CH₂- or #-C(=O)-NR⁵-CH₂-;
each W is independently direct bond, -CH₂-O-C(=O))-# or -C(=O)-O-#;
each R¹ is independently H, alkyl or aryl;
R² is a direct bond or a linker;
each R³ is independently a direct bond or a linker;
each R⁴ is independently H, an optionally substituted alkyl or an optionally substituted alkenyl;
each R⁵ is independently H, alkyl, aryl or a group according to the following formula: 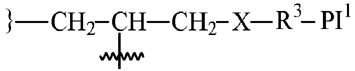 wherein X, R³ and PI¹ are as defined above and the symbol } represents the point of attachment to the nitrogen atom;
the symbol * represents a point of attachment to R³;
the symbol 4 represents a point of attachment to R².

In formula (XXI), the value of n₁ is 0, 1, 2, 3 or 4. In particular, the value of n₁ may be 0, 1 or 2, more particularly n₁ may be 0.

In formula (XXI), the value of n₂ is 0, 1, 2, 3 or 4. In particular, the value of n₂ may be 2, 3 or 4, more particularly n₂ may be 2 or 3.

In formula (XXI), the sum n₁ + n₂ is equal to 1, 2, 3 or 4. In particular, the sum n₁ + n₂ may be equal to 2, 3 or 4, more particularly the sum n₁ + n₂ may be equal to 2 or 3. If the sum n₁ + n₂ is equal to 1, then n₁ is preferably 0, n₂ is preferably 1, V is preferably #-NR⁵-CH₂- or 4-C(=O)-NR⁵-CH₂- and R⁵ is preferably a group according to the following formula:

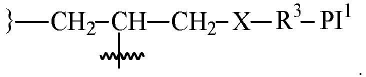

In one embodiment, the value of n₁ may be 0 and the value of n₂ may be 1, 2, 3 or 4. In particular, the value of n₁ may be 0, the value of n₂ may be 2 or 3. In another embodiment, the value of n₂ may be 0 and the value of n₁ may be 2, 3 or 4. In particular, the value of n₂ may be 0, the value of n₁ may be 2. In another embodiment, the value of n₁ may be 1, 2 or 3 and the value of n₂ may be 1, 2 or 3. In particular, the value of n₁ may be 1 and the value of n₂ may be 1 or 2.

In a preferred embodiment, n₂ is other than 0 and R² bears at least one moiety corresponding to the following formula: 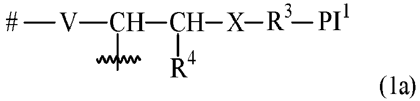 wherein PI¹, V, X, R³ and R⁴ are as defined herein and the symbol 4 represents a point of attachment to R².

R² may bear at least one moiety of formula (1a) wherein V is #-O-CH₂- and R⁴ is H. Such a moiety may be derived from the ring-opening of a glycidyl ether group.

R² may bear at least one moiety of formula (1a) wherein V is #-(C=O)-O-CH₂- and R⁴ is H. Such a moiety may be derived from the ring-opening of a glycidyl ester group.

R² may bear at least one moiety of formula (1a) wherein V is #-NR⁵-CH₂-, R⁴ is H and R⁵ is as defined above. Such a moiety may be derived from the ring-opening of a glycidyl amine group.

R² may bear at least one moiety of formula (1a) wherein V is #-(C=O)-NR⁵-CH₂-, R⁴ is H and R⁵ is as defined above. Such a moiety may be derived from the ring-opening of a glycidyl amide group.

R² may bear at least one moiety of formula (1a) wherein V is a direct bond and R⁴ is H, an optionally substituted alkyl or an optionally substituted alkenyl. Such a moiety may be derived from the ring-opening of an epoxy group which is derived from the epoxidation of a non-cyclic carbon-carbon double bond.

R² may bear 1, 2, 3 or 4 or four moieties of formula (1a). Such moieties may be identical or different from one another. For example, R² may bear 2, 3 or 4 moieties of formula (1a) wherein V is #-O-CH₂- and R⁴ is H. Alternatively, R² may bear 2, 3 or 4 moieties of formula (1a) wherein V is #-(C=O)-O-CH₂- and R⁴ is H. Alternatively, R² may bear 1 or 2 moieties of formula (1a) wherein V is #-NR⁵-CH₂- and R⁴ is H. Alternatively, R² may bear 1 or 2 moieties of formula (1a) wherein V is #-(C=O)-NR⁵-CH₂- and R⁴ is H. Alternatively, R² may bear two or three moieties of formula (1a) wherein V is a direct bond and R⁴ is H or an optionally substituted alkyl. Alternatively, R² may bear at least two moieties of formula (1a), wherein the moieties differ from one another due to the nature of V and/or R⁴.

In another embodiment, n₁ is other than 0 and R² bears at least one moiety according to the following formula (2a): 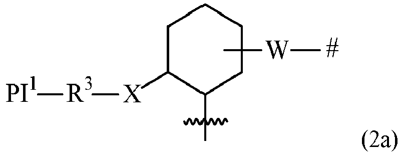 wherein PI¹, X, W and R³ are as defined herein and the symbol 4 represents a point of attachment to R².

Such a moiety may be derived from the ring-opening of a cycloaliphatic epoxide group.

R² may bear at least one moiety of formula (2a) wherein W is -CH₂-O-C(=O))-#.

R² may bear at least one moiety of formula (2a) wherein W is -C(=O)-O-#.

R² may bear at least one moiety of formula (2a) wherein W is a direct bond.

R² may bear 2, 3 or 4 or four moieties of formula (2a). Such moieties may be identical or different from one another. For example, R² may bear 2, 3 or 4 moieties of formula (2a) wherein W is -CH₂-O-C(=O))-#. Alternatively, R² may bear 2, 3 or 4 moieties of formula (2a) wherein W is -C(=O)-O-#. Alternatively, R² may bear at least two moieties of formula (2a), wherein the moieties differ from one another due to the nature of W.

In yet another embodiment, n₁ and n₂ are both other than 0 and R² bears at least one moiety according to formula (1a) and at least one moiety according to formula (2a). In particular, R² may bear 1 moiety according to formula (1a) wherein V is a direct bond and R⁴ is H and 1 moiety of formula (2a) wherein W is a direct bond. Alternatively, R² may bear 1 moiety according to formula (1a) wherein V is #-O-CH₂- and R⁴ is H and one moiety of formula (2a) wherein W is a direct bond. Alternatively, R² may bear 2 moieties according to formula (1a) wherein V is #-(C=O)-O-CH₂- and R⁴ is H and 1 moiety of formula (2a) wherein W is a direct bond.

In particular, formula (XXI) may correspond to one of the following formulae (XXII) to (XXXV): 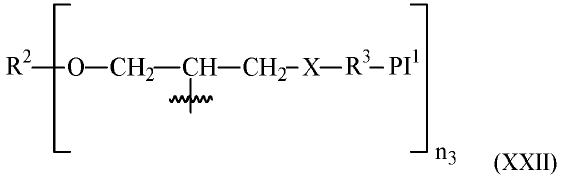 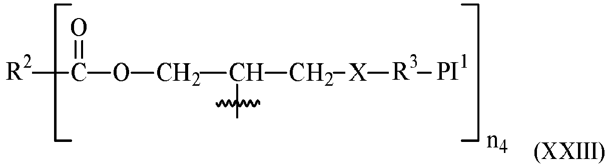 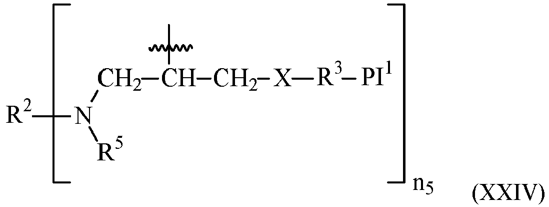 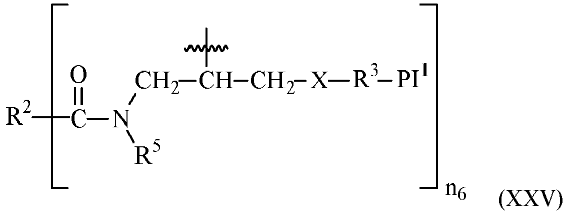 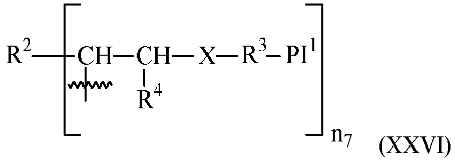 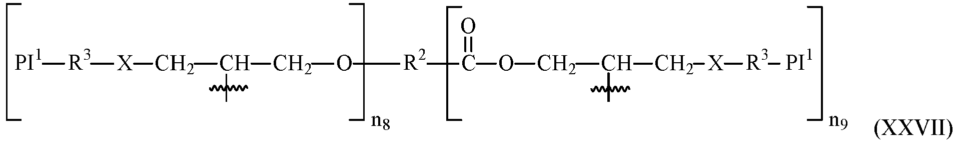 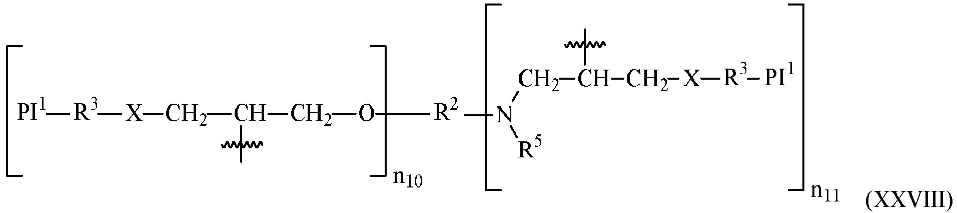 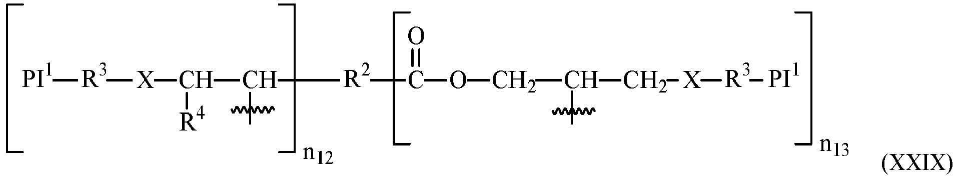 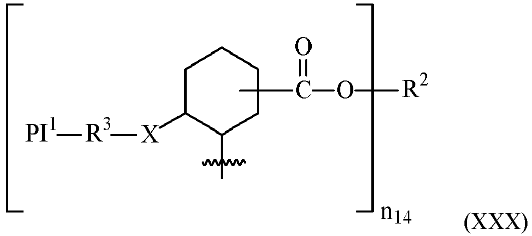 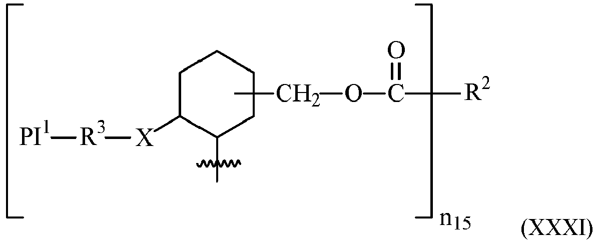 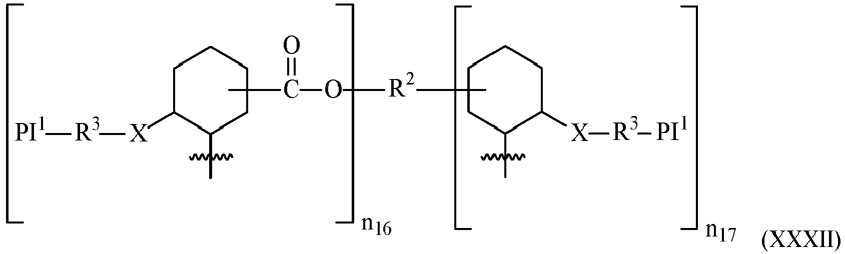 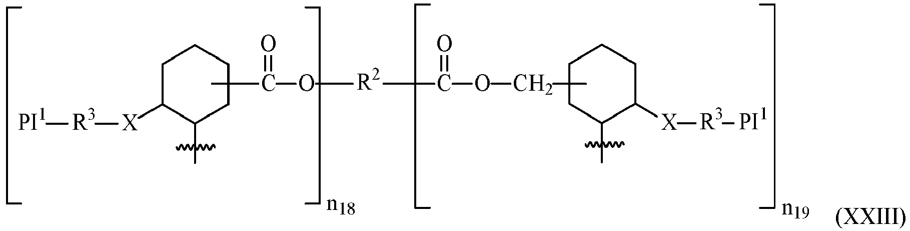 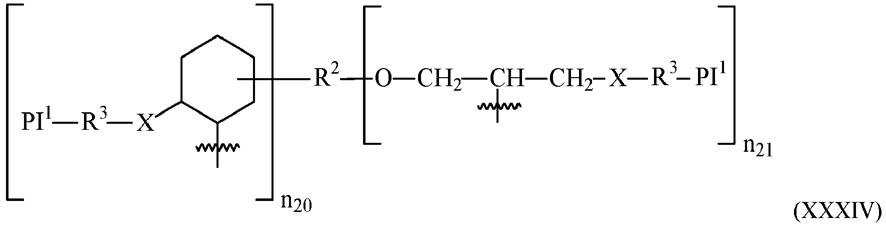 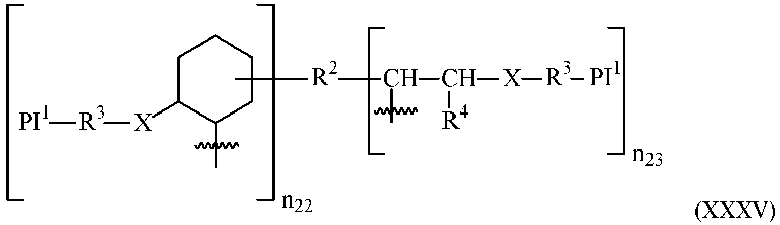 wherein
PI¹, X, R², R³, R⁴ and R⁵ are as defined herein;
n₃, n₄, n₇, n₁₄ and n₁₅ are independently 2, 3 or 4, preferably 2 or 3;
n₅ and n₆ are independently 1 or 2, preferably 2;
n₈ is 1, 2, or 3 preferably 2;
n₉ is 1, 2 or 3, preferably 1;
n₁₀, n₁₁, n₁₆, n₁₇, n₁₈, n₁₉, n₂₀, n₂₁, n₂₂ and n₂₃ are independently 1 or 2, preferably 1;
n₁₂ and n₁₃ are independently 1, 2 or 3, preferably 1.

Chromophore moieties according to one of formulae (XXII), (XXIII), (XXIV), (XXVI), (XXVII), (XXVIII) and (XXIX) are preferred. Chromophore moieties according to one of formulae (XXII), (XXIII), (XXIV) and (XXVI) are particularly preferred.

In formulae (XXI) to (XXXV), each R³ is independently a direct bond or a linker.

In particular, each R³ may independently be a direct bond or a divalent linker comprising 1 to 15, preferably 1 to 10, carbon atoms. Said linker may further optionally comprise one or more heteroatoms, such as O, N or S. For example, the linker may be a hydrocarbon linker optionally comprising one or more bonds selected from -O-, -S-, -NR₁-, -C(=O)-, -O-C(=O)-, -C(=O)-O-, -NR₁-C(=O)-, -C(=O)-NR₁-, -O-C(=O)-O-, -NR₁-C(=O)-O-, -O-C(=O)-NR₁-, and combinations thereof, wherein R₁ is H, alkyl or aryl.

More particularly, each R³ may independently be:
- a direct bond;
- an acyclic linker which may be saturated or unsaturated, in particular an acyclic linker having from 1 to 6, from 1 to 4 or from 1 to 2 carbon atoms; or
- a cyclic linker which may be aromatic or non-aromatic, monocyclic or polycyclic, in particular a cyclic linker having from 6 to 10, from 7 to 9 or from 7 to 8 carbon atoms.

More particularly still, each R³ may independently be selected from direct bond, C1-C6 alkylene, C1-C6 oxyalkylene, C1-C6 alkenylene, C1-C6 thioalkylene, C1-C6 ketoalkylene or C1-C6 aminoalkylene.

Even more particularly, each R³ may independently be selected from direct bond, alkylene of formula (C-11), oxyalkylene of formula (C-12), thioalkylene of formula (C-13), ketoalkylene of formula (C-14) or aminoalkylene of formula (C-15): 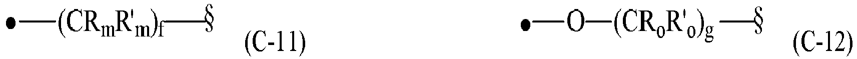 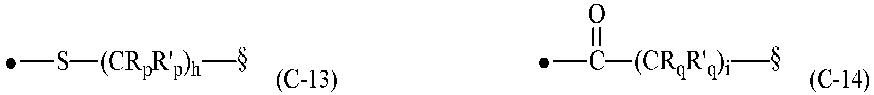 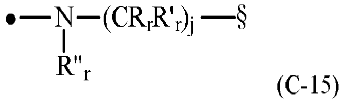 wherein
each Rₘ, R'ₘ, Rₒ, R'ₒ, Rₚ, R'ₚ, R_{q}, R'_{q}, Rᵣ and R'ᵣ is independently H or an optionally substituted alkyl, in particular H;
R"ᵣ is H or an optionally substituted alkyl;
f, g, h and j are independently 1, 2, 3, 4, 5 or 6, in particular 1 or 2;
i is 1, 2, 3, 4 or 5, in particular 1;
the symbol ● represents a point of attachment to the PI¹ moiety;
the symbol § represents a point of attachment to the X moiety.

Even more particularly still, each R³ may independently be selected from direct bond, alkylene of formula (C-11), oxyalkylene of formula (C-12) as defined above and aminoalkylene of formula (C-15) as defined above.

Even more particularly yet, each R³ may independently be selected from direct bond, alkylene of formula (C-11) and oxyalkylene of formula (C-12) as defined above.

In formulae (XXI) to (XXXV), each linker R³ is connected to X. X may correspond to the residue of an epoxide-reactive group, i.e. a group that is capable of opening an epoxide ring. Examples of epoxide reactive groups are alcohols, thiols, primary and secondary amines, carboxylic acids and amides.

In formulae (XXI) to (XXXV), each X is independently -NR¹-, -O-, -S-, *-C(=O)-O-, or ^{∗}-C(=O)-NR¹-; each R¹ is independently H, alkyl or aryl; and the symbol * represents a point of attachment to R³.

In particular, each X may independently be -NR¹-, -O-, or *-C(=O)-O-. More particularly each X may independently be -NR¹- or ^{∗}-C(=O)-O-.

In formulae (XXI) to (XXXV), each R² is independently a direct bond or a linker. When R² is a linker, the linker may be divalent, trivalent or tetravalent. When R² is a linker, the linker may be aliphatic or aromatic, in particular aliphatic. R² may comprise 0 to 20, in particular 0 to 15, more particularly 0 to 10, carbon atoms. R² may comprise 0 to 2, in particular 0 to 1, more particularly 0, oxygen atoms. R² may comprise 0 to 2, in particular 0 to 1, more particularly 0, heteroatoms atoms selected from O, N or S. The molecular weight of R² may be lower than 500 g/mol, in particular lower than 400 g/mol, more particularly lower than 300 g/mol, even more particularly lower than 250 g/mol, more particularly still lower than 200 g/mol.

In particular, each R² may independently be a direct bond or a linker selected from the group consisting of alkylene, heteroatom-containing alkylene, alkenylene, heteroatom-containing alkenylene, cycloalkylene, heterocycloalkylene, arylene, heteroarylene, and combinations thereof.

More particularly, each R² may independently be a direct bond or a linker selected from the group consisting of alkylene, heteroatom-containing alkylene, cycloalkylene, arylene, heteroarylene and combinations thereof.

In one embodiment, R² may be an aromatic linker such as arylene, heteroarylene or combinations thereof. In such a case, R² is preferably arylene.

For example, R² may be represented by one of the following formulae (3) to (9): 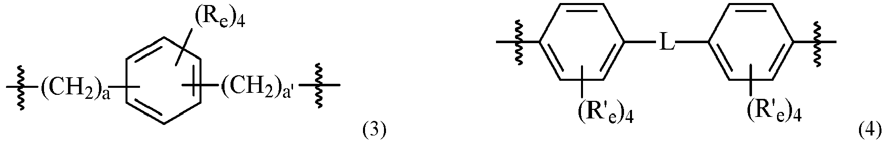 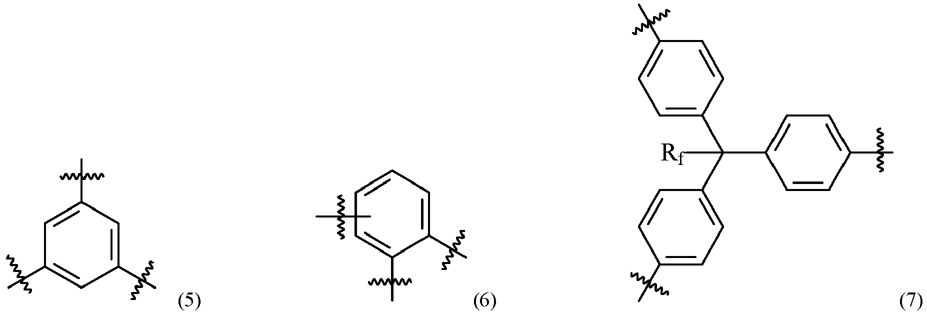 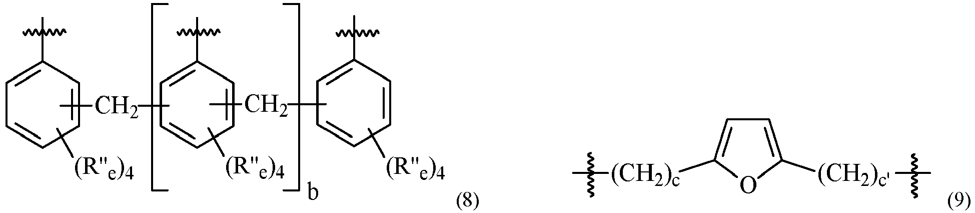
wherein L is a direct bond or a linker;
Rₑ, R'ₑ and R"ₑ are independently selected from H, alkyl, cycloalkyl, aryl, alkaryl, aralkyl, alkoxy, -C(=O)O-Alkyl and a halogen atom;
R_{f} is H or methyl;
a, a', c and c' are independently 0 or 1;
b is 1 or 2.

In particular, R² may be represented by one of formulae (3) or (4) as defined above, preferably by formula (4). When R² is represented by formula (4), L may be selected from direct bond, alkylene, -CR'₃R'₄-, -C(=O)-, -C(=O)-O-Alk-O-C(=O)-, -SO-, -SO₂-, -C(=CCl₂)- and -CR'₅R'₆-Ph-CR'₇R'₈-; wherein
R'₃ and R'₄ are independently selected from H, alkyl, cycloalkyl, aryl, haloalkyl and perfluoroalkyl, or R'₃ and R'₄, with the carbon atoms to which they are attached, may form a ring;
R'₅, R'₆, R'₇ and R'₈ are independently selected from H, alkyl, cycloalkyl, aryl, haloalkyl and perfluoroalkyl;
Alk is an alkylene;
Ph is a phenylene optionally substituted with one or more groups selected from alkyl, cycloalkyl, aryl and a halogen atom.

More particularly R² may correspond to the residue of an optionally substituted aromatic polyol, polyacid, polyamine, hydroxy-acid or hydroxy-amine (without the OH, COOH and/or amino groups).

For example R² may be the residue of an optionally substituted aromatic polyol, polyacid, polyamine, hydroxy-acid or hydroxy-amine comprising 1 to 3, preferably 1 or 2, aromatic rings, such as pyrocatechol, resorcinol, cardol, (hydroxymethyl)phenol, benzenedimethanol, a hydroxybenzoic acid, phthalic acid, terephthalic acid, isophthalic acid, naphthalene dicarboxylic acid, a bisphenol, a biphenyl diol, phloroglucinol, pyrogallol, tris(hydroxyphenyl)methane, tris(hydroxyphenyl)ethane, trimellitic acid, gallic acid, a condensation product of an optionally substituted aromatic alcohol and formaldehyde (also referred to as novolak), phenylene diamine, toluylene diamine, xylylene diamine, diaminobiphenyl, diaminodiphenyl ether, diaminodiphenyl methane, diaminodiphenyl sulfone, aminophenol, (aminophenoxy)phenol.

Even more particularly, R² may be the residue of an optionally substituted bisphenol. Examples of suitable bisphenols are bisphenol A, bisphenol AP, bisphenol AF, bisphenol B, bisphenol BP, bisphenol C, bisphenol C2, bisphenol F, bisphenol G, bisphenol M, bisphenol S, bisphenol P, bisphenol PH, bisphenol TMC, bisphenol-Z, dinitrobisphenol A, tetrabromobisphenol A and combinations thereof.

In another embodiment, R² may be an aliphatic linker, such as alkylene, heteroatom-containing alkylene, cycloalkylene, heterocycloalkylene, or combinations thereof. In such a case, R² is preferably alkylene, heteroatom-containing alkylene, cycloalkylene or combinations thereof.

For example, R² may be represented by one of the following formulae (10) to (20): 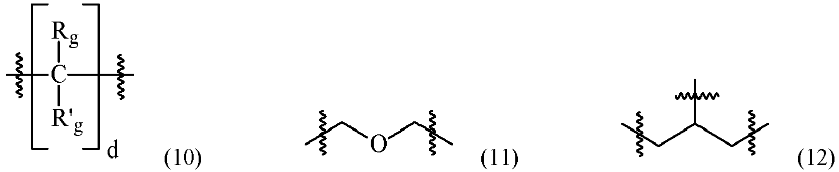 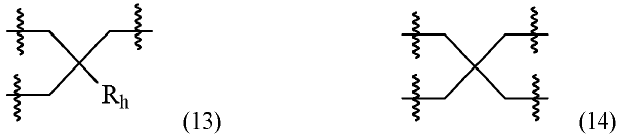 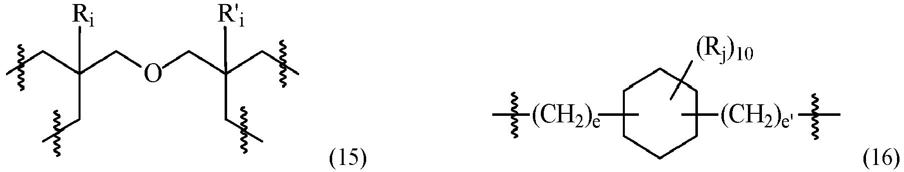 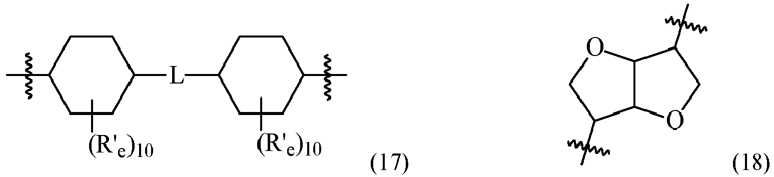 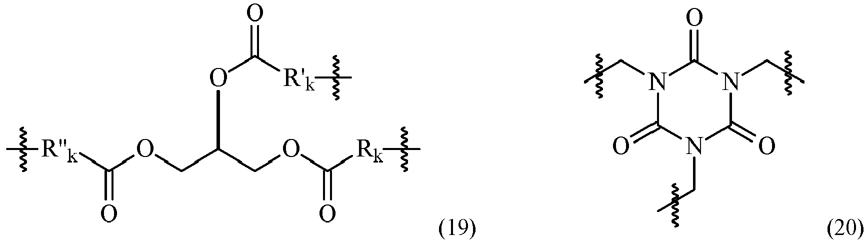 wherein
R'ₑ and L are as defined above for formula (4);
each R_{g}, R'_{g}, Rₕ, Rᵢ and R'ᵢ is independently H or alkyl;
each Rⱼ is independently H, alkyl, cycloalkyl, aryl, alkaryl, aralkyl, alkoxy, -C(=O)O-Alkyl and a halogen atom;
Rₖ, R'ₖ and R"ₖ are independently alkylene or alkenylene;
d is 1 to 12;
e and e' are independently 0 or 1.

In particular, R² may be represented by one of formulae (10), (11), (12), (13), (15), (16), (17) or (19) as defined above, preferably by one of formulae (10), (11) or (12).

More particularly, R² may correspond to the residue of an optionally substituted aliphatic polyol, polyacid, polyamine, hydroxy-acid or hydroxy-amine (without the OH, COOH and/or amino groups).

For example, R² may be the residue of an optionally substituted aliphatic polyol, polyacid, polyamine, hydroxy-acid or hydroxy-amine selected from ethylene glycol, 1,2- or 1,3-propylene glycol, 1,2-, 1,3- or 1,4-butylene glycol, 1,5-pentanediol, 1,6-hexanediol, 1,7-heptanediol, 1,8-octanediol, 1,9-nonanediol, 1,10-decanediol, 1,12-dodecanediol, 2-methyl-1,3-propanediol, neopentyl glycol, 2,2-diethyl-1,3-propanediol, 3-methyl-1,5-pentanediol, 3,3-dimethyl-1,5-pentanediol, 2,4-diethyl-1,5-pentanediol, 3,3-butylethyl-1,5-pentane diol, cyclohexanediol, cyclohexane-1,4-dimethanol, norbornene dimethanol, norbornane dimethanol, tricyclodecanediol, tricyclodecane dimethanol, hydrogenated bisphenol A, B, F or S, trimethylolmethane, trimethylolethane, trimethylolpropane, di(trimethylolpropane), pentaerythritol, glycerol, a dianhydrohexitol (i.e. isosorbide, isomannide, isoidide), a hydroxylated vegetable oil, tris(2-hydroxyethyl)isocyanurate, 1,2-ethylenediamine, 1,3-propylenediamine, 1,4-tetramethylenediamine, 1,5-pentamethylenediamine, 1,6-hexamethylenediamine, 1,8-octamethylenediamine, 1,12-dodecamethylenediamine, isophoronediamine, diaminocyclohexane, methylcyclohexane diamine, bis(aminomethyl)cyclohexane, diaminodecahydronaphthalene, dimethyldiaminodicyclohexylmethane, diaminodicyclohexylmethane, bis(aminomethyl)norbornane, malonic acid, succinic acid, 2-methylsuccinic acid, 2,2-dimethylsuccinic acid, glutaric acid, 3,3-diethylglutaric acid, adipic acid, pimelic acid, suberic acid, azelaic acid, sebacic acid, dodecanedioic acid, citric acid, 1,2-, 1,3- or 1,4 cyclohexane dicarboxylic acid, glycolic acid, 12-hydroxystearic acid, ethanolamine, diethanolamine.

Alternatively, R² may correspond to the residue of an epoxidized polyunsaturated compound, i.e. a compound having at least two non-aromatic carbon-carbon double bonds which have been epoxidized. As used herein, the term "residue of epoxidized polyunsaturated compound" means the residue that is obtained by removing the terminal groups containing an epoxide ring from the compound. For example, if the epoxidized polyunsaturated compound has the following formula: 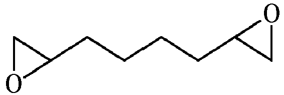 then the residue of the epoxidized polyunsaturated compound corresponds to the following formula:

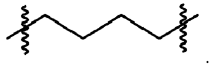

Alternatively, R² may correspond to the residue of an epoxidized oil. As used herein, the term "residue of an epoxidized oil" means the residue that is obtained by removing the terminal groups containing an epoxide from the oil. For example, if the epoxidized oil has the following formula: 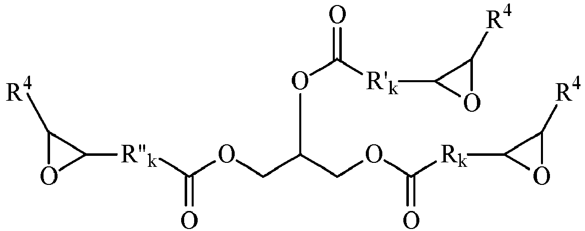 wherein
each R⁴ is independently H, an optionally substituted alkyl or an optionally substituted alkenyl; Rₖ, R'ₖ and R"ₖ are independently alkylene or alkenylene;
then the residue of the epoxidized oil corresponds to the following formula:

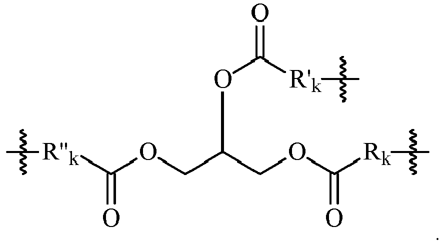

In formulae (XXI), (XXVI), (XXIX) and (XXXV), each R⁴ is independently H, an optionally substituted alkyl or an optionally substituted alkenyl.

In one embodiment, each R⁴ is H. In another embodiment, each R⁴ is an optionally substituted alkyl or an optionally substituted alkenyl. In yet another embodiment, part of the R⁴ groups are H and the other part of the R⁴ groups are an optionally substituted alkyl or an optionally substituted alkenyl. Preferably, each R⁴ is H.

In formulae (XXIV), (XXV) and (XXVIII), each R⁵ is independently H, alkyl, aryl or a group according to the following formula: 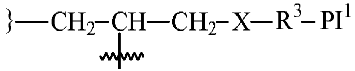 wherein X, R³ and PI¹ are as defined above and the symbol } represents the point of attachment to the nitrogen atom.

In particular, each R⁵ may be H, alkyl or a group according to the following formula:

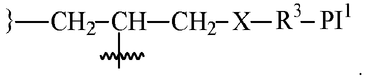

In formulae (XXI) to (XXXV), each PI¹ is preferably independently according to formula (VIII) or (XI) as defined above and E in formula (XI) is preferably S. In formula (XXa), each PI¹ is more preferably independently according to formula (VIIIa) or (XIa) as defined above.

In a particularly preferred embodiment, the self-crosslinkable urethane (meth)acrylate comprises at least one chromophore moiety Q according to one of the following formulae (XXII), (XXIII), (XXIV) or (XXVI): 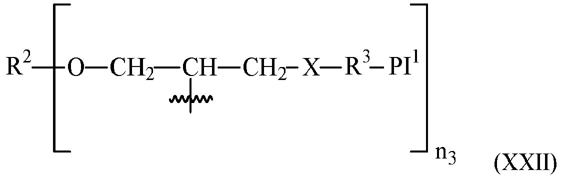 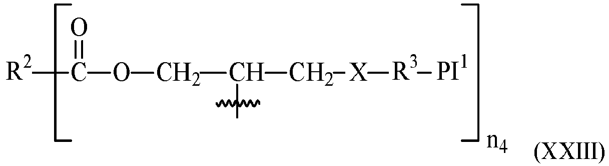 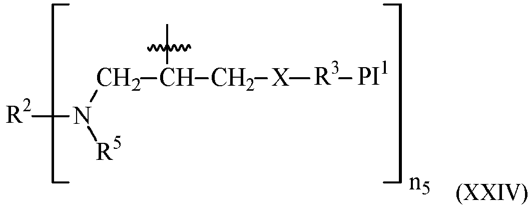 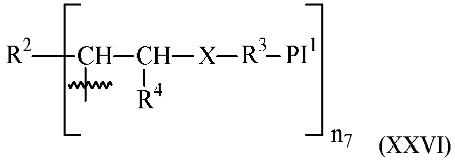 wherein
n₃, n₄, n₅ and n₇ are independently 2, 3 or 4, preferably 2 or 3;
each PI¹ is independently a monovalent photoinitiator moiety PI according to formula (VIII) or (XI) as defined above, preferably each PI¹ is a monovalent photoinitiator moiety PI according to formula (Villa) or (XIa) as defined above;
each X is independently -NR¹-, -O- or ^{∗}-C(=O)-O-, preferably -NR¹- or ^{∗}-C(=O)-O-;
each R¹ is independently H or alkyl;
each R² is independently an alkylene or a heteroatom-containing alkylene;
each R³ is independently a direct bond, an alkylene of formula (C-11) as defined above, an oxyalkylene of formula (C-12) as defined above or an aminoalkylene of formula (C-15) as defined above, preferably a direct bond, an alkylene of formula (C-11) or an oxyalkylene of formula (C-12);
each R⁴ is H;
each R⁵ is independently H, alkyl or a group according to the following formula: 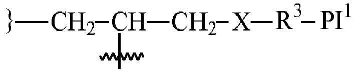 wherein X, R³ and PI¹ are as defined above and the symbol } represents the point of attachment to the nitrogen atom;
the symbol * represents a point of attachment to R³.

In a particularly preferred embodiment, the self-crosslinkable urethane (meth)acrylate comprises at least one chromophore moiety Q according to one of the following formulae (XXb1)-(XXb9):

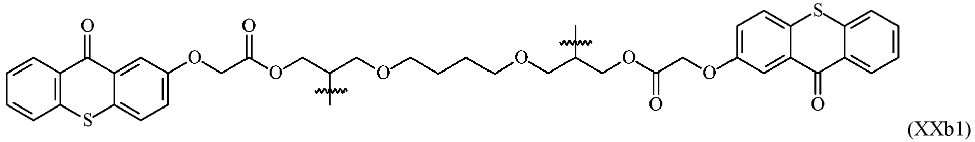

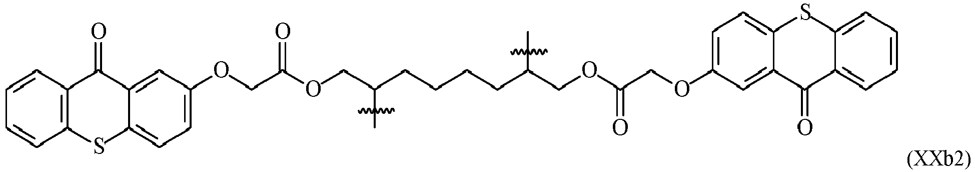

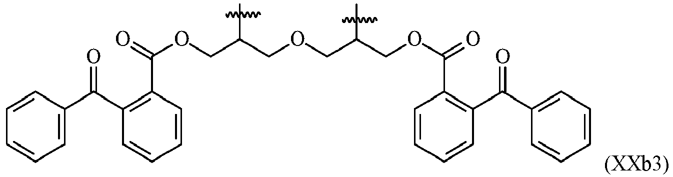

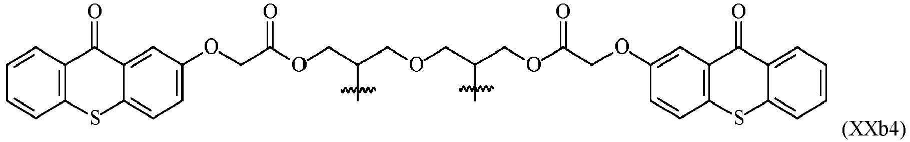

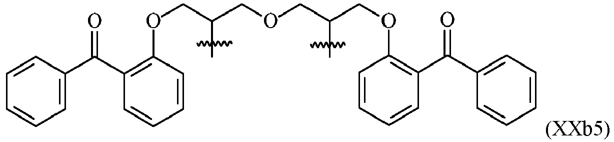

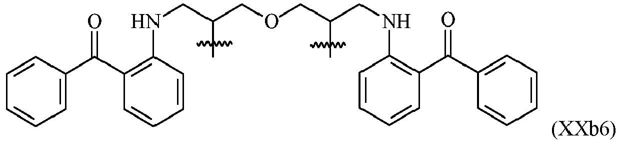

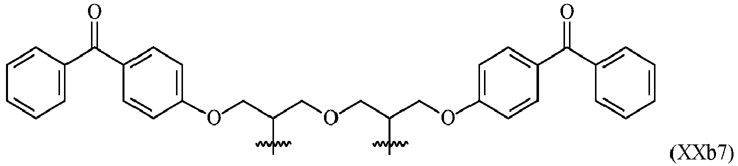

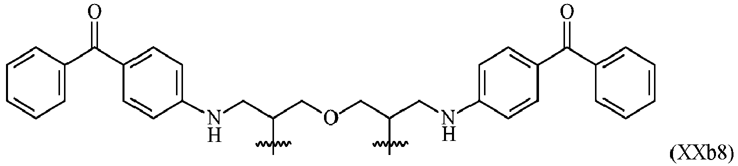

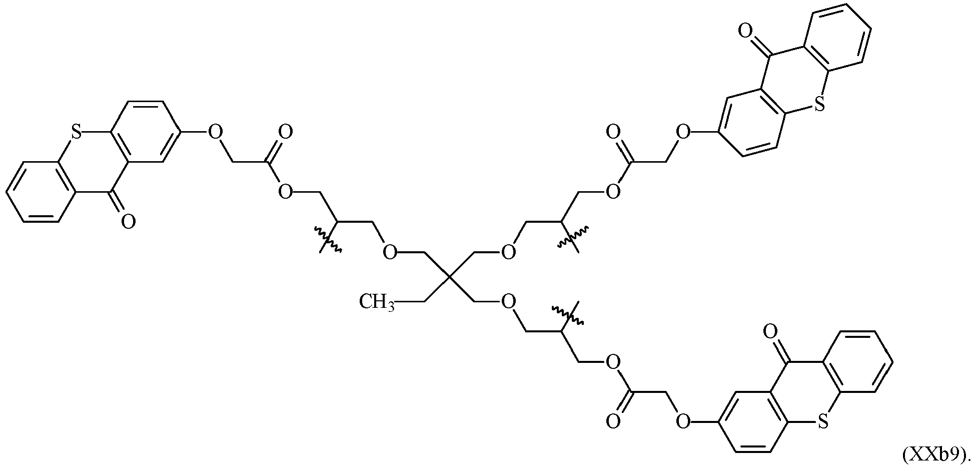

In another embodiment, the self-crosslinkable urethane (meth)acrylate of the invention may comprise at least one chromophore moiety Q according to formula (XXb) wherein said chromophore moiety Q has a single photoinitiator moiety PI and at least two points of attachment to the backbone of the self-crosslinkable urethane (meth)acrylate.

In particular, the self-crosslinkable urethane (meth)acrylate of the invention may comprise at least one chromophore moiety Q according to one of the following formulae (XXXVI) to (XXXXI): 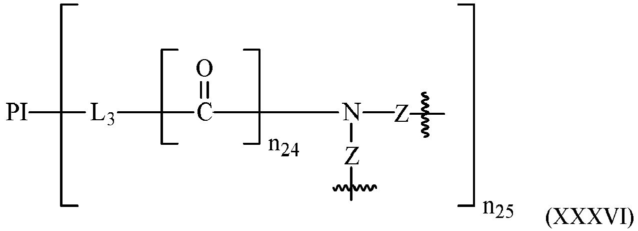 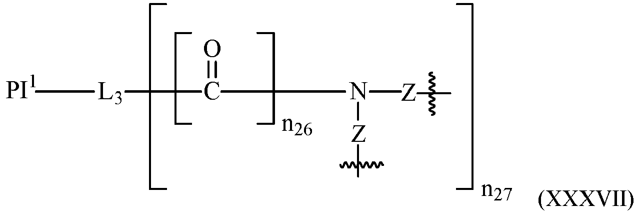 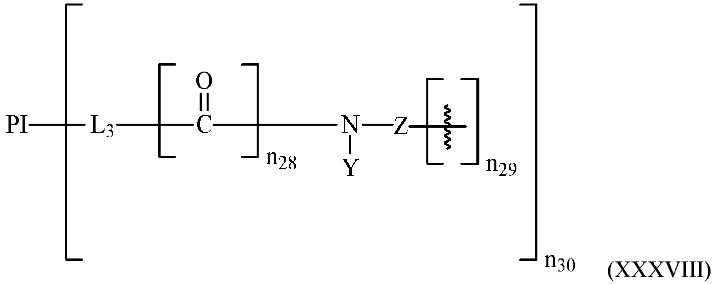 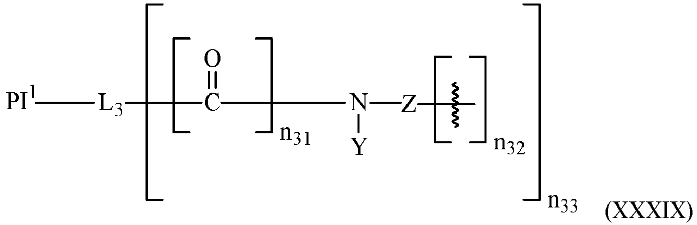 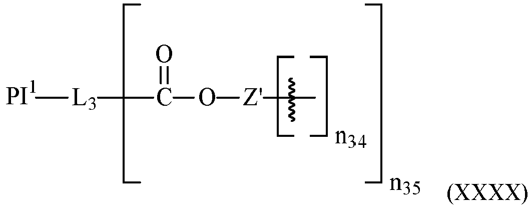 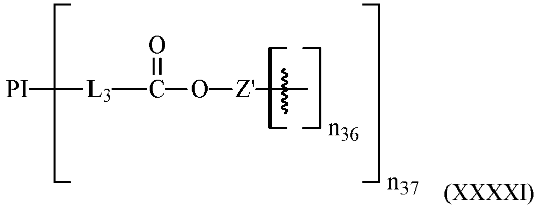 wherein
each PI is independently a monovalent or divalent photoinitiator moiety PI as defined above;
each PI¹ is independently a monovalent photoinitiator moiety PI as defined above;
each L₃ is independently selected from a direct bond or a linker comprising 1 to 20 carbon atoms;
each Y is independently H or alkyl;
each Z and Z' is independently a linker comprising 1 to 20 carbon atoms;
each n₂₄, n₂₆, n₂₈ and n₃₁ is independently 0 or 1, in particular 1;
each n₂₉, n₃₂, n₃₄ and n₃₆ is independently 1, 2 or 3, in particular 1;
each n₂₇ and n₃₃ is independently 1, 2 or 3; in particular 1 or 2;
each n₂₅ and n₃₀ is independently 1 or 2;
n₃₅ is 1, 2 or 3, in particular 2;
n₃₇ is 1 or 2, in particular 2.

In formula (XXXVI), n₂₅ is preferably 1 or 2.

In formula (XXXVII), n₂₇ is preferably 1 or 2.

In formula (XXXVIII), when n₂₉ is 1 then n₃₀ is preferably 2.

In formula (XXXVIII), when n₃₀ is 1 then n₂₉ is preferably 2 or 3.

In formula (XXXIX), when n₃₂ is 1 then n₃₃ is preferably 2.

In formula (XXXIX), when n₃₃ is 1 then n₃₂ is preferably 2 or 3.

In formula (XXXX), when n₃₄ is 1 then n₃₅ is preferably 2.

In formula (XXXX), when n₃₅ is 1 then n₃₄ is preferably 2 or 3.

In formula (XXXXI), when n₃₆ is 1 then n₃₇ is preferably 2.

In formula (XXXX), when n₃₇ is 1 then n₃₆ is preferably 2 or 3.

In formulae (XXXVI) to (XXXXI), each L₃ is independently selected from a direct bond or a linker comprising 1 to 20 carbon atoms.

In particular, each L₃ may independently be selected from direct bond, C1-C6 alkylene, C1-C6 oxyalkylene, C1-C6 alkenylene, C1-C6 thioalkylene, C1-C6 ketoalkylene, C1-C6 ketoalkenylene, C7-C13 ketoarylene, C6-C13 ketocycloalkylene.

More particularly, each L₃ may independently be selected from direct bond, alkylene of formula (C-16), oxyalkylene of formula (C-17), thioalkylene of formula (C-18), ketoalkylene of formula (C-19), aminoalkylene of formula (C-20) or alkylene of formula (C-21): 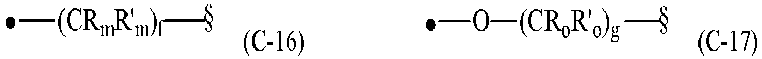 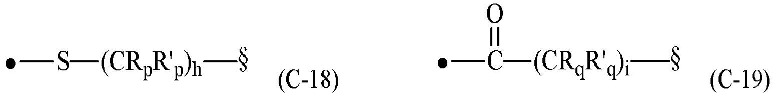 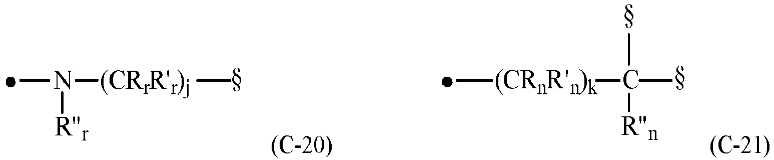 wherein
each Rₘ, R'ₘ, Rₙ, R'ₙ Rₒ, R'ₒ, Rₚ, R'ₚ, R_{q}, R'_{q}, Rᵣ and R'ᵣ is independently H or an optionally substituted alkyl, in particular H;
R"ᵣ and R"ₙ are independently H or an optionally substituted alkyl;
f, g, h and j are independently 1, 2, 3, 4, 5 or 6, in particular 1 or 2;
i and k are independently 1, 2, 3, 4 or 5, in particular 1;
the symbol ● represents a point of attachment to the PI or PI¹ moiety;
the symbol § represents a point of attachment to the moieties other than PI or PI¹.

In formulae (XXXVIII) and (XXXIX), each Y is independently H or alkyl.

In particular, each Y may independently be alkyl, more particularly methyl or ethyl.

In formulae (XXXVI) to (XXXIX), each Z is independently a linker comprising 1 to 20 carbon atoms.

In particular, each Z may independently be an alkylene.

More particularly, each Z may independently be an alkylene according to one of the following formulae (C-22), (C-23) or (C-24): 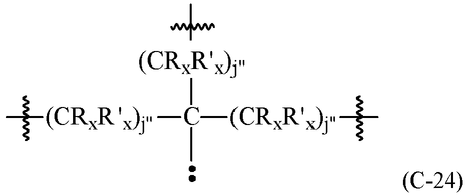 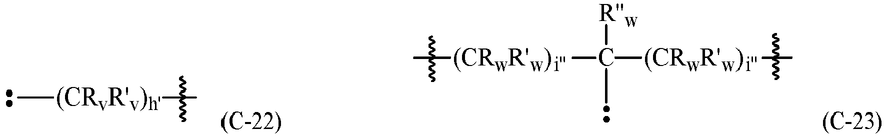
wherein each Rᵥ, R'ᵥ, R_{w}, R'_{w}, Rₓ, R'ₓ is independently H or alkyl, in particular H;
R"_{w} is H or alkyl, in particular alkyl;
h' is 1, 2, 3, 4, 5 or 6, in particular 2;
each i" is independently 1, 2 or 3, in particular 1;
each j" is independently 1, 2 or 3, in particular 1;
the symbol ● represents a point of attachment to the nitrogen or oxygen atom.

Even more particularly, each Z may independently be an alkylene of formula (C-22) as defined above.

In formulae (XXXX) and (XXXXI), each Z' is independently a linker comprising 1 to 20 carbon atoms.

In particular, each Z' may independently be selected from an aliphatic or aromatic hydrocarbon linker, a polyether linker, a polyester linker, a polycarbonate linker, a polyorganosiloxane linker, a polydiene linker, an isocyanurate linker, and combinations thereof.

More particularly, each Z' may independently be selected from an aliphatic or aromatic hydrocarbon linker, a polyether linker, a polyester linker and combinations thereof.

Even more particularly each Z' may independently be selected from an alkylene, an alkoxylated alkylene and a polycaprolactone linker.

In a particularly preferred embodiment, the self-crosslinkable urethane (meth)acrylate of the invention may comprise at least one chromophore moiety Q according to one of the following formulae (XXXXII) to (XXXXIV): 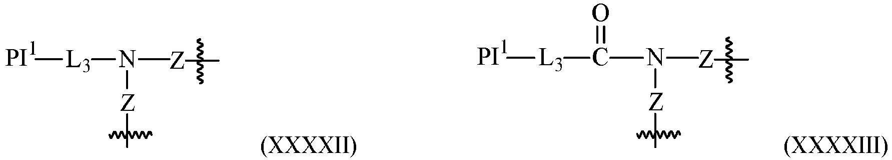 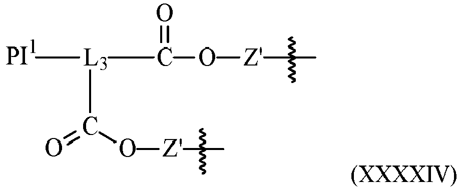 wherein
each PI¹ is independently a monovalent photoinitiator moiety PI according to formula (VIII) or (XI) as defined above, preferably each PI¹ is a monovalent photoinitiator moiety PI according to formula (Villa) or (XIa) as defined above;
each L₃ is independently selected from a direct bond, an alkylene of formula (C-16) as defined above, an oxyalkylene of formula (C-17) as defined above, a thioalkylene of formula (C-18) as defined above, or an alkylene of formula (C-21) as defined above;
each Z is independently an alkylene of formula (C-22) as defined above;
each Z' is independently an alkylene or a polyether linker.

In a particularly preferred embodiment, the self-crosslinkable urethane (meth)acrylate comprises at least one chromophore moiety Q according to one of the following formulae (XXb 10)-(XXb26):

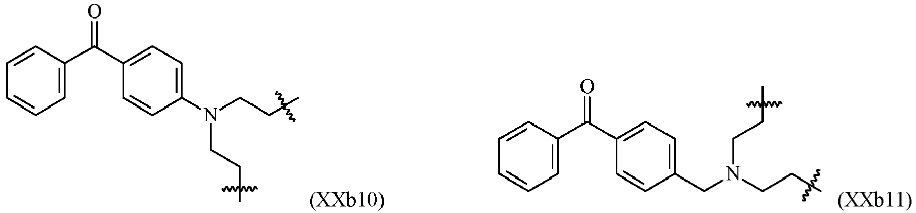

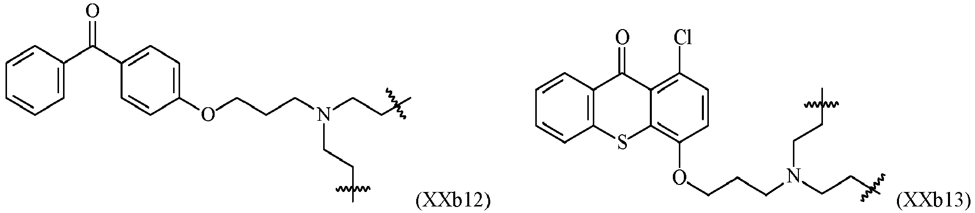

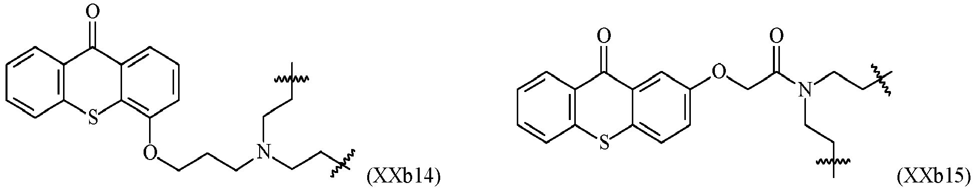

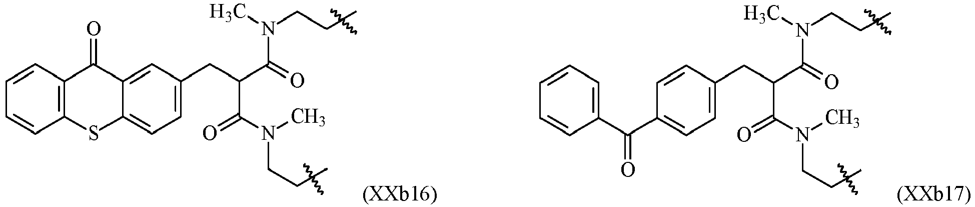

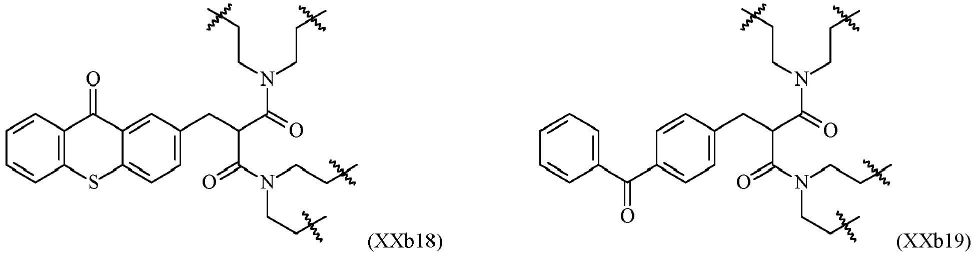

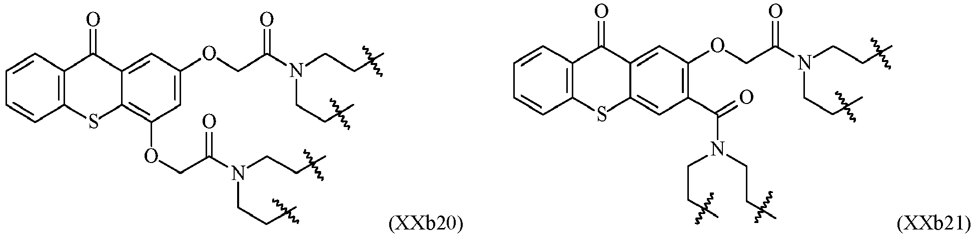

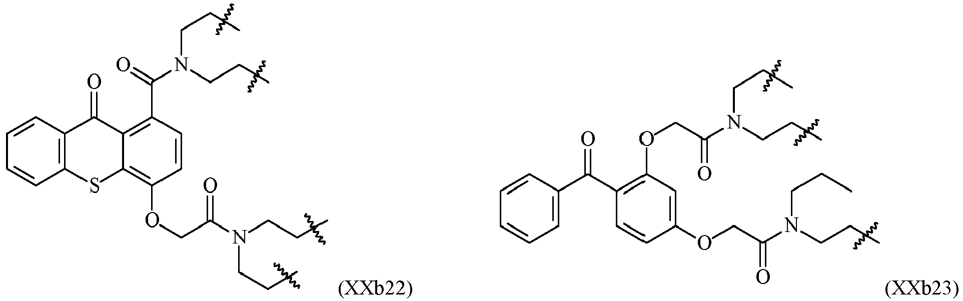

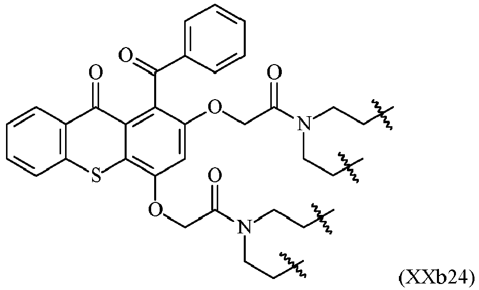

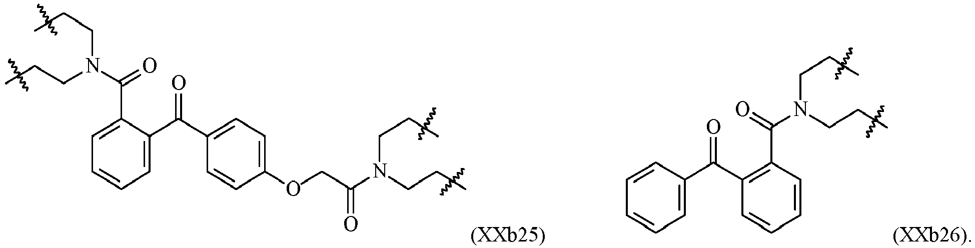

The total amount of chromophore moieties Q in the self-crosslinkable urethane (meth)acrylate of the invention may represent from 0.5 to 20%, in particular from 1 to 15%, more particularly from 1.5 to 10%, of the total weight of the self-crosslinkable urethane (meth)acrylate.

### Chain-extending moiety EXT

The self-crosslinkable urethane (meth)acrylate of the invention may optionally comprise at least one chain-extending moiety EXT. The self-crosslinkable urethane (meth)acrylate of the invention may optionally comprise more than one chain-extending moiety EXT. When this is the case, the chain-extending moieties may be identical or distinct from one another.

Alternatively, the self-crosslinkable urethane (meth)acrylate of the invention may be substantially free of a chain-extending moiety EXT.

As used herein, a chain-extending moiety means a moiety other than a (meth)acrylate-functionalized moiety ACR, a polyurethane moiety UU and a chromophore moiety Q. A chain-extending moiety EXT is therefore free of a photoinitiator moiety PI as defined above, free of a (meth)acrylate group and free of a urethane bond. A chain-extending moiety may be introduced in the self-crosslinkable urethane (meth)acrylate in order to increase its number average molecular weight and/or to tailor its mechanical properties and/or to introduce a hydrogen donor functional group to act as a co-initiator for the chromophore moiety Q.

Each chain-extending moiety EXT may correspond to the residue of a polyol (without the OH groups), preferably each chain-extending moiety EXT may correspond to the residue of a diol (without the OH groups).

Examples of suitable polyols are detailed in component d) of the preparation process of the self-crosslinkable urethane (meth)acrylate of the invention.

The self-crosslinkable urethane (meth)acrylate may comprise at least one chain-extending moiety EXT which is a linker (preferably a divalent or trivalent linker) selected from an aliphatic or aromatic hydrocarbon linker, a polyether linker, a polyester linker, a polycarbonate linker, a polyorganosiloxane linker, a polydiene linker, and combinations thereof.

The self-crosslinkable urethane (meth)acrylate may comprise at least one chain-extending moiety EXT which is the residue of a polyol (without the OH groups), in particular the residue of a diol (without the OH groups).

The self-crosslinkable urethane (meth)acrylate may comprise at least one chain-extending moiety EXT which is the residue of a polyol selected from the residue of a polymeric polyol, the residue of a non-polymeric polyol or the residue of an amino-functional polyol.

In particular, the self-crosslinkable urethane (meth)acrylate may comprise at least one chain-extending moiety EXT which is the residue of a polymeric polyol or the residue of a non-polymeric polyol, preferably the residue of a polymeric diol or the residue of a non-polymeric diol.

As used herein, the term "polymeric polyol" means a polymer bearing two or more isocyanate-reactive hydroxyl groups per molecule. As used herein, the term "polymeric diol" means a polymer bearing two isocyanate-reactive hydroxyl groups per molecule. As used herein, the term "non-polymeric polyol" means a non-polymeric compound bearing two or more isocyanate-reactive hydroxyl groups per molecule. As used herein, the term "non-polymeric diol" means a non-polymeric compound bearing two isocyanate-reactive hydroxyl groups per molecule. In the context of the present invention, the term "polymer" means a compound containing five or more repeating units per molecule and the term "nonpolymeric compound" means a compound containing up to four repeating units per molecule (and thus both monomeric compounds and oligomeric compounds containing 2 to 4 repeating units per molecule). For instance, ethylene glycol, diethylene glycol, triethylene glycol and tetraethylene glycol are all examples of non-polymeric diols, whereas polyethylene glycol containing five or more oxyalkylene repeating units is an example of a polymeric diol.

The self-crosslinkable urethane (meth)acrylate may comprise at least one chain-extending moiety EXT which is the residue of a polymeric polyol (without the OH groups). Alternatively, the self-crosslinkable urethane (meth)acrylate of the invention may be substantially free of a chain-extending moiety EXT which is the residue of a polymeric polyol (without the OH groups).

The molecular weight of the residue of a polymeric polyol may be varied as may be needed or desired in order to achieve particular properties in the self-crosslinkable urethane (meth)acrylate. The number average molecular weight of the residue of the polymeric polyol may be at least 300, at least 350, or at least 400 g/mol. The number average molecular weight of the residue of the polymeric polyol may be less than 5000, less than 4500, or less than 4000 g/mol. For example, the polymeric polyol may have a number average molecular weight of from 300 to 5000 g/mol, from 350 to 4500 g/mol or from 400 to 4000 g/mol.

The polymer portion of the polymeric polyol may be comprised of a plurality of repeating units such as oxyalkylene units, ester units, carbonate units, acrylic units, alkylene units or the like or combinations thereof.

In particular, the self-crosslinkable urethane (meth)acrylate of the invention may comprise at least one chain-extending moiety EXT which is the residue of a polymeric diol (without the OH groups).

More particularly, the self-crosslinkable urethane (meth)acrylate may comprise at least one chain-extending moiety EXT which is the residue of a polymeric diol selected from the residue of a polyether diol, the residue of a polyester diol, the residue of a polycarbonate diol, the residue of a polyorganosiloxane diol (e.g., the residue of a polydimethylsiloxane diol), the residue of a polydiene diol including fully or partially hydrogenated polydiene diols (e.g., the residue of a polybutadiene diol).

Even more particularly, the self-crosslinkable urethane (meth)acrylate may comprise at least one chain-extending moiety EXT which is the residue of a polyether diol or a polyester diol.

More particularly still, the self-crosslinkable urethane (meth)acrylate may comprise at least one chain-extending moiety EXT which is the residue of a polyether diol selected from a polyethylene glycol, a poly(1,2-propylene glycol), a poly(1,3-propylene glycol), a poly(1,4-butylene glycol) and combinations thereof, or the residue of a polyester diol selected from a poly(caprolactone), a poly(lactide), a poly(alkylene glycol adipate) and a poly(alkylene glycol succinate).

The self-crosslinkable urethane (meth)acrylate of the invention may comprise at least one chain-extending moiety EXT which is the residue of a non-polymeric polyol (without the OH groups). Alternatively, the self-crosslinkable urethane (meth)acrylate of the invention may be substantially free of a chain-extending moiety EXT which is the residue of a non-polymeric polyol (without the OH groups).

In particular, the self-crosslinkable urethane (meth)acrylate may comprise at least one chain-extending moiety EXT which is the residue of a non-polymeric diol.

More particularly, the self-crosslinkable urethane (meth)acrylate may comprise at least one chain-extending moiety EXT which is the residue of a non-polymeric aliphatic diol.

Even more particularly, the self-crosslinkable urethane (meth)acrylate may comprise at least one chain-extending moiety EXT which is the residue of a non-polymeric aliphatic diol selected from ethylene glycol, di-, tri- or tetraethylene glycol, 1,2- or 1,3-propylene glycol, di-, tri- or tetra(1,2-propylene glycol), di-, tri- or tetra(1,3-propylene glycol), 1,2-, 1,3- or 1,4-butylene glycol, di-, tri- or tetra(1,4-butylene glycol), 1,5-pentanediol, 1,6-hexanediol, 1,7-heptanediol, 1,8-octanediol, 1,9-nonanediol, 1,10-decanediol, 1,12-dodecanediol, 2-methyl-1,3-propanediol, 2,2-dimethyl-1,3-propanediol, 2,2-diethyl-1,3-propanediol, 2-methyl-2-ethyl-1,3-propanediol, 3-methyl-1,5-pentanediol, 3,3-dimethyl-1,5-pentanediol, 2,4-diethyl-1,5-pentanediol, 3-butyl-3-ethyl-1,5-pentane diol, 2,2,4-trimethyl 1,5-pentanediol, cyclohexanediol, cyclohexane-1,4-dimethanol, norbornene dimethanol, norbornane dimethanol, tricyclodecanediol, tricyclodecane dimethanol, hydrogenated bisphenol A, B, F or S, a dianhydrohexitol (i.e. isosorbide, isomannide, isoidide), a hydrogenated dimer fatty acid (i.e. a diol obtained by dimerizing one or more unsaturated fatty acids such as oleic acid or linoleic acid and then hydrogenating the resulting product to convert the carboxylic acid groups into hydroxyl groups, for example Pripol^{®} 2033 from Croda) as well as the alkoxylated (i.e. ethoxylated and/or propoxylated) derivatives thereof with up to four oxyalkylene units.

The self-crosslinkable urethane (meth)acrylate may comprise at least one chain-extending moiety EXT corresponding to the following formula (XXXXV): 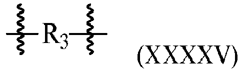
wherein R³ is a divalent linker selected from one of formula (Iib) to (Vib):

   -(CR₂₂R'₂₂)ₘ- (Iib)

   -[(CR₂₃R'₂₃)ₙ-O]ₒ-(CR₂₃R'₂₃)ₙ- (IIIb)

   -[(CR₂₄R'₂₄)ₚ-O]_{q}-(CR₂₅R'₂₅)ᵣ-[O-(CR₂₆R'₂₆)_{p'}]_{q'}- (Ivb)

   -[(CR₂₇R'₂₇)ₛ-C(=O)-O]ₜ-(CR₂₈R'₂₈)ᵤ-[O-(CR₂₈R'₂₈)ᵤ]ᵤ-[O-C(=O)-(CR₂₇R'₂₇)ₛ]_{t'}- (Vb)

   -[(CR₂₉R'₂₉)ᵥ-O-C(=O)-(CR₃₀R'₃₀)_{w}-C(=O)-O]ₓ-(CR₂₉R'₂₉)ᵥ- (Vib)
R₂₂, R'₂₂, R₂₅, R'₂₅, R₂₉, R'₂₉, R₃₀ and R'₃₀ are independently H or alkyl;
R₂₃, R'₂₃, R₂₄, R'₂₄, R₂₆, R'₂₆, R₂₇, R'₂₇, R₂₈ and R'₂₈ are independently H or methyl;
m is 2 to 50;
n, p and p' are independently 2 to 4;
o is 1 to 20;
q and q' are independently 0 to 20 with the proviso that at least one of q and q' is not 0 ;
r is 2 to 20;
s is 3 to 12;
t and t' are independently 1 to 20;
u is 2 to 8;
u' is 0 to 10;
v is 2 to 20;
w is 2 to 30;
x is 1 to 20.

The self-crosslinkable urethane (meth)acrylate may comprise at least one chain-extending moiety EXT which comprises a tertiary amine group. Said tertiary amine group may act as an amine synergist moiety to increase the rate and efficiency of photoinitiation by the chromophore moiety included in the backbone of the self-crosslinkable urethane (meth)acrylate.

The chain-extending moiety EXT which comprises a tertiary amine group may be the residue of an amino-functional polyol (without the OH groups).

Accordingly, the self-crosslinkable urethane (meth)acrylate may comprise at least one chain-extending moiety EXT which is the residue of an amino-functional polyol, in particular the residue of an amino-functional polyol selected from triethanol amine, N-methyldiethanol amine, N-ethyldiethanol amine, N-propyl diethanolamine, N-butyl diethanol amine, N-t-butyl diethanolamine, trimethanol amine, N-methyl dimethanol amine, 3-(dimethylamino)-1,2-propanediol, 3-(diethylamino)-1,2-propanediol, 3-(dipropylamino)-1,2-propanediol, 2-(dimethylamino)propane-1,3-diol, bis(2-hydroxyethyl)dodecylamine, bis(2-hydroxyethyl)octadecylamine, N,N-dioctadecyl-N',N'-bis(2-hydroxyethyl)-1,3-diaminopropane, 3-morpholino-1,2-propanediol, 3-piperidino-1,2-propanediol, 3-pyrrolidino-1-yl-1,2- propanediol, and an aminobenzamide diol according to the following formula (XXXXVI): 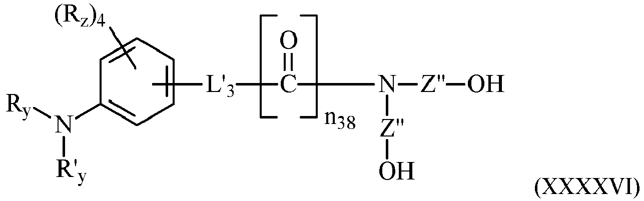 wherein
R_{y} and R'_{y} are independently an optionally substituted optionally substituted group selected from alkyl, alkenyl, alkynyl, aryl, aralkyl, alkaryl and heteroaryl;
each R_{z} is independently H, F, Cl, Br, I, -OR*, -SR*, -N(R^{∗})₂, -NO₂, -CN, -C(=O)R*, -O-C(=O)R*, -C(=O)OR*, -C(=O)N(R^{∗})₂, -NR*-C(=O)-R*, -SO₂-N(R^{∗})₂, or an optionally substituted group selected from the group consisting of alkyl, heteroatom-containing alkyl, cycloalkyl, heterocycloalkyl, alkenyl, alkynyl, aryl, aralkyl, alkaryl and heteroaryl;
each R* is independently H or an optionally substituted group selected from alkyl, heteroatom-containing alkyl, cycloalkyl, heterocycloalkyl, alkenyl, alkynyl, aryl, aralkyl, alkaryl and heteroaryl;
L'₃ is as defined above for L₃;
each Z" is independently as defined above for Z;
n₃₈ is 0 or 1.

The total amount of chain-extending moieties EXT in the self-crosslinkable urethane (meth)acrylate of the invention may represent from 0 to 84.5%, in particular from 0.1 to 75%, more particularly from 1 to 65%, of the total weight of the self-crosslinkable urethane (meth)acrylate.

In one embodiment, the total amount of chain-extending moieties EXT in the self-crosslinkable urethane (meth)acrylate of the invention may represent less than 0.2%, in particular less than 0.1%, more particularly 0%, of the total weight of the self-crosslinkable urethane (meth)acrylate. Such amounts may for example be obtained when the self-crosslinkable urethane (meth)acrylate is substantially free of a chain-extending moiety.

In one embodiment the total amount of chain-extending moieties EXT in the self-crosslinkable urethane (meth)acrylate of the invention may represent from 0.1 to 20%, in particular from 0.2 to 15%, more particularly from 0.5 to 10%, of the total weight of the self-crosslinkable urethane (meth)acrylate. Such amounts may for example be obtained when the self-crosslinkable urethane (meth)acrylate comprises a chain-extending moiety derived from a non-polymeric polyol.

In another embodiment the total amount of chain-extending moieties EXT in the self-crosslinkable urethane (meth)acrylate of the invention may represent from 15 to 84.5%, in particular from 20 to 75%, more particularly from 25 to 65%, of the total weight of the self-crosslinkable urethane (meth)acrylate. Such amounts may for example be obtained when the self-crosslinkable urethane (meth)acrylate comprises a chain-extending moiety derived from a polymeric polyol.

### Structure of the self-crosslinkable urethane (meth)acrylate

The structure of the self-crosslinkable urethane (meth)acrylate may be linear or branched, depending on the nature of the reactants used to prepare the self-crosslinkable urethane (meth)acrylate.

If the polyurethane moiety UU is derived from a diisocyanate and if both the chromophore moiety Q and the optional chain-extending moiety EXT are derived from a diol, then then the resulting self-crosslinkable urethane (meth)acrylate is linear.

If the polyurethane moiety UU is derived from a triisocyanate or if the chromophore moiety Q is derived from a triol or a tetraol, then then the resulting self-crosslinkable urethane (meth)acrylate is branched.

Each (meth)acrylate-functionalized moiety ACR may be a terminal moiety attached to a single polyurethane moiety UU. The (meth)acrylate-functionalized moiety ACR may thus serve as an end-cap moiety of the self-crosslinkable urethane (meth)acrylate.

Each chromophore moiety Q may be attached to at least one polyurethane moiety UU. When the chromophore moiety Q is derived from a photoinitiator compound bearing a single OH group, then said chromophore moiety Q may be a terminal moiety attached to a single polyurethane moiety UU. When the chromophore moiety Q is derived from a photoinitiator compound bearing at least two OH groups, then said chromophore moiety Q may be attached to at least two distinct polyurethane moieties UU (one for each OH group borne by the photoinitiator compound).

Each chain-extending moiety EXT may be attached to two distinct polyurethane moieties UU.

Each polyurethane moiety UU may be positioned between two, three or four moieties independently selected from a (meth)acrylate-functionalized moiety ACR, a chromophore moiety Q and a chain-extending moiety EXT.

When the self-crosslinkable urethane (meth)acrylate comprises one or more chromophore moieties Q and one or more chain-extending moieties EXT, the Q and EXT moieties may be randomly positioned with respect to the polyurethane moieties UU. For example, the one or more chain-extending moieties and the chromophore moieties Q may be randomly disposed along the polyurethane backbone and linked together by the polyurethane moieties UU. By varying the synthetic method used to prepare the self-crosslinkable urethane (meth)acrylate, it is also possible for the oligomer to have a more ordered structure. For example, the chain-extending moieties EXT may be clustered near the center of the polyurethane backbone, with the chromophore moieties being positioned towards each end of the polyurethane backbone, or vice versa.

The self-crosslinkable urethane (meth)acrylate may comprise a chromophore moiety Q attached to at least two distinct polyurethane moieties UU. This is the case when the chromophore moiety Q is derived from a photoinitiator compound bearing 2, 3 or 4 OH groups.

In particular, the self-crosslinkable urethane (meth)acrylate may correspond to the following structure (A): 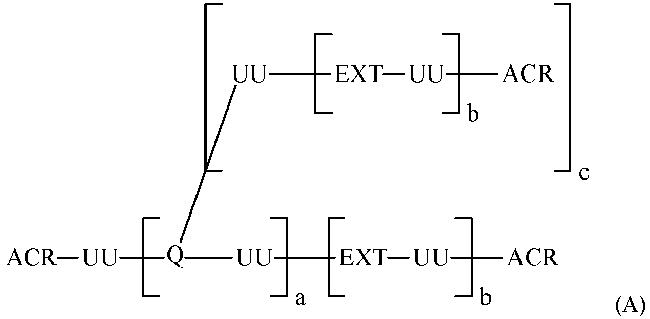
wherein ACR, UU, Q and EXT are as defined above;
a is an integer from 1 to 100;
b is an integer from 0 to 100;
c is an integer equal to 0, 1 or 2, preferably 0 or 1.

The self-crosslinkable urethane (meth)acrylate may comprise at least one chromophore moiety Q attached to a single polyurethane moiety UU. This is the case when the chromophore moiety Q is derived from a photoinitiator compound bearing a single OH group.

In particular, the self-crosslinkable urethane (meth)acrylate may correspond to the following structure (B): 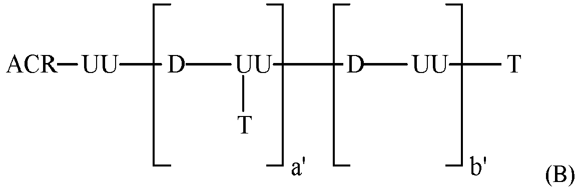 wherein
each D is independently Q or EXT;
each T is independently ACR or Q;
ACR, UU, Q and EXT are as defined in any one of claims 1 to 30;
a' is an integer from 0 to 100;
b' is an integer from 0 to 100;
wherein at least one of a' and b' is not 0; and
at least one of D and T is Q.

When a' is other than 0, then the self-crosslinkable urethane (meth)acrylate comprises at least one polyurethane moiety UU derived from a triisocyanate. In such a case, the self-crosslinkable urethane (meth)acrylate may or may not further comprise at least one polyurethane moiety UU derived from a diisocyanate (i.e. b' may be either other than 0 or b' may be equal to 0). Preferably, when a' is other than 0, then the at least one chromophore moiety Q is derived from a monool or a diol.

The self-crosslinkable urethane (meth)acrylate may not comprise an effective amount of a dye moiety, preferably the self-crosslinkable urethane (meth)acrylate does not comprise any dye moiety.

As used herein, an "effective amount of dye moiety" is understood to mean an amount of dye moiety in the self-crosslinkable urethane (meth)acrylate sufficient to impart a color different from the color of the self-crosslinkable urethane (meth)acrylate not including any such dye moiety, such that the different color is detectable by the naked eye.

Examples of dye moieties are moieties derived from an azo dye, a phthalocyanine dye, an anthraquinone dye, a di- or tricyanovinyl dye, a methine dye, an aniline dye, an indoaniline dye, such as those described in US 2014/0316060 A1.

The number-average molecular weight of the self-crosslinkable urethane (meth)acrylate may be from 500 to 50,000 g/mol, or from 1,000 to 10,000 g/mol or from 2,000 to 8,000 g/mol. The number average molecular weight of the self-crosslinkable urethane (meth)acrylate may be varied as may be desired in order to impart certain characteristics to the oligomer itself and to cured compositions prepared from the oligomers. Generally speaking, if the self-crosslinkable urethane (meth)acrylate comprises at least one chain extending moiety EXT, the number average molecular weight is typically somewhat higher. For example, if the self-crosslinkable urethane (meth)acrylate comprises at least one chain extending moiety EXT, it may have a number average molecular weight of from 3,000 g/mol to 50,000 g/mol. If the self-crosslinkable urethane (meth)acrylate does not comprise a chain extending moiety EXT, it may have a number average molecular weight of from 500 g/mol to 5,000 g/mol, for example.

The self-crosslinkable urethane (meth)acrylate may be obtained by a reacting the following components (in one step or in successive steps):
a) a hydroxyl-functionalized (meth)acrylate component;
b) a polyisocyanate component;
c) a hydroxyl-functionalized photoinitiator component; and
d) optionally, a polyol component.

Components a), b), c) and d) may be as detailed below in the process for the preparation of the self-crosslinkable urethane (meth)acrylate.

### Process for the preparation of the self-crosslinkable urethane (meth)acrylate

The invention also relates to a process for the preparation of a self-crosslinkable urethane (meth)acrylate. The self-crosslinkable urethane (meth)acrylate obtained with the process of the invention may be as defined above.

The process for the preparation of a self-crosslinkable urethane (meth)acrylate comprises reacting:
a) a hydroxyl-functionalized (meth)acrylate component;
b) a polyisocyanate component;
c) a hydroxyl-functionalized photoinitiator component; and
d) optionally, a polyol component.

The reaction between components a), b) c) and d) may be conducted in a single step or in successive steps. When successive steps are used, the process may comprise a first step of reacting components b) and c) and optionally component d) to form an isocyanate-terminated prepolymer and a second step) of reacting said isocyanate-terminated prepolymer with component a). Alternatively, the process may comprise a first step of reacting components a) and b) to form an isocyanate-functionalized (meth)acrylate compound and a second step) of reacting said isocyanate-functionalized (meth)acrylate compound with component c) and optionally component d).

The process may be carried out in the presence of one or more additional components e) as detailed below.

The process may be carried out at a temperature from 30 to 90°C, preferably from 50 to 80°C.

The process may be carried out until the NCO value is less than 1 wt%, preferably less than 0.5%, more preferably less than 0.1%, based on the total weight of the reaction mixture. If necessary, an additional amount of component a) can be introduced into the reaction mixture to reach the target NCO value.

Components a), b), c), d) and e) may be as detailed below.

### a) Hydroxyl-functionalized (meth)acrylate component

One of the components used to prepare the self-crosslinkable urethane (meth)acrylate of the invention is a hydroxyl-functionalized (meth)acrylate component, also referred to as component a).

A hydroxyl-functionalized (meth)acrylate component comprises or consists of at least one hydroxyl-functionalized (meth)acrylate compound. A hydroxyl-functionalized (meth)acrylate component may comprise or consist of a mixture of hydroxyl-functionalized (meth)acrylate compounds.

A hydroxyl-functionalized (meth)acrylate compound is a compound bearing at least one (meth)acrylate group and a single OH group.

The reaction of component a) with the other components used prepare the self-crosslinkable urethane (meth)acrylate may result in the incorporation of one or more (meth)acrylate-functionalized moieties ACR in the backbone of the self-crosslinkable urethane (meth)acrylate. The preferential embodiments disclosed above for the (meth)acrylate-functionalized moiety ACR equally apply to component a).

Component a) may comprise a hydroxyl-functionalized (meth)acrylate compound bearing 1 to 5 (meth)acrylate groups, in particular 1 to 3 (meth)acrylate groups, more particularly 1 (meth)acrylate group.

In particular, component a) may comprise a hydroxyl-functionalized (meth)acrylate compound according to the following formula (Ia): 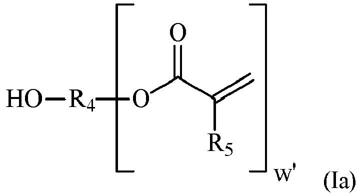 wherein
R₄, R₅ and w' are as defined above for the (meth)acrylate-functionalized moiety ACR. All the preferred embodiments for R₄, R₅ and w' described above for the (meth)acrylate-functionalized moiety ACR equally apply to the compound of formula (Ia).

Examples of suitable hydroxyl-functionalized (meth)acrylate compounds include a hydroxyalkyl (meth)acrylate (for example 2-hydroxyethyl (meth)acrylate, 2-hydroxypropyl (meth)acrylate, 3-hydroxypropyl (meth)acrylate, 2-hydroxybutyl (meth)acrylate, 3-hydroxybutyl (meth)acrylate, 4-hydroxybutyl (meth)acrylate, neopentyl glycol mono(meth)acrylate or 1,6-hexanediol mono(meth)acrylate), 2-hydroxy-3-phenoxypropyl (meth)acrylate, 3-chloro-2-hydroxypropyl (meth)acrylate, tris(2-hydroxyethyl)isocyanurate mono- and di(meth)acrylate, di-, tri-, tetra- or polyethylene glycol mono(meth)acrylate, di-, tri-, tetra- or poly(1,2-propylene glycol) mono(meth)acrylate, di-, tri-, tetra- or poly(1,3-propylene glycol) mono(meth)acrylate, di-, tri-, tetra- or poly(1,4-butylene glycol) mono(meth)acrylate, glycerin mono- and di(meth)acrylate, 2-hydroxy-1-acryloxy-3-(meth)acryloxypropane, trimethylolpropane mono- and di(meth)acrylate, di(trimethylolpropane) mono-, di- and tri(meth)acrylate, trimethylolethane mono- and di(meth)acrylate, pentaerythritol mono-, di- and tri(meth)acrylate, dipentaerythritol mono-, di-, tri-, tetra- and penta(meth)acrylate, as well as the alkoxylated (e.g., ethoxylated and/or propoxylated) derivatives thereof and the (poly)caprolactone derivatives thereof obtained by ring-opening polymerization of ε-caprolactone initiated with one of the aforementioned hydroxyl-functionalized (meth)acrylate compounds (i.e. a (poly)caprolactone (meth)acrylate such as (poly)caprolactone 2-hydroxyethyl (meth)acrylate according to the following formula: CH₂=CR₅-C(=O)-O-CH₂-CH₂-[O-(C=O)-(CH₂)₅]ₙ-OH wherein R₅ is H or methyl and t is 1 to 20), and combinations thereof.

Preferably, component a) may comprise a hydroxyl-functionalized (meth)acrylate compound selected from a hydroxyalkyl (meth)acrylate, a (poly)caprolactone (meth)acrylate and mixtures thereof.

The total amount of component a) used to prepare the self-crosslinkable urethane (meth)acrylate of the invention may be from 5 to 85%, in particular from 10 to 80%, more particularly from 15 to 75%, by weight of component a) based on the total amount of components a), b), c) and d).

In one embodiment, the total amount of component a) used to prepare the self-crosslinkable urethane (meth)acrylate of the invention may be from 50 to 85%, in particular from 55 to 80%, more particularly from 60 to 75%, by weight of component a) based on the total amount of components a), b), c) and d). Such amounts may for example be used when component a) comprises a (poly)caprolactone (meth)acrylate.

In another embodiment, the total amount of component a) used to prepare the self-crosslinkable urethane (meth)acrylate of the invention may be from 5 to 40%, in particular from 10 to 35%, more particularly from 15 to 30%, by weight of component a) based on the total amount of components a), b), c) and d). Such amounts may for example be used when component a) comprises a hydroxyalkyl (meth)acrylate.

### b) Polyisocyanate component

One of the components used to prepare the self-crosslinkable urethane (meth)acrylate of the invention is a polyisocyanate component, also referred to as component b).

A polyisocyanate component comprises or consists of at least one polyisocyanate compound. A polyisocyanate component may comprise or consist of a mixture of polyisocyanate compounds.

A polyisocyanate compound is a compound bearing at least two isocyanate -(N=C=O) groups.

The reaction of component b) with the other components used prepare the self-crosslinkable urethane (meth)acrylate may result in the incorporation of one or more polyurethane moieties UU in the backbone of the self-crosslinkable urethane (meth)acrylate. The preferential embodiments disclosed above for the polyurethane moiety UU equally apply to component b).

Component b) may comprise a polyisocyanate compound bearing 2 to 3 isocyanate groups. In particular, component b) may comprise a diisocyanate compound according to the following formula (VIIa) and/or a triisocyanate compound according to the following formula (VIIb):

O=C=N-R₁-N=C=O (VIIa)

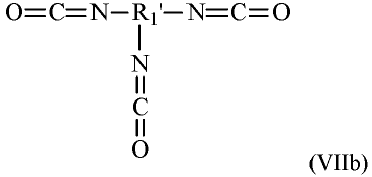

wherein R₁ and R₁' are as defined above for the polyurethane moiety UU. All the preferred embodiments for R₁ and R₁' described above for the polyurethane moiety UU equally apply to the compounds of formula (VIIa) and (VIIb).

Suitable examples of diisocyanate compounds according to formula (VIIa) include 2,4- and 2,6-toluene diisocyanate (TDI), isophorone diisocyanate (IPDI - corresponding to 3-isocyanatomethyl-3,5,5-trimethylcyclohexylisocyanate), methylene diisocyanate, ethylene diisocyanate, 1,2- or 1,3-propylene diisocyanate, 1,2-, 1,3 or 1,4-butylene diisocyanate, 1,5-pentamethylene diisocyanate (PDI), 1,6-hexamethylene diisocyanate (HDI), 2,2,4- and 2,4,4-trimethylhexamethylene diisocyanate (TMDI), 1,10-decylene diisocyanate, 1,12-dodecylene diisocyanate, 1,18-octadecylene diisocyanate, 2,2'-, 2,4'- and 4,4'-diphenylmethane diisocyanate (MDI), 2,2'-, 2,4'- and 4,4'-dicyclohexylmethane diisocyanate (H12MDI), benzidine diisocyanate, 3,3'-dimethyl-4,4'-biphenyl diisocyanate, dianisidine diisocyanate, 3,3'-dimethyl-4,4'-diphenylmethane diisocyanate, 1,3- and 1,4-phenylene diisocyanate, 1,4- and 1,5-naphtalene diisocyanate (NDI), 1,4- and 9,10-anthracene diisocyanate, 1,3- and 1,4-cyclohexane diisocyanate, 1-methyl-2,4-diisocyanatocyclohexane, 1-methyl-2,6-diisocyanatocyclohexane, 1,3 and 1,4-bis(isocyanatomethyl)cyclohexane, m-tetramethylxylylene diisocyanate, m-xylylene diisocyanate, 4-methoxy-1,3-phenylene diisocyanate, 4-ethoxy-1,3-phenylene diisocyanate, 5,6-dimethyl-1,3-phenylene diisocyanate, 2,4'- or 4,4'-diisocyanate diphenyl ether, lysine diisocyanate, dimer acid diisocyanate, dimers of the aforementioned diisocyanates (in particular uretdione or allophanate dimers), as well as polyurea or polyurethane prepolymers functionalized with isocyanate functional groups, and combinations thereof.

Suitable examples of triisocyanate compounds according to formula (VIIb) include 1,6,1 1-undecane triisocyanate, triphenylmethane triisocyanate, 2,4,6-tolylene triisocyanate, 2,4,4'-triisocyanate diphenyl ether, trimers of the diisocyanates detailed above for formula (VIIa) (in particular isocyanurate or biuret trimers), polymeric derivatives of the diisocyanates detailed above for formula (VIIa), and combinations thereof.

As used herein, an isocyanurate trimer of a diisocyanate of formula O=C=N-R₁-N=C=O corresponds to the following formula: 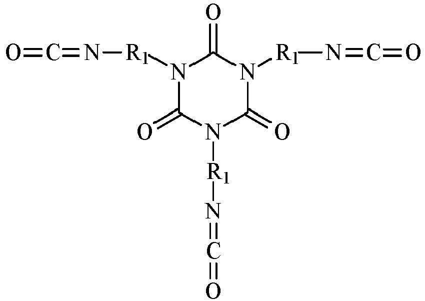 wherein R₁ is as defined above.

As used herein, a biuret trimer of a diisocyanate of formula O=C=N-R₁-N=C=O corresponds to the following formula: 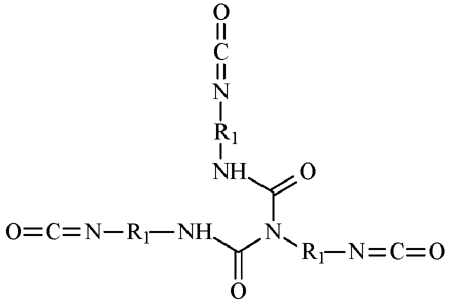 wherein R₁ is as defined above.

Preferably, component b) comprises a polyisocyanate compound selected from isophorone diisocyanate, 2,2'-, 2,4'- and 4,4'-dicyclohexylmethane diisocyanate, an isocyanurate trimer of hexamethylene diisocyanate, and combinations thereof.

The total amount of component b) used to prepare the self-crosslinkable urethane (meth)acrylate of the invention may be from 10 to 65%, in particular from 15 to 60%, more particularly from 20 to 55%, by weight of component b) based on the total amount of components a), b), c) and d).

### c) Hydroxyl-functionalized photoinitiator component

One of the components used to prepare the self-crosslinkable urethane (meth)acrylate of the invention is a hydroxyl-functionalized photoinitiator component, also referred to as component c).

A hydroxyl-functionalized photoinitiator component comprises or consists of at least one hydroxyl-functionalized photoinitiator. A hydroxyl-functionalized photoinitiator component may comprise or consist of a mixture of hydroxyl-functionalized photoinitiators.

The reaction of component c) with the other components used prepare the self-crosslinkable urethane (meth)acrylate may result in the incorporation of one or more chromophore moieties Q in the backbone of the self-crosslinkable urethane (meth)acrylate. The preferential embodiments disclosed above for the chromophore moiety Q equally apply to component c).

A hydroxyl-functionalized photoinitiator is a compound bearing at least one hydroxyl group (in particular 1, 2, 3 or 4 hydroxy groups) and at least one chromophore moiety (in particular 1, 2, 3 or 4 chromophore moieties). The at least one chromophore moiety of the hydroxyl-functionalized photoinitiator may be as defined above for the chromophore moiety Q.

A hydroxyl-functionalized photoinitiator may be a monool (i.e. a photoinitiator bearing a single OH group), a diol (i.e. a photoinitiator bearing two OH groups), a triol (i.e. a photoinitiator bearing three OH groups) or a tetraol (i.e. a photoinitiator bearing four OH groups).

In particular, a hydroxyl-functionalized photoinitiator may bear:
- a single OH group and a single chromophore moiety;
- two OH groups and a single chromophore moiety;
- three OH groups and a single chromophore moiety;
- four OH groups and a single chromophore moiety;
- two OH groups and two chromophore moieties;
- three OH groups and three chromophore moieties; or
- four OH groups and four chromophore moieties.

Each chromophore moiety may independently comprise at least one monovalent or divalent photoinitiator moiety PI selected from a benzophenone moiety, a thioxanthone moiety, a xanthone moiety, an acridone moiety, a camphorquinone moiety, a benzil moiety, a coumarin moiety, a ketocoumarin moiety, derivatives thereof and combinations thereof, preferably a monovalent or divalent photoinitiator moiety PI selected from a benzophenone moiety or a thioxanthone moiety.

In particular, each chromophore moiety may independently comprise a monovalent photoinitiator moiety PI corresponding to one of formulae (VIII), (XI), (XIV), (XVII), (XVIII) or (XIX), or at least one divalent photoinitiator moiety PI corresponding to one of formulae (XII), (XIII), (XV) or (XVI). Formulae (VIII), (IX), (X) (XI), (XII), (XIII), (XIV), (XV), (XVI), (XVII), (XVIII) and (XIX) are as defined above for the chromophore moiety Q.

More particularly, each chromophore moiety may independently comprise at least one monovalent photoinitiator moiety PI corresponding to one of formulae (VIII) or (XI) wherein E is S, or at least one divalent photoinitiator moiety PI corresponding to one of formulae (IX), (X), (XII) or (XIII) wherein E is S.

More particularly yet, each chromophore moiety may independently comprise at least one monovalent photoinitiator moiety PI corresponding to one of formulae (Villa) or (XIa) as defined above for the chromophore moiety Q, or at least one divalent photoinitiator moiety PI corresponding to one of formulae (IXa), (Xa), (XIIa) or (XIIIa) as defined above for the chromophore moiety Q.

Even more particularly, each chromophore moiety may independently comprise at least one monovalent photoinitiator moiety PI corresponding to formula (VIII) or (XI) wherein E is S, or at least one divalent photoinitiator moiety PI corresponding to formula (X) or (XIII) wherein E is S.

Yet even more particularly, each chromophore moiety may independently comprise at least one monovalent photoinitiator moiety PI corresponding to formula (VIIIa) or (XIa) as defined above for the chromophore moiety Q, or at least one divalent photoinitiator moiety PI corresponding to formula (Xa) or (XIIIa) as defined above for the chromophore moiety Q.

The one or more OH groups and the one or more chromophore moieties of the hydroxyl-functionalized photoinitiator may be connected by one or more linkers.

In particular, component c) may comprise at least one hydroxyl-functionalized photoinitiator according to one of the following formulae (XXd), (XXe) or (XXf):

HO-L₁-PI¹ (XXd)

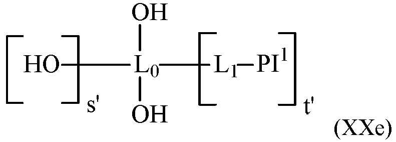

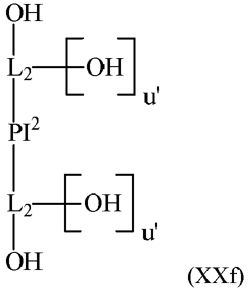

wherein L₀, L₁, L₂, PI¹, PI², s', t' and u' are as defined above for the chromophore moiety Q according to one of formulae (XXa), (XXb) and (XXc).

Component c) may comprise at least one hydroxyl-functionalized photoinitiator according to formula (XXd) as defined above. A hydroxyl-functionalized photoinitiator according to formula (XXd) may have a single chromophore moiety and a single OH group.

In formula (XXd), L₁ and PI¹ may be as defined above for the chromophore moiety Q according to formula (XXa). All the preferred embodiments for L₁ and PI¹ described above for the chromophore moiety Q according to formula (XXa) equally apply to the photoinitiator of formula (XXd).

In particular, component c) may comprise at least one hydroxyl-functionalized photoinitiator corresponding to the following formulae (XXd1) or (XXd2) 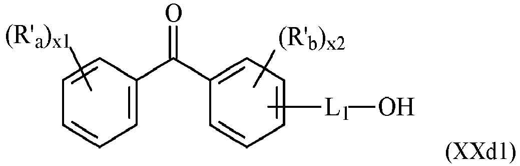 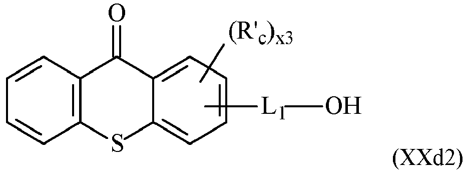 wherein R'ₐ, R'_{b}, R'_{c}, x1, x2, x3 and L₁ are as defined above for the chromophore moiety Q according to one of formulae (XXa1) and (XXa2).

More particularly, component c) may comprise at least one hydroxyl-functionalized photoinitiator corresponding to one of the following formulae (XXd3)-(XXd17):

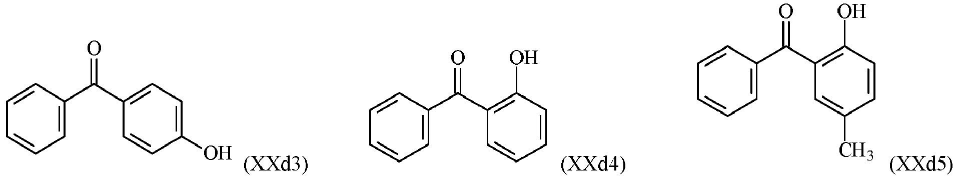

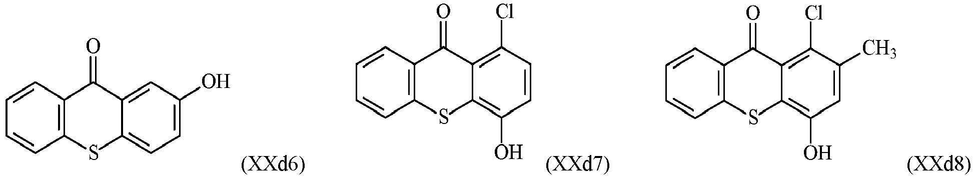

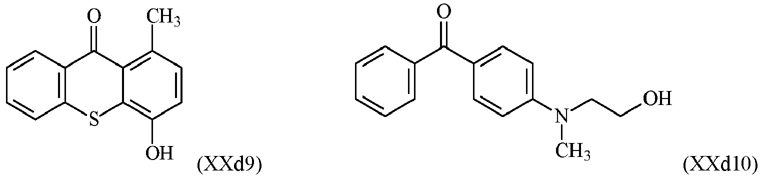

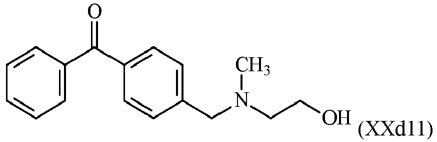

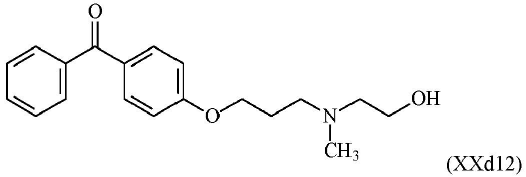

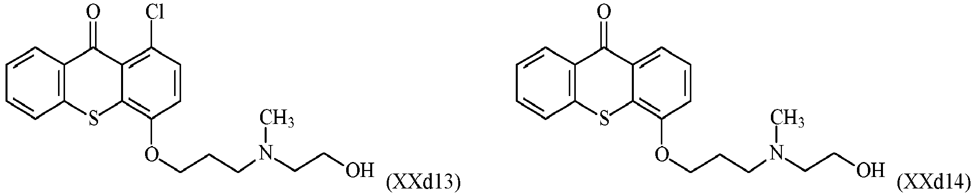

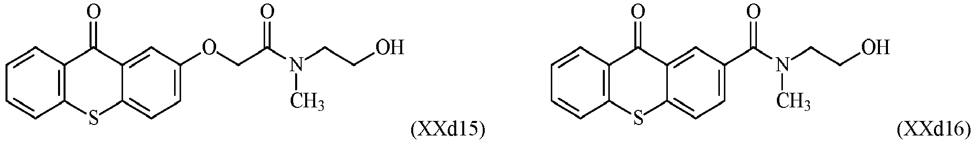

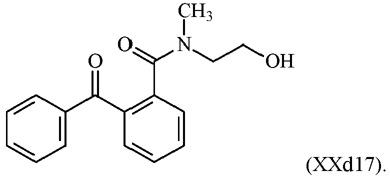

Component c) may comprise at least one hydroxyl-functionalized photoinitiator according to formula (XXf) as defined above. A hydroxyl-functionalized photoinitiator according to formula (XXf) as defined above may bear a single chromophore moiety and at least two OH groups, preferably two OH groups.

In formula (XXf), L₂, u' and PI² may be as defined above for the chromophore moiety Q according to formula (XXc). All the preferred embodiments for L₂, u' and PI² described above for the chromophore moiety Q according to formula (XXc) equally apply to the photoinitiator of formula (XXf).

In particular, component c) may comprise at least one hydroxyl-functionalized photoinitiator corresponding to one of the following formulae (XXf1)-(XXf4) 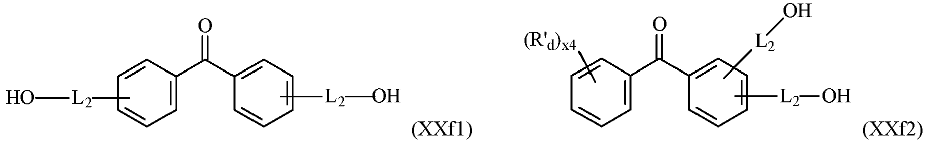 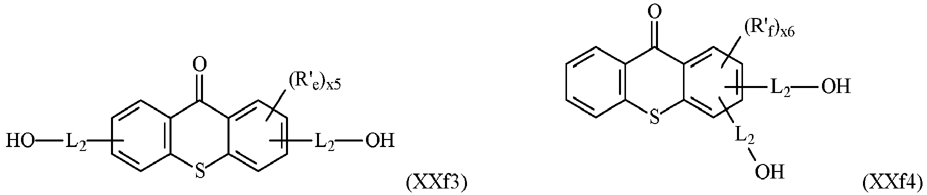 wherein R'_{d}, R'ₑ, R'_{f}, x4, x5, x6 and L₂ are as defined above for the chromophore moiety Q according to one of formulae (XXc1)-(XXc4).

More particularly, component c) may comprise at least one hydroxyl-functionalized photoinitiator according to one of the following formulae (XXf5)-(XXf13):

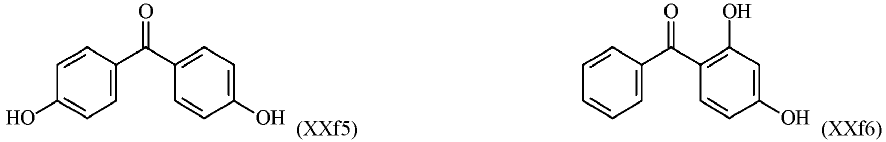

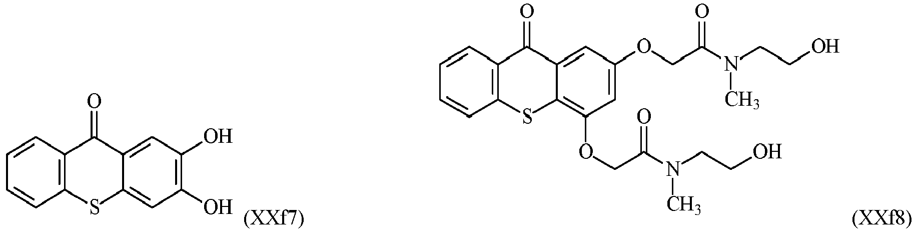

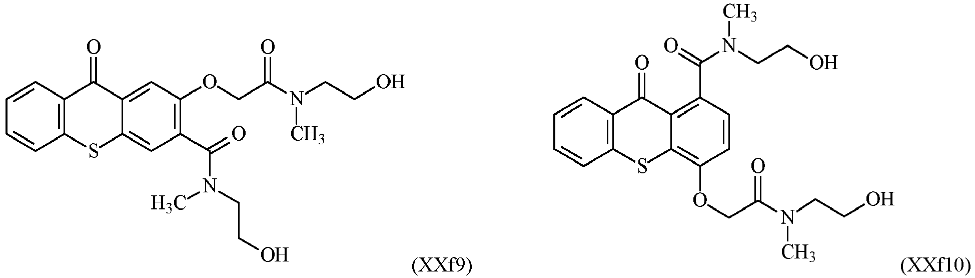

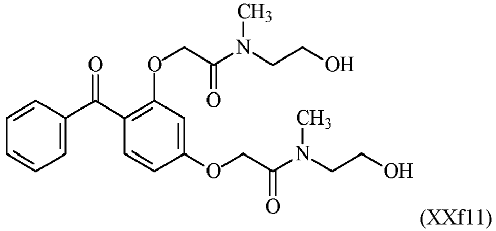

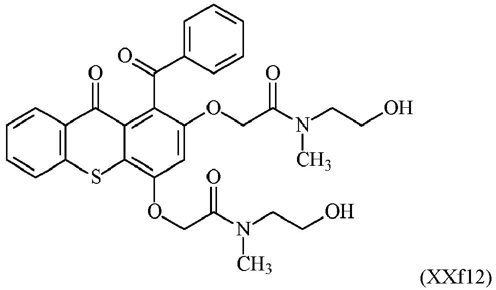

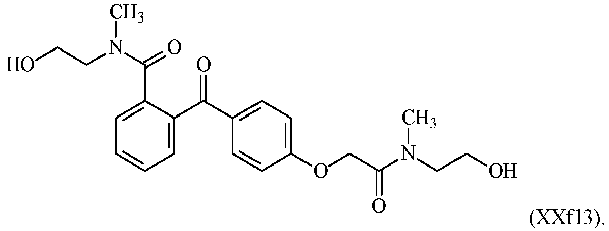

Component c) may comprise at least one hydroxyl-functionalized photoinitiator according to formula (XXe) as defined above. A hydroxyl-functionalized photoinitiator according to formula (XXe) may have at least one photoinitiator moiety PI¹ and at least two OH groups.

In one embodiment, component c) may comprise at least one hydroxyl-functionalized photoinitiator according to formula (XXe) wherein said hydroxyl-functionalized photoinitiator bears at least two photoinitiator moieties PI¹ and at least two OH groups.

In particular, component c) may comprise at least one hydroxyl-functionalized photoinitiator according to formula (XXe) wherein said hydroxyl-functionalized photoinitiator bears a number of photoinitiator moieties PI¹ that is equal to the number of OH groups.

More particularly, component c) may comprise at least one hydroxyl-functionalized photoinitiator according to formula (XXIa): 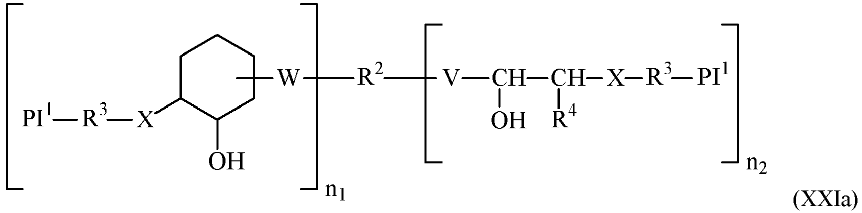 wherein n₁, n₂, PI¹, V, W, X, R², R³ and R⁴ are as defined above for the chromophore moiety Q according to formula (XXI).

In particular, the hydroxyl-functionalized photoinitiator of formula (XXIa) may correspond to one of the following formulae (XXIIa) to (XXXVa): 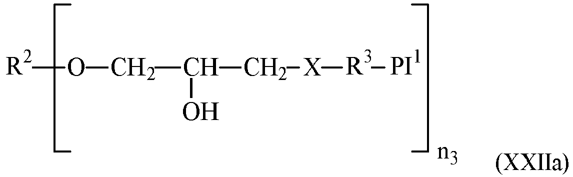 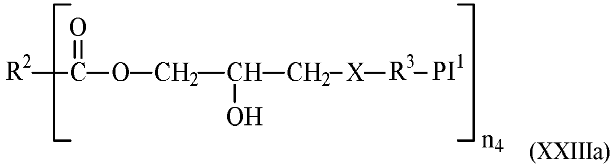 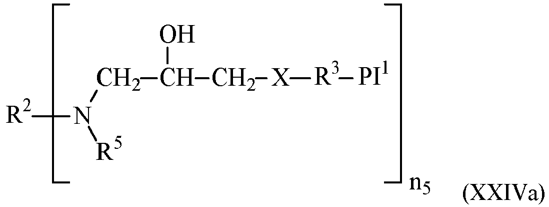 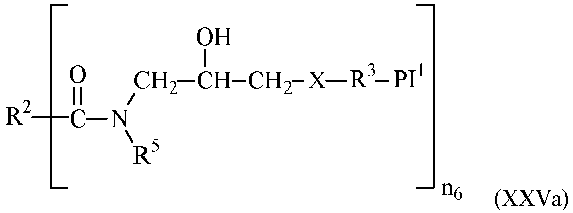 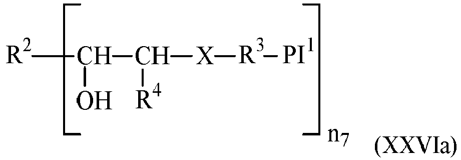 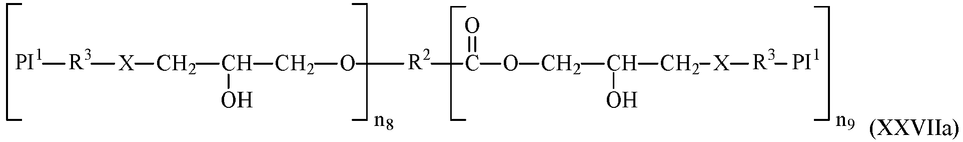 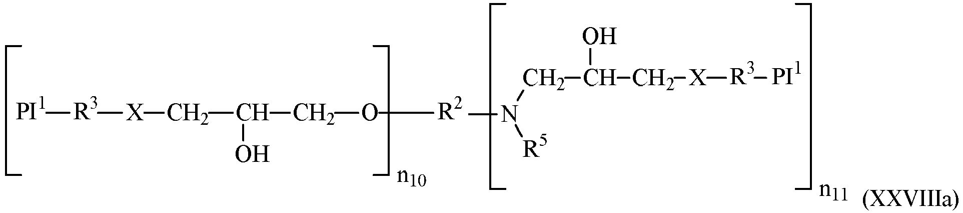 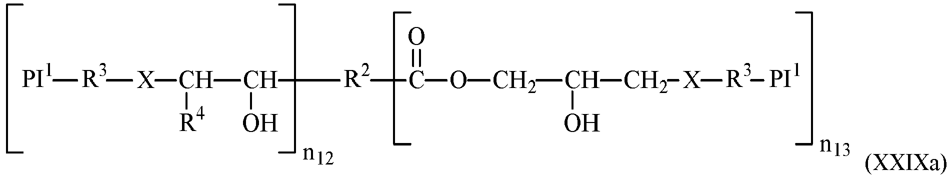 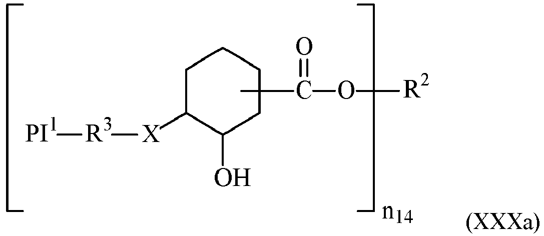 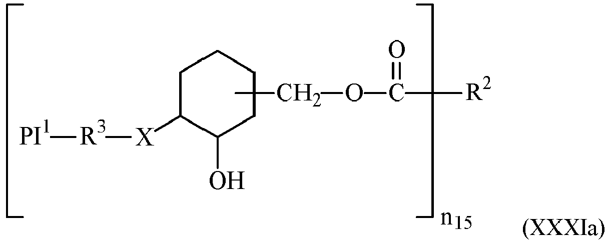 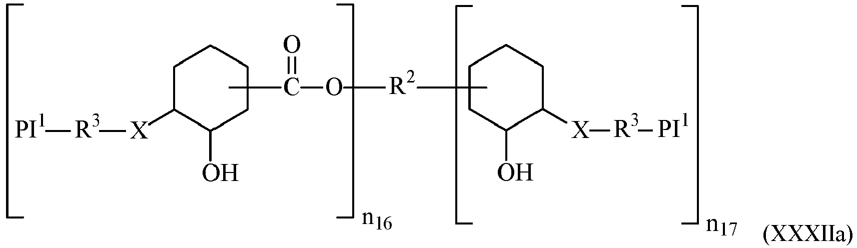 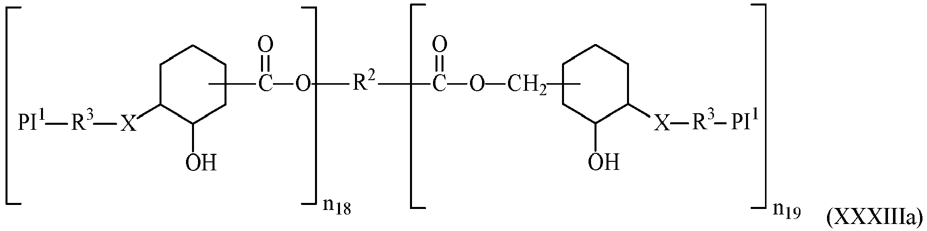 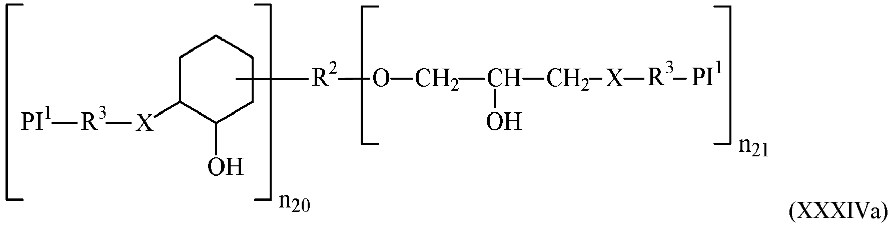 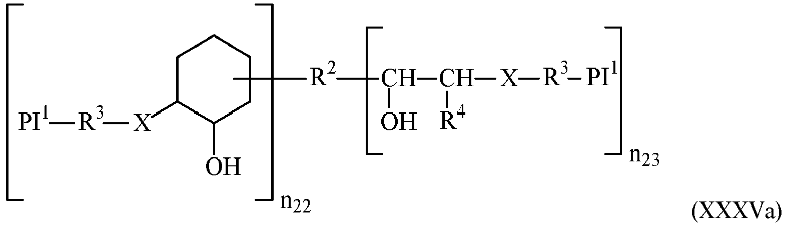
wherein n₃, n₄, n₅, n₆, n₇, n₈, n₉, n₁₀, n₁₁, n₁₂, n₁₃, m₁₄, n₁₅, n₁₆, n₁₇, n₁₈, n₁₉, n₂₀, n₂₁, n₂₂, n₂₃, PI¹, X, R², R³ and R⁴ are as defined above for the chromophore moiety Q according to one of formulae (XXII)-(XXXV);
each R⁵ is independently H, alkyl, aryl or a group according to the following formula: 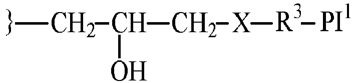 wherein X, R³ and PI¹ are as defined above and the symbol } represents the point of attachment to the nitrogen atom.

All the preferred embodiments for n₃, n₄, n₅, n₆, n₇, n₈, n₉, n₁₀, n₁₁, n₁₂, n₁₃, n₁₄, n₁₅, n₁₆, n₁₇, n₁₈, n₁₉, n₂₀, n₂₁, n₂₂, n₂₃, PI¹, X, R², R³, R⁴ and R⁵ described above for the chromophore moiety Q according to according to one of formulae (XXII)-(XXXV) equally apply to the photoinitiators of formulae (XXIIa)-(XXXVa).

Hydroxyl-functionalized photoinitiators according to one of formulae (XXIIa), (XXIIIa), (XXIVa), (XXVIa), (XXVIIa), (XXVIIIa) or (XXIXa) are preferred. Hydroxyl-functionalized photoinitiators according to one of formulae (XXIIa), (XXIIIa), (XXIVa) or (XXVIa) are particularly preferred.

More particularly, component c) comprises a hydroxyl-functionalized photoinitiator according to one of the following formulae (XXIIa), (XXIIIa), (XXIVa) or (XXVIa): 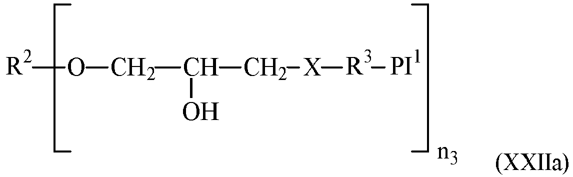 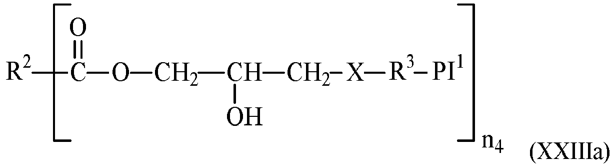 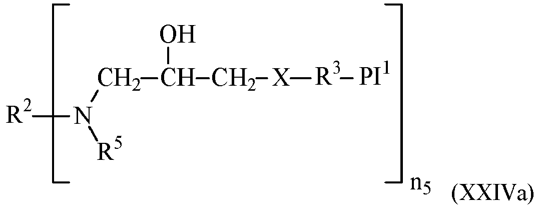 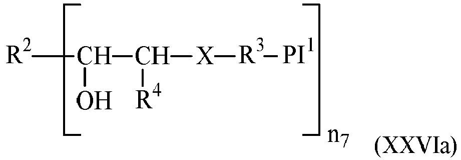 wherein
n₃, n₄, n₅ and n₇ are independently 2, 3 or 4, preferably 2 or 3;
each PI¹ is independently a monovalent photoinitiator moiety PI according to formula (VIII) or (XI) as defined above, preferably each PI¹ is a monovalent photoinitiator moiety PI according to formula (Villa) or (XIa) as defined above;
each X is independently -NRl-, -O- or *-C(=O)-O-, preferably -NR¹- or ^{∗}-C(=O)-O-;
each R¹ is independently H or alkyl;
each R² is independently an alkylene or a heteroatom-containing alkylene;
each R³ is independently a direct bond, an alkylene of formula (C-11) as defined above, an oxyalkylene of formula (C-12) as defined above or an aminoalkylene of formula (C-15) as defined above, preferably a direct bond, an alkylene of formula (C-11) or an oxyalkylene of formula (C-12) as defined above;
each R⁴ is H;
each R⁵ is independently H, alkyl or a group according to the following formula: 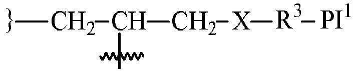 wherein X, R³ and PI¹ are as defined above and the symbol } represents the point of attachment to the nitrogen atom;
the symbol * represents a point of attachment to R³.

Even more particularly, component c) comprises at least one hydroxyl-functionalized photoinitiator according to one of the following formulae (XXe 1)-(XXe9):

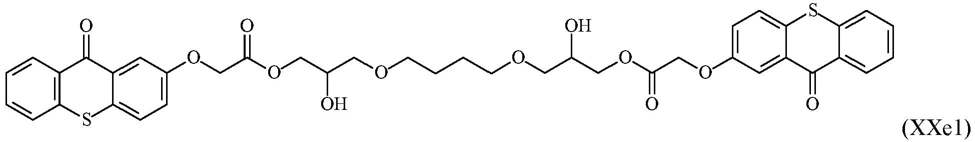

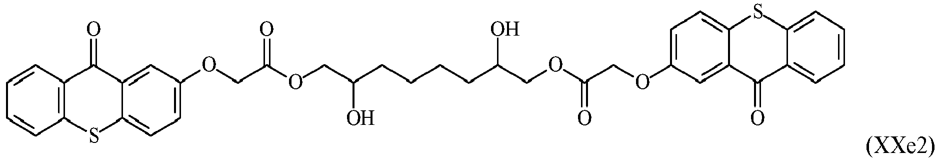

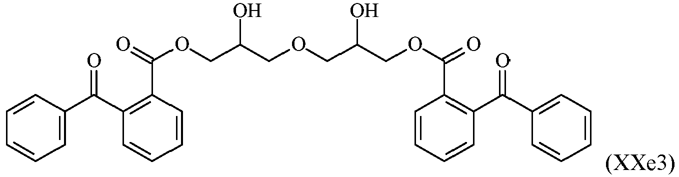

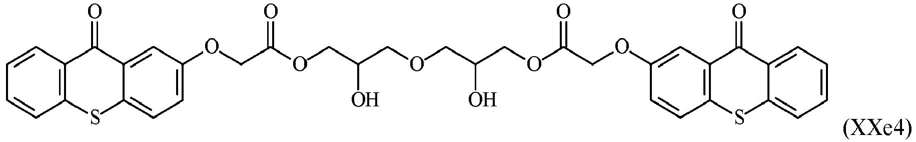

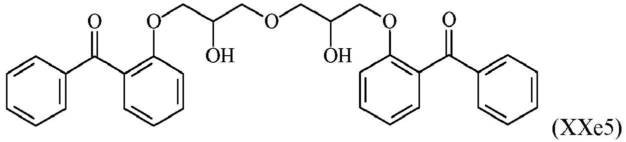

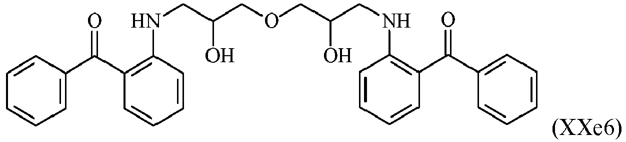

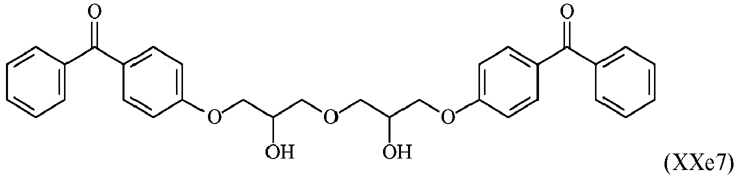

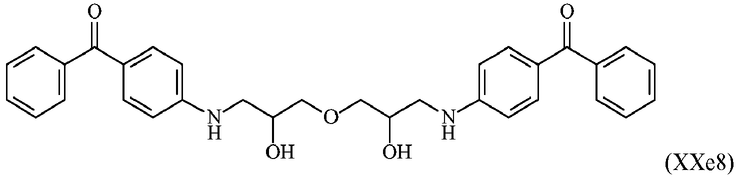

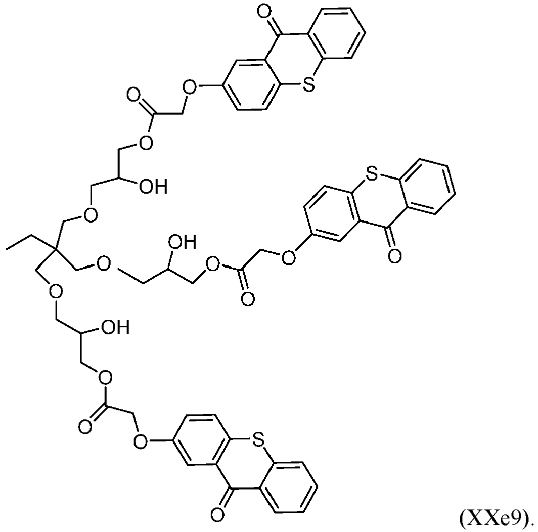

In another embodiment, component c) may comprise at least one hydroxyl-functionalized photoinitiator according to formula (XXe) wherein said hydroxyl-functionalized photoinitiator has a single photoinitiator moiety PI and at least two OH groups.

In particular, component c) may comprise a hydroxyl-functionalized photoinitiator according to one of the following formulae (XXXVIa) to (XXXXIa): 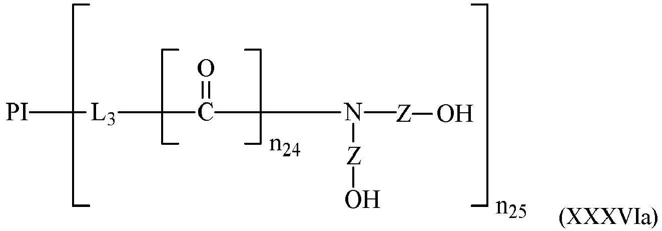 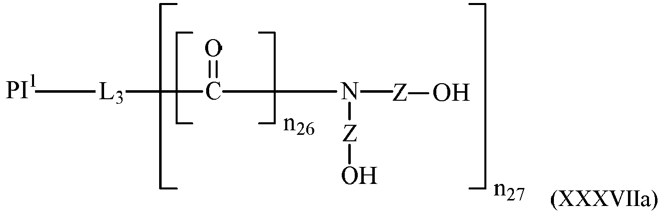 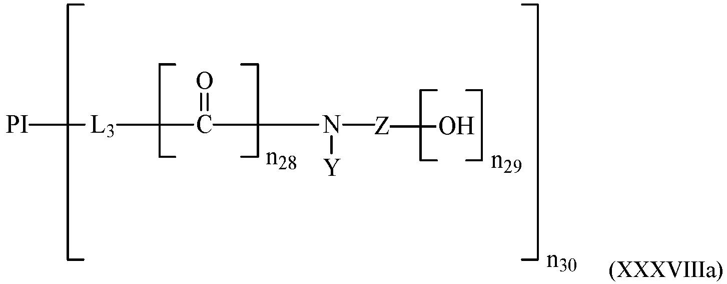 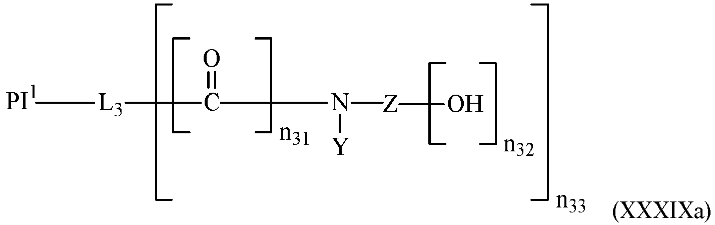 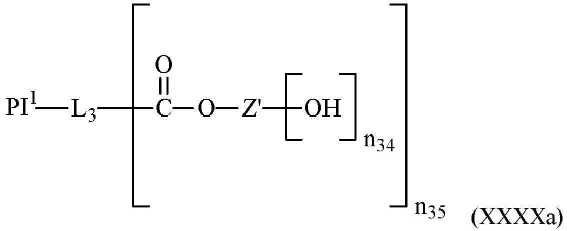 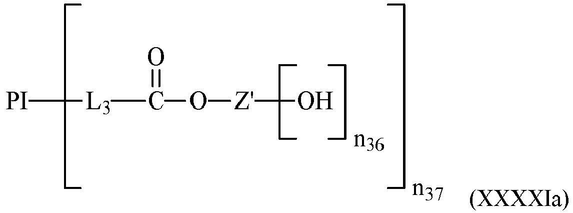 wherein PI, PI¹, L₃, Y, Z, Z', n₂₄, n₂₅, n₂₆, n₂₇, n₂₈, n₂₉, n₃₀, n₃₁, n₃₂, n₃₃, n₃₄, n₃₅, n₃₆ and n₃₇ are as defined above for the chromophore moiety Q according to one of formulae (XXXVI) to (XXXXI).

All the preferred embodiments for PI, PI¹, L₃, Y, Z, Z', n₂₄, n₂₅ n₂₆, n₂₇ n₂₈ n₂₉ n₃₀ n₃₁ n₃₂ n₃₃ n₃₄ n₃₅ n₃₆ and n₃₇ described above for the chromophore moiety Q according to according to one of formulae (XXXVI) to (XXXXI) equally apply to the photoinitiators of formulae (XXXVIa) to (XXXXIa).

More particularly, component c) may comprise a hydroxyl-functionalized photoinitiator according to one of the following formulae (XXXXIIa) to (XXXXIVa): 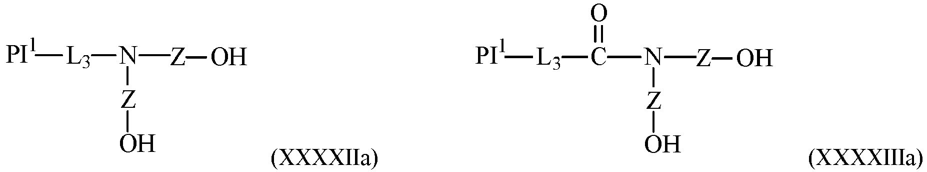 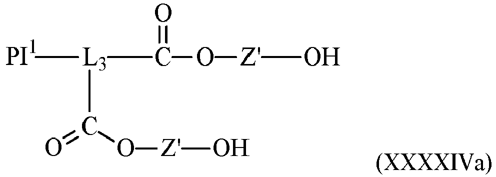 wherein
each PI¹ is independently a monovalent photoinitiator moiety PI according to formula (VIII) or (XI) as defined above, preferably each PI¹ is a monovalent photoinitiator moiety PI according to formula (Villa) or (XIa) as defined above;
each L₃ is independently selected from a direct bond, an alkylene of formula (C-16) as defined above, an oxyalkylene of formula (C-17) as defined above, a thioalkylene of formula (C-18) as defined above, or an alkylene of formula (C-21) as defined above;
each Z is independently an alkylene of formula (C-22) as defined above,
each Z' is independently an alkylene or a polyether linker.

Even more particularly, component c) may comprise a hydroxyl-functionalized photoinitiator according to one of the following formulae (XXe 10)-(XXe26):

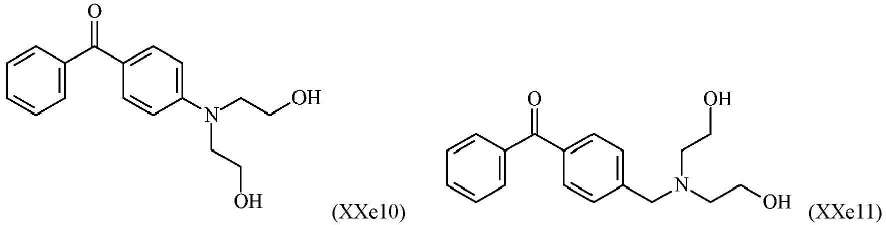

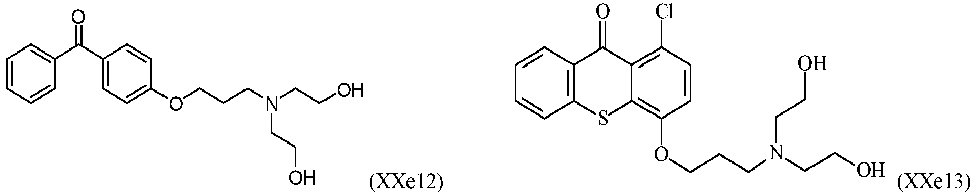

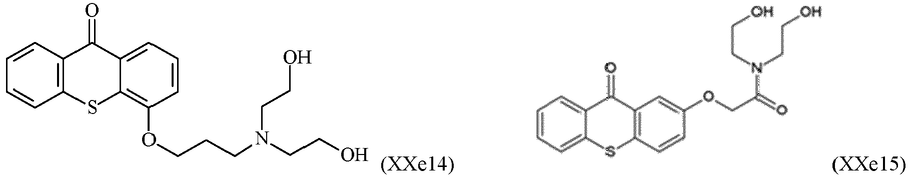

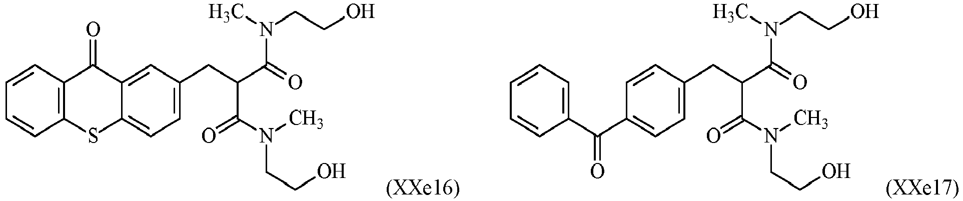

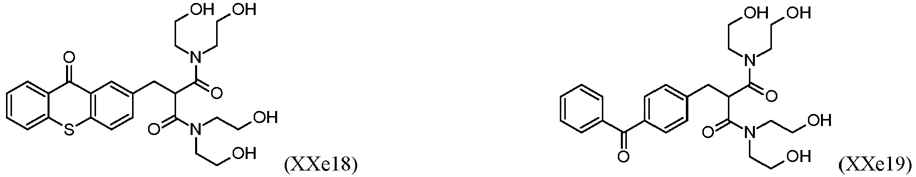

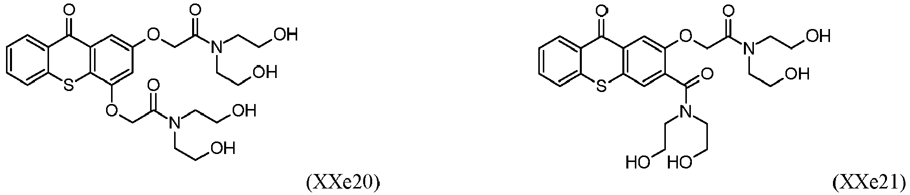

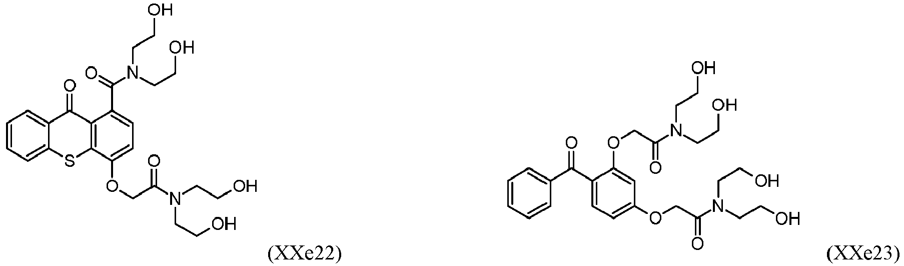

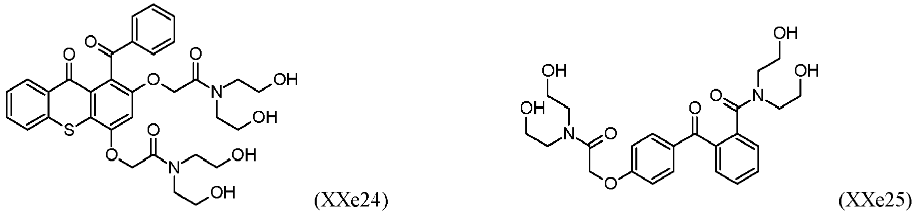

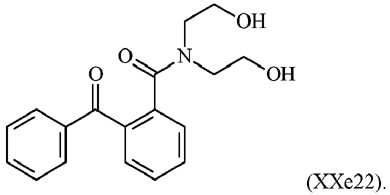

The total amount of component c) used to prepare the self-crosslinkable urethane (meth)acrylate of the invention may be from 0.5 to 20%, in particular from 1 to 15%, more particularly from 1.5 to 10%, by weight of component c) based on the total amount of components a), b), c) and d).

### d) Optional polyol component

One of the components that may optionally be used to prepare the self-crosslinkable urethane (meth)acrylate of the invention is a polyol component, also referred to as component d).

A polyol component comprises or consists of at least one polyol. A polyol component may comprise or consist of a mixture of polyols.

A polyol is a compound bearing at least two hydroxyl groups (in particular 2, 3 or 4 hydroxy groups).

The polyol component d) is distinct from components a), b) and c). Accordingly, component d) may be free of a hydroxyl-functionalized (meth)acrylate compound, a polyisocyanate compound and a hydroxyl-functionalized photoinitiator.

The reaction of component d) with the other components used prepare the self-crosslinkable urethane (meth)acrylate may result in the incorporation of one or more chain-extending moieties EXT in the backbone of the self-crosslinkable urethane (meth)acrylate. The preferential embodiments disclosed above for the chain-extending moiety EXT equally apply to component d).

Component d) may be used in the preparation process of the self-crosslinkable urethane (meth)acrylate in order to increase its number average molecular weight and/or to tailor its mechanical properties and/or to introduce a hydrogen donor functional group to act as a co-initiator for the chromophore moiety Q.

Alternatively, component d) may not be used in the preparation process of the self-crosslinkable urethane (meth)acrylate.

Component d) may comprise at least one polyol (preferably a diol or triol) selected from an aliphatic polyol, an aromatic polyol, a polyether polyol, a polyester polyol, a polycarbonate polyol, a polyorganosiloxane polyol, a polydiene polyol, and combinations thereof.

Component d) may comprise at least one polyol, in particular a diol, as defined above for the chain-extending moiety EXT.

Component d) may comprise at least one polyol selected from a polymeric polyol, a non-polymeric polyol, or an amino-functional polyol, as defined above for the chain-extending moiety EXT.

In particular, component d) may comprise at least one polymeric polyol or at least one non-polymeric polyol, preferably at least one polymeric diol or at least one non-polymeric diol.

Component d) may comprise at least one polymeric polyol. Alternatively, component d) may be substantially free of a polymeric polyol.

The molecular weight of the polymeric polyol may be varied as may be needed or desired in order to achieve particular properties in the self-crosslinkable urethane (meth)acrylate. The number average molecular weight of the polymeric polyol may be at least 330, at least 350, or at least 400 g/mol. The number average molecular weight of the residue of the polymeric polyol may be less than 5000, less than 4500, or less than 4000 g/mol. For example, the polymeric polyol may have a number average molecular weight of from 330 to 5000 g/mol, from 350 to 4500 g/mol or from 400 to 4000 g/mol.

The polymer portion of the polymeric polyol may be comprised of a plurality of repeating units such as oxyalkylene units, ester units, carbonate units, acrylic units, alkylene units or the like or combinations thereof.

In particular, component d) may comprise at least one polymeric diol.

More particularly, component d) may comprise at least one polymeric diol selected from a polyether diol, a polyester diol, a polycarbonate diol, a polyorganosiloxane diol (e.g., a polydimethylsiloxane diol), a polydiene diol including fully or partially hydrogenated polydiene diols (e.g., a polybutadiene diol).

Even more particularly, component d) may comprise at least one polyether diol or at least one polyester diol.

More particularly still, component d) may comprise at least one polyether diol selected from a polyethylene glycol, a poly(1,2-propylene glycol), a poly(1,3-propylene glycol), and a poly(1,4-butylene glycol), or at least one polyester diol selected from a poly(caprolactone), a poly(lactide), a poly(alkylene glycol adipate) and a poly(alkylene glycol succinate).

Component d) may comprise at least one non-polymeric polyol. Alternatively, component d) may be substantially free of a non-polymeric polyol.

In particular, component d) may comprise at least one non-polymeric diol.

More particularly, component d) may comprise at least one non-polymeric aliphatic diol.

Even more particularly, component d) may comprise at least one non-polymeric aliphatic diol selected from ethylene glycol, di-, tri- or tetraethylene glycol, 1,2- or 1,3-propylene glycol, di-, tri- or tetra(1,2-propylene glycol), di-, tri- or tetra(1,3-propylene glycol), 1,2-, 1,3- or 1,4-butylene glycol, di-, tri- or tetra(1,4-butylene glycol), 1,5-pentanediol, 1,6-hexanediol, 1,7-heptanediol, 1,8-octanediol, 1,9-nonanediol, 1,10-decanediol, 1,12-dodecanediol, 2-methyl-1,3-propanediol, 2,2-dimethyl-1,3-propanediol, 2,2-diethyl-1,3-propanediol, 2-methyl-2-ethyl-1,3-propanediol, 3-methyl-1,5-pentanediol, 3,3-dimethyl-1,5-pentanediol, 2,4-diethyl-1,5-pentanediol, 3-butyl-3-ethyl-1,5-pentane diol, 2,2,4-trimethyl 1,5-pentanediol, cyclohexanediol, cyclohexane-1,4-dimethanol, norbornene dimethanol, norbornane dimethanol, tricyclodecanediol, tricyclodecane dimethanol, hydrogenated bisphenol A, B, F or S, a dianhydrohexitol (i.e. isosorbide, isomannide, isoidide), a hydrogenated dimer fatty acid (i.e. a diol obtained by dimerizing one or more unsaturated fatty acids such as oleic acid or linoleic acid and then hydrogenating the resulting product to convert the carboxylic acid groups into hydroxyl groups, for example Pripol^{®} 2033 from Croda) as well as the alkoxylated (i.e. ethoxylated and/or propoxylated) derivatives thereof with up to four oxyalkylene units.

Component d) may comprise a polyol corresponding to the following formula (XXXXVa):

HO-R₃-OH (XXXXVa)

wherein R³ is as defined above for the chain-extending moiety EXT according to formula (XXXXV).

Component d) may comprise at least one polyol which comprises a tertiary amine group (also referred to as an amino-functional polyol). Said tertiary amine group may act as an amine synergist moiety to increase the rate and efficiency of photoinitiation by the chromophore moiety included in the backbone of the self-crosslinkable urethane (meth)acrylate.

In particular, component d) may comprise at least one amino-functional polyol selected from triethanol amine, N-methyldiethanol amine, N-ethyldiethanol amine, N-propyl diethanolamine, N-butyl diethanol amine, N-t-butyl diethanolamine, trimethanol amine, N-methyl dimethanol amine, 3-(dimethylamino)-1,2-propanediol, 3-(diethylamino)-1,2-propanediol, 3-(dipropylamino)-1,2-propanediol, 2-(dimethylamino)propane-1,3-diol, bis(2-hydroxyethyl)dodecylamine, bis(2-hydroxyethyl)octadecylamine, N,N-dioctadecyl-N',N'-bis(2-hydroxyethyl)-1,3-diaminopropane, 3-morpholino-1,2-propanediol, 3-piperidino-1,2-propanediol, 3-pyrrolidino-1-yl-1,2- propanediol, and an aminobenzamide diol according to the following formula (XXXXVI): 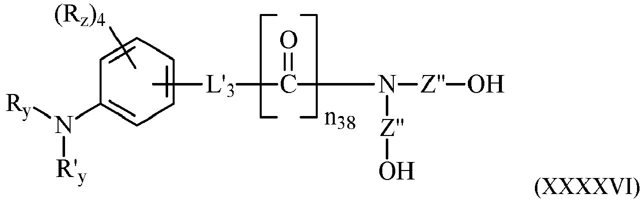 wherein R_{y}, R'_{y}, R_{z}, L'₃, Z" and n₃₈ are as defined in the chain-extending moiety according to formula (XXXXVI).

The total amount of component d) used to prepare the self-crosslinkable urethane (meth)acrylate of the invention may be from 0 to 84.5%, in particular from 0.1 to 75%, more particularly from 1 to 65%, by weight of component d) based on the total amount of components a), b), c) and d).

In one embodiment, the total amount of component d) used to prepare the self-crosslinkable urethane (meth)acrylate of the invention may be less than 0.2%, in particular less than 0.1%, more particularly 0%, by weight of component d) based on the total amount of components a), b), c) and d). Such amounts may for example be used when the process of preparation of the self-crosslinkable urethane (meth)acrylate of the invention is substantially free of component d).

In one embodiment, the total amount of component d) used to prepare the self-crosslinkable urethane (meth)acrylate of the invention may be from 0.1 to 20%, in particular from 0.2 to 15%, more particularly from 0.5 to 10%, by weight of component d) based on the total amount of components a), b), c) and d). Such amounts may for example be used when component d) comprises a non-polymeric polyol.

In another embodiment, the total amount of component d) used to prepare the self-crosslinkable urethane (meth)acrylate of the invention may be from 15 to 84.5%, in particular from 20 to 75%, more particularly from 25 to 65%, by weight of component d) based on the total amount of components a), b), c) and d). Such amounts may for example be used when component d) comprises a polymeric polyol.

### e) Other optional components

The preparation process of the self-crosslinkable urethane (meth)acrylate may involve the use of components other than components a), b), c) and d).

The process may be carried out in the presence of a catalyst. Alternatively, the process may not require the presence of a catalyst.

When present, the catalyst may be any substance which is capable of catalyzing the reaction between a hydroxyl group and an isocyanate group to form a urethane linkage. The catalyst may accelerate the rate at which such reaction takes place at a given temperature and/or achieve a target degree of completion of such reaction at a temperature which is lower than the temperature at which the target degree of completion is achieved in the absence of any urethane catalyst.

Any of the tin-based catalysts known in the art may be utilized (e.g., dibutyltin dilaurate). However, according to certain embodiments, a non-tin catalyst or a combination of non-tin catalysts is used.

Suitable non-tin catalysts include, for example, one or more non-tin catalysts selected from the group consisting of carboxylate complexes of bismuth (such as bismuth octoate or bismuth neodecanoate); acetylacetonate complexes of zirconium; acetylacetonate complexes of hafnium; acetylacetonate complexes of titanium; beta-diketiminate complexes of zirconium; beta-diketiminate complexes of hafnium; beta-diketiminate complexes of titanium; amidinate complexes of zirconium; amidinate complexes of hafnium; amidinate complexes of titanium; carboxylate complexes of zinc; tertiary amines; imidazoles; N-heterocyclic carbenes; tetraalkylammonium (pseudo)halides; phosphines; and combinations thereof.

Typically, the catalyst may be utilized in an amount which is from 0.0001 to 0.1% by weight, based on the total weight of components a), b), c) and d).

The process may be carried out in the presence of a solvent. Alternatively, the process may not require the presence of a solvent. As used herein, the term "solvent" means a non-reactive organic solvent, i.e. a solvent that does not react with the other components used to prepare the self-crosslinkable urethane (meth)acrylate.

Examples of suitable solvents include aliphatic hydrocarbons such n-pentane, n-hexane, n-heptane, octane cyclohexane or methylcyclohexane; aromatic hydrocarbons such as benzene toluene or xylene; halogenated hydrocarbons such as dichloromethane, chloroform or trichlororethane; ketones such as acetone, methyl ethyl ketone, methylpropylketone, diethylketone, methylisobutylketone, ethylbutylketone, cyclopentanone or cyclohexanone; esters such as methyl formate, butyl formate, methyl acetate, ethyl acetate, propyl acetate or butyl acetate; ethers such as diethyl ether, diisopropyl ether, dibutyl ether, ethylene glycol diethyl ether, tetrahydrofuran or tetrahydropyrane; carbonates such as diethylcarbonate; and combinations thereof.

Preferably, the process does not involve the use of a solvent. When a solvent is used, the total amount of solvent may be from 5 to 150% by weight based on the total weight of components a), b), c) and d).

The process may be carried out in the presence of a stabilizer such as an antioxidant, a light blocker/absorber or a polymerization inhibitor. Alternatively, the process may not require the presence of a stabilizer.

Stabilizers may be introduced during the preparation process of the self-crosslinkable urethane (meth)acrylate, to protect against unwanted reactions during the preparation process and/or during storage of the resulting product. A stabilizer may be a compound or substance which retards or prevents reaction or curing of actinically-polymerizable functional groups present in a composition in the absence of actinic radiation. However, it will be advantageous to select an amount and type of stabilizer such that the self-crosslinkable urethane (meth)acrylate remains capable of being cured when exposed to actinic radiation (that is, the stabilizer does not prevent radiation curing of the composition). The stabilizer may, in particular be a free radical stabilizer (i.e. a stabilizer which functions by inhibiting free radical reactions).

Any of the stabilizers known in the art related to (meth)acrylate-functionalized compounds may be utilized in the present invention. Quinones represent a particularly preferred type of stabilizer which can be employed in the context of the present invention. As used herein, the term "quinone" includes both quinones and hydroquinones as well as ethers thereof such as monoalkyl, monoaryl, monoaralkyl and bis(hydroxyalkyl) ethers of hydroquinones. Hydroquinone monomethyl ether (MeHQ) is an example of a suitable stabilizer which can be utilized. Other stabilizers known in the art such as hydroquinone (HQ), 4-tert-butylcatechol (TBC), 3,5-di-tertiobutyl-4-hydroxytoluene (BHT), phenothiazine (PTZ), pyrogallol, phosphite compounds, triphenyl antimony and tin(II) salts.

Preferably, the process involves the use of a stabilizer such as MeHQ or BHT. When a stabilizer is used, the total amount of stabilizer may be from 0.01 to 1% by weight based on the total weight of components a), b), c) and d).

The preparation process of the self-crosslinkable urethane (meth)acrylate may not involve the use of a component other than component a), component b), component c), component d), a catalyst, a solvent and a stabilizer.

In particular, the preparation process of the self-crosslinkable urethane (meth)acrylate may not involve the use of a dye component. Accordingly, components a), b), c) and d) may not comprise a dye moiety.

A dye component comprises or consists of at least one NCO-reactive dye. A dye component may comprise or consist of a mixture of NCO-reactive dyes.

A NCO-reactive dye is a dye bearing at least one group which is reactive towards isocyanate groups such as for example a hydroxyl group, a thiol group or an amino group. The term "dye" as used herein means a compound that imparts a color different from the color of the self-crosslinkable urethane (meth)acrylate not including any such dye moiety, such that the different color is detectable by the naked eye colorant having a solubility of 10 mg/L or more in the medium in which it is introduced at 25°C.

The NCO-reactive dye may be selected from an azo dye, a phthalocyanine dye, an anthraquinone dye, a di- or tricyanovinyl dye, a methine dye, an aniline dye, an indoaniline dye, such as those described in US 2014/0316060 A1.

### Polymerization process

The self-crosslinkable urethane (meth)acrylate of the invention may be used in a polymerization process, i.e. a process for polymerizing (e.g. curing) one or more ethylenically unsaturated compounds.

The process for polymerizing one or more ethylenically unsaturated compounds comprises contacting one or more ethylenically unsaturated compounds with the self-crosslinkable urethane (meth)acrylate according to the invention and irradiating the mixture, in particular with UV, near-UV, visible, infrared, near-infrared and/or electron beam radiation.

In particular, the polymerization process does not involve the use of a radical initiator or initiating system other than the self-crosslinkable urethane (meth)acrylate of the invention.

Examples of radical initiators and initiating systems include photoinitiators, peroxides, redox systems, azo-compounds.

A photoinitiator is a compound capable of absorbing light and generating a reactive species useful to initiate a polymerisation reaction. Non-limiting types of photoinitiators include, for example, benzoins, benzoin ethers, acetophenones, α-hydroxy acetophenones, benzil, benzil ketals, phosphine oxides, acylphosphine oxides, α-hydroxyketones, phenylglyoxylates, α-aminoketones, benzophenones, thioxanthones, xanthones, acridine derivatives, phenazine derivatives, quinoxaline derivatives, triazine compounds, benzoyl formates, aromatic oximes, metallocenes, acylsilyl or acylgermanyl compounds, camphorquinones, polymeric derivatives thereof, and mixtures thereof. Examples of specific photoinitiators include, but are not limited to, benzoin ethers, benzoin, benzoin methyl ether, benzoin ethyl ether, benzoin isopropyl ether, alpha-methylbenzoin, alpha-phenylbenzoin, Michler's ketone, acetophenones such as 2,2-dialkoxybenzophenones and 1-hydroxyphenyl ketones, benzophenone, 4,4'-bis-(diethylamino) benzophenone, acetophenone, 2,2-diethyloxyacetophenone, , 2-isopropylthioxanthone, thioxanthone, diethyl thioxanthone, 1,5-acenaphthylene, benzil, α-hydroxyketone, 2,4,6-trimethylbenzoyldiphenyl phosphine oxide, 2,2-dimethoxy-1,2-diphenylethanone, 1-hydroxycyclohexyl phenyl ketone, 2-methyl-1-[4-(methylthio) phenyl]-2-morpholinopropanone, 2-hydroxy-2-methyl-1-phenyl-propanone, oligomeric α-hydroxy ketone, benzoyl phosphine oxides, phenylbis(2,4,6-trimethylbenzoyl)phosphine oxide, ethyl(2,4,6-trimethylbenzoyl)phenyl phosphinate, anisoin, (benzene) tricarbonylchromium, benzoin isobutyl ether, benzophenone/1-hydroxycyclohexyl phenyl ketone 50/50 blend, 3,3',4,4'-benzophenonetetracarboxylic dianhydride, 4-benzoylbiphenyl, 2-benzyl-2-(dimethylamino)-4'-morpholinobutyrophenone, 4,4'-bis(diethylamino)benzophenone, 4,4'-bis(dimethylamino)benzophenone, camphorquinone, 2-chlorothioxanthen-9-one, dibenzosuberenone, 4,4'-dihydroxybenzophenone, , 4-(dimethylamino)benzophenone, 4,4'-dimethylbenzil, 2,5-dimethylbenzophenone, 3,4-dimethylbenzophenone, diphenyl(2,4,6-trimethylbenzoyl)phosphine oxide /2-hydroxy-2-methylpropiophenone 50/50 blend, 4'-ethoxyacetophenone, 2,4,6-trimethylbenzoyldiphenylphosphine oxide,, 3'-hydroxyacetophenone, 4'-hydroxyacetophenone, 3-hydroxybenzophenone, 4-hydroxybenzophenone, 2-methylbenzophenone, 3-methylbenzophenone, methybenzoylformate, phenanthrenequinone, 4'-phenoxyacetophenone, (cumene)cyclopentadienyl iron(ii) hexafluorophosphate, 9,10-diethoxy and 9,10-dibutoxyanthracene, 2-ethyl-9,10-dimethoxyanthracene, and combinations thereof.

A peroxide may be defined as a compound comprising an oxygen-oxygen single bond. The peroxide may be selected from a persulfate, hydrogen peroxide (H₂O₂), an organic hydroperoxide, a peracid, or a mixture thereof. Non-limiting examples of peroxides include ammonium persulfate, potassium persulfate, sodium persulfate, hydrogen peroxide, cumene hydroperoxide, t-butyl hydroperoxide, acetyl peroxide, benzoyl peroxide, lauroyl peroxide; peracetic acid and perbenzoic acid.

A redox system may be defined as a mixture of a peroxide and a reducing agent which promotes the decomposition of the peroxide. Examples of suitable reducing agents include ferrous compounds, carboxylic acids and/or sodium metabisulfite.

An azo-compound may be defined as a compound comprising a nitrogen-nitrogen double bond. Examples of suitable azo-compounds include 2,2'-azobisisobutyronitrile, 4,4'-azobis(4-cyanovaleric acid) or 2,2'-azobis(2-methylbutyronitrile).

The ethylenically unsaturated compound(s) that are polymerized with the polymerization process may be as defined below.

### Curable composition

The self-crosslinkable urethane (meth)acrylate disclosed herein is especially useful for applications requiring very low extractables and fast cure time when exposed to actinic radiation. In particular, the self-crosslinkable urethane (meth)acrylate may be used in curable compositions to obtain a coating (in particular a scratch-resistant wood coating, a concrete coating or a plastic coating), an ink, a varnish, an encapsulation or potting material, a 3D-printed article, a molded article, a sealant, an adhesive, a nail polish or a dental material. The self-crosslinkable urethane (meth)acrylate is especially useful to obtain thick parts or thick coatings.

The curable composition of the invention comprises the self-crosslinkable urethane (meth)acrylate according to the invention, referred to as component A) and optionally a polymerizable component other than component A), referred to as component B).

The curable composition of the invention may comprise from 1 to 100%, from 5 to 95%, from 10 to 90%, from 15 to 85%, from 20 to 80%, from 25 to 75%, from 30 to 70%, from 35 to 65% or from 40 to 60%, by weight of component A) based on the total weight of components A) and B).

The curable composition of the invention may comprise from 0 to 99%, from 0 to 95%, from 10 to 90%, from 15 to 85%, from 20 to 80%, from 25 to 75%, from 30 to 70%, from 35 to 65% or from 40 to 60%, by weight of component B) based on the total weight of components A) and B).

The curable composition of the invention may comprise from 1 to 100%, from 5 to 95%, from 5 to 90%, from 10 to 85%, from 10 to 80%, from 15 to 75%, from 15 to 70%, from 20 to 65% or from 20 to 60% by weight of component A) based on weight of the curable composition.

The curable composition of the invention may comprise from 0 to 99%, from 5 to 95%, from 10 to 95%, from 15 to 90%, from 20 to 90%, from 25 to 85%, from 30 to 85%, from 35 to 80% or from 40 to 80%, by weight of component B) based on weight of the curable composition.

### Polymerizable component

The curable composition of the invention may optionally comprise a polymerizable component B).

A polymerizable component comprises or consists of one or more ethylenically unsaturated compounds. A polymerizable component may comprise or consist of one or more ethylenically unsaturated compounds.

Component B) is distinct from Component A). Accordingly, the one or more ethylenically unsaturated compounds of component B) do not comprise a chromophore moiety.

Component B) may comprise one or more ethylenically unsaturated compounds selected from a (meth)acrylate-functionalized monomer, a (meth)acrylate-functionalized oligomer and mixtures thereof. In particular, component B) may comprise one or more (meth)acrylate functionalized monomers.

As used herein, the term "(meth)acrylate-functionalized monomer" means a monomer comprising a (meth)acrylate group, in particular an acrylate group. The term "(meth)acrylate-functionalized oligomer" means an oligomer comprising a (meth)acrylate group, in particular an acrylate group.

Component B) may comprise at least one (meth)acrylate-functionalized monomer. Component B) may comprise a mixture of (meth)acrylate-functionalized monomers.

A (meth)acrylate-functionalized monomer may have a molecular weight of less than 600 g/mol, in particular from 100 to 550 g/mol, more particularly 200 to 500 g/mol.

A (meth)acrylate-functionalized monomer may have 1 to 6 (meth)acrylate groups, in particular 1 to 3 (meth)acrylate groups.

Component B) may comprise a mixture of (meth)acrylate-functionalized monomers having different functionalities. For example component B) may comprise a mixture of a (meth)acrylate-functionalized monomer containing a single acrylate or methacrylate group per molecule (referred to herein as "mono(meth)acrylate-functionalized compounds") and a (meth)acrylate-functionalized monomer containing 2 or more, preferably 2 or 3, acrylate and/or methacrylate groups per molecule (referred to herein as "poly(meth)acrylate-functionalized compounds").

In particular, component B) may comprise a mono(meth)acrylate-functionalized monomer. The mono(meth)acrylate-functionalized monomer may advantageously function as a reactive diluent and reduce the viscosity of the composition.

Examples of suitable mono(meth)acrylate-functionalized monomers include, but are not limited to, mono-(meth)acrylate esters of aliphatic alcohols (wherein the aliphatic alcohol may be straight chain, branched or alicyclic and may be a mono-alcohol, a di-alcohol or a polyalcohol, provided only one hydroxyl group is esterified with (meth)acrylic acid); mono-(meth)acrylate esters of aromatic alcohols (such as phenols, including alkylated phenols); mono-(meth)acrylate esters of alkylaryl alcohols (such as benzyl alcohol); mono-(meth)acrylate esters of oligomeric and polymeric glycols such as diethylene glycol, triethylene glycol, dipropylene glycol, tripropylene glycol, polyethylene glycol, and polypropylene glycol); mono-(meth)acrylate esters of monoalkyl ethers of glycols and oligoglycols; mono-(meth)acrylate esters of alkoxylated (e.g., ethoxylated and/or propoxylated) aliphatic alcohols (wherein the aliphatic alcohol may be straight chain, branched or alicyclic and may be a mono-alcohol, a di-alcohol or a polyalcohol, provided only one hydroxyl group of the alkoxylated aliphatic alcohol is esterified with (meth)acrylic acid); mono-(meth)acrylate esters of alkoxylated (e.g., ethoxylated and/or propoxylated) aromatic alcohols (such as alkoxylated phenols); caprolactone mono(meth)acrylates; and the like.

The following compounds are specific examples of mono(meth)acrylate-functionalized monomers suitable for use in component B): methyl (meth)acrylate; ethyl (meth)acrylate; n-propyl (meth)acrylate; n-butyl (meth)acrylate; isobutyl (meth)acrylate; n-hexyl (meth)acrylate; 2-ethylhexyl (meth)acrylate; n-octyl (meth)acrylate; isooctyl (meth)acrylate; n-decyl (meth)acrylate; n-dodecyl (meth)acrylate; tridecyl (meth)acrylate; tetradecyl (meth)acrylate; hexadecyl (meth)acrylate; 2-hydroxyethyl (meth)acrylate; 2- and 3-hydroxypropyl (meth)acrylate; 2-methoxyethyl (meth)acrylate; 2-ethoxyethyl (meth)acrylate; 2- and 3-ethoxypropyl (meth)acrylate; tetrahydrofurfuryl (meth)acrylate; alkoxylated tetrahydrofurfuryl (meth)acrylate; 2-(2-ethoxyethoxy)ethyl (meth)acrylate; cyclohexyl (meth)acrylate; glycidyl (meth)acrylate; isodecyl (meth)acrylate; lauryl (meth)acrylate; 2-phenoxyethyl (meth)acrylate; alkoxylated phenol (meth)acrylates; alkoxylated nonylphenol (meth)acrylates; cyclic trimethylolpropane formal (meth)acrylate; isobornyl (meth)acrylate; tricyclodecanemethanol (meth)acrylate; tert-butylcyclohexanol (meth)acrylate; trimethylcyclohexanol (meth)acrylate; diethylene glycol monomethyl ether (meth)acrylate; diethylene glycol monoethyl ether (meth)acrylate; diethylene glycol monobutyl ether (meth)acrylate; triethylene glycol monoethyl ether (meth)acrylate; ethoxylated lauryl (meth)acrylate; methoxy polyethylene glycol (meth)acrylates; hydroxyl ethylbutyl urethane (meth)acrylates; 3-(2-hydroxyalkyl)oxazolidinone (meth)acrylates; and combinations thereof.

Component B) may comprise a poly(meth)acrylate-functionalized monomer.

A poly(meth)acrylate-functionalized monomer may have from 2 to 6 (meth)acrylate groups, in particular from 2 to 6 acrylate groups.

Examples of suitable poly(meth)acrylate-functionalized monomers include acrylate and methacrylate esters of polyols. Examples of suitable polyols are as listed above for the optional polyol component d) used to prepare the self-crosslinkable urethane (meth)acrylate of the invention. Such polyols may be fully or partially esterified (with (meth)acrylic acid, (meth)acrylic anhydride, (meth)acryloyl chloride or the like), provided they contain at least two (meth)acrylate functional groups per molecule.

Exemplary poly(meth)acrylate-functionalized monomers may include bisphenol A di(meth)acrylate; hydrogenated bisphenol A di(meth)acrylate; ethylene glycol di(meth)acrylate; diethylene glycol di(meth)acrylate; triethylene glycol di(meth)acrylate; tetraethylene glycol di(meth)acrylate; polyethylene glycol di(meth)acrylate; propylene glycol di(meth)acrylate; dipropylene glycol di(meth)acrylate; tripropylene glycol di(meth)acrylate; tetrapropylene glycol di(meth)acrylate; polypropylene glycol di(meth)acrylate; polytetramethylene glycol di(meth)acrylate; 1,2-butanediol di(meth)acrylate; 2,3-butanediol di(meth)acrylate; 1,3-butanediol di(meth)acrylate; 1,4-butanediol di(meth)acrylate; 1,5-pentanediol di(meth)acrylate; 1,6-hexanediol di(meth)acrylate; 1,8-octanediol di(meth)acrylate; 1,9-nonanediol di(meth)acrylate; 1,10-nonanediol di(meth)acrylate; 1,12-dodecanediol di(meth)acrylate; neopentyl glycol di(meth)acrylate; 2-methyl-2,4-pentanediol di(meth)acrylate; polybutadiene di(meth)acrylate; cyclohexane-1,4-dimethanol di(meth)acrylate; tricyclodecane dimethanol di(meth)acrylate; metallic di(meth)acrylates; modified metallic di(meth)acrylates; glyceryl di(meth)acrylate; glyceryl tri(meth)acrylate; trimethylolethane tri(meth)acrylate; trimethylolethane di(meth)acrylate; trimethylolpropane tri(meth)acrylate; trimethylolpropane di(meth)acrylate; pentaerythritol di(meth)acrylate; pentaerythritol tri(meth)acrylate; pentaerythritol tetra(meth)acrylate, di(trimethylolpropane) diacrylate; di(trimethylolpropane) triacrylate; di(trimethylolpropane) tetraacrylate, sorbitol penta(meth)acrylate; di(pentaerythritol) tetraacrylate; di(pentaerythritol) pentaacrylate; di(pentaerythritol) hexa(meth)acrylate; tris (2-hydroxyethyl) isocyanurate tri(meth)acrylate; as well as the alkoxylated (e.g., ethoxylated and/or propoxylated) derivatives thereof; and combinations thereof.

In a preferred embodiment, component B) comprises at least one poly(meth)acrylate-functionalized monomer selected from glycerol tri(meth)acrylate; diglycerol tetra(meth)acrylate, triglycerol penta(meth)acrylate, tetraglycerol hexa(meth)acrylate, trimethylolethane tri(meth)acrylate; trimethylolpropane tri(meth)acrylate; pentaerythritol tetra(meth)acrylate, di(trimethylolpropane) tetraacrylate, sorbitol penta(meth)acrylate; di(pentaerythritol) hexa(meth)acrylate; tris (2-hydroxyethyl) isocyanurate tri(meth)acrylate, as well as the alkoxylated (e.g., ethoxylated and/or propoxylated) derivatives thereof, and combinations thereof.

Component B) may comprise a urethane (meth)acrylate monomer.

A urethane (meth)acrylate monomer may have from 2 to 10 (meth)acrylate groups and at least two urethane bonds.

In particular, component B) may comprise a urethane (meth)acrylate monomer according to the following formula (XXXXVII). 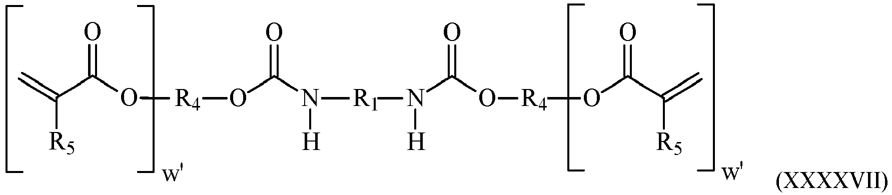 wherein
R₄, R₅ and w' are as defined above for the (meth)acrylate-functionalized moiety ACR;
R₁ is as defined above for the polyisocyanate moiety UU.

Component B) may comprise 0 to 100%, in particular 5 to 90%, more particularly 10 to 80%, even more particularly 15 to 75%, more particularly still 20 to 70% by weight of (meth)acrylate-functionalized monomer based on the total weight of the component B). In particular, component B) may comprise 50 to 100% or 55 to 100% or 60 to 100% or 65 to 100% or 70 to 100%, by weight of (meth)acrylate-functionalized monomer based on the total weight of component B).

Component B) may comprise a (meth)acrylate-functionalized oligomer. Component B) may comprise a mixture of (meth)acrylate-functionalized oligomers.

A (meth)acrylate-functionalized oligomer may be selected in order to enhance the flexibility, strength and/or modulus, among other attributes, of a cured polymer prepared by curing the curable composition of the invention.

A (meth)acrylate functionalized oligomer may have 1 to 18 (meth)acrylate groups, in particular 2 to 6 (meth)acrylate groups, more particularly 2 to 6 acrylate groups.

A (meth)acrylate functionalized oligomer may have a number average molecular weight equal or more than 600 g/mol, in particular 800 to 15,000 g/mol, more particularly 1,000 to 5,000 g/mol.

In particular, component B) may comprise a (meth)acrylate-functionalized oligomer selected from the group consisting of epoxy (meth)acrylates, polyester (meth)acrylates, polyether (meth)acrylates, urethane (meth)acrylates, (meth)acrylated poly(meth)acrylates and mixtures thereof.

Non-limiting examples of epoxy (meth)acrylates are the reaction products of an epoxide (such as glycidyl ethers, glycidyl esters, cycloaliphatic epoxides or epoxides obtained by epoxidation of mono- and/or polyunsaturated compounds) with a (meth)acrylating agent (such as (meth)acrylic acid, (meth)acrylic anhydride, (meth)acryloyl chloride or combinations thereof). The epoxide may be selected from 1,2,3,4-diepoxybutane; 1,2,4,5-diepoxypentane; 1,2,5,6-diepoxyhexane; 1,2,7,8-diepoxyoctane; 1,2,9,10-diepoxydecane; bisphenol A diglycidyl ether, bisphenol F diglycidyl ether, bisphenol S diglycidyl ether, brominated bisphenol A diglycidyl ether, brominated bisphenol F diglycidyl ether, brominated bisphenol S diglycidyl ether, epoxy novolak resin, hydrogenated bisphenol A diglycidyl ether, hydrogenated bisphenol F diglycidyl ether, hydrogenated bisphenol S diglycidyl ether, 3,4-epoxycyclohexylmethyl-3',4'-epoxycyclohexanecarboxylate, 2-(3,4-epoxycyclohexyl-5,5-spiro-3,4-epoxy)cyclohexane-1,4-dioxane, bis(3,4-epoxycyclohexylmethyl)adipate, vinylcyclohexene oxide, 4-vinylepoxycyclohexane, bis(3,4-epoxy-6-methylcyclohexylmethyl)adipate,3,4-epoxy-6-methylcyclohexy 1-3',4'-epoxy-6'-methylcyclohexanecarboxylate, methylenebis(3,4-epoxycyclohexane), dicyclopentadiene diepoxide, di(3,4-epoxycyclohexylmethyl) ether of ethylene glycol, ethylenebis(3, 4-epoxycyclohexanecarboxylate), ethylene glycol diglycidyl ether, 1,2- or 1,3-propylene glycol diglycidyl ether, 1,2-, 1,3- or 1,4-butanediol diglycidyl ether, 1,5-pentanediol diglycidyl ether, 1,6-hexanediol diglycidyl ether, 1,7-hexanediol diglycidyl ether, 1,8-octanediol diglycidyl ether, 1,9-nonanediol diglycidyl ether, 1,10-decanediol diglycidyl ether, 1,12-dodecanediol diglycidyl ether, 2-methyl-1,3-propanediol diglycidyl ether, neopentyl glycol diglycidyl ether, 2,2-diethyl-1,3-propane diol diglycidyl ether, 3-methyl-1,5-pentanediol diglycidyl ether, 3,3-dimethyl-1,5-pentanediol diglycidyl ether, 2,4-diethyl-1,5-pentanediol diglycidyl ether, 3,3-butylethyl-1,5-pentane diol diglycidyl ether, di-, tri- or tetra(ethylene glycol) diglycidyl ether, di-, tri- or tetra(1,2-propylene glycol) diglycidyl ether, di-, tri- or tetra(1,3-propylene glycol) diglycidyl ether, di-, tri- or tetra(1,4-butylene glycol) diglycidyl ether, a polyethylene glycol) diglycidyl ether, a polypropylene glycol) diglycidyl ether, a poly(trimethylene glycol) diglycidyl ether, a poly(tetramethylene glycol) diglycidyl ether, a polyethylene glycol-co-propylene glycol) diglycidyl ether, glycerol triglycidyl ether, a polyglycerol polyglycidyl ether, trimethylolmethane triglycidyl ether, trimethylolethane triglycidyl ether, trimethylolpropane triglycidyl ether, di(trimethylolpropane) tetraglycidyl ether, pentaerythritol tetraglycidyl ether, diglycidyl cyclohexanedicarboxylate, cyclohexane diglycidyl ether, cyclohexane-1,4-dimethanol diglycidyl ether, tricyclodecane dimethanol diglycidyl ether, isosorbide diglycidyl ether, pyrocatechol diglycidyl ether, resorcinol diglycidyl ether, cardol diglycidyl ether, phloroglucinol triglycidyl ether, pyrogallol triglycidyl ether, tris(hydroxyphenyl)methane triglycidyl ether, tris(hydroxyphenyl)ethane triglycidyl ether, diglycidyl phthalate, diglycidyl terephthalate, diglycidyl isophthalate, polyglycidyl ethers of a polyether polyol obtained by the addition of one or more alkylene oxides to an aliphatic polyhydric alcohol such as ethylene glycol, propylene glycol, and glycerol, diglycidyl esters of aliphatic long-chain (C6-C22) dibasic acids, monoglycidyl ethers of aliphatic higher alcohols, monoglycidyl ethers of phenol, cresol, butyl phenol, or polyether alcohols obtained by the addition of alkylene oxide to these compounds, glycidyl esters of higher fatty acids, an epoxidized vegetable oil (such as epoxidized soybean oil and epoxidized linseed oil), epoxybutylstearic acid, epoxyoctylstearic acid, epoxidized polybutadiene, triglycidyl isocyanurate and the like.

Non-limiting examples of polyester (meth)acrylates are the reaction products of a hydroxyl group-terminated polyester polyol with a (meth)acrylating agent (such as (meth)acrylic acid, (meth)acrylic anhydride, (meth)acryloyl chloride or combinations thereof). The reaction process may be conducted such that a significant concentration of residual hydroxyl groups remain in the polyester (meth)acrylate or may be conducted such that all or essentially all of the hydroxyl groups of the polyester polyol have been (meth)acrylated. The polyester polyols can be made by polycondensation reactions of a polyhydroxyl functional component (in particular a diol) and a polycarboxylic acid functional compound (in particular, a dicarboxylic acid or anhydride). To prepare the polyester (meth)acrylates, the hydroxyl groups of the polyester polyol are then partially or fully esterified by reacting with the (meth)acrylating agent. Polyester (meth)acrylates may also be synthesized by reacting a hydroxyl-containing (meth)acrylate such as a hydroxyalkyl (meth)acrylate (e.g., hydroxyethyl acrylate) with a polycarboxylic acid. The polyhydroxyl functional and polycarboxylic acid functional components can each have linear, branched, cycloaliphatic or aromatic structures and can be used individually or as mixtures.

Non-limiting examples of polyether (meth)acrylates are the condensation reaction products of a polyetherol which is a polyether polyol with a (meth)acrylating agent (such as (meth)acrylic acid, (meth)acrylic anhydride, (meth)acryloyl chloride or combinations thereof). Suitable polyetherols can be linear or branched substances containing ether bonds and terminal hydroxyl groups. Polyetherols can be prepared by ring opening polymerization of epoxides and other oxygen-containing heterocyclic compounds (e.g., ethylene oxide, 1,2-propylene oxide, butene oxide, tetrahydrofuran and combinations thereof) with a starter molecule. Suitable starter molecules include water, hydroxyl functional materials, polyester polyols and amines. Polyetherols may also be obtained by the condensation of diols such as glycols.

Non-limiting examples of urethane (meth)acrylates are the condensation reaction products of at least one polyisocyanate (e.g., diisocyanate, triisocyanate), at least one polyol (such as a polyether polyol or a polyester polyol) and a hydroxyl-functionalized (meth)acrylate (such as 2-hydroxyethyl (meth)acrylate or 3-hydroxypropyl (meth)acrylate) to provide terminal (meth)acrylate groups. For example, the urethane (meth)acrylate may contain two, three, four or more (meth)acrylate groups per molecule. The order of addition of the components to prepare the urethane (meth)acrylate is well known in the art. For example, the hydroxyl-functionalized (meth)acrylate may be first reacted with the polyisocyanate to obtain an isocyanate-functionalized (meth)acrylate, which is then reacted with the polyol. In yet another embodiment, the polyisocyanate may be first reacted with the polyol to obtain an isocyanate-functionalized polyol, which is thereafter reacted with a hydroxyl-functionalized (meth)acrylate. Alternatively, all the components may be combined and reacted at the same time.

Non-limiting examples of (meth)acrylated poly(meth)acrylates are substances having an oligomeric (meth)acrylic backbone which is functionalized with one or (meth)acrylate groups (which may be at a terminus of the oligomer or pendant to the acrylic backbone). The (meth)acrylic backbone may be a homopolymer, random copolymer or block copolymer comprised of repeating units of (meth)acrylic monomers. The (meth)acrylic monomers may be any monomeric (meth)acrylate such as C1-C6 alkyl (meth)acrylates as well as functionalized (meth)acrylates such as (meth)acrylates bearing hydroxyl, carboxylic acid and/or epoxy groups. (Meth)acrylated poly(meth)acrylates may be prepared using any procedures known in the art, such as by oligomerizing (meth)acrylic monomers, at least a portion of which are functionalized with hydroxyl, carboxylic acid and/or epoxy groups (e.g., hydroxyalkyl(meth)acrylates, (meth)acrylic acid, glycidyl (meth)acrylate) to obtain a functionalized poly(meth)acrylate, which is then reacted with one or more (meth)acrylate-containing reactants to introduce the desired (meth)acrylate functional groups.

Component B) may comprise 0 to 100%, in particular 10 to 95%, more particularly 20 to 90%, even more particularly 25 to 85%, more particularly still 30 to 80% by weight of (meth)acrylate-functionalized oligomer based on the total weight of the component B). In particular, component B) may comprise 0 to 50% or 0 to 45% or 0 to 40% or 0 to 35% or 0 to 30%, by weight of (meth)acrylate-functionalized oligomer based on the total weight of component B).

Component B) may comprise one or more ethylenically unsaturated compounds other than a (meth)acrylate-functionalized monomer or oligomer. Examples of such ethylenically unsaturated compounds include:
- polyvinylic and/or polyallylic monomers (in particular divinyl benzene, 1,4-butanediol divinyl ether, tri(ethylene glycol) divinyl ether, diallyl ether, glycerol diallyl ether, glycerol triallyl ether, trimethylolpropane diallyl ether, trimethylolpropane triallyl ether, pentaerythritol triallyl ether, pentaerythritol tetraallyl ether, diallyl phthalate, triallyl isocyanurate, 2,4,6-triallyloxy-1,3,5-triazine, glyoxal bis(diallyl acetal) and mixtures thereof);
- vinyl esters of carboxylic acids (in particular vinyl acetate, vinyl propionate, vinyl hexanoate, vinyl 2-ethylhexanoate, vinyl octanoate, vinyl pelargonate, vinyl laurate, vinyl stearate, a vinyl ester of versatic acid and mixtures thereof);
- vinyl ethers (in particular vinyl methyl ether, vinyl ethyl ether, vinyl n-butyl ether, vinyl isobutyl ether and mixtures thereof, ethylene glycol divinyl ether, triethylene glycol divinyl ether and trimethylolpropane trivinyl ether);
- cycloaliphatic vinyl monomers (in particular vinylcyclohexane);
- olefins (in particular ethylene, propene, 1-butene, isobutylene, diisobutylene, 1-nonene, 1-decene and mixtures thereof);
- conjugated dienes (in particular butadiene, isoprene, pentadiene, chlorodiene and mixtures thereof);
- vinyl aromatic monomers (in particular styrene, alpha-methylstyrene, tert-butylstyrene, ortho-, meta-, and para-methylstyrene, ortho-, meta- and para-ethylstyrene, o-methyl-p-isopropylstyrene, p-chlorostyrene, p-bromostyrene, o,p-dichlorostyrene, o,p-dibromostyrene, ortho-, meta- and para-methoxystyrene, optionally substituted indenes, optionally substituted vinylnaphthalenes, acenaphthylene, diphenylethylene, vinyl anthracene and mixtures thereof);
- mono- or dicarboxylic acid monomers, cyclic anhydride monomers and salts thereof (in particular 3-butenoic acid, crotonic acid, vinyl acetic acid, fumaric acid, maleic acid, maleic anhydride, tetrahydrophthalic acid, tetrahydrophthalic anhydride, itaconic acid, mesaconic acid, citraconic acid, glutaconic acid, muconic acid and mixtures thereof);
- unsaturated polymers such as polybutadiene;
- as well as the alkoxylated (e.g., ethoxylated and/or propoxylated) derivatives thereof
- and mixtures thereof.

### Other components

The curable composition of the invention may further comprise one or more additives and/or one or more solvents as detailed below.

### Additives

The curable composition of the present invention may further comprise an additive. The curable composition may comprise a mixture of additives.

In particular, the additive may be selected from an initiator or initiating system, sensitizers, amine synergists, stabilizers (such as antioxidants, light blockers/absorbers or polymerization inhibitors), foam inhibitors, flow or leveling agents, colorants, pigments, dispersants (wetting agents, surfactants), slip additives, fillers, thixotropic agents, matting agents, impact modifiers, waxes and mixtures thereof; and any other additive conventionally used in the coating, sealant, adhesive, molding, 3D printing or ink arts.

The curable composition may comprise an initiator or initiating system other than the self-crosslinkable urethane (meth)acrylate of the invention. The initiator or initiating system may be as defined above in the polymerization process of the invention.

Preferably the curable composition may be substantially free of or free of an initiator or initiating system other than the self-crosslinkable urethane (meth)acrylate of the invention.

If an initiator is to be added in the curable composition, it may be selected from a photoinitiator having Norrish type I activity and/or Norrish type II activity, more particularly a radical photoinitiator having Norrish type I activity. Such photoinitiators may be as defined above in the polymerization process of the invention.

In particular, the photoinitiator may be selected from a benzophenone such as SpeedCure^{®} BP (benzophenone), SpeedCure^{®} 7005 (polymeric benzophenone), SpeedCure^{®} 7006 (polymeric benzophenone), SpeedCure^{®} EMK (4,4'-bis(diethylamino)benzophenone) or SpeedCure^{®} BMS (4-benzoyl-4'-methyldiphenyl sulphide); a thioxanthone such as SpeedCure^{®} 7010 (polymeric thioxanthone), SpeedCure^{®} ITX (isopropyl thioxanthone), SpeedCure^{®} DETX (2,4-diethylthioxanthone) or SpeedCure^{®} CPTX (1-chloro-4-propoxythioxanthone); an α-hydroxy acetophenone such as SpeedCure^{®} 73 (2-hydroxy-2-methyl-1-phenylpropanone); an acylphosphine oxide such as SpeedCure^{®} BPO (phenyl bis(2,4,6-trimethylbenzoyl)-phosphine oxide), SpeedCure^{®} TPO (2,4,6-trimethylbenzoyldiphenylphosphine oxide) or SpeedCure^{®} TPO-L (ethyl (2,4,6-trimethylbenzoyl)phenyl phosphinate); a phenylglyoxylate such as SpeedCure^{®} MBF (methylbenzoylformate); and mixtures thereof.

The curable composition may comprise a stabilizer such as an antioxidant, a light blocker/absorber or a polymerization inhibitor. Such stabilizers are as defined above for the optional components used in the preparation process of the self-crosslinkable urethane (meth)acrylate.

The concentration of stabilizer in the curable composition will vary depending upon the particular stabilizer or combination of stabilizers selected for use and also on the degree of stabilization desired and the susceptibility of components in the curable compositions towards degradation in the absence of stabilizer. Typically, however, the curable composition is formulated to comprise from 5 to 5000 ppm stabilizer.

The curable composition may comprise an amine synergist. Alternatively, the composition may be substantially free of amine synergist. As used herein, the term "amine synergist" refers to an amine synergist other than the self-crosslinkable urethane (meth)acrylate of the invention. An amine synergist may be a compound comprising a hydrogen atom in alpha position to a heteroatom such as N or S. Amine synergists may be added to a curable composition to effect a faster cure rate if desired. Amine synergists may in particular act as hydrogen donors for Norrish type II chromophores. Non limiting examples of amine synergists include tertiary amines and polythiols such as ethylhexyl-dimethylaminobenzoate (SpeedCure^{®} EHA), triethanolamine, pentaerythritoltetrakis-3-mercaptopropionate or amino-acrylates obtained by reaction of a primary or secondary amine with an acrylate-functionalized monomer or oligomer (such as CN174 (Sartomer) and CN186 (Sartomer)).

The curable composition may be substantially free of a dye. For examples, the curable composition may comprise less than 1%, in particular less than 0.5% or even 0% by weight of a dye based on the weight of the curable composition.

The curable composition may comprise a colorant. As used herein the term "colorant" means a substance other than a dye that imparts color to the curable composition. The colorant may be a pigment. The term "pigment" is defined in DIN 55943, as a colorant that is practically insoluble in the application medium under the pertaining ambient conditions, hence having a solubility of less than 10 mg/L therein at 25°C. The term "C.I." is used as an abbreviation for Colour Index.

In particular, the composition may comprise a pigment selected from organic and/or inorganic pigments. If the pigment is not a self-dispersible pigment, the curable composition preferably also contains a dispersant, more preferably a polymeric dispersant. The pigment may be black, cyan, magenta, yellow, red, orange, violet, blue, green, brown and mixtures thereof. Pigments may be chosen from those disclosed by HERBST, Willy, et al. Industrial Organic Pigments, Production, Properties, Applications. 3rd edition. Wiley - VCH, 2004. ISBN 3527305769.

The curable composition of the invention may comprise a dispersant. The dispersant may be used to disperse an insoluble material such as a pigment or filler in the curable composition.

### Solvents

The curable composition of the invention may comprise a solvent. The solvent may be as defined above for the preparation process of the self-crosslinkable urethane (meth)acrylate.

Advantageously, the curable composition of the present invention may be formulated to be substantially free of a solvent. For example, the curable composition may contain little or no solvent, e.g., less than 10 %, or less than 5 %, or less than 1 %, or even 0 % by weight of solvent, based on the total weight of the curable composition.

According to some embodiments, the curable composition is a liquid at 25°C. In various embodiments of the invention, the curable compositions described herein are formulated to have a viscosity of less than 10,000 mPa.s, or less than 5,000 mPa.s, or less than 1,000 mPa.s, or less than 500 mPa.s, or less than 250 mPa.s, or even less than 100 mPa.s as measured at 25°C using a Brookfield viscometer, model DV-II, using a 27 spindle (with the spindle speed varying typically between 20 and 200 rpm, depending on viscosity). In advantageous embodiments of the invention, the viscosity of the curable composition is from 10 to 10,000 mPa.s, or from 10 to 5,000 mPa.s, or from 10 to 1,000 mPa.s, or from 10 to 500 mPa.s, or from 10 to 250 mPa.s, or from 10 to 100 mPa.s at 25°C.

### Formulations

The curable compositions described herein may be compositions that are to be subjected to curing by means of free radical polymerization. In particular embodiments, the curable compositions may be cured by exposing the curable composition to actinic radiation, in particular UV, near-UV, visible, infrared, near-infrared and/or electron beam radiation.

The curable composition of the invention may be a coating composition, an ink composition, a varnish composition, an encapsulation or potting composition, a 3D-printing composition, a molding composition, a sealant composition, an adhesive composition, a nail polish composition or a dental composition.

End use applications for the curable compositions include, but are not limited to, inks, coatings, adhesives, additive manufacturing resins (such as 3D printing resins), molding resins, sealants, composites, antistatic layers, electronic applications, recyclable materials, smart materials capable of detecting and responding to stimuli, packaging materials, personal care articles, nail polishes, articles for use in agriculture, water or food processing, or animal husbandry, and biomedical materials. The curable compositions of the invention thus find utility in the production of biocompatible articles. Such articles may, for example, exhibit high biocompatibility, low cytotoxicity and/or low extractables.

The composition according to the invention may in particular be used to obtain a cured product according to the following processes.

### Process for the preparation of a cured product

The process for the preparation of a cured product according to the invention comprises curing the curable composition of the invention. In particular, the curable composition may be cured by exposing the composition to actinic radiation. More particularly, the curable composition may be cured by exposing the composition to UV, near-UV, visible, infrared infrared, near-infrared and/or electron beam radiation. The curable composition may advantageously be cured by exposing the composition to a LED light source.

Curing may be accelerated or facilitated by supplying energy to the curable composition, such as by heating the curable composition. Thus, the cured product may be deemed as the reaction product of the curable composition, formed by curing. A curable composition may be partially cured by exposure to actinic radiation, with further curing being achieved by heating the partially cured article. For example, a product formed from the curable composition may be heated at a temperature of from 40°C to 120°C for a period of time from 5 minutes to 12 hours.

Prior to curing, the curable composition may be applied to a substrate surface in any known conventional manner, for example, by spraying, jetting, knife coating, roller coating, casting, drum coating, dipping, and the like and combinations thereof. Indirect application using a transfer process may also be used.

The substrate on which the curable composition is applied and cured may be any kind of substrate. Suitable substrates are detailed below. When used as an adhesive, the curable composition may be placed between two substrates and then cured, the cured composition thereby bonding the substrates together to provide an adhered article. Curable compositions in accordance with the present invention may also be formed or cured in a bulk manner (e.g., the curable composition may be cast into a suitable mold and then cured).

The substrate may be a ceramic, metallic, mineral, cellulosic, animal-based or polymeric substrate. The substrate may also be a part of a human body, such as a tooth or a nail.

The substrate may be porous or substantially non-porous. The substrates may be transparent, translucent or opaque.

Examples of ceramic substrates include alumina-based ceramics and zirconia-based ceramics. Examples of metallic substrates include titanium, gold, silver, copper, brass, steel and bronze. Examples of mineral substates include glass, asbestos and basalt. Examples of cellulosic substrates include plain paper or resin coated paper (e.g. polyethylene or polypropylene coated paper). There is no real limitation on the type of paper and it includes newsprint paper, magazine paper, office paper, wallpaper but also paper of higher grammage, usually referred to as boards, such as white lined chipboard, corrugated board and packaging board. Further examples of cellulosic substrates include bamboo, cotton, flax, hemp, jute, lyocell, modal, rayon, raffia, ramie and sisal. Examples of cellulosic substrates include wool, fur, silk and leather. Examples of polymeric substrates include polyethylene, polypropylene, polycarbonate, polyvinyl chloride, polyethylene terephthalate, polyethylene naphthalate, polylactide, polyamide, polyimide, polyacrylonitrile, polyurethane, acrylonitrile butadiene styrene.

There is no restriction on the shape of the substrate. It can be a sheet, a film, a non-woven or woven fiber mat or a three dimensional object.

In particular, the substrate may be selected from a food and beverage packaging, a pharmaceutical packaging, a textile, a nail, a tooth, a medical device, a food and beverage processing equipment, a water pipe or a toy.

The cured product obtained with the process of the invention may be a coating (in particular a scratch-resistant wood coating, a concrete coating or a plastic coating), an ink, a varnish, an encapsulation or potting material, a 3D-printed article, a molded article, a sealant, an adhesive, a nail polish or a dental material.

### Process of 3D printing

A 3D-printed article may, in particular, be obtained with a 3D printing process that comprises printing a 3D article with the composition of the invention. In particular, the process may comprise printing a 3D article layer by layer or continuously.

Three-dimensional (3D) printing (also referred to as additive manufacturing) is a process in which a 3D digital model is manufactured by the accretion of construction material. The 3D printed object is created by utilizing the computer-aided design (CAD) data of an object through sequential construction of two dimensional (2D) layers or slices that correspond to cross-sections of 3D objects. The radiation can be in the form of electromagnetic waves or an electron beam. The most commonly applied energy source is UV, near-UV, visible, infrared and/or near-infrared radiation.

A plurality of layers of a curable composition in accordance with the present invention may be applied to a substrate surface; the plurality of layers may be simultaneously cured (by exposure to a single dose of radiation, for example) or each layer may be successively cured before application of an additional layer of the curable composition.

Non-limiting examples of suitable 3D printing processes include stereolithography (SLA); digital light process (DLP); liquid crystal device (LCD); inkjet head (or multjet) printing; Continuous Liquid Interface Production (CLIP); extrusion type processes such as continuous fiber 3D printing and cast-in-motion 3D printing; and volumetric 3D printing. The building method may be "layer by layer" or continuous. The liquid may be in a vat, or deposited with an inkjet or gel deposition, for example.

Stereolithography and other photocurable 3D printing methods typically apply low intensity light sources to radiate each layer of a photocurable resin to form the desired article. As a result, photocurable resin polymerization kinetics and the green strength of the printed article are important criteria if a particular photocurable resin will sufficiently polymerize (cure) when irradiated and have sufficient green strength to retain its integrity through the 3D printing process and post-processing.

The curable compositions of the invention may be used as 3D printing resin formulations, that is, compositions intended for use in manufacturing three-dimensional articles using 3D printing techniques. Such three-dimensional articles may be free-standing/self-supporting and may consist essentially of or consist of a composition in accordance with the present invention that has been cured. The three-dimensional article may also be a composite, comprising at least one component consisting essentially of or consisting of a cured composition as previously mentioned as well as at least one additional component comprised of one or more materials other than such a cured composition (for example, a metal component or a thermoplastic component or inorganic filler or fibrous reinforcement). The curable compositions of the present invention are particularly useful in digital light printing (DLP), although other types of three-dimensional (3D) printing methods may also be practiced using the inventive curable compositions (e.g., SLA, inkjet, multi-jet printing, piezoelectric printing, actinically-cured extrusion, and gel deposition printing). The curable compositions of the present invention may be used in a three-dimensional printing operation together with another material which functions as a scaffold or support for the article formed from the curable composition of the present invention.

Thus, the curable compositions of the present invention are useful in the practice of various types of three-dimensional fabrication or printing techniques, including methods in which construction of a three-dimensional object is performed in a step-wise or layer-by-layer manner. In such methods, layer formation may be performed by solidification (curing) of the curable composition under the action of exposure to radiation, such as visible, UV or other actinic irradiation. For example, new layers may be formed at the top surface of the growing object or at the bottom surface of the growing object. The curable compositions of the present invention may also be advantageously employed in methods for the production of three-dimensional objects by additive manufacturing wherein the method is carried out continuously. For example, the object may be produced from a liquid interface. Suitable methods of this type are sometimes referred to in the art as "*continuous liquid interface (or interphase) product (or printing)*" ("CLIP") methods. Such methods are described, for example, in WO 2014/126830; WO 2014/126834; WO 2014/126837; and Tumbleston et al., "Continuous Liquid Interface Production of 3D Objects", Science Vol. 347, Issue 6228, pp. 1349-1352 (March 20, 2015.

The curable composition may be supplied by ejecting it from a printhead rather than supplying it from a vat. This type of process is commonly referred to as inkjet or multijet 3D printing. One or more UV curing sources mounted just behind the inkjet printhead cures the curable composition immediately after it is applied to the build surface substrate or to previously applied layers. Two or more printheads can be used in the process which allows application of different compositions to different areas of each layer. For example, compositions of different colors or different physical properties can be simultaneously applied to create 3D printed parts of varying composition. In a common usage, support materials - which are later removed during post-processing - are deposited at the same time as the compositions used to create the desired 3D printed part. The printheads can operate at temperatures from about 25°C up to about 100°C. Viscosities of the curable compositions are less than 30 mPa.s at the operating temperature of the printhead.

The process for the preparation of a 3D-printed article may comprise the steps of:
a) providing (e.g., coating) a first layer of a curable composition in accordance with the present invention onto a surface;
b) curing the first layer, at least partially, to provide a cured first layer;
c) providing (e.g., coating) a second layer of the curable composition onto the cured first layer;
d) curing the second layer, at least partially, to provide a cured second layer adhered to the cured first layer; and
e) repeating steps c) and d) a desired number of times to build up the three-dimensional article.

Alternatively, the process for the preparation of a 3D-printed article may comprise the steps of:
a) providing a carrier and an optically transparent member having a build surface, the carrier and build surface defining a build region therebetween;
b) filling the build region with a composition as defined above;
c) continuously or intermittently curing part of the composition in the build region according to the method as defined above to form a cured composition; and
d) continuously or intermittently advancing the carrier away from the build surface to form the 3D-printed article from the cured composition.

After the 3D article has been printed, it may be subjected to one or more post-processing steps. The post-processing steps can be selected from one or more of the following steps removal of any printed support structures, washing with water and/or organic solvents to remove residual resins, and post-curing using thermal treatment and/or actinic radiation either simultaneously or sequentially. The post-processing steps may be used to transform the freshly printed article into a finished, functional article ready to be used in its intended application.

### Process of inkjet printing

The process of inkjet printing according to the invention comprises jetting the curable composition of the invention onto a substrate.

The substrate on which the curable composition is jetted may be any kind of substrate. Suitable substrates are as detailed above.

The curable composition may be jetted by one or more print heads ejecting small droplets in a controlled manner through nozzles onto a substrate moving relative to the print head(s).

The print head may be a piezoelectric head or a continuous type print head.

The inkjet printing process may be carried out in a single pass or with a multi-pass printing mode.

The inkjet printing process may further comprise a UV-curing step. In inkjet printing, the UV curing device may be arranged in combination with the print head of the inkjet printer, travelling therewith so that the liquid UV curable inkjet ink is exposed to curing radiation very shortly after been jetted.

In a particularly preferred embodiment, the UV curing step is performed using UV LED light sources.

For facilitating curing, the inkjet printer may include one or more oxygen depletion units. The oxygen depletion units place a blanket of nitrogen or other relatively inert gas (e.g. CO₂), with adjustable position and adjustable inert gas concentration, in order to reduce the oxygen concentration in the curing environment.

### Process of coating a nail

The process of coating a nail according to the invention comprises applying the curable composition of the invention on a nail, and curing the composition on the nail.

### Uses

The self-crosslinkable urethane (meth)acrylate of the invention may be used to obtain a cured product having a reduced amount of extractables. In particular, the cured product may be a coating (in particular a scratch-resistant wood coating, a concrete coating or a plastic coating), an ink, a varnish, an encapsulation or potting material, a 3D-printed article, a molded article, a sealant, an adhesive, a nail polish or a dental material.

The reduction in the amount of extractables may be assessed in comparison with a cured product obtained with a conventional photoinitiator.

The extractables may be any component that migrates from the cured product. In particular, the extractables may be a photoinitiator or a residue thereof.

Migration in inkjet inks may occur in different ways:
- Penetration Migration - through the substrate to the reverse-side of the print;
- Set-off Migration - from the printed side of a substrate to the reverse side of the substrate while stacked or stored on a roll;
- Vapor-phase migration - evaporation of volatile compounds when heated;
- Condensation Extraction - condensation of critical compounds when cooked or sterilized.

The amount of extractables may be determined quantitatively using a suitable analytical method such as liquid chromatography mass spectroscopy (LC-MS). For example, the curable composition can be applied in 12 µm thickness film on a glass substrate, and crosslinked using a UV Hg lamp. Resulting cured films are removed from the glass plate, weighed and soaked in solvent such as acetonitrile or dichloromethane. The liquid fraction is finally evaporated and the residue, corresponding to the extractables part, is weighed, allowing to determine the amount of product that is uncured (not trapped within the photocured network).

Analytical methods such as nuclear magnetic resonance (NMR), liquid chromatography mass spectroscopy (LC-MS) or gas chromatography mass spectroscopy (GC-MS) may then be used to identify the nature of the extractables and refine their corresponding content.

In particular, the cured product may have less than 5%, less than 2%, less than 1%, less than 0.5%, less than 0.25% or less than 0.1%, by weight of extractables based on the weight of the cured product.

The curable composition of the invention may be used to obtain a coating, an ink, a varnish, an encapsulation or potting material, a 3D-printed article, a molded article, a sealant, an adhesive, a nail polish or a dental material.

Within this specification, embodiments have been described in a way which enables a clear and concise specification to be written, but it is intended and will be appreciated that embodiments may be variously combined or separated without departing from the invention. For example, it will be appreciated that all preferred features described herein are applicable to all aspects of the invention described herein.

Although the invention is illustrated and described herein with reference to specific embodiments, the invention is not intended to be limited to the details shown. Rather, various modifications may be made in the details within the scope and range of equivalents of the claims and without departing from the invention.

### ASPECTS

The invention may be as defined in any one of the following Aspects:
Aspect 1. A self-crosslinkable urethane (meth)acrylate comprising:
   at least one (meth)acrylate-functionalized moiety ACR;
   at least two polyurethane moieties UU;
   at least one chromophore moiety Q;
   optionally at least one chain-extending moiety EXT.
Aspect 2. The self-crosslinkable urethane (meth)acrylate according to Aspect 1, wherein each (meth)acrylate-functionalized moiety ACR independently bears 1 to 5 (meth)acrylate groups, in particular 1 to 3 (meth)acrylate groups, more particularly 1 (meth)acrylate group.
Aspect 3. The self-crosslinkable urethane (meth)acrylate according to Aspect 1 or 2, wherein each (meth)acrylate-functionalized moiety ACR is independently according to the following formula (I): 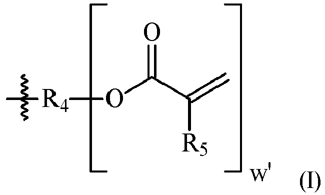 wherein
   R₄ is a (w'+1)valent linker;
   R₅ is H or methyl;
   w' is 1 to 5, in particular 1 to 3, more particularly 1.
Aspect 4. The self-crosslinkable urethane (meth)acrylate according to Aspect 3, wherein R⁴ is a (w'+1)valent linker selected from an aliphatic or aromatic hydrocarbon linker, a polyether linker, a polyester linker, a polycarbonate linker, a polyorganosiloxane linker, a polydiene linker, an isocyanurate linker, and combinations thereof; in particular an aliphatic or aromatic hydrocarbon linker, a polyether linker, a polyester linker and combinations thereof; more particularly an alkylene, an alkoxylated alkylene, a polycaprolactone linker and combinations thereof.
Aspect 5. The self-crosslinkable urethane (meth)acrylate of Aspect 3 or 4, wherein R⁴ is a divalent linker selected from one of formula (IIa) to (VIa):

   -(CR₂₂R'₂₂)ₘ- (IIa)

   -[(CR₂₃R'₂₃)ₙ-O]ₒ-(CR₂₃R'₂₃)ₙ- (IIIa)

   -[(CR₂₄R'₂₄)ₚ-O]_{q}-(CR_{25R}'₂₅)ᵣ-[O-(CR_{26R}'₂₆)_{p'}]_{q'}- (IVa)

   -[(CR₂₇R'₂₇)ₛ-C(=O)O]ₜ-(CR₂₈R'₂₈)ᵤ-^{∗} (Va)

   -[(CR₂₉R'₂₉)ᵥ-O-C(=O)-(CR₃R'₃₀),-C(=O)-O]ₓ-(CR₂₉R'₂₉)ᵥ- (VIa)

   wherein
   R₂₂, R'₂₂, R₂₅, R'₂₅, R₂₉, R'₂₉, R₃₀ and R'₃₀ are independently H or alkyl;
   R₂₃, R'₂₃, R₂₄, R'₂₄, R₂₆, R'₂₆, R₂₇, R'₂₇, R₂₈ and R'₂₈ are independently H or methyl;
   m is 2 to 50;
   n, p and p' are independently 2 to 4;
   o is 1 to 20;
   q and q' are independently 0 to 20 with the proviso that at least one of q and q' is not 0 ;
   r is 2 to 20;
   s is 3 to 12;
   t is 1 to 20;
   u is 2 to 8;
   v is 2 to 20;
   w is 2 to 30;
   x is 1 to 20;
   the symbol * represents the point of attachment to the (meth)acrylate group;
   in particular, R₄ is a divalent linker selected from an alkylene such as 1,2-ethylene, 1,2- or 1,3-propylene, 1,2-, 1,3- or 1,4-butylene, 1,5-pentylene, 1,6-hexylene, 1,8-octylene, 1,9-nonylene, 1,10-decylene, 1,12-dodecylene, 1,18-octadecylene, 2-methyl-1,3-propanediyl, 2,2-diethyl-1,3-propanediyl, 3-methyl-1,5-pentanediyl, 3,3-dimethyl-1,5-pentanediyl, 2,2-dimethyl-1,3-propanediyl, 2,4-diethyl-1,5-pentanediyl; an alkoxylated derivative of the aforementioned alkylenes; an esterified derivative of the aforementioned alkylenes; a residue of a di-, tri-, tetra- or polyoxyalkylene (without the OH groups) such as a residue of di-, tri- or tetraethylene glycol, di-, tri- or tetrapropylene glycol, di-, tri- or tetrabutylene glycol, polyethylene glycol, polypropylene glycol, polybutylene glycol, poly(ethylene glycol-co-propylene glycol); and a residue of a polyester polyol (without the OH groups).
Aspect 6. The self-crosslinkable urethane (meth)acrylate according to any one of Aspects 1 to 5, wherein the total amount of (meth)acrylate-functionalized moieties ACR in the self-crosslinkable urethane (meth)acrylate represents from 5 to 85%, in particular from 10 to 80%, more particularly from 15 to 75%, of the total weight of the self-crosslinkable urethane (meth)acrylate.
Aspect 7. The self-crosslinkable urethane (meth)acrylate according to any one of Aspects 1 to 6, wherein each polyurethane moiety UU independently bears 2 to 3 urethane bonds.
Aspect 8. The self-crosslinkable urethane (meth)acrylate according to any one of Aspects 1 to 7, wherein each polyurethane moiety UU is independently according to one of the following formula (VIIa) or (VIIb): 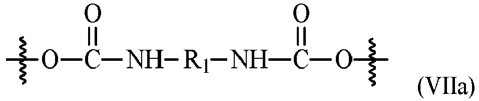 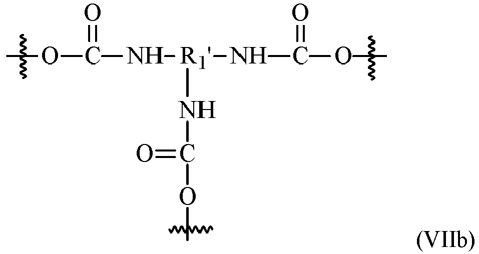 wherein
   R₁ and R₁' are independently an aliphatic linker or an aromatic linker.
Aspect 9. The self-crosslinkable urethane (meth)acrylate according to Aspect 8, wherein R₁ is selected from one of the following formulae: 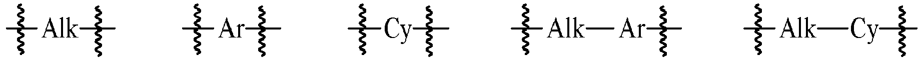 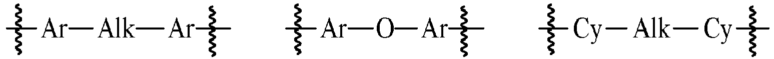 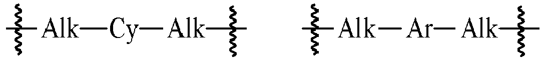 wherein :
   Alk is a linear or branched alkylene, in particular methylene, 1,2-ethylene, 1,2- or 1,3-propylene, 1,2-, 1,3- or 1,4-butylene, 1,5-pentylene, 1,6-hexylene, 2,2,4- or 2,4-,4-trimethylhexylene, 1,8-octylene, 1,9-nonylene, 1,10-decylene, 1,12-dodecylene, 1,18-octadecylene;
   Ar is an optionally substituted arylene, in particular an optionally substituted arylene selected from phenylene, tolylene, biphenylene, naphthylene, anthracenylene;
   Cy is an optionally substituted cycloalkylene, in particular an optionally substituted cyclohexylene.
Aspect 10. The self-crosslinkable urethane (meth)acrylate according to Aspect 8 or 9, wherein R₁' may be selected from one of the following formulae: 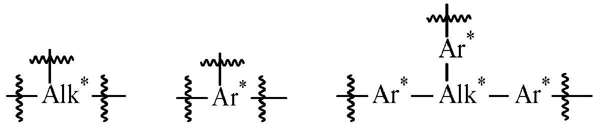 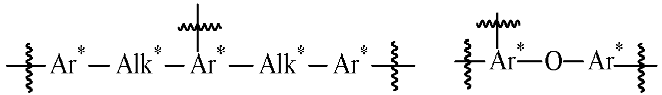 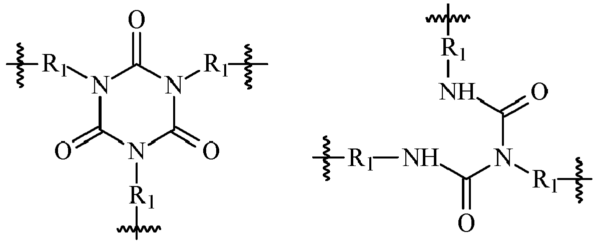 wherein
   Alk* is a linear or branched alkylene, in particular methylene, methanetriyl, undecane-1,6,1 1-triyl;
   Ar* is an optionally substituted arylene, in particular an optionally substituted arylene selected from phenylene, tolylene and biphenylene;
   R₁ is as defined above for formula (VIIa), in particular 1,6-hexamethylene.
Aspect 11. The self-crosslinkable urethane (meth)acrylate according to any one of Aspects 1 to 10, wherein each polyurethane moiety UU is independently selected from:
   - a moiety of formula (VIIa) wherein R₁ is selected from one of the following formulae: 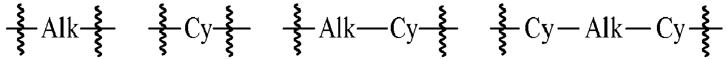 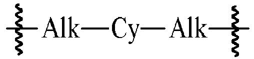 wherein
      Alk is a linear or branched alkylene, in particular methylene, 1,2-ethylene, 1,2- or 1,3-propylene, 1,2-, 1,3- or 1,4-butylene, 1,5-pentylene, 1,6-hexylene, 2,2,4- or 2,4,4-trimethylhexylene, 1,8-octylene, 1,9-nonylene, 1,10-decylene, 1,12-dodecylene, 1,18-octadecylene;
      Cy is an optionally substituted cycloalkylene, in particular an optionally substituted cyclohexylene;
   - a moiety of (VIIb) wherein R'₁ corresponds to the following formula: 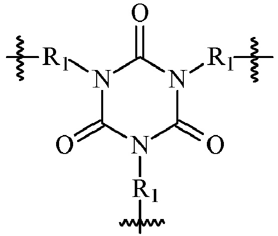 and R₁ is a linear or branched alkylene, in particular 1,6-hexylene;
   and mixtures thereof.
Aspect 12. The self-crosslinkable urethane (meth)acrylate according to any one of Aspects 1 to 11, wherein the total amount of polyurethane moieties UU in the self-crosslinkable urethane (meth)acrylate represents from 10 to 65%, in particular from 15 to 60%, more particularly from 20 to 55%, of the total weight of the self-crosslinkable urethane (meth)acrylate.
Aspect 13. The self-crosslinkable urethane (meth)acrylate according to any one of Aspects 1 to 12, wherein each chromophore moiety Q independently comprises at least one monovalent or divalent photoinitiator moiety PI selected from a benzophenone moiety, a thioxanthone moiety, a xanthone moiety, an acridone moiety, a camphorquinone moiety, a benzil moiety, a coumarin moiety, a ketocoumarin moiety, derivatives thereof and combinations thereof, preferably at least one monovalent or divalent photoinitiator moiety PI selected from a benzophenone moiety or a thioxanthone moiety.
Aspect 14. The self-crosslinkable urethane (meth)acrylate according to any one of Aspects 1 to 13, wherein each chromophore moiety Q independently comprises at least one monovalent photoinitiator moiety PI corresponding to one of the following formulae (VIII), (XI), (XIV), (XVII), (XVIII) or (XIX), or at least one divalent photoinitiator moiety PI corresponding to one of the following formulae (IX), (X), (XII), (XIII), (XV) or (XVI): 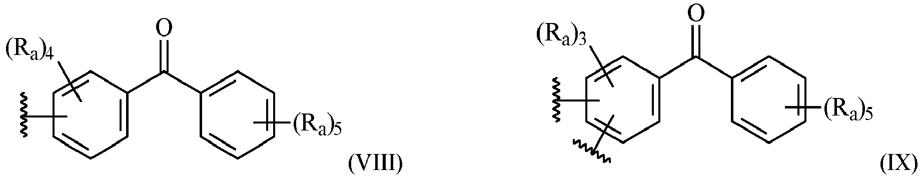 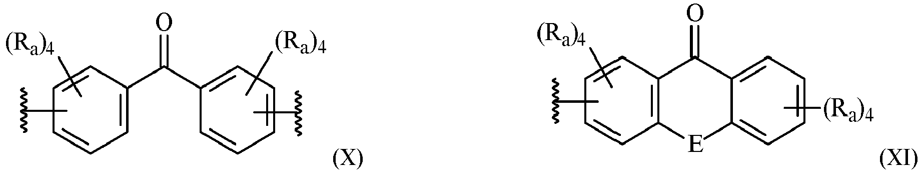 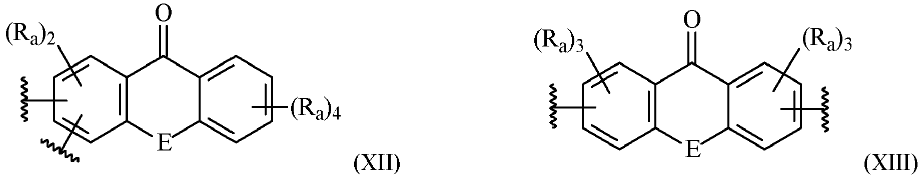 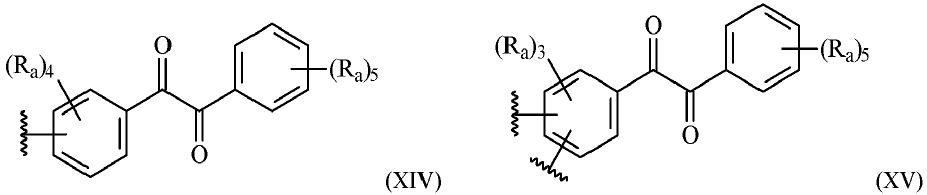 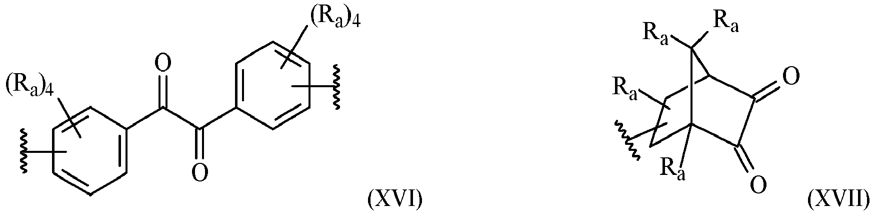 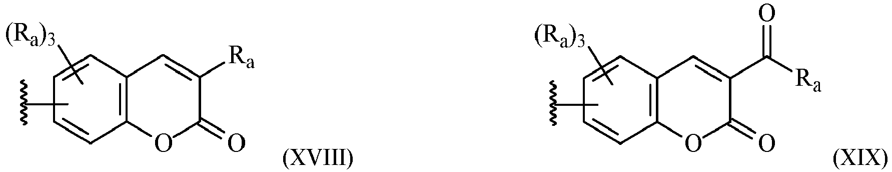 wherein
   each E is independently S, O or NR, in particular S;
   R is H, an optionally substituted alkyl or an optionally substituted aryl;
   each Rₐ is independently H, F, Cl, Br, I, -OR_{b}, -SR_{b}, -N(R_{b})₂, -NO₂, -CN, -C(=O)R_{b}, -O-C(=O)R_{b}, -C(=O)OR_{b}, -C(=O)N(R_{b})₂, -NR_{b}-C(=O)-R_{b}, -SO₂-N(R_{b})₂, or an optionally substituted group selected from the group consisting of alkyl, heteroatom-containing alkyl, cycloalkyl, heterocycloalkyl, alkenyl, alkynyl, aryl, aralkyl, alkaryl and heteroaryl; or two adjacent Rₐ groups may form, with the carbon atoms to which they are attached, a 5 to 8 membered ring; each R_{b} is independently H or an optionally substituted group selected from alkyl, heteroatom-containing alkyl, cycloalkyl, heterocycloalkyl, alkenyl, alkynyl, aryl, aralkyl, alkaryl and heteroaryl.
Aspect 15. The self-crosslinkable urethane (meth)acrylate according to Aspect 14, wherein each chromophore moiety Q independently comprises at least one monovalent photoinitiator moiety PI corresponding to one of formulae (VIII) or (XI) wherein E is S, or at least one divalent photoinitiator moiety PI corresponding to one of formulae (IX), (X), (XII) or (XIII) wherein E is S; preferably each chromophore moiety Q independently comprises at least one monovalent photoinitiator moiety PI corresponding to one of the following formulae (Villa) or (XIa), or at least one divalent photoinitiator moiety PI corresponding to one of the following formulae (IXa), (Xa), (XIIa) or (XIIIa): 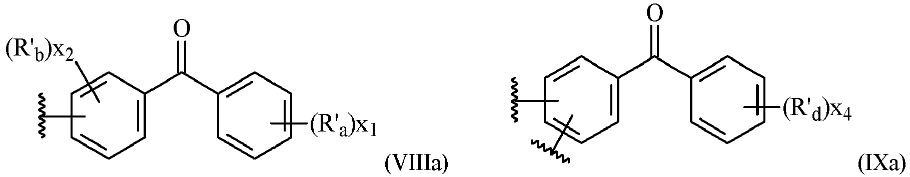 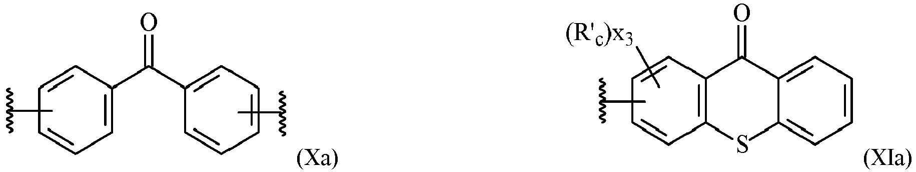 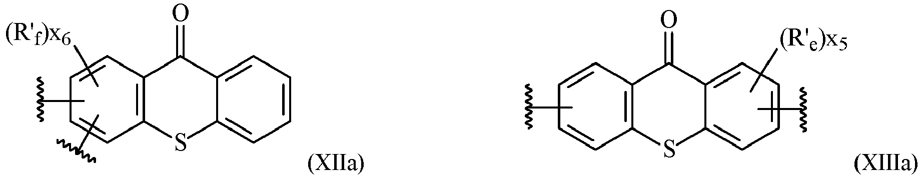 wherein
   R'ₐ and R'_{d} are independently alkyl, Ar, -S-Ar or -O-Ar wherein Ar is aryl, in particular methyl, Ph, -S-Ph or -O-Ph wherein Ph is phenyl;
   R'_{b} is alkyl, in particular methyl;
   R'_{c}, R'ₑ and R'_{f} are independently is halogen, alkoxy, alkyl or -C(=O)-Ar wherein Ar is aryl, in particular F, Cl, methyl, ethyl, isopropyl or -C(=O)-Ph wherein Ph is phenyl;
   x₁ and x₄ are independently 0, 1, 2 or 3, in particular 0 or 1;
   x₂, x₃ and x₅ are independently 0, 1 or 2, in particular 0 or 1;
   x₆ is 0 or 1.
Aspect 16. The self-crosslinkable urethane (meth)acrylate according to any one of Aspects 1 to 15, wherein each chromophore moiety Q is independently according to one of the following formulae (XXa), (XXb) or (XXc): 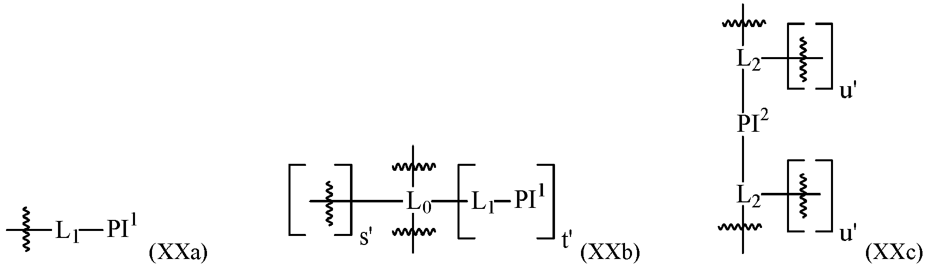 wherein
   L₀ is a (s'+t'+2)valent linker;
   each L₁ is independently a divalent linker;
   each L₂ is independently a (u'+2)valent linker;
   each PI¹ is independently a monovalent photoinitiator moiety PI as defined in any one of Aspects 13 to 15;
   each PI² is independently a divalent photoinitiator moiety PI as defined in any one of Aspects 13 to 15;
   s' is 0, 1 or 2; in particular 0 or 1;
   t' is 1, 2, 3 or 4; in particular 2 or 3;
   each u' is independently 0 or 1, in particular 0.
Aspect 17. The self-crosslinkable urethane (meth)acrylate according to Aspect 16, wherein each L₁ and L₂ is independently selected from a direct bond or a linker comprising 1 to 20, preferably 1 to 10, carbon atoms, said linker optionally comprising one or more heteroatoms, such as O, N or S; in particular each L₁ and L₂ is independently selected from a direct bond or a hydrocarbon linker optionally comprising one or more bonds selected from -O-, -S-, -NR₁-, -C(=O)-, -O-C(=O)-, -C(=O)-O-, -NR₁-C(=O)-, -C(=O)-NR₁-, -O-C(=O)-O-, and combinations thereof, wherein R₁ is H, alkyl or aryl.
Aspect 18. The self-crosslinkable urethane (meth)acrylate according to Aspect 16 or 17, wherein L₀ is a linker comprising 1 to 20, preferably 1 to 10, carbon atoms, said linker optionally comprising one or more heteroatoms, such as O, N or S; in particular, L₀ is a hydrocarbon linker optionally comprising one or more bonds selected from -O-, -S-, -NRi-, -C(=O)-, -O-C(=O)-, -C(=O)-O-, -NR₁-C(=O)-, -C(=O)-NRi-, -O-C(=O)-O-, and combinations thereof, wherein R₁ is H, alkyl or aryl.
Aspect 19. The self-crosslinkable urethane (meth)acrylate according to any one of Aspects 1 to 18, wherein the self-crosslinkable urethane (meth)acrylate comprises at least one chromophore moiety Q according to formula (XXa) as defined in Aspect 16, wherein
   L₁ is preferably selected from direct bond, alkylene of formula (C-1), oxyalkylene of formula (C-2), thioalkylene of formula (C-3), ketoalkylene of formula (C-4), aminoalkylene of formula (C-5), carboxyalkylene of formula (C-6), amidoalkylene of formula (C-7), alkylene-aminoalkylene of formula (C-8), oxyalkylene-aminoalkylene of formula (C-9), oxyalkylene-amidoalkylene of formula (C-10): 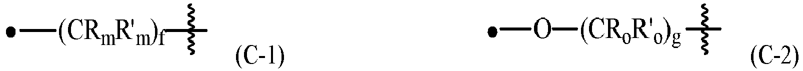 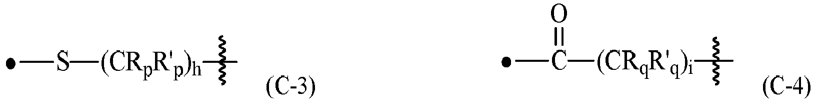 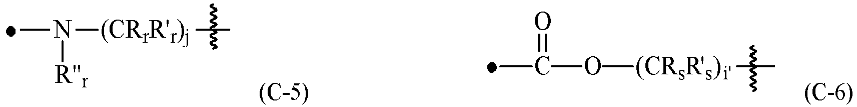 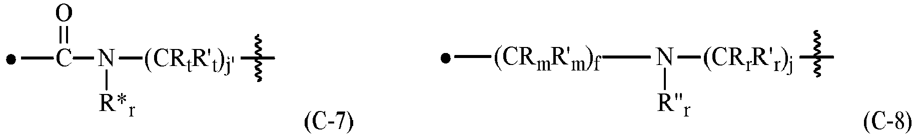 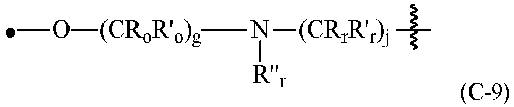 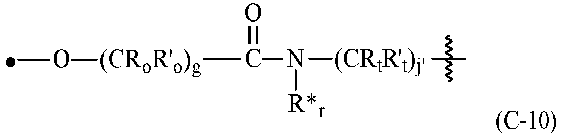
   wherein
   each Rₘ, R'ₘ, Rₒ, R'ₒ, Rₚ, R'ₚ, R_{q}, R'_{q}, Rᵣ, R'ᵣ, Rₛ, R'ₛ, Rₜ and R'ₜ is independently H or an optionally substituted alkyl, in particular H;
   R"r and R^{∗}ᵣ are independently H or an optionally substituted alkyl;
   f, g, h, j and j' are independently 1, 2, 3, 4, 5 or 6, in particular 1 or 2;
   i and i' are independently 1, 2, 3, 4 or 5, in particular 1;
   the symbol ● represents a point of attachment to the PI moiety;
   PI¹ is preferably according to formula (VIII) or (XI) as defined in Aspect 14 wherein E in formula (XI) is S; more preferably PI¹ is according to formula (VIIIa) or (XIa) as defined in Aspect 15.
Aspect 20. The self-crosslinkable urethane (meth)acrylate according to any one of Aspects 1 to 19, wherein the self-crosslinkable urethane (meth)acrylate comprises at least one chromophore moiety Q corresponding to the following formulae (XXa1) or (XXa2) 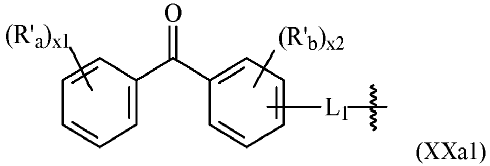 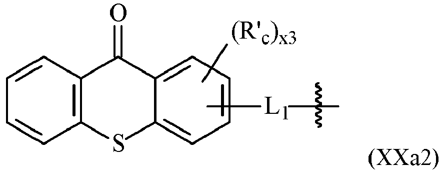 wherein
   R'ₐ is alkyl, Ar, -S-Ar or -O-Ar wherein Ar is aryl, in particular methyl, Ph, -S-Ph or -O-Ph wherein Ph is phenyl;
   R'_{b} is alkyl, in particular methyl;
   R'_{c} is halogen, alkoxy, alkyl or -C(=O)-Ar wherein Ar is aryl, in particular F, Cl, methyl, ethyl, isopropyl or -C(=O)-Ph wherein Ph is phenyl;
   x1 is 0, 1, 2 or 3, in particular 0 or 1;
   x2 is 0, 1 or 2, in particular 0 or 1;
   x3 is 0, 1, or 2, in particular 0 or 1;
   L₁ is selected from direct bond, alkylene of formula (C-1), oxyalkylene of formula (C-2), aminoalkylene of formula (C-5), amidoalkylene of formula (C-7), alkylene-aminoalkylene of formula (C-8), oxyalkylene-aminoalkylene of formula (C-9) or oxyalkylene-amidoalkylene of formula (C-10).
Aspect 21. The self-crosslinkable urethane (meth)acrylate according to any one of Aspects 1 to 20, wherein the self-crosslinkable urethane (meth)acrylate comprises at least one chromophore moiety Q corresponding to one of the following formulae (XXa3)-(XXa17): 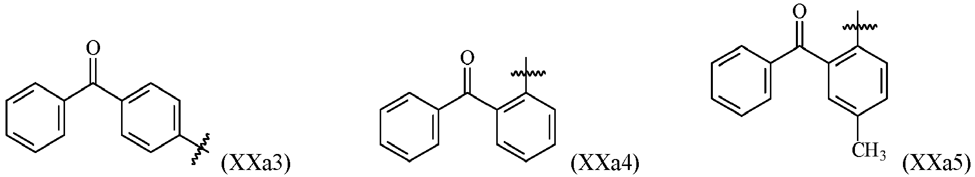 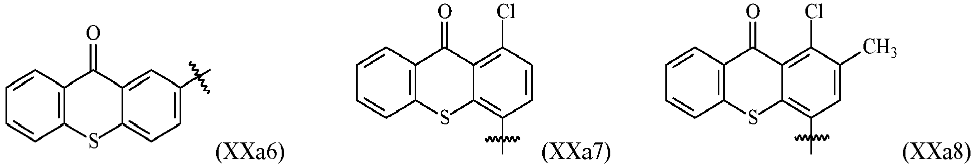 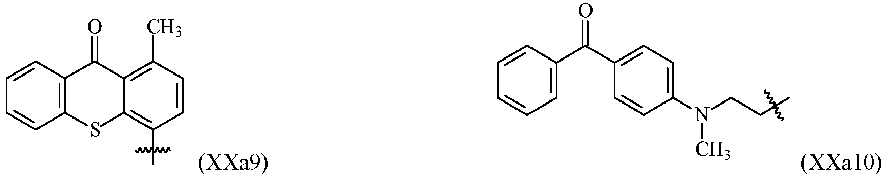 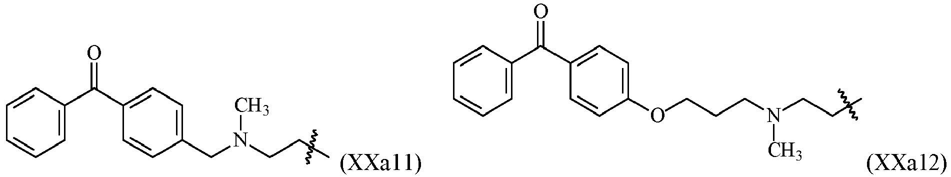 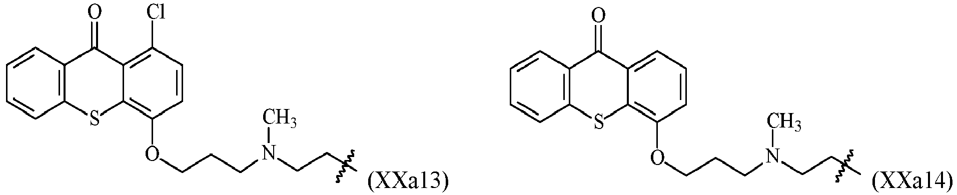 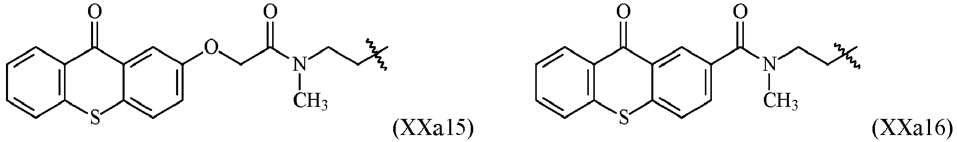 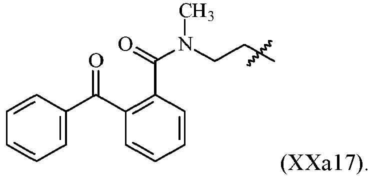
Aspect 22. The self-crosslinkable urethane (meth)acrylate according to any one of Aspects 1 to 21, wherein the self-crosslinkable urethane (meth)acrylate comprises at least one chromophore moiety Q according to formula (XXc) as defined in Aspect 16, wherein
   each L₂ is preferably selected from direct bond, alkylene of formula (C-1) as defined in Aspect 19, oxyalkylene of formula (C-2) as defined in Aspect 19, thioalkylene of formula (C-3) as defined in Aspect 19, ketoalkylene of formula (C-4) as defined in Aspect 19, aminoalkylene of formula (C-5) as defined in Aspect 19, carboxyalkylene of formula (C-6) as defined in Aspect 19, amidoalkylene of formula (C-7) as defined in Aspect 19, alkylene-aminoalkylene of formula (C-8) as defined in Aspect 19, oxyalkylene-aminoalkylene of formula (C-9) as defined in Aspect 19, or oxyalkylene-amidoalkylene of formula (C-10) as defined in Aspect 19,
   each u' is preferably 0;
   PI² is preferably according to formula (IX), (X), (XII) or (XIII) as defined in Aspect 14 wherein E in formula (XII) and (XIII) is S; more preferably PI² is according to formula (IXa), (Xa), (XIIa) or (XIIIa) as defined in Aspect 15.
Aspect 23. The self-crosslinkable urethane (meth)acrylate according to any one of Aspects 1 to 22, wherein the self-crosslinkable urethane (meth)acrylate comprises at least one chromophore moiety Q according to one of the following formulae (XXc1)-(XXc4): 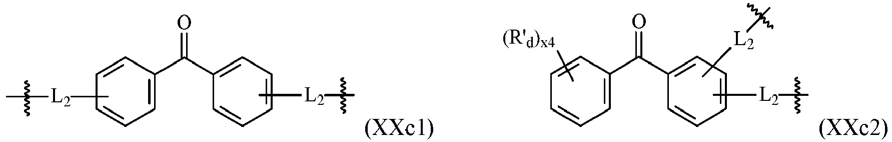 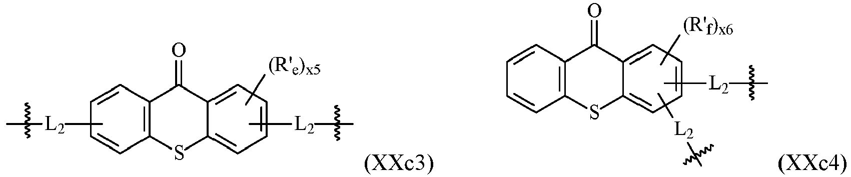 wherein
   R'_{d} is alkyl, Ar, -S-Ar or -O-Ar wherein Ar is aryl, in particular methyl, Ph, -S-Ph or -O-Ph wherein Ph is phenyl;
   R'ₑ and R'_{f} are independently halogen, alkoxy, alkyl or -C(=O)-Ar wherein Ar is aryl, in particular F, Cl, methyl, ethyl, isopropyl or -C(=O)-Ph wherein Ph is phenyl;
   x4 is 0, 1, 2 or 3, in particular 0 or 1;
   x5 is 0, 1 or 2, in particular 0 or 1;
   x6 is 0 or 1;
   each L₂ is independently selected from direct bond, amidoalkylene of formula (C-7) as defined in Aspect 19, or oxyalkylene-amidoalkylene of formula (C-10) as defined in Aspect 19.
Aspect 24. The self-crosslinkable urethane (meth)acrylate according to any one of Aspects 1 to 23, wherein the self-crosslinkable urethane (meth)acrylate comprises at least one chromophore moiety Q according to one of the following formulae (XXc5)-(XXc13): 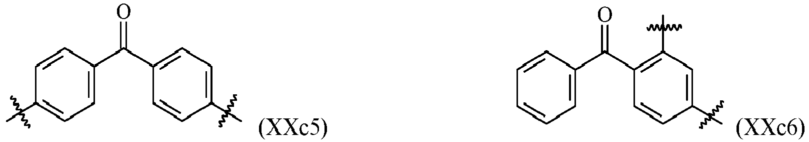 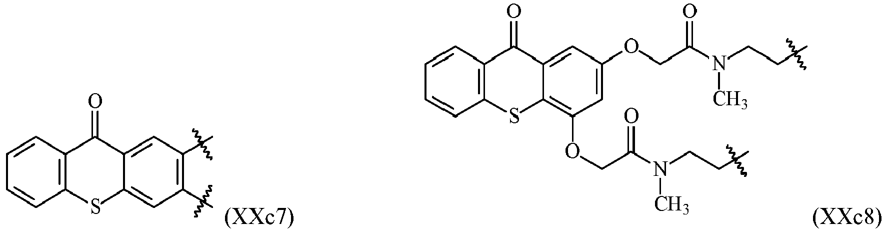 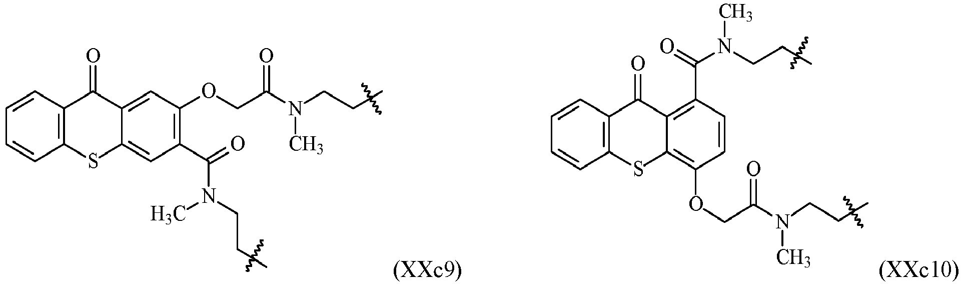 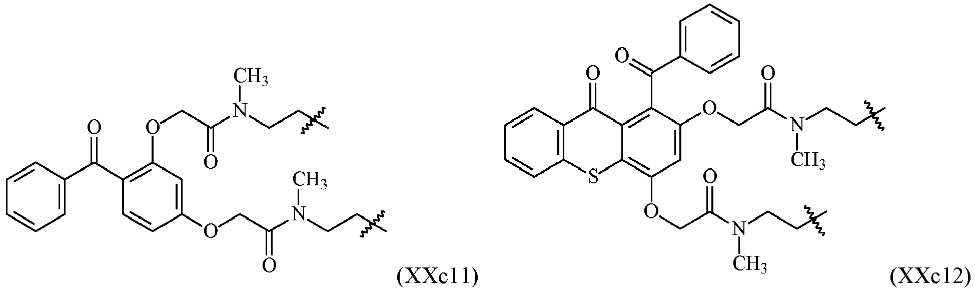 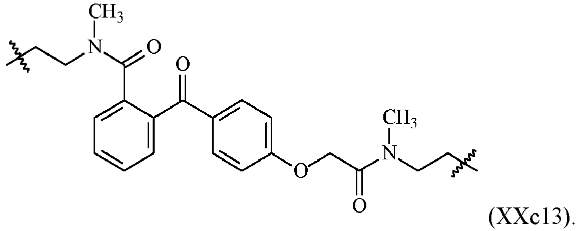
Aspect 25. The self-crosslinkable urethane (meth)acrylate according to any one of Aspects 1 to 24, wherein the self-crosslinkable urethane (meth)acrylate comprises at least one chromophore moiety Q according to the following formula (XXI): 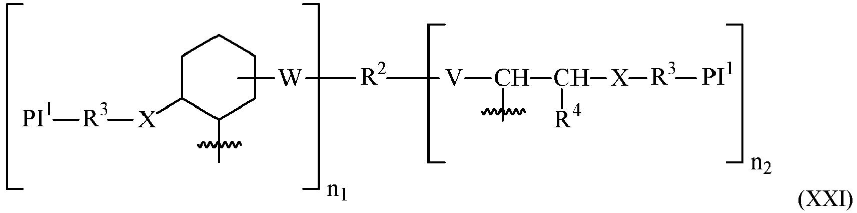 wherein
   n₁ is 0, 1, 2, 3 or 4;
   n₂ is 0, 1, 2, 3 or 4;
   the sum n₁ + n₂ is equal to 1, 2, 3 or 4;
   each PI¹ is independently a monovalent photoinitiator moiety PI as defined in any one of Aspects 13 to 15;
   each X is independently -NR¹-, -O-, -S-, *-C(=O)-O-, or ^{∗}-C(=O)-NR¹-;
   each V is independently direct bond, #-O-CH₂-, #-C(=O)-O-CH₂-, #-NR⁵-CH₂- or #-C(=O)-NR⁵-CH₂-;
   each W is independently direct bond, -CH₂-O-C(=O))-# or -C(=O)-O-#;
   each R¹ is independently H, alkyl or aryl;
   R² is a direct bond or a linker;
   each R³ is independently a direct bond or a linker;
   each R⁴ is independently H, an optionally substituted alkyl or an optionally substituted alkenyl;
   each R⁵ is independently H, alkyl, aryl or a group according to the following formula: 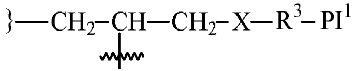 wherein X, R³ and PI¹ are as defined above and the symbol } represents the point of attachment to the nitrogen atom;
   the symbol * represents a point of attachment to R³;
   the symbol # represents a point of attachment to R².
Aspect 26. The self-crosslinkable urethane (meth)acrylate according to any one of Aspects 1 to 25, wherein the self-crosslinkable urethane (meth)acrylate comprises at least one chromophore moiety Q according to one of the following formulae (XXII) to (XXXV): 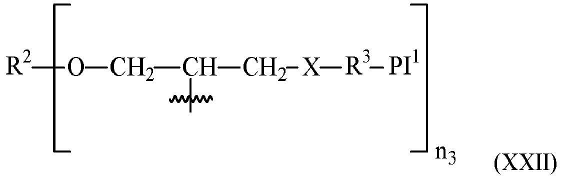 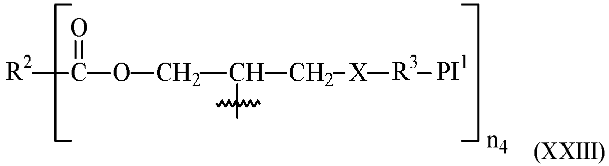 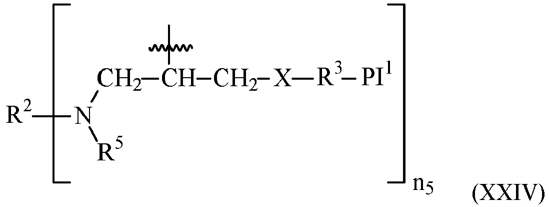 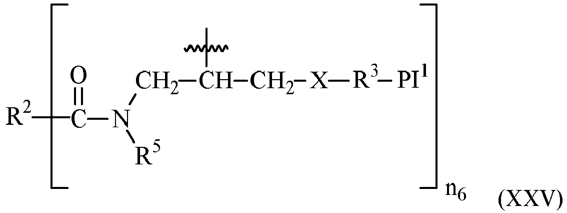 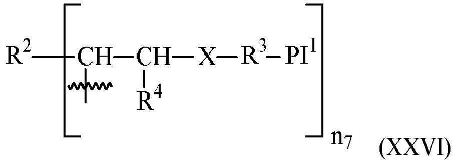 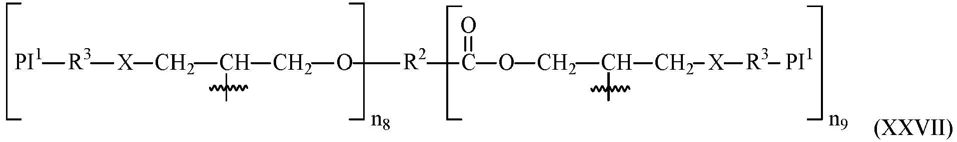 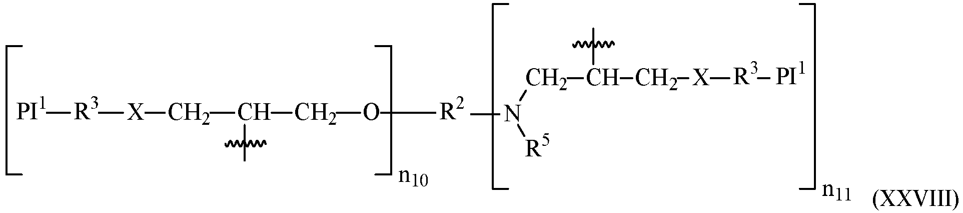 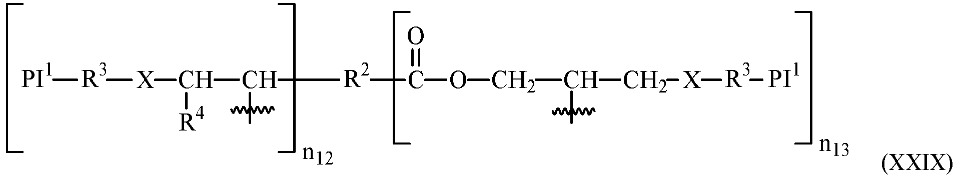 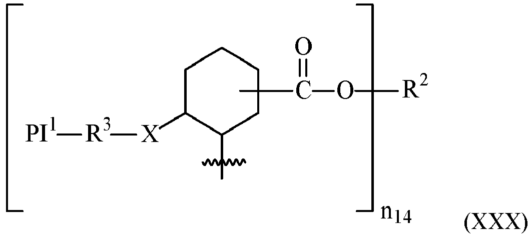 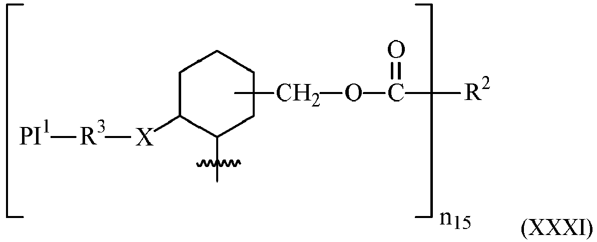 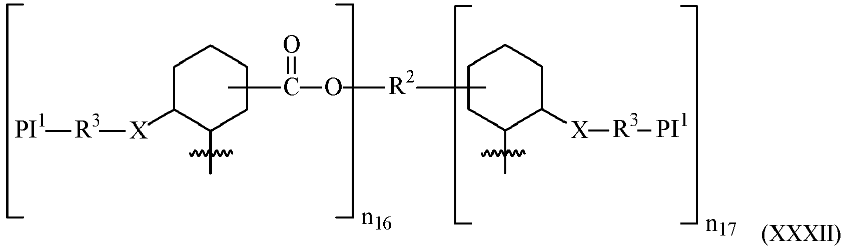 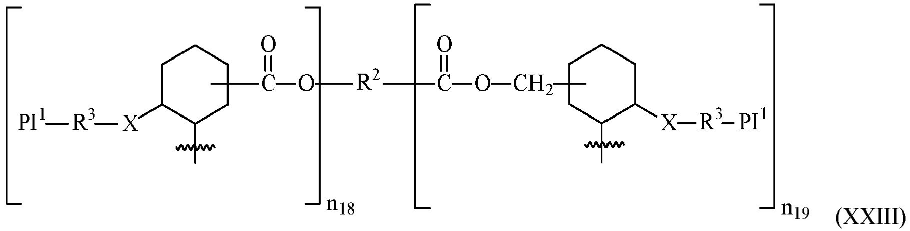 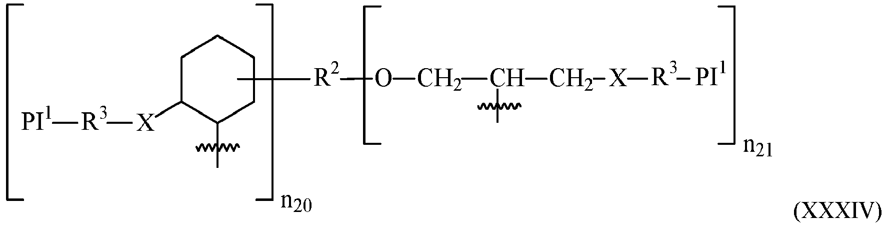 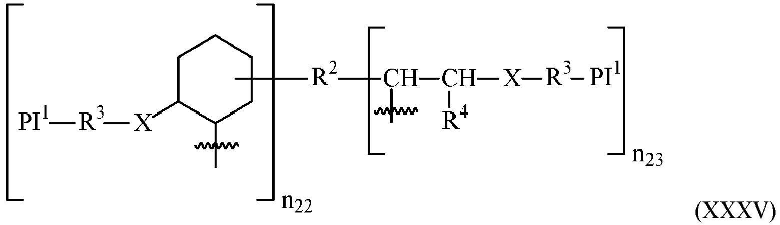 wherein
   PI¹, X, R², R³, R⁴ and R⁵ are as defined in Aspect 25;
   n₃, n₄, n₇, n₁₄ and n₁₅ are independently 2, 3 or 4, preferably 2 or 3;
   n₅ and n₆ are independently 1 or 2, preferably 2;
   n₈ is 1, 2, or 3 preferably 2;
   n₉ is 1, 2 or 3, preferably 1;
   n₁₀, n₁₁, n₁₆, n₁₇, n₁₈, n₁₉, n₂₀, n₂₁, n₂₂ and n₂₃ are independently 1 or 2, preferably 1;
   n₁₂ and n₁₃ are independently 1, 2 or 3, preferably 1.
Aspect 27. The self-crosslinkable urethane (meth)acrylate according to Aspect 26, wherein each R³ is independently selected from direct bond, C1-C6 alkylene, C1-C6 oxyalkylene, C1-C6 alkenylene, C1-C6 thioalkylene, C1-C6 ketoalkylene or C1-C6 aminoalkylene; in particular each R³ is independently selected from direct bond, alkylene of formula (C-11), oxyalkylene of formula (C-12), thioalkylene of formula (C-13), ketoalkylene of formula (C-14) or aminoalkylene of formula (C-15):

   •-(CRₘR'ₘ)_{f}-§ (C-11)

   •-O-(CRₒR'ₒ)_{g}-§ (C-12)

   •-S-(CRₚR'ₚ)ₕ-§ (C-13)

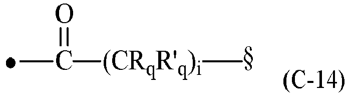

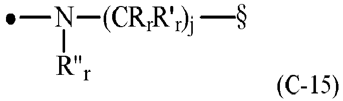

   wherein
   each Rₘ, R'ₘ, Rₒ, R'ₒ, Rₚ, R'ₚ, R_{q}, R'_{q}, Rᵣ and R'ᵣ is independently H or an optionally substituted alkyl, in particular H;
   R"ᵣ is H or an optionally substituted alkyl;
   f, g, h and j are independently 1, 2, 3, 4, 5 or 6, in particular 1 or 2;
   i is 1, 2, 3, 4 or 5, in particular 1;
   the symbol ● represents a point of attachment to the PI¹ moiety;
   the symbol § represents a point of attachment to the X moiety.
Aspect 28. The self-crosslinkable urethane (meth)acrylate according to Aspect 26 or 27, wherein each X is independently -NR¹-, -O- or ^{∗}-C(=O)-O-; in particular each X is independently -NR¹- or *-C(=O)-O-.
Aspect 29. The self-crosslinkable urethane (meth)acrylate according to any one of Aspects 26 to 28, wherein R² is a direct bond or a linker selected from the group consisting of alkylene, heteroatom-containing alkylene, alkenylene, heteroatom-containing alkenylene, cycloalkylene, heterocycloalkylene, arylene, heteroarylene, and combinations thereof;
   in particular R² is a direct bond or a linker selected from the group consisting of alkylene, heteroatom-containing alkylene, cycloalkylene, arylene, heteroarylene and combinations thereof.
Aspect 30. The self-crosslinkable urethane (meth)acrylate according to any one of Aspects 1 to 29, wherein the self-crosslinkable urethane (meth)acrylate comprises at least one chromophore moiety Q according to one of the following formulae (XXII), (XXIII), (XXIV) or (XXVI): 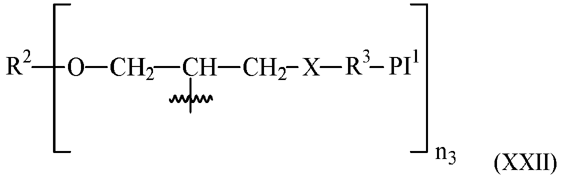 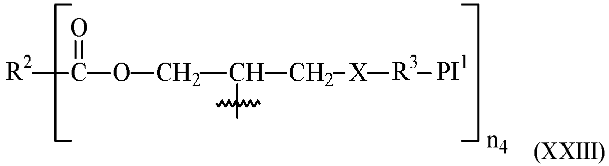 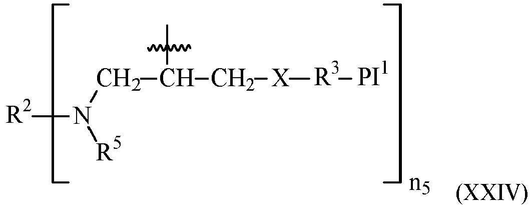 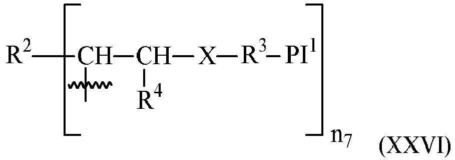 wherein
   n₃, n₄, n₅ and n₇ are independently 2, 3 or 4, preferably 2 or 3;
   each PI¹ is independently a monovalent photoinitiator moiety PI according to formula (VIII) or (XI) as defined in Aspect 14, preferably each PI¹ is a monovalent photoinitiator moiety PI according to formula (Villa) or (XIa) as defined in Aspect 15;
   each X is independently -NR¹-, -O- or ^{∗}-C(=O)-O-, preferably -NR¹- or ^{∗}-C(=O)-O-;
   each R¹ is independently H or alkyl;
   each R² is independently an alkylene or a heteroatom-containing alkylene;
   each R³ is independently a direct bond, an alkylene of formula (C-11) as defined in Aspect 27, an oxyalkylene of formula (C-12) as defined in Aspect 27 or an aminoalkylene of formula (C-15) as defined in Aspect 27, preferably a direct bond, an alkylene of formula (C-11) or an oxyalkylene of formula (C-12);
   each R⁴ is H;
   each R⁵ is independently H, alkyl or a group according to the following formula: 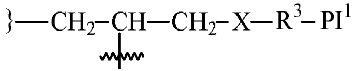 wherein X, R³ and PI¹ are as defined above and the symbol } represents the point of attachment to the nitrogen atom;
   the symbol * represents a point of attachment to R³.
Aspect 31. The self-crosslinkable urethane (meth)acrylate according to any one of Aspects 1 to 30, wherein the self-crosslinkable urethane (meth)acrylate comprises at least one chromophore moiety Q according to one of the following formulae (XXb1)-(XXb9): 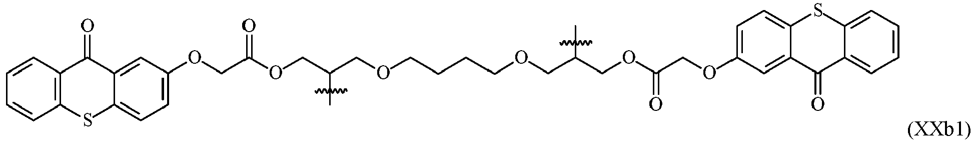 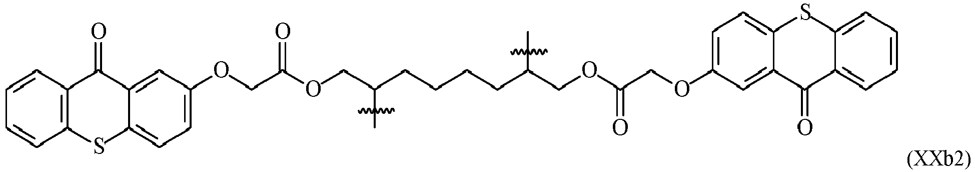 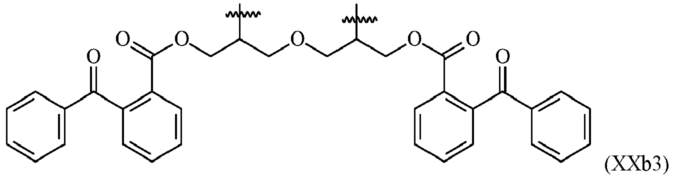 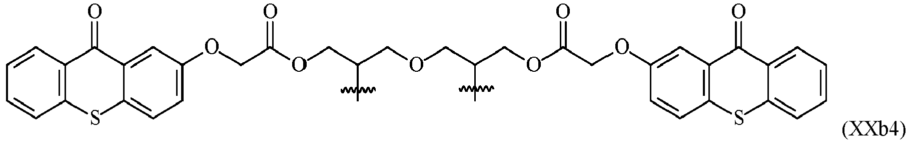 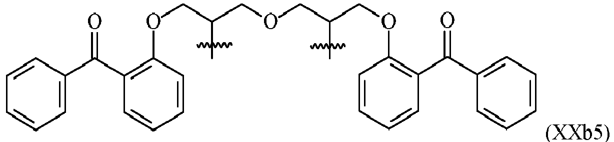 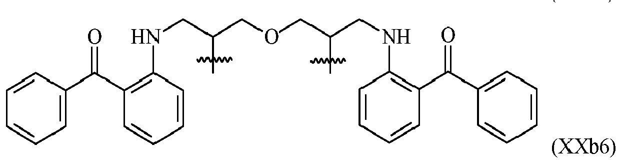 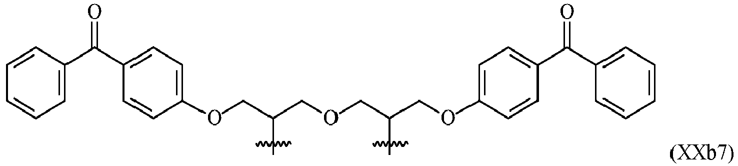 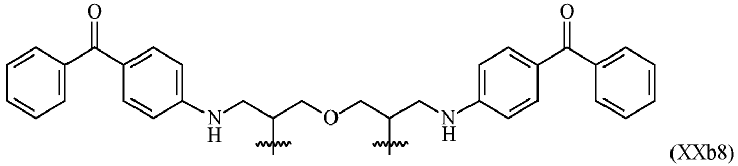 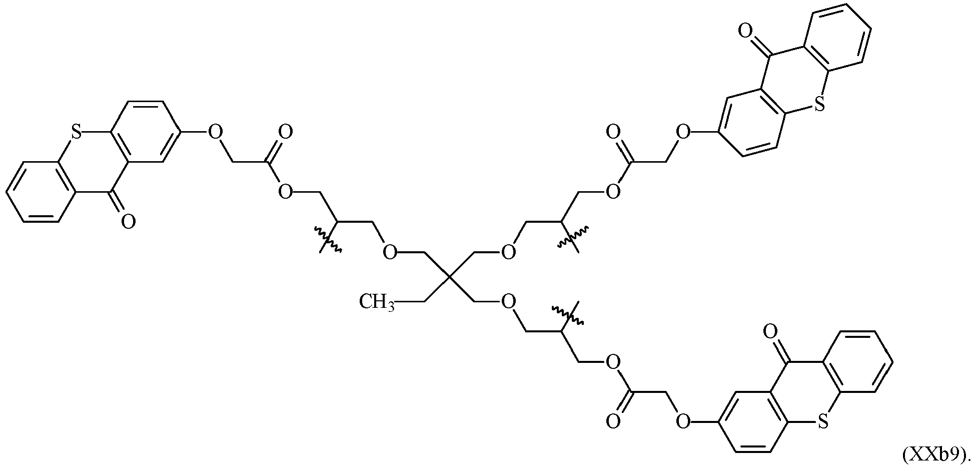
Aspect 32. The self-crosslinkable urethane (meth)acrylate according to any one of Aspects 1 to 31, wherein the self-crosslinkable urethane (meth)acrylate comprises at least one chromophore moiety Q according to one of the following formulae (XXXVI) to (XXXXI): 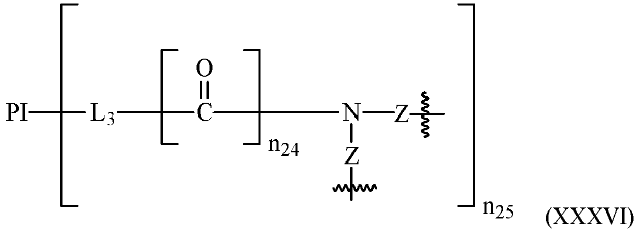 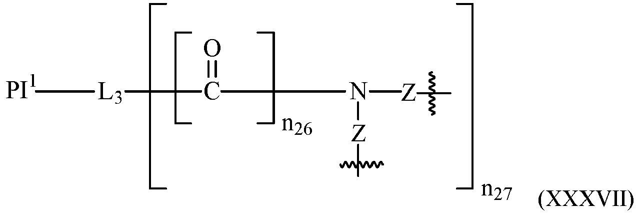 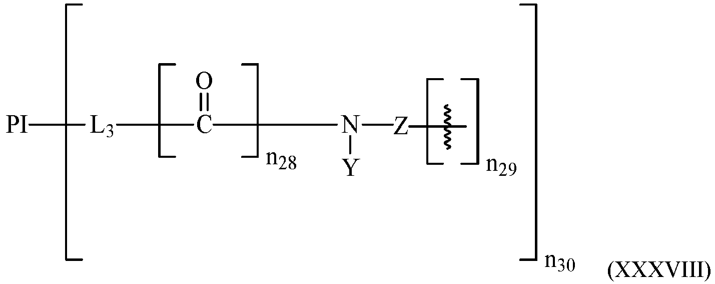 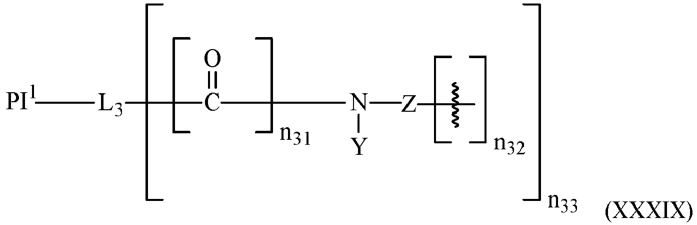 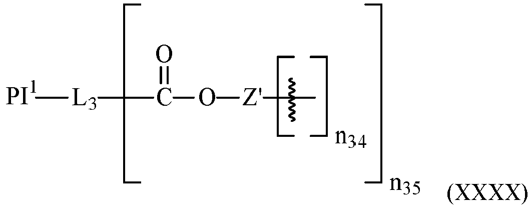 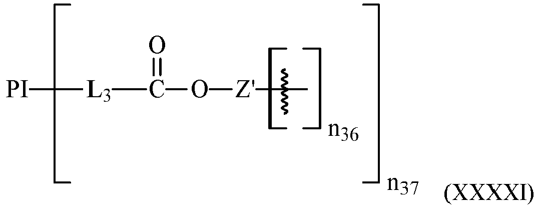 wherein
   each PI is independently a monovalent or divalent photoinitiator moiety PI as defined in any one of Aspects 13 to 15;
   each PI¹ is independently a monovalent photoinitiator moiety PI as defined in any one of Aspects 13 to 15;
   each L₃ is independently selected from a direct bond or a linker comprising 1 to 20 carbon atoms;
   each Y is independently H or alkyl;
   each Z and Z' is independently a linker comprising 1 to 20 carbon atoms;
   each n₂₄, n₂₆, n₂₈ and n₃₁ is independently 0 or 1, in particular 1;
   each n₂₉, n₃₂, n₃₄ and n₃₆ is independently 1, 2 or 3, in particular 1;
   each n₂₂ and n₃₃ is independently 1, 2 or 3; in particular 1 or 2;
   each n₂₈ and n₃₀ is independently 1 or 2, in particular 1;
   n₃₅ is 1, 2 or 3, in particular 2;
   n₃₇ is 1 or 2, in particular 2.
Aspect 33. The self-crosslinkable urethane (meth)acrylate according to Aspect 32, wherein each L₃ is independently selected from direct bond, C1-C6 alkylene, C1-C6 oxyalkylene, C1-C6 alkenylene, C1-C6 thioalkylene, C1-C6 ketoalkylene, C1-C6 ketoalkenylene, C7-C13 ketoarylene, C6-C13 ketocycloalkylene; more particularly each L₃ is independently selected from direct bond, alkylene of formula (C-16), oxyalkylene of formula (C-17), thioalkylene of formula (C-18), ketoalkylene of formula (C-19), aminoalkylene of formula (C-20) or alkylene of formula (C-21):

   •-(CRₘR'ₘ)_{f}-§ (C-16)

   •-O-(CRₒR'ₒ)_{g}-§ (C-17)

   •-S-(CRₚR'ₚ)ₕ-§ (C-18)

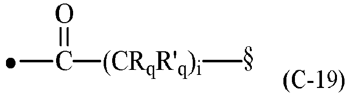

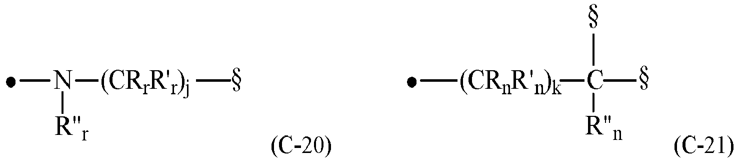

   wherein
   each Rₘ, R'ₘ, Rₙ, R'ₙ Rₒ, R'ₒ, Rₚ, R'ₚ, R_{q}, R'_{q}, Rᵣ and R'ᵣ is independently H or an optionally substituted alkyl, in particular H;
   R"ᵣ and R"ₙ are independently H or an optionally substituted alkyl;
   f, g, h and j are independently 1, 2, 3, 4, 5 or 6, in particular 1 or 2;
   i and k are independently 1, 2, 3, 4 or 5, in particular 1;
   the symbol ● represents a point of attachment to the PI or PI¹ moiety;
   the symbol § represents a point of attachment to the moieties other than PI or PI¹.
Aspect 34. The self-crosslinkable urethane (meth)acrylate according to Aspect 32 or 33, wherein each Y is independently an alkyl, in particular methyl or ethyl.
Aspect 35. The self-crosslinkable urethane (meth)acrylate according to any one of Aspects 32 to 34, wherein each Z is independently an alkylene; in particular each Z is independently an alkylene according to one of the following formulae (C-22), (C-23) or (C-24), more particularly each Z is independently an alkylene of formula (C-22): 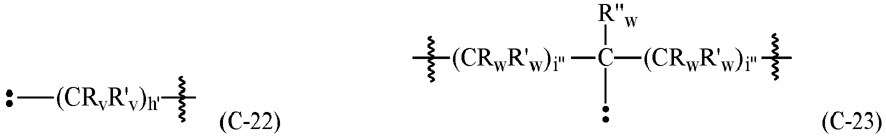 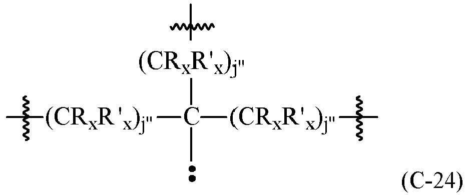
   wherein each Rᵥ, R'ᵥ, R_{w}, R'_{w}, Rₓ, R'ₓ is independently H or alkyl, in particular H;
   R"_{w} is H or alkyl, in particular alkyl;
   h' is 1, 2, 3, 4, 5 or 6, in particular 2;
   each i" is independently 1, 2 or 3, in particular 1;
   each j" is independently 1, 2 or 3, in particular 1;
   the symbol 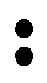 represents a point of attachment to the nitrogen or oxygen atom.
Aspect 36. The self-crosslinkable urethane (meth)acrylate according to any one of Aspects 32 to 35, wherein each Z' is independently selected from an aliphatic or aromatic hydrocarbon linker, a polyether linker, a polyester linker, a polycarbonate linker, a polyorganosiloxane linker, a polydiene linker, an isocyanurate linker, and combinations thereof; in particular each Z' is independently selected from an aliphatic or aromatic hydrocarbon linker, a polyether linker, a polyester linker and combinations thereof; more particularly each Z' is independently selected from an alkylene, an alkoxylated alkylene and a polycaprolactone linker.
Aspect 37. The self-crosslinkable urethane (meth)acrylate according to any one of Aspects 1 to 36, wherein the self-crosslinkable urethane (meth)acrylate of the invention comprises at least one chromophore moiety Q according to one of the following formulae (XXXXII) to (XXXXIV): 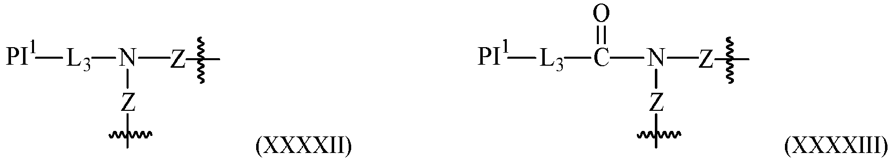 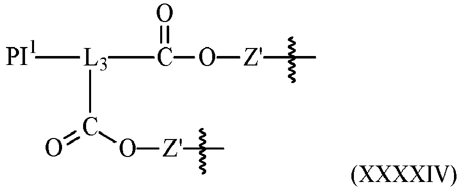 wherein
   each PI¹ is independently a monovalent photoinitiator moiety PI according to formula (VIII) or (XI) as defined in Aspect 14, preferably each PI¹ is a monovalent photoinitiator moiety PI according to formula (Villa) or (XIa) as defined in Aspect 15;
   each L₃ is independently selected from a direct bond, an alkylene of formula (C-16) as defined in Aspect 33, an oxyalkylene of formula (C-17) as defined in Aspect 33, a thioalkylene of formula (C-18) as defined in Aspect 33, or an alkylene of formula (C-21) as defined in Aspect 33;
   each Z is independently an alkylene of formula (C-22) as defined in Aspect 35,
   each Z' is independently an alkylene or a polyether linker.
Aspect 38. The self-crosslinkable urethane (meth)acrylate according to any one of Aspects 1 to 37, wherein the self-crosslinkable urethane (meth)acrylate comprises at least one chromophore moiety Q according to one of the following formulae (XXb10)-(XXb26): 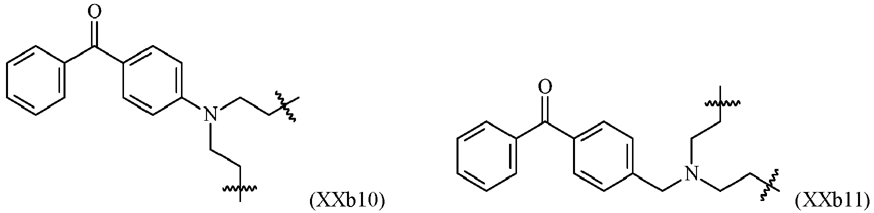 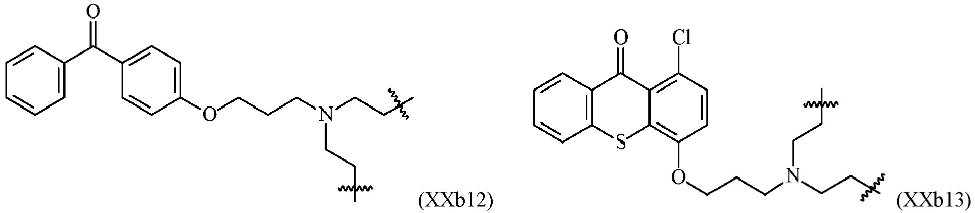 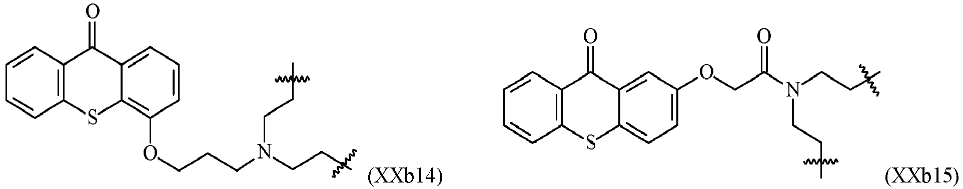 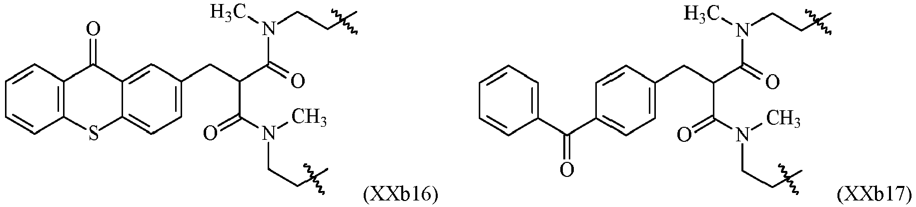 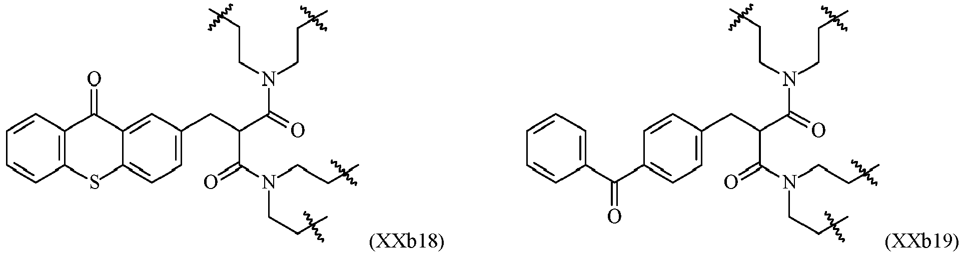 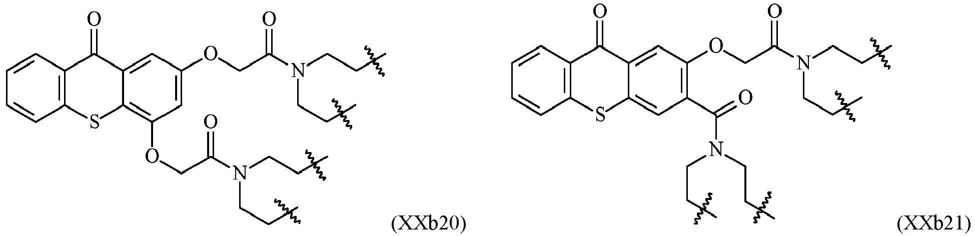 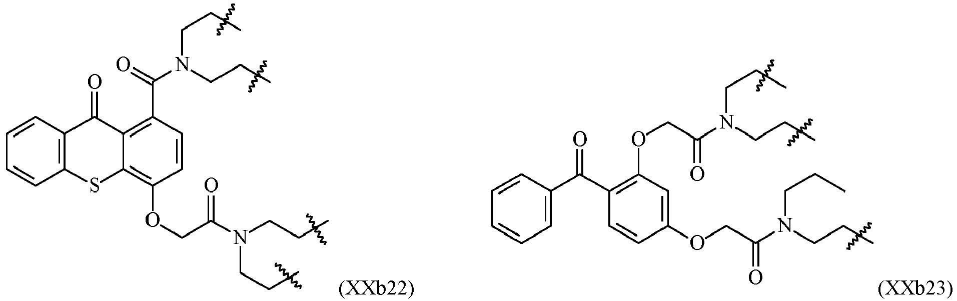 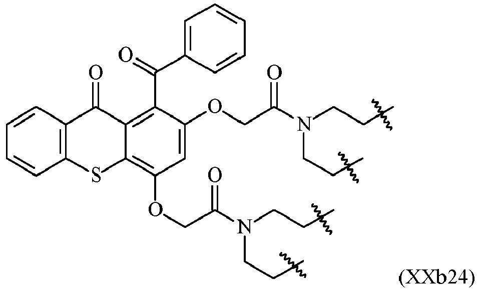 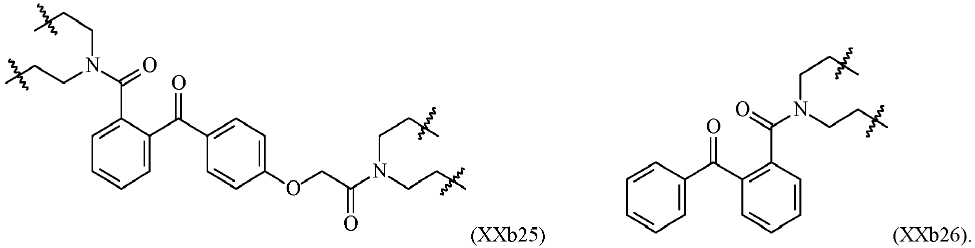
Aspect 39. The self-crosslinkable urethane (meth)acrylate according to any one of Aspects 1 to 38, wherein the total amount of chromophore moieties Q in the self-crosslinkable urethane (meth)acrylate represents from 0.5 to 20%, in particular from 1 to 15%, more particularly from 1.5 to 10%, of the total weight of the self-crosslinkable urethane (meth)acrylate.
Aspect 40. The self-crosslinkable urethane (meth)acrylate according to any one of Aspects 1 to 39, wherein the self-crosslinkable urethane (meth)acrylate comprises at least one chain-extending moiety EXT which is the residue of a polyol selected from the residue of a polymeric polyol, the residue of a non-polymeric polyol or the residue of an amino-functional polyol.
Aspect 41. The self-crosslinkable urethane (meth)acrylate according to any one of Aspects 1 to 40, wherein the self-crosslinkable urethane (meth)acrylate comprises at least one chain-extending moiety EXT which is the residue of a polymeric diol, in particular the residue of a polyether diol or a polyester diol, more particularly the residue of a polyether diol selected from a polyethylene glycol, a poly(1,2-propylene glycol), a poly(1,3-propylene glycol), a poly(1,4-butylene glycol) and combinations thereof, or the residue of a polyester diol selected from a poly(caprolactone), a poly(lactide), a poly(alkylene glycol adipate) and a poly(alkylene glycol succinate).
Aspect 42. The self-crosslinkable urethane (meth)acrylate according to any one of Aspects 1 to 41, wherein the self-crosslinkable urethane (meth)acrylate comprises at least one chain-extending moiety EXT which is the residue of a non-polymeric diol, in particular the residue of a non-polymeric aliphatic diol, more particularly the residue of a non-polymeric aliphatic diol selected from ethylene glycol, di-, tri- or tetraethylene glycol, 1,2- or 1,3-propylene glycol, di-, tri- or tetra(1,2-propylene glycol), di-, tri- or tetra(1,3-propylene glycol), 1,2-, 1,3- or 1,4-butylene glycol, di-, tri- or tetra(1,4-butylene glycol), 1,5-pentanediol, 1,6-hexanediol, 1,7-heptanediol, 1,8-octanediol, 1,9-nonanediol, 1,10-decanediol, 1,12-dodecanediol, 2-methyl-1,3-propanediol, 2,2-dimethyl-1,3-propanediol, 2,2-diethyl-1,3-propanediol, 2-methyl-2-ethyl-1,3-propanediol, 3-methyl-1,5-pentanediol, 3,3-dimethyl-1,5-pentanediol, 2,4-diethyl-1,5-pentanediol, 3-butyl-3-ethyl-1,5-pentane diol, 2,2,4-trimethyl 1,5-pentanediol, cyclohexanediol, cyclohexane-1,4-dimethanol, norbomene dimethanol, norbornane dimethanol, tricyclodecanediol, tricyclodecane dimethanol, hydrogenated bisphenol A, B, F or S, a dianhydrohexitol (i.e. isosorbide, isomannide, isoidide), a hydrogenated dimer fatty acid (i.e. a diol obtained by dimerizing one or more unsaturated fatty acids such as oleic acid or linoleic acid and then hydrogenating the resulting product to convert the carboxylic acid groups into hydroxyl groups, for example Pripol^{®} 2033 from Croda) as well as the alkoxylated (i.e. ethoxylated and/or propoxylated) derivatives thereof with up to four oxyalkylene units.
Aspect 43. The self-crosslinkable urethane (meth)acrylate according to any one of Aspects 1 to 42, wherein the self-crosslinkable urethane (meth)acrylate comprises at least one chain-extending moiety EXT which comprises a tertiary amine group, in particular at least one chain-extending moiety EXT which is the residue of an amino-functional polyol selected from triethanol amine, N-methyldiethanol amine, N-ethyldiethanol amine, N-propyl diethanolamine, N-butyl diethanol amine, N-t-butyl diethanolamine, trimethanol amine, N-methyl dimethanol amine, 3-(dimethylamino)-1,2-propanediol, 3-(diethylamino)-1,2-propanediol, 3-(dipropylamino)-1,2-propanediol, 2-(dimethylamino)propane-1,3-diol, bis(2-hydroxyethyl)dodecylamine, bis(2-hydroxyethyl)octadecylamine, N,N-dioctadecyl-N',N'-bis(2-hydroxyethyl)-1,3-diaminopropane, 3-morpholino-1,2-propanediol, 3-piperidino-1,2-propanediol, 3-pyrrolidino-1-yl-1,2- propanediol, and an aminobenzamide diol according to the following formula (XXXXVI): 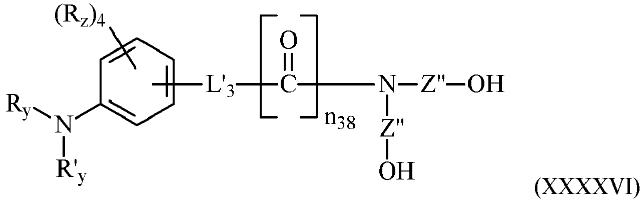 wherein
   R_{y} and R'_{y} are independently an optionally substituted optionally substituted group selected from alkyl, alkenyl, alkynyl, aryl, aralkyl, alkaryl and heteroaryl;
   each R_{z} is independently H, F, Cl, Br, I, -OR^{∗}, -SR^{∗}, -N(R^{∗})₂, -NO₂, -CN, -C(=O)R^{∗}, -O-C(=O)R^{∗}, -C(=O)OR^{∗}, -C(=O)N(R^{∗})₂, -NR^{∗}-C(=O)-R^{∗}, -SO₂-N(R^{∗})₂, or an optionally substituted group selected from the group consisting of alkyl, heteroatom-containing alkyl, cycloalkyl, heterocycloalkyl, alkenyl, alkynyl, aryl, aralkyl, alkaryl and heteroaryl;
   each R* is independently H or an optionally substituted group selected from alkyl, heteroatom-containing alkyl, cycloalkyl, heterocycloalkyl, alkenyl, alkynyl, aryl, aralkyl, alkaryl and heteroaryl;
   L'₃ is as defined for L₃ in Aspect 32 or 33;
   each Z" is independently as defined for Z in Aspect 32 or 35;
   n₃₈ is 0 or 1.
Aspect 44. The self-crosslinkable urethane (meth)acrylate according to any one of Aspects 1 to 43, wherein the total amount of chain-extending moieties EXT in the self-crosslinkable urethane (meth)acrylate represents from 0 to 84.5%, in particular from 0.1 to 75%, more particularly from 1 to 65%, of the total weight of the self-crosslinkable urethane (meth)acrylate.
Aspect 45. The self-crosslinkable urethane (meth)acrylate according to any one of Aspects 1 to 44, wherein each (meth)acrylate-functionalized moiety ACR is a terminal moiety attached to a single polyurethane moiety UU.
Aspect 46. The self-crosslinkable urethane (meth)acrylate according to any one of Aspects 1 to 45, wherein the self-crosslinkable urethane (meth)acrylate comprises a chromophore moiety Q attached to at least two distinct polyurethane moieties UU.
Aspect 47. The self-crosslinkable urethane (meth)acrylate according to any one of Aspects 1 to 46, wherein the self-crosslinkable urethane (meth)acrylate corresponds to the following structure (A): 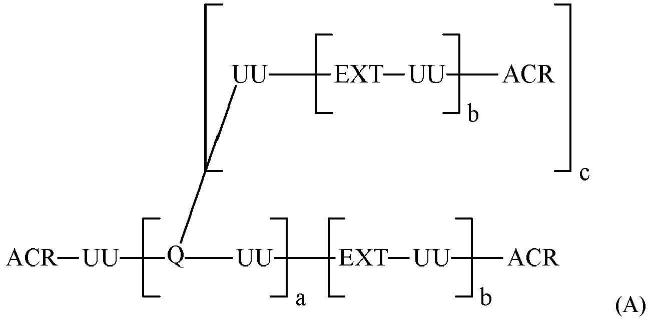
   wherein ACR, UU, Q and EXT are as defined in any one of Aspects 1 to 44;
   a is an integer from 1 to 100;
   b is an integer from 0 to 100;
   c is an integer equal to 0, 1 or 2, preferably 0 or 1.
Aspect 48. The self-crosslinkable urethane (meth)acrylate according to any one of Aspects 1 to 45, wherein the self-crosslinkable urethane (meth)acrylate comprises at least one chromophore moiety Q attached to a single polyurethane moiety UU.
Aspect 49. The self-crosslinkable urethane (meth)acrylate according to any one of Aspects 1 to 45 and 48, wherein the self-crosslinkable urethane (meth)acrylate corresponds to the following structure (B): 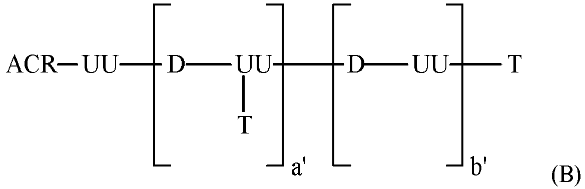 wherein
   each D is independently Q or EXT;
   each T is independently ACR or Q;
   ACR, UU, Q and EXT are as defined in any one of Aspects 1 to 44;
   a' is an integer from 0 to 100;
   b' is an integer from 0 to 100;
   wherein at least one of a' and b' is not 0; and
   at least one of D and T is Q.
Aspect 50. The self-crosslinkable urethane (meth)acrylate of any one of Aspects 1 to 49, wherein the self-crosslinkable urethane (meth)acrylate does not comprise an effective amount of a dye moiety, preferably the self-crosslinkable urethane (meth)acrylate does not comprise any dye moiety.
Aspect 51. A process for the preparation of a self-crosslinkable urethane (meth)acrylate, wherein the process comprises reacting:
   a) a hydroxyl-functionalized (meth)acrylate component;
   b) a polyisocyanate component;
   c) a hydroxyl-functionalized photoinitiator component; and
   d) optionally, a polyol component.
Aspect 52. The process according to Aspect 51, wherein component a) comprises at least one hydroxyl-functionalized (meth)acrylate compound bearing 1 to 5 (meth)acrylate groups, in particular 1 to 3 (meth)acrylate groups, more particularly 1 (meth)acrylate group.
Aspect 53. The process according to Aspect 51 or 52, wherein component a) comprises at least one hydroxyl-functionalized (meth)acrylate compound according to the following formula (Ia): 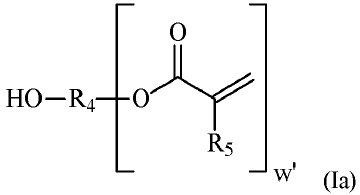 wherein
   R₄, R₅ and w' are as defined in any one of Aspects 3 to 5.
Aspect 54. The process according to any one of Aspects 51 to 53, wherein component a) comprises at least one hydroxyl-functionalized (meth)acrylate compound selected from a hydroxyalkyl (meth)acrylate (for example 2-hydroxyethyl (meth)acrylate, 2-hydroxypropyl (meth)acrylate, 3-hydroxypropyl (meth)acrylate, 2-hydroxybutyl (meth)acrylate, 3-hydroxybutyl (meth)acrylate, 4-hydroxybutyl (meth)acrylate, neopentyl glycol mono(meth)acrylate or 1,6-hexanediol mono(meth)acrylate), 2-hydroxy-3-phenoxypropyl (meth)acrylate, 3-chloro-2-hydroxypropyl (meth)acrylate, tris(2-hydroxyethyl)isocyanurate mono- and di(meth)acrylate, di-, tri-, tetra- or polyethylene glycol mono(meth)acrylate, di-, tri-, tetra- or poly(1,2-propylene glycol) mono(meth)acrylate, di-, tri-, tetra- or poly(1,3-propylene glycol) mono(meth)acrylate, di-, tri-, tetra- or poly(1,4-butylene glycol) mono(meth)acrylate, glycerin mono- and di(meth)acrylate, 2-hydroxy-1-acryloxy-3-(meth)acryloxypropane, trimethylolpropane mono- and di(meth)acrylate, di(trimethylolpropane) mono-, di- and tri(meth)acrylate, trimethylolethane mono- and di(meth)acrylate, pentaerythritol mono-, di- and tri(meth)acrylate, dipentaerythritol mono-, di-, tri-, tetra- and penta(meth)acrylate, as well as the alkoxylated (e.g., ethoxylated and/or propoxylated) derivatives thereof and the (poly)caprolactone derivatives thereof obtained by ring-opening polymerization of ε-caprolactone initiated with one of the aforementioned hydroxyl-functionalized (meth)acrylate compounds (i.e. a (poly)caprolactone (meth)acrylate such as (poly)caprolactone 2-hydroxyethyl (meth)acrylate according to the following formula: CH₂=CR₅-C(=O)-O-CH₂-CH₂-[O-(C=O)-(CH₂)₅]ₙ-OH wherein R₅ is H or methyl and t is 1 to 20), and combinations thereof.
Aspect 55. The process according to any one of Aspects 51 to 54, wherein the total amount of component a) used to prepare the self-crosslinkable urethane (meth)acrylate is from 5 to 85%, in particular from 10 to 80%, more particularly from 15 to 75%, by weight of component a) based on the total amount of components a), b), c) and d).
Aspect 56. The process according to any one of Aspects 51 to 55, wherein component b) comprises at least one polyisocyanate compound bearing 2 to 3 isocyanate groups.
Aspect 57. The process according to any one of Aspects 51 to 56, wherein component b) comprises at least one diisocyanate compound according to the following formula (VIIa) and/or at least one triisocyanate compound according to the following formula (VIIb):

   O=C=N-R₁-N=C=O (VIIa)

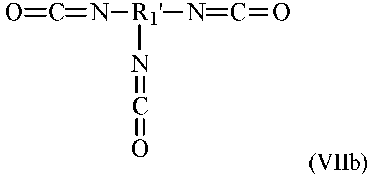

   wherein R₁ and R₁' are as defined in any one of Aspects 8 to 10.
Aspect 58. The process according to any one of Aspects 51 to 57, wherein component b) comprises:
   - at least one diisocyanate selected from 2,4- and 2,6-toluene diisocyanate (TDI), isophorone diisocyanate (IPDI - corresponding to 3-isocyanatomethyl-3,5,5-trimethylcyclohexylisocyanate), methylene diisocyanate, ethylene diisocyanate, 1,2- or 1,3-propylene diisocyanate, 1,2-, 1,3 or 1,4-butylene diisocyanate, 1,5-pentamethylene diisocyanate (PDI), 1,6-hexamethylene diisocyanate (HDI), 2,2,4- and 2,4,4-trimethylhexamethylene diisocyanate (TMDI), 1,10-decylene diisocyanate, 1,12-dodecylene diisocyanate, 1,18-octadecylene diisocyanate, 2,2'-, 2,4'- and 4,4'-diphenylmethane diisocyanate (MDI), 2,2'-, 2,4'- and 4,4'-dicyclohexylmethane diisocyanate (H12MDI), benzidine diisocyanate, 3,3'-dimethyl-4,4'-biphenyl diisocyanate, dianisidine diisocyanate, 3,3'-dimethyl-4,4'-diphenylmethane diisocyanate, 1,3- and 1,4-phenylene diisocyanate, 1,4- and 1,5-naphtalene diisocyanate (NDI), 1,4- and 9,10-anthracene diisocyanate, 1,3- and 1,4-cyclohexane diisocyanate, 1-methyl-2,4-diisocyanatocyclohexane, 1-methyl-2,6-diisocyanatocyclohexane, 1,3 and 1,4-bis(isocyanatomethyl)cyclohexane, m-tetramethylxylylene diisocyanate, m-xylylene diisocyanate, 4-methoxy-1,3-phenylene diisocyanate, 4-ethoxy-1,3-phenylene diisocyanate, 5,6-dimethyl-1,3-phenylene diisocyanate, 2,4' or 4,4'-diisocyanate diphenyl ether, lysine diisocyanate, dimer acid diisocyanate, dimers of the aforementioned diisocyanates (in particular uretdione or allophanate dimers), as well as polyurea or polyurethane prepolymers functionalized with isocyanate functional groups, and combinations thereof; and/or
   - at least one triisocyanate selected from 1,6,11-undecane triisocyanate, triphenylmethane triisocyanate, 2,4,6-tolylene triisocyanate, 2,4,4'-triisocyanate diphenyl ether, trimers of the aforementioned diisocyanates (in particular isocyanurate or biuret trimers), polymeric derivatives of the aforementioned diisocyanates, and combinations thereof.
Aspect 59. The process according to any one of Aspects 51 to 58, wherein the total amount of component b) used to prepare the self-crosslinkable urethane (meth)acrylate is from 10 to 65%, in particular from 15 to 60%, more particularly from 20 to 55%, by weight of component b) based on the total amount of components a), b), c) and d).
Aspect 60. The process according to any one of Aspects 51 to 59, wherein component c) comprises at least one hydroxyl-functionalized photoinitiator comprising at least one monovalent or divalent photoinitiator moiety PI as defined in any one of Aspects 13 to 15.
Aspect 61. The process according to any one of Aspects 51 to 60, wherein component c) comprises at least one hydroxyl-functionalized photoinitiator which is a monool, a diol, a triol or a tetraol.
Aspect 62. The process according to any one of Aspects 51 to 61, wherein component c) comprises at least one hydroxyl-functionalized photoinitiator according to one of the following formulae (XXd), (XXe) or (XXf):

   HO-L₁-PI¹ (XXd)

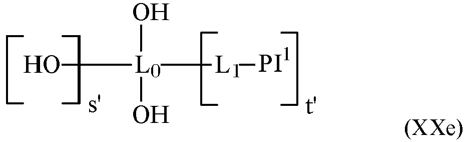

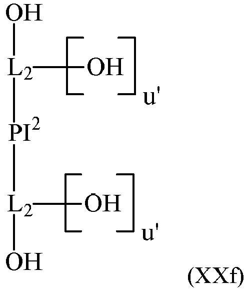

   wherein
   L₀, L₁ and L₂ are as defined in any one of Aspects 16 to 19, 22 and 23;
   each PI¹ is independently a monovalent photoinitiator moiety PI as defined in any one of Aspects 13 to 15;
   each PI² is independently a divalent photoinitiator moiety PI as defined in any one of Aspects 13 to 15;
   s', t' and u' are as defined in Aspect 16.
Aspect 63. The process according to any one of Aspects 51 to 62, wherein component c) comprises at least one hydroxyl-functionalized photoinitiator according to formula (XXd) as defined in Aspect 62 and wherein L₁ and PI¹ are preferably as defined in Aspect 19.
Aspect 64. The process according to any one of Aspects 51 to 63, wherein component c) comprises at least one hydroxyl-functionalized photoinitiator according to the following formulae (XXd1) or (XXd2) 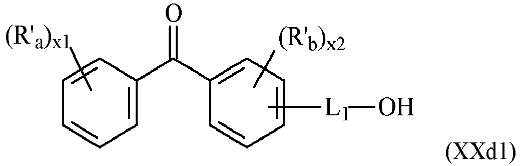 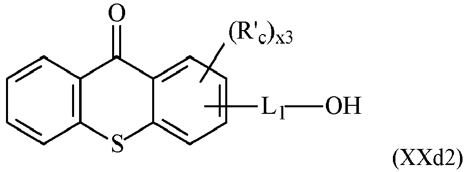 wherein R'ₐ, R'_{b}, R'_{c}, x1, x2, x3 and L₁ are as defined in Aspect 20.
Aspect 65. The process according to any one of Aspects 51 to 64, wherein component c) comprises at least one hydroxyl-functionalized photoinitiator corresponding to one of the following formulae (XXd3)-(XXd17): 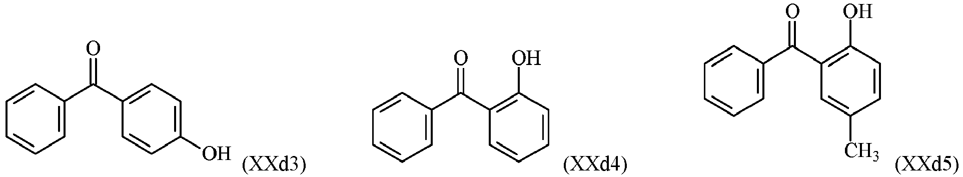 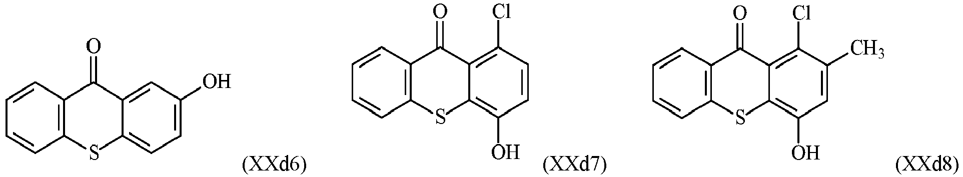 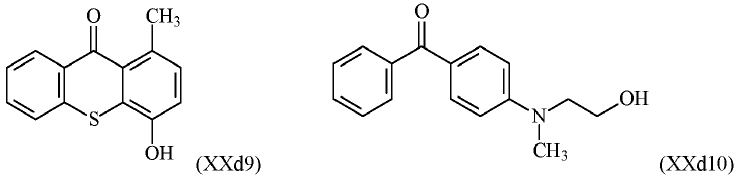 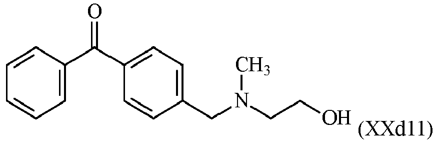 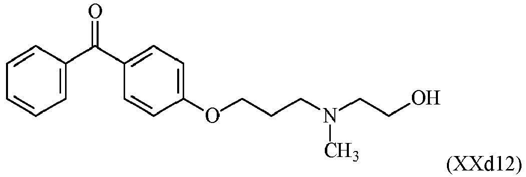 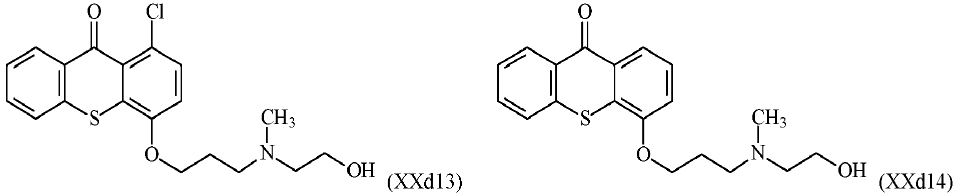 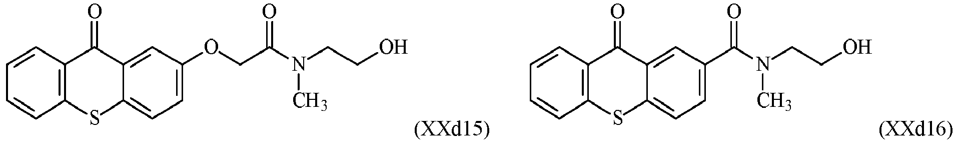 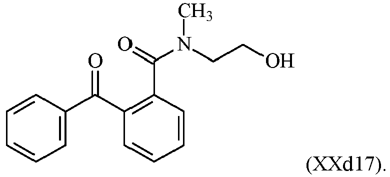
Aspect 66. The process according to any one of Aspects 51 to 65, wherein component c) comprises a hydroxyl-functionalized photoinitiator according to formula (XXf) as defined in Aspect 62 wherein each L₂, u' and PI² are as defined in Aspect 22.
Aspect 67. The process according to any one of Aspects 51 to 66, wherein component c) comprises at least one hydroxyl-functionalized photoinitiator corresponding to one of the following formulae (XXf1)-(XXf4) 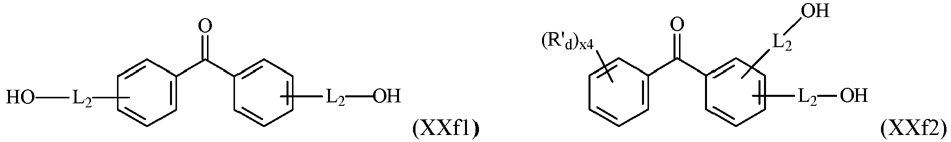 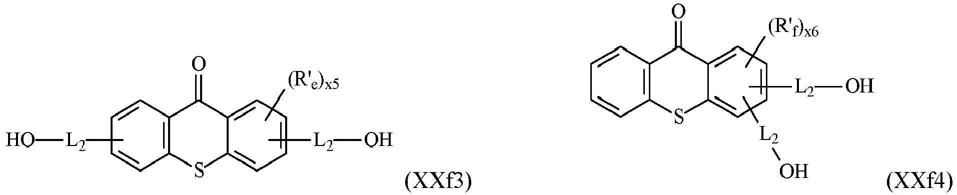 wherein R'_{d}, R'ₑ, R'_{f}, x4, x5, x6 and L₂ are as defined in Aspect 23.
Aspect 68. The process according to any one of Aspects 51 to 67, wherein component c) comprises at least one hydroxyl-functionalized photoinitiator according to one of the following formulae (XXf5)-(XXf13): 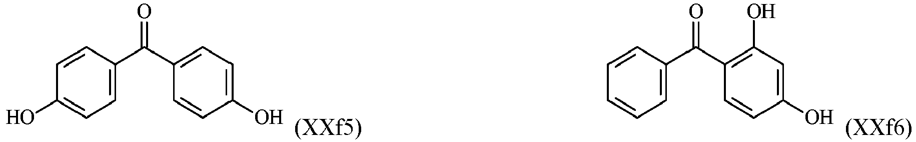 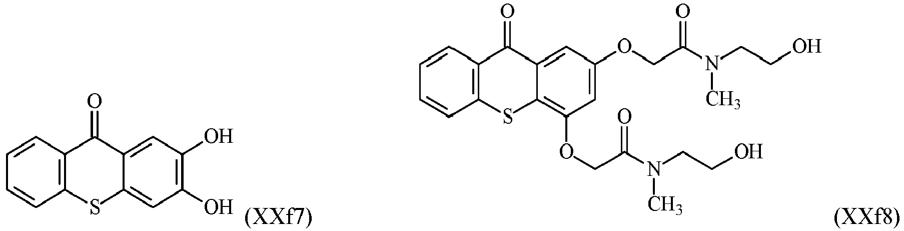 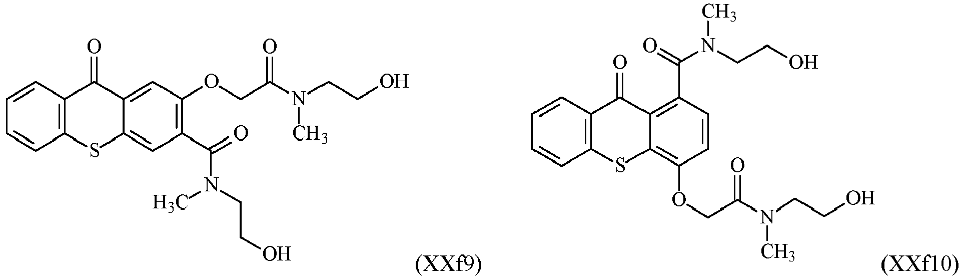 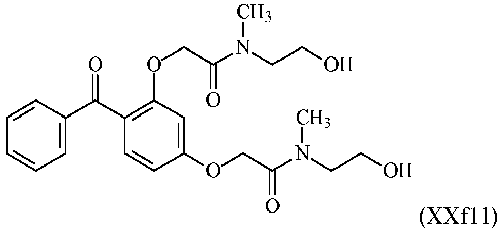 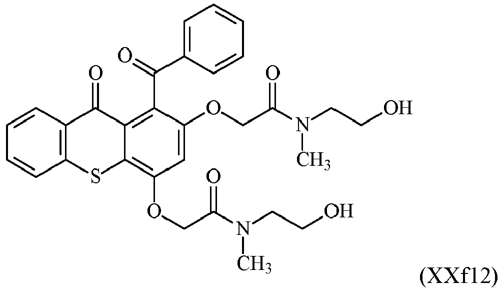 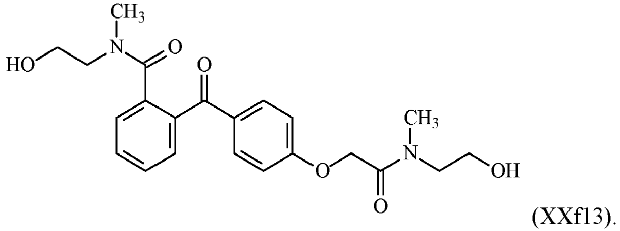
Aspect 69. The process according to any one of Aspects 51 to 68, wherein component c) comprises at least one hydroxyl-functionalized photoinitiator according to formula (XXe) as defined in Aspect 62, and the hydroxyl-functionalized photoinitiator preferably bears a number of photoinitiator moieties PI¹ that is equal to the number of OH groups.
Aspect 70. The process according to any one of Aspects 51 to 69, wherein component c) comprises at least one hydroxyl-functionalized photoinitiator according to formula (XXIa): 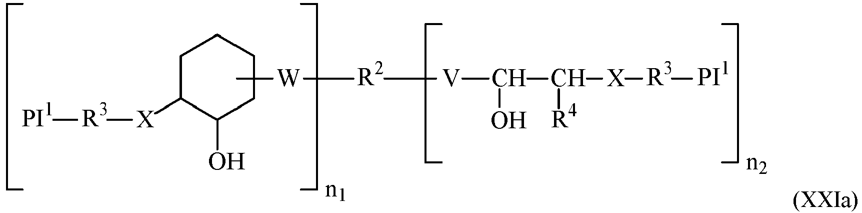 wherein n₁, n₂, PI¹, V, W, X, R², R³ and R⁴ are as defined in Aspect 25.
Aspect 71. The process according to any one of Aspects 51 to 70, wherein component c) comprises at least one hydroxyl-functionalized photoinitiator according to one of the following formulae (XXIIa) to (XXXVa): 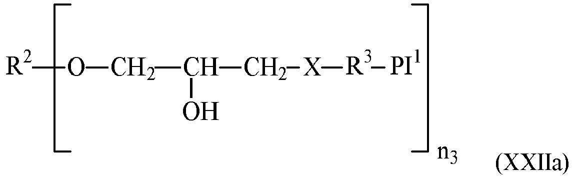 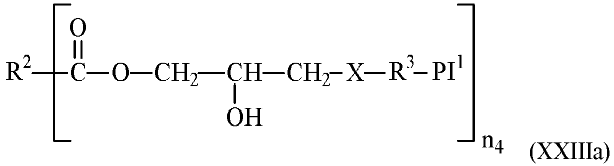 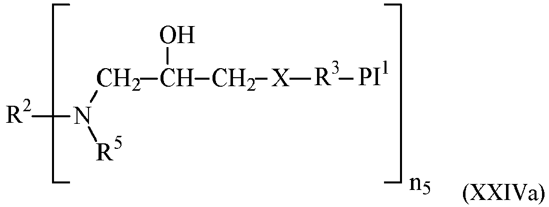 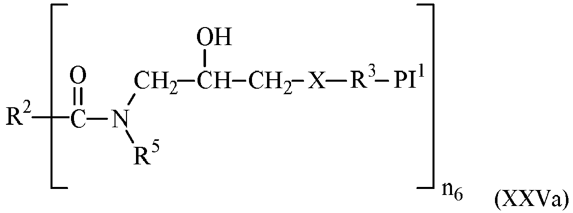 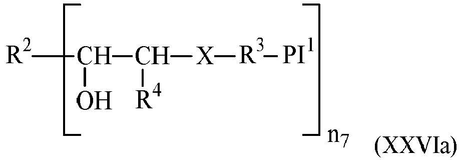 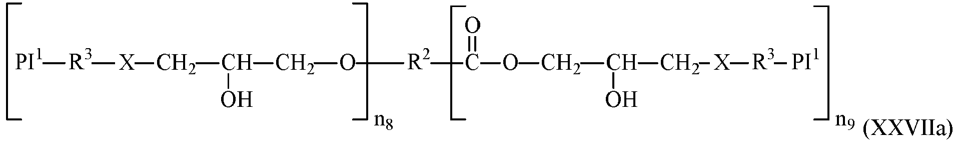 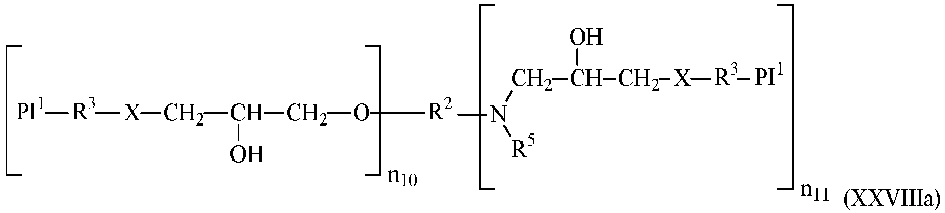 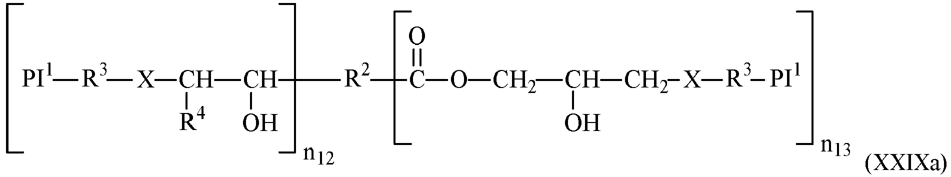 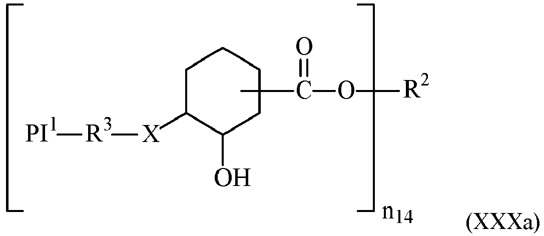 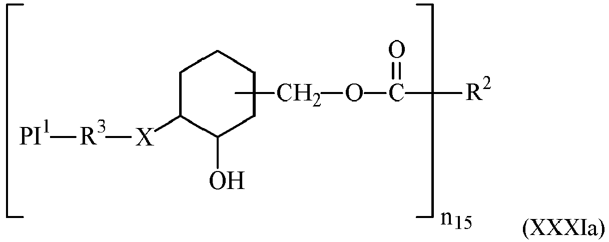 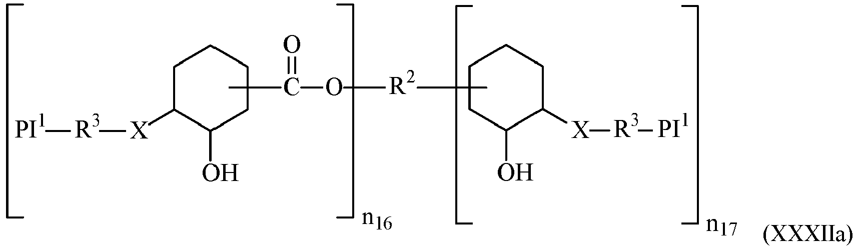 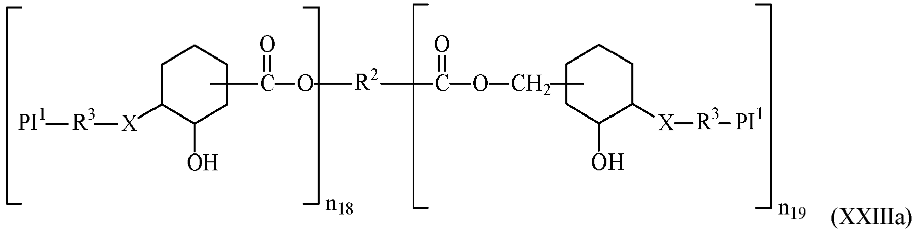 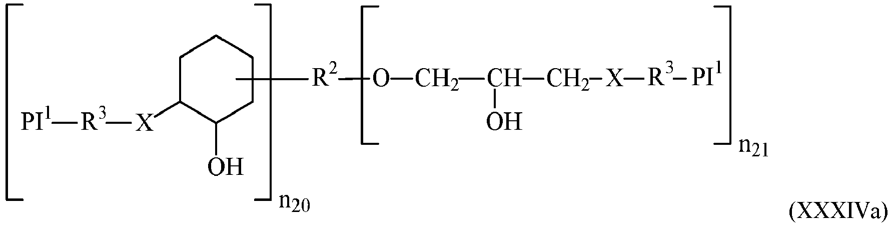 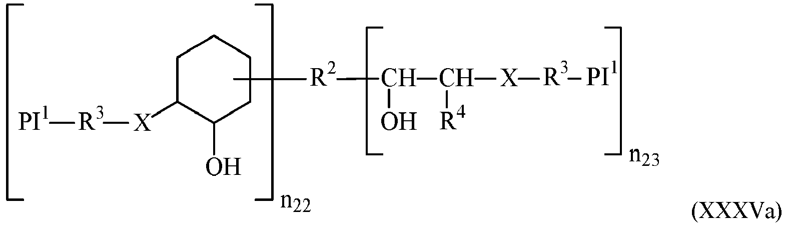
   wherein n₃, n₄, n₅, n₆, n₇, n₈, n₉, n₁₀, n₁₁, n₁₂, n₁₃, n₁₄, n₁₅, n₁₆, n₁₇, n₁₈, n₁₉, n₂₀, n₂₁, n₂₂, n₂₃, PI¹, X, R², R³ and R⁴ are as defined in any one of Aspects 26 to 29;
   each R⁵ is independently H, alkyl, aryl or a group according to the following formula: 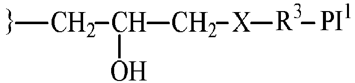 wherein X, R³ and PI¹ are as defined above and the symbol } represents the point of attachment to the nitrogen atom.
Aspect 72. The process according to any one of Aspects 51 to 71, wherein component c) comprises at least one hydroxyl-functionalized photoinitiator according to one of the following formulae (XXIIa), (XXIIIa), (XXIVa) or (XXVIa): 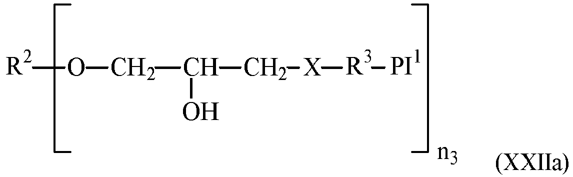 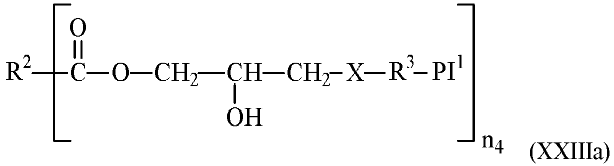 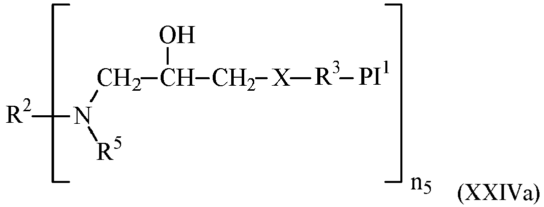 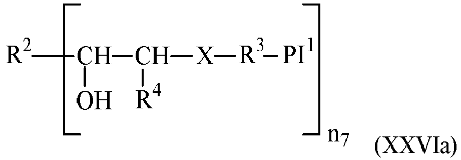 wherein
   n₃, n₄, n₅, n₇, PI¹, X, R², R³, R⁴ are as defined in Aspect 30;
   each R⁵ is independently H, alkyl or a group according to the following formula: 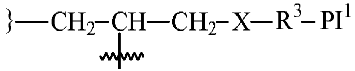 wherein X, R³ and PI¹ are as defined above and the symbol } represents the point of attachment to the nitrogen atom.
Aspect 73. The process according to any one of Aspects 51 to 72, wherein component c) comprises at least one hydroxyl-functionalized photoinitiator according to one of the following formulae (XXe1)-(XXe9): 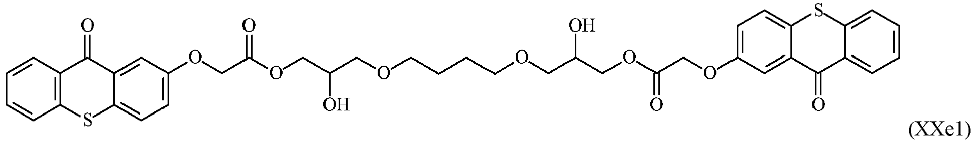 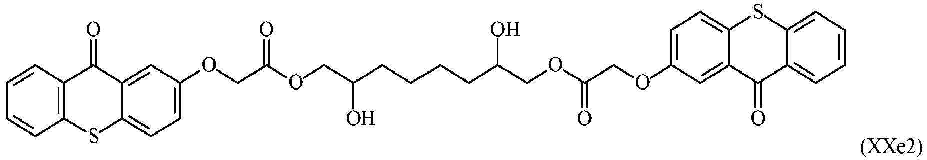 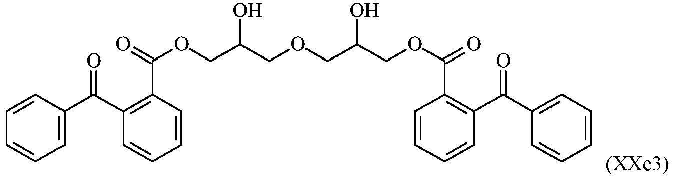 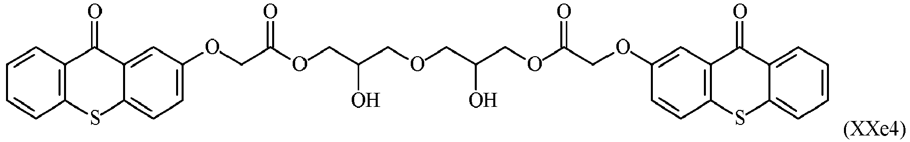 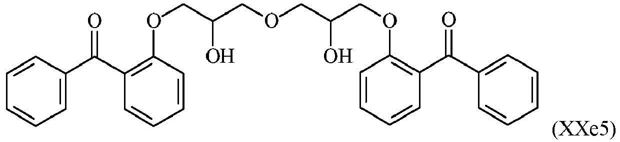 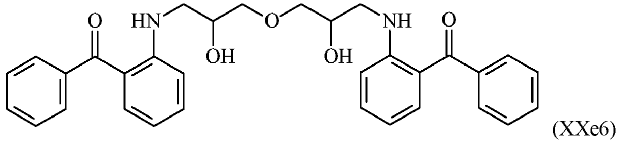 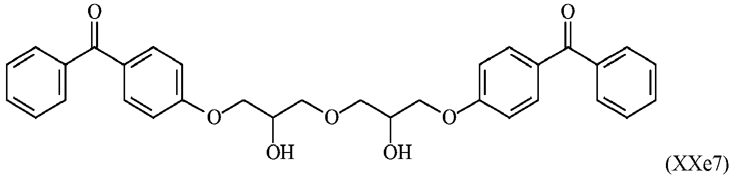 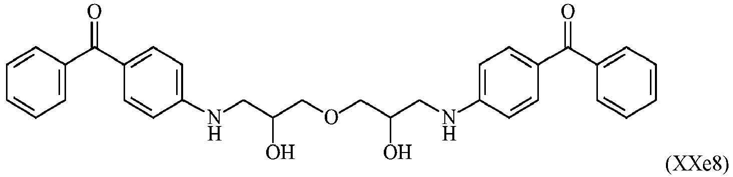 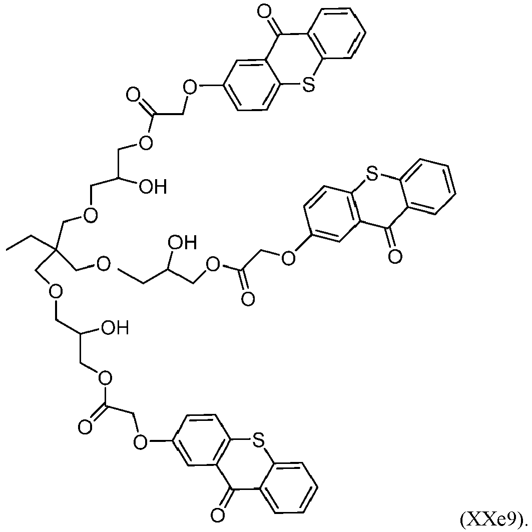
Aspect 74. The process according to any one of Aspects 51 to 73, wherein component c) comprises at least one hydroxyl-functionalized photoinitiator according to one of the following formulae (XXXVIa) to (XXXXIa): 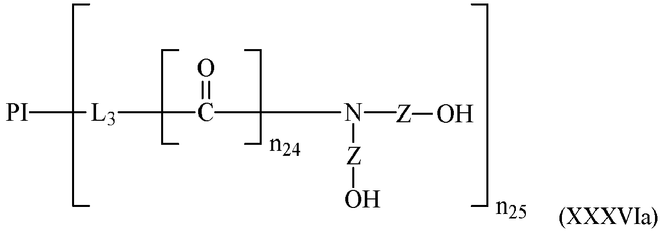 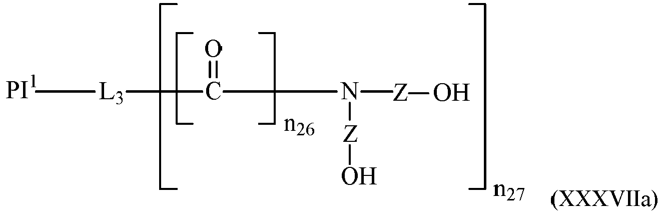 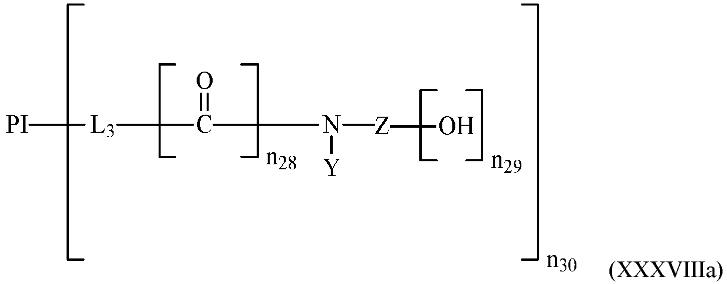 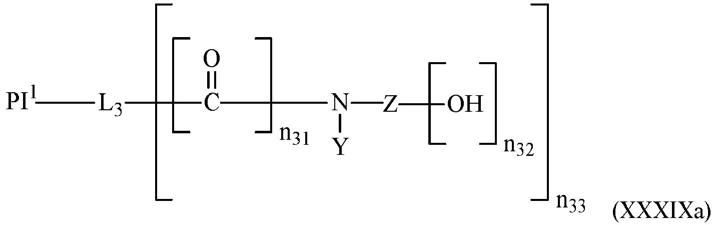 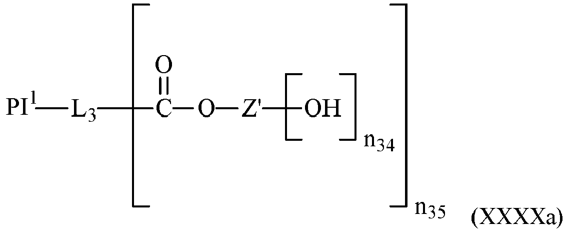 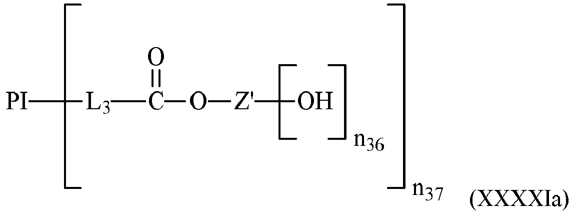 wherein PI, PI¹, L₃, Y, Z, Z', n₂₄, n₂₅, n₂₆, n₂₇, n₂₈, n₂₉, n₃₀, n₃₁, n₃₂, n₃₃, n₃₄, n₃₅, n₃₆ and n₃₇ are as defined in any one of Aspects 32 to 36.
Aspect 75. The process according to any one of Aspects 51 to 74, wherein component c) comprises at least one hydroxyl-functionalized photoinitiator according to one of the following formulae (XXXXIIa) to (XXXXIVa): 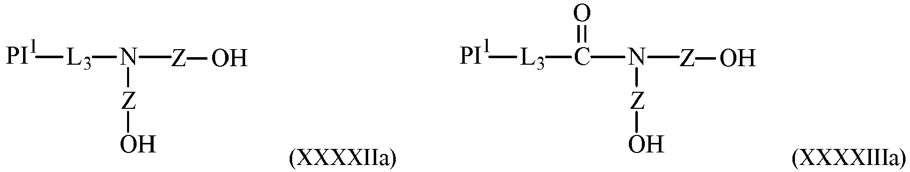 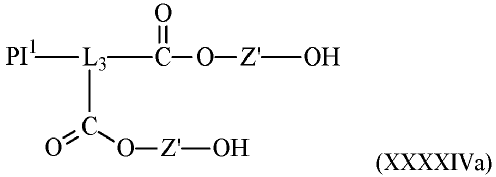 wherein PI¹, L₃ Z and Z' are as defined in Aspect 37.
Aspect 76. The process according to any one of Aspects 51 to 75, wherein component c) comprises at least one hydroxyl-functionalized photoinitiator according to one of the following formulae (XXe10)-(XXe26): 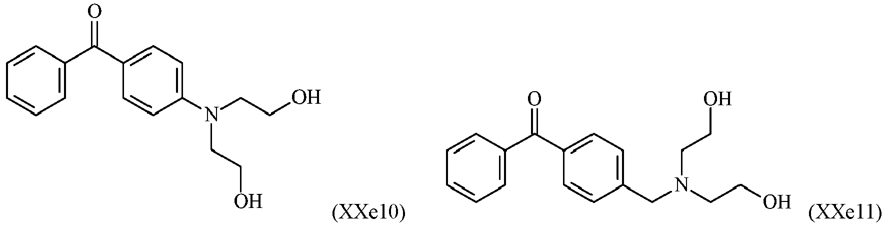 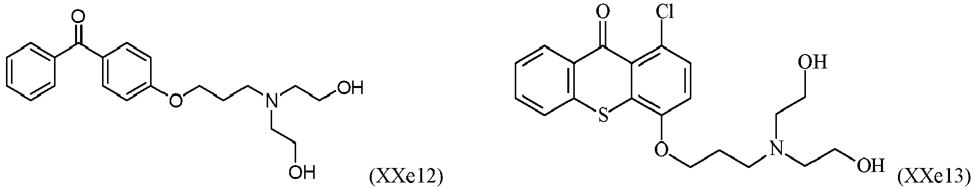 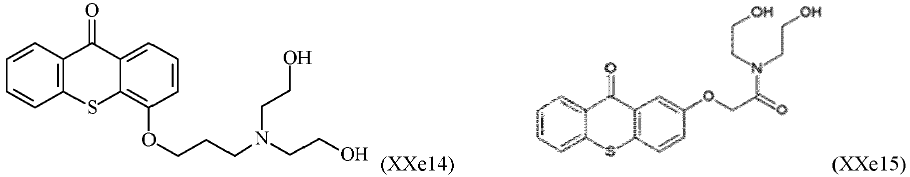 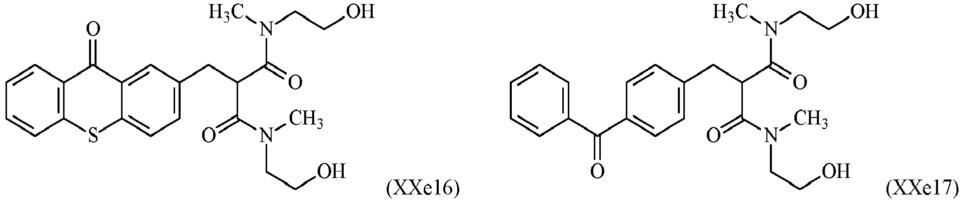 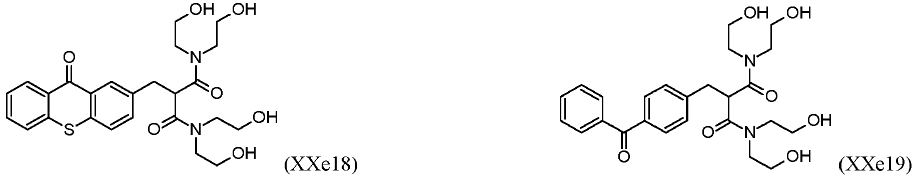 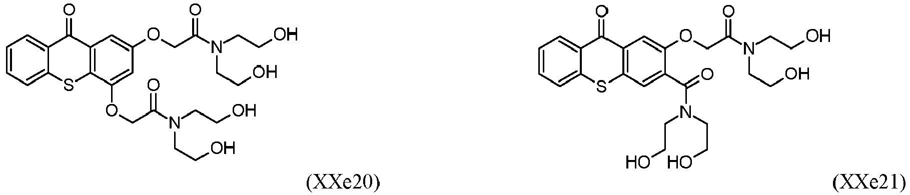 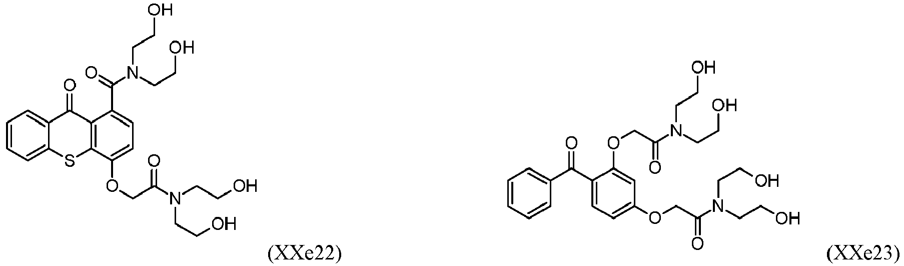 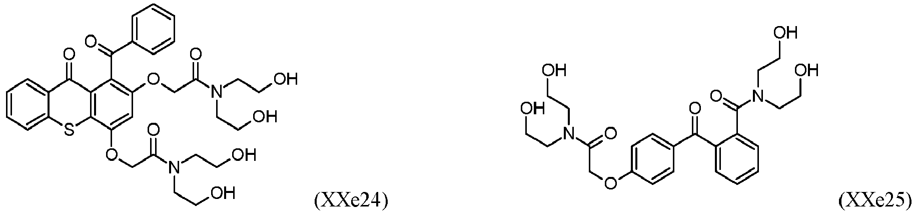 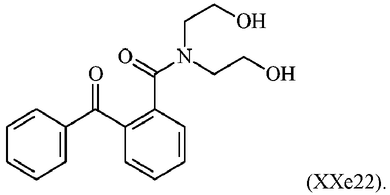
Aspect 77. The process according to any one of Aspects 51 to 76, wherein the total amount of component c) used to prepare the self-crosslinkable urethane (meth)acrylate is from 0.5 to 20%, in particular from 1 to 15%, more particularly from 1.5 to 10%, by weight of component c) based on the total amount of components a), b), c) and d).
Aspect 78. The process according to any one of Aspects 51 to 77, wherein component d) comprises at least one polyol selected from a polymeric polyol, a non-polymeric polyol or an amino-functional polyol.
Aspect 79. The process according to any one of Aspects 51 to 78, wherein component d) comprises at least one polymeric diol, in particular at least one polyether diol or at least one polyester diol, more particularly at least one polyether diol selected from a polyethylene glycol, a poly(1,2-propylene glycol), a poly(1,3-propylene glycol), a poly(1,4-butylene glycol) and combinations thereof, or at least one polyester diol selected from a poly(caprolactone) diol, a poly(lactide) diol, a poly(alkylene glycol adipate) diol and a poly(alkylene glycol succinate) diol.
Aspect 80. The process according to any one of Aspects 51 to 79, wherein component d) comprises at least one non-polymeric diol, in particular at least one non-polymeric aliphatic diol, more particularly at least one non-polymeric aliphatic diol selected from ethylene glycol, di-, tri-or tetraethylene glycol, 1,2- or 1,3-propylene glycol, di-, tri- or tetra(1,2-propylene glycol), di-, tri- or tetra(1,3-propylene glycol), 1,2-, 1,3- or 1,4-butylene glycol, di-, tri- or tetra(1,4-butylene glycol), 1,5-pentanediol, 1,6-hexanediol, 1,7-heptanediol, 1,8-octanediol, 1,9-nonanediol, 1,10-decanediol, 1,12-dodecanediol, 2-methyl-1,3-propanediol, 2,2-diethyl-1,3-propanediol, 3-methyl-1,5-pentanediol, 3,3-dimethyl-1,5-pentanediol, 2,4-diethyl-1,5-pentanediol, 3,3-butylethyl-1,5-pentane diol, cyclohexanediol, cyclohexane-1,4-dimethanol, norbomene dimethanol, norbornane dimethanol, tricyclodecanediol, tricyclodecane dimethanol, hydrogenated bisphenol A, B, F or S, a dianhydrohexitol (i.e. isosorbide, isomannide, isoidide), a hydrogenated dimer fatty acid, as well as the alkoxylated (i.e. ethoxylated and/or propoxylated) derivatives thereof.
Aspect 81. The process according to any one of Aspects 51 to 80, wherein component d) comprises at least one amino-functional polyol, in particular at least one amino-functional polyol selected from triethanol amine, N-methyldiethanol amine, N-ethyldiethanol amine, N-propyl diethanolamine, N-butyl diethanol amine, N-t-butyl diethanolamine, trimethanol amine, N-methyl dimethanol amine, 3-(dimethylamino)-1,2-propanediol, 3-(diethylamino)-1,2-propanediol, 3-(dipropylamino)-1,2-propanediol, 2-(dimethylamino)propane-1,3-diol, bis(2-hydroxyethyl)dodecylamine, bis(2-hydroxyethyl)octadecylamine, N,N-dioctadecyl-N',N'-bis(2-hydroxyethyl)-1,3-diaminopropane, 3-morpholino-1,2-propanediol, 3-piperidino-1,2-propanediol, 3-pyrrolidino-1-yl-1,2- propanediol, and an aminobenzamide diol according to the following formula (XXXXVI): 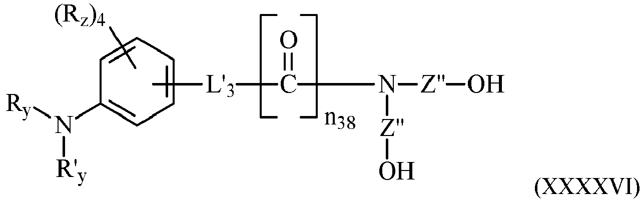 wherein R_{y}, R'_{y}, R_{z}, L'₃, Z" and n₃₈ are as defined in Aspect 43.
Aspect 82. The process according to any one of Aspects 51 to 81, wherein the total amount of component d) used to prepare the self-crosslinkable urethane (meth)acrylate is from 0 to 84.5%, in particular from 0.1 to 75%, more particularly from 1 to 65%, by weight of component d) based on the total amount of components a), b), c) and d).
Aspect 83. The process according to any one of Aspects 51 to 82, wherein components a), b), c) and d) do not comprise a dye moiety.
Aspect 84. A process for polymerizing one or more ethylenically unsaturated compounds, wherein the process comprises contacting one or more ethylenically unsaturated compounds with the self-crosslinkable urethane (meth)acrylate according to any one of Aspects 1 to 50 or prepared with the process according to any one of Aspects 51 to 83, and irradiating the mixture, in particular with UV, near-UV, visible, infrared, near-infrared and/or electron beam radiation.
Aspect 85. The process according to Aspect 84, wherein the polymerization process does not involve the use of a radical initiator or initiating system other than the self-crosslinkable urethane (meth)acrylate according to any one of Aspects 1 to 50 or prepared with the process according to any one of Aspects 51 to 83.
Aspect 86. A curable composition comprising:
   A) a self-crosslinkable urethane (meth)acrylate according to any one of Aspects 1 to 50 or prepared with the process according to any one of Aspects 51 to 83; and
   optionally B) a polymerizable component other than A).
Aspect 87. The curable composition according to Aspect 86, wherein the curable composition comprises from 1 to 100%, from 5 to 95%, from 5 to 90%, from 10 to 85%, from 10 to 80%, from 15 to 75%, from 15 to 70%, from 20 to 65% or from 20 to 60% by weight of component A) based on weight of the curable composition.
Aspect 88. The curable composition according to Aspect 86 or 87, wherein the curable composition comprises 0 to 99%, from 5 to 95%, from 10 to 95%, from 15 to 90%, from 20 to 90%, from 25 to 85%, from 30 to 85%, from 35 to 80% or from 40 to 80%, by weight of component B) based on weight of the curable composition.
Aspect 89. A curable composition according to any one of Aspects 86 to 88, wherein component B) comprises at least one (meth)acrylate-functionalized monomer, more preferably at least one poly(meth)acrylate-functionalized monomer.
Aspect 90. A curable composition according to any one of Aspects 86 to 89, wherein component B) comprises at least one poly(meth)acrylate-functionalized monomer selected from glycerol tri(meth)acrylate; diglycerol tetra(meth)acrylate, triglycerol penta(meth)acrylate, tetraglycerol hexa(meth)acrylate, trimethylolethane tri(meth)acrylate; trimethylolpropane tri(meth)acrylate; pentaerythritol tetra(meth)acrylate, di(trimethylolpropane) tetraacrylate, sorbitol penta(meth)acrylate; di(pentaerythritol) hexa(meth)acrylate; tris (2-hydroxyethyl) isocyanurate tri(meth)acrylate, as well as the alkoxylated (e.g., ethoxylated and/or propoxylated) derivatives thereof, and combinations thereof.
Aspect 91. A curable composition according to any one of Aspects 86 to 90, wherein component B) comprises a urethane (meth)acrylate monomer, in particular a urethane (meth)acrylate monomer according to the following formula (XXXXVII): 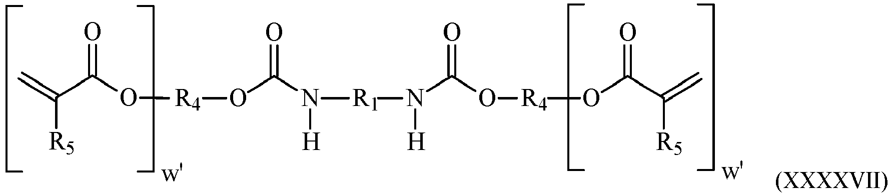 wherein
   R₄, R₅ and w' are as defined in any one of Aspects 3 to 5;
   R₁ is as defined in Aspect 8 or 9.
Aspect 92. The curable composition according to any one of Aspects 86 to 91, wherein component B) comprises 0 to 100%, in particular 5 to 90%, more particularly 10 to 80%, even more particularly 15 to 75%, more particularly still 20 to 70% by weight of (meth)acrylate-functionalized monomer based on the total weight of the component B).
Aspect 93. The curable composition according to any one of Aspects 86 to 92, wherein the curable composition is substantially free of an initiator or initiating system other than component A).
Aspect 94. The curable composition according to any one of Aspects 86 to 73, wherein the curable composition is substantially free of a dye.
Aspect 95. A process for the preparation of a cured product, comprising curing the curable composition according to any one of Aspects 86 to 94, preferably by exposing the curable composition to actinic radiation such as UV, near-UV, visible, infrared near-infrared and/or electron beam radiation.
Aspect 96. A substrate on which the curable composition of any one of Aspects 86 to 94 has been applied and cured, in particular the substrate is a food and beverage packaging, a pharmaceutical packaging, a textile, a nail, a tooth, a medical device, a food and beverage processing equipment, a water pipe or a toy.
Aspect 97. A use of the self-crosslinkable urethane (meth)acrylate according to any one of Aspects 1 to 50 or prepared with the process according to any one of Aspects 51 to 83, to obtain a cured product having a reduced amount of extractables.

The invention will be illustrated in more detail with reference to the following Examples, but it should be understood that the invention is not deemed to be limited thereto.

### EXAMPLES

### Methods:

PhotoDSC: PhotoDSC was performed using a TA Instruments Q800 DSC equipped with photo accessory. Samples were irradiated with a Hg-arc based light output via a liquid silica glass optical light guide. Light intensity at the samples was approximately 290 mW/cm² based on measurements made with an EIT Power Puck II. Samples were irradiated until the polymerization exotherm was complete, typically 3-4 minutes. Time from initial irradiance to peak exotherm rate was measured and reported as the onset-to-peak time. Polymerization enthalpy was measured as the area under the total polymerization curve. Samples were UV cured under nitrogen flow and under air to observe the effects of the atmosphere on polymerization behavior.

NCO titration: Isocyanate titrations were performed colorimetrically by reacting the resin with excess amine in the form of a 0.2N toluene solution of dibutyl amine. Isopropyl alcohol and bromocresol green indicator are then added and the excess amine is back titrated with 0.1N HCl to a yellow endpoint.

### Example 1: Self-crosslinkable urethane (meth)acrylate according to the invention

The self-crosslinkable urethane (meth)acrylate of Example 1 was prepared as follows using the amounts of reagents shown in Table 1 below. Caprolactone acrylate (SR495B, Sartomer Americas) was combined a hydroxyl-functionalized photoinitiator of formula (XXe12) as shown below (available as SpeedCure^{®} 9001 from Arkema) in a 500 mL brown bottle and placed in an 80°C oven until all of the photoinitiator dissolved. To this solution was added butylated hydroxytoluene as antioxidant (BHT), dibutyltindilaurate catalyst (DBTDL) and isophorone diisocyanate (IPDI) in amounts according to Table 1. The reaction was mixed well and allowed to exotherm slightly, after which it was placed in an 80°C oven to complete the reaction. The isocyanate value (NCO wt. %) was tested by titration until it stalled at around 3.6 weight %. Additional SR495B was added to the reactor (part 2) and the reaction mixture was again placed in the 80°C oven for several hours at which point the NCO value had reached less than 0.1 wt%.

**Table 1: Example 1 reagents loading**

| **Component** | **MW** | **Grams** | **Mole** |
|---|---|---|---|
| (1) SR495B, part 1 | 337.4 | 102.6 | 0.304 |
| (2) Photoinitiator (XXe12)^{∗} | 343 | 3.9 | 0.011 |
| (3) BHT (Aldrich) | | 0.19 | |
| (4) IPDI (Covestro) | 222.29 | 51.6 | 0.232 |
| (5) DBTDL | | 0.11 | |
| (6) SR495B, part 2 | | 43.5 | 0.129 |
| Total SR495B amount | | 146.1 | 0.433 |
| Total | | 201.9 | |

| | | | |
|---|---|---|---|
| ^{∗} Photoinitiator (XXe12) corresponds to the following formula: | | | |

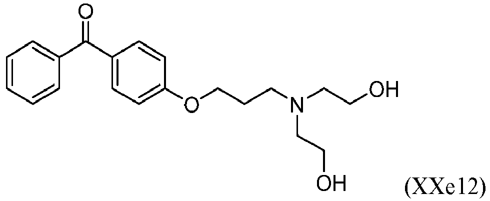

### Example 2: Self-crosslinkable urethane (meth)acrylate according to the invention

The self-crosslinkable urethane (meth)acrylate of Example 2 was prepared in the same way as Example 1, except that dicyclohexylmethane diisocyanate (Desmodur W, Covestro) was used as the diisocyanate component and the amounts of the reagents were adjusted as shown below in Table 2.

**Table 2: Example 2 reagents loading**

| **Component** | **MW** | **Grams** | **Mole** |
|---|---|---|---|
| (1) SR495B, part 1 | 337.4 | 104.5 | 0.31 |
| (2) Photoinitiator (XXe12) | 343 | 8.6 | 0.025 |
| (3) BHT (Aldrich) | | 0.19 | |
| (4) Desmodur W (Covestro) | 262 | 66.3 | 0.253 |
| (5) DBTDL | | 0.11 | |
| (6) SR495B, part 2 | | 43.72 | 0.13 |
| Total SR495B amount | | 148.22 | 0.44 |
| Total | | 223.42 | |

### Example 3: Self-crosslinkable urethane (meth)acrylate according to the invention

The self-crosslinkable urethane (meth)acrylate of Example 3 was prepared in the same way as Example 1, except that dicyclohexylmethane diisocyanate (Desmodur W, Covestro) was used as the diisocyanate component and the amounts of the reagents were adjusted as shown below in Table 3.

**Table 3: Example 3 reagents loading**

| **Component** | **MW** | **Grams** | **Mole** |
|---|---|---|---|
| (1) SR495B, part 1 | 337.4 | 102.7 | 0.304 |
| (2) Photoinitiator (XXe12) | 343 | 3.6 | 0.01 |
| (3) BHT (Aldrich) | | 0.19 | |
| (4) Desmodur W (Covestro) | 262 | 53.8 | 0.205 |
| (5) DBTDL | | 0.11 | |
| (6) SR495B, part 2 | | 26.27 | 0.078 |
| Total SR495B amount | | 128.97 | 0.382 |
| Total | | 186.67 | |

The reaction scheme for Example 3 is shown below:

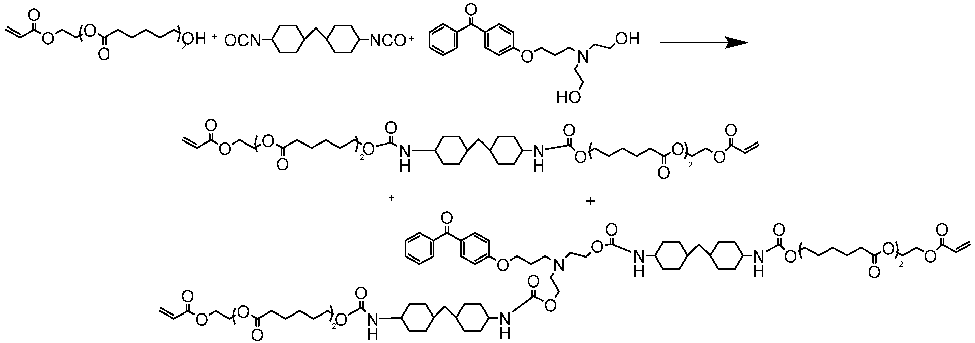

### Example 4: Self-crosslinkable urethane (meth)acrylate according to the invention

The self-crosslinkable urethane (meth)acrylate of Example 4 was prepared in the same way as Example 1, except that the amounts of the reagents were adjusted as shown below in Table 4.

**Table 4: Example 4 reagents loading**

| **Component** | **MW** | **Grams** | **Mole** |
|---|---|---|---|
| (1) SR495B, part 1 | 337.4 | 110.3 | 0.327 |
| (2) Photoinitiator (XXe12) | 343 | 7.8 | 0.0227 |
| (3) BHT (Aldrich) | | 0.19 | |
| (4) IPDI (Covestro) | 222.29 | 51.6 | 0.232 |
| (5) DBTDL | | 0.11 | |
| (6) SR495B, part 2 | | 28.18 | 0.0835 |
| Total SR495B amount | | 138.48 | 0.4105 |
| Total | | 198.18 | |

### Example 5: Self-crosslinkable urethane (meth)acrylate according to the invention

A hydroxyl-functionalized photoinitiator of formula (XXe 1) was prepared by the reaction of 2-carboxymethylthioxanthone with 1,4-butanediol diglycidyl ether. More details of preparation of this chromophore bearing component are disclosed in patent application EP23305849.4 filed on May 30, 2023, the entire contents of which is incorporated herein for all purposes.

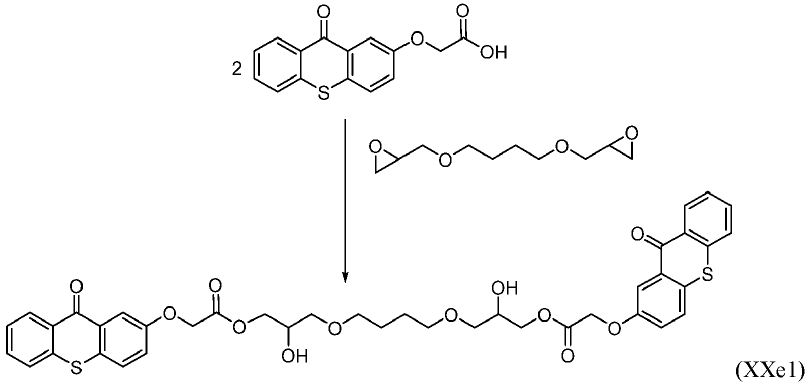

First, the hydroxyl-functionalized photoinitiator of formula (XXe1) was prepared by mixing 2-carboxymethylthioxanthone (CMTX from Arkema, 31.8 gram, 0.1054 mole); 1,4-butanediol diglycidyl ether (GE-21 from CVC, 15 gram, 0.1289 mole); benzyltriethylammonium chloride (BTEAC from Aldrich, 0.14g, 0.3 wt%) as catalyst and 50 wt% cyclopentanone as process solvent in a three-neck flask equipped with mechanical stirring under nitrogen, and heating to 125°C. The reaction was then run at 125°C until shut down criteria were reached at acid value (AV) < 2 mg KOH/gm and epoxy value (EV) < 2 mg KOH/gm were attained after about after 3 hours. The AV or EV was adjusted by adding more 2-carboxymethylthioxanthone or 1,4-butanediol diglycidyl ether as the reaction proceeded as needed. The hydroxyl-functionalized photoinitiator of formula (XXe1) was a liquid and exhibited acceptable FT-IR and ¹H NMR spectral features.

The self-crosslinkable urethane (meth)acrylate of Example 5 was then prepared as follows using the amounts of reagents shown in Table 5 below. SR495B (part 1), the photoinitiator of formula (XXe1) pre-dissolved in cyclopentanone, BHT and Desmodur W were combined into a ½ liter resin kettle equipped with air sparge, agitator, thermocouple, temperature controller, heating mantle, side arm and condenser. This mixture was agitated for 10 minutes, and then DBTDL was added. The exotherm was allowed to reach 75°C, and heat was applied if needed. The reaction mixture was held at 75°C for 1 hour. The intermediate isocyanate (NCO) level was checked until it reached 4.5 wt% (4.4 - 4.8 wt%) or it stalled. SR495B (part 2) was added. The mixture was agitated at 85°C until NCO wt. % was below 0.1 wt. %. SR495B (part 3) and/or (part 4) were added if needed to achieve the desired NCO value of less than 0.1 wt% of NCO.

**Table 5: Example 5 reagents loading**

| **Component** | **MW** | **Grams** | **Mole** |
|---|---|---|---|
| (1) SR495B, part 1 | 337.4 | 98.42 | 0.2917 |
| (2) Photoinitiator (XXe1)^{∗} (50 wt. % in cyclopentanone) | 813 | 18.2 | 0.0112 |
| (3) BHT (Aldrich) | | 0.19 | |
| (4) Desmodur W (Covestro) | 262 | 66.1 | 0.2523 |
| (5) DBTDL | | 0.15 | |
| (6) SR495B, part 2 | | 59.52 | 0.1764 |
| (7) SR495B, part 3 | | 8.54 | 0.0253 |
| (8) SR495B, part 4 | | 2.09 | 0.0062 |
| Total SR495B amount | | 168.57 | 0.4996 |
| Total | | 253.21 | |

| | | | |
|---|---|---|---|
| * Photoinitiator (XXe1) with 50 wt% cyclopentanone and has a hydroxyl value of 69.1 mg KOH/gm by titration. | | | |

The reaction scheme for Example 5 is shown below.

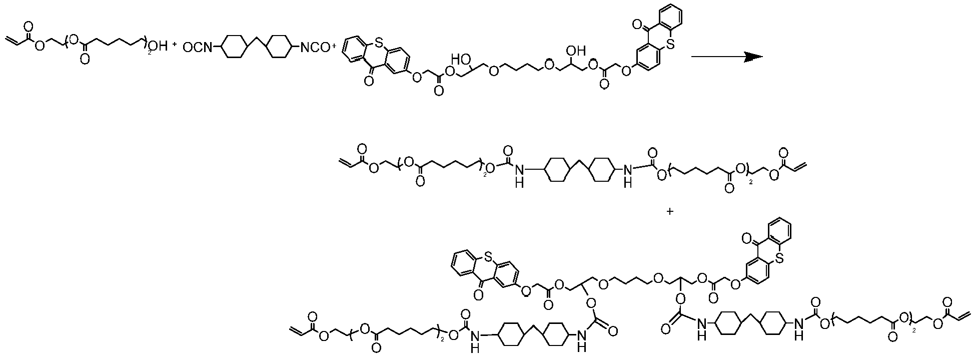

### Example 6: Self-crosslinkable urethane (meth)acrylate according to the invention

The self-crosslinkable urethane (meth)acrylate of Example 6 was prepared according to the procedure of Example 5, except that the diisocyanate was isophorone diisocyanate (IPDI, Wanhua) and the amounts of the reagents were adjusted as shown below in Table 6.

**Table 6: Example 6 reagent loading**

| **Component** | **MW** | **Grams** | **Mole** |
|---|---|---|---|
| (1) SR495B, part 1 | 337.4 | 115.09 | 0.3411 |
| (2) Photoinitiator (XXe1)^{∗} (50 wt. % in cyclopentanone) | 813 | 16.2 | 0.01 |
| (3) BHT (Aldrich) | | 0.2 | |
| (4) IPDI (Wanhua) | 222.29 | 51.6 | 0.2321 |
| (5) DBTDL | | 0.16 | |
| (6) SR495B, part 2 | | 35.36 | 0.1048 |
| (7) SR495B, part 3 | | 2.09 | 0.0062 |
| Total SR495B amount | | 152.54 | 0.4521 |
| Total | | 220.7 | |

| | | | |
|---|---|---|---|
| * Photoinitiator (XXe1) with 50 wt% cyclopentanone and has a hydroxyl value of 69.1 mg KOH/gm by titration. | | | |

### Example 7: Self-crosslinkable urethane (meth)acrylate according to the invention

The self-crosslinkable urethane (meth)acrylate of Example 7 was prepared as follows using the amounts of reagents shown in Table 7 below. Irganox^{®} 1035 antioxidant, BHT antioxidant, DBTDL catalyst and IPDI were combined into a 0.5 liter resin kettle equipped with air sparge, agitator, thermocouple, temperature controller, heating mantle, side arm and condenser. 2-hydroxyethyl acrylate (HEA) was fed into the resin kettle through an addition funnel over the course of 2 hours. The reaction mixture was allowed to exotherm to 60°C. The mixture was held at 60°C for 30 minutes, and then the NCO wt. % was checked to confirm that it was about 8.5 to 8.9 wt. % (8.7 wt. %). Then the hydroxyl-functionalized photoinitiator (XXe1) and melted poly(neopentyl glycol adipate) having MW of 500 g/mol (Fomrez^{®} 55-225 - polyester diol from Chemtura) were premixed at a 1:1 ratio by weight. The premix was added to the reactor over the course of several minutes, and the mixture was allowed to exotherm to 80°C. Heat was applied as needed to maintain a reaction temperature of approximately 80°C. The rest of the melted Fomrez^{®} 55-225 was added in three increments at 15 minute intervals. The reaction mixture was allowed to reach 90°C, applying heat as needed. The reaction mixture was held until the NCO weight % was less than 0.06.

**Table 7: Example 7 reagent loading**

| **Component** | **MW** | **Grams** | **Mole** |
|---|---|---|---|
| (1) IPDI (Evonik) | 222.29 | 129.0 | 0.5803 |
| (2) Irganox 1035 | | 0.35 | |
| (3) BHT (Aldrich) | | 0.14 | |
| (4) DBTDL | | 0.32 | |
| (5) HEA (Nippon Shokubai) | | 81.77 | 0.7042 |
| (6) Photoinitiator (XXe1)^{∗} (50 wt. % in cyclopentanone) | 819 | 27.88 | 0.017 |
| (7) Fomrez^{®} 55-225 | 500 | 112.84 | 0.2257 |
| | | | |
| Total | | 352.3 | |

| | | | |
|---|---|---|---|
| * Photoinitiator (XXe1) with 50 wt% cyclopentanone and has a hydroxyl value of 69.1 mg KOH/gm by titration. | | | |

### Example 8: Self-crosslinkable urethane (meth)acrylate according to the invention

The self-crosslinkable urethane (meth)acrylate of Example 8 was prepared as follows using the amounts of reagents shown in Table 8 below. Triphenyl antimony (TPA), methylhydroquinone (MeHQ), DBTDL(catalyst) and IPDI were combined in a ½ liter resin kettle equipped with air sparge, agitator, thermocouple, temperature controller, heating mantle, side arm and condenser. 2-hydroxyethyl acrylate (HEA) was fed into the reaction mixture through an addition funnel over the course of 2 hours. The reaction mixture was allowed to exotherm to 60°C. The reaction mixture was held at 60°C for 30 minutes. The weight % of NCO was checked by titration to confirm is about 12.4% (desired range 12.0 - 12.8%). Next, the hydroxyl-functionalized photoinitiator (XXe1) and pre-melted poly(tetrahydrofuran) (PolyTHF 650 - polyether diol from BASF) at a ratio of 1:2 by weight were pre-mixed and added to the reactor. The reaction was allowed to exotherm to 75°C, and then heat was applied as needed. The rest of the PolyTHF 650 was added in 3 increments, at 15 minute intervals. The mixture was allowed to exotherm to reach 80°C, and heat was applied if needed. The reaction mixture was held at 80°C until NCO wt. % was confirmed to be less than 0.06 wt%.

**Table 8: Example 8 reagent loading**

| **Component** | **MW** | **Grams** | **Mole** |
|---|---|---|---|
| (1) IPDI (Evonik) | 222.29 | 102.3 | 0.4602 |
| (2) TPA | | 0.31 | |
| (3) MeHQ (Aldrich) | | 0.06 | |
| (4) DBTDL | | 0.14 | |
| (5) HEA (Nippon Shokubai) | | 53.5 | 0.4607 |
| (6) Photoinitiator (XXe1) (50 wt. % in cyclopentanone) | 813 | 26 | 0.016 |
| (7) PolvTHF650 | 650 | 143.4 | 0.2206 |
| (8) DBTDL | | 0.14 | |
| (9) MeHQ (Aldrich) | | 0.06 | |
| | | | |
| Total | | 325.91 | |

| | | | |
|---|---|---|---|
| * Photoinitiator (XXe1) with 50 wt% cyclopentanone and has a hydroxyl value of 69.1 mg KOH/gm by titration. | | | |

### Example 9: UV cured gel content study of the self-crosslinkable urethane (meth)acrylates of Examples 5-8

The self-crosslinkable urethane (meth)acrylates of Examples 5-8 were coated onto an aluminum substrate using a drawdown bar to produce nominally 50 µm thick films. No discrete photoinitiator was added to the coated film material. The films were UV cured using either a Fusion H bulb (300 W/in, dose∼1JUVA/cm²) or a Phoseon 8W/cm² 395 nm LED. Films were removed from the substrate, weighed, and soaked in THF for >24 h. The remaining solid was isolated, dried at 65°C/4h, and re-weighed to calculate gel content.

All the self-crosslinkable urethane (meth)acrylates exhibited high inherent reactivity as judged by the high gel contents tabulated in Table 9 below. The high gel contents using the 395 nm LED exemplify the excellent activity of the resins at the longer wavelengths typical of common industrial LED light sources. This study exemplifies that the self-crosslinkable urethane (meth)acrylates of the invention can efficiently serve the role of both photoinitiator and curable oligomer. The examples also show that the hydrogen donor functionality needed for Type II photoinitiation can be supplied by the resin backbone itself, and added amine synergists or other H-donors are not required to obtain efficient cure.

**Table 9: Gel contents of the self-crosslinkable urethane (meth)acrylates of Examples 5-8, neat.**

| **Sample #** | **Gel Content H Bulb (% gel)** | **Gel Content 395 nm LED (% gel)** |
|---|---|---|
| Example 5 | 73 | 84 |
| Example 6 | 67 | 75 |
| Example 7 | 80 | 76 |
| Example 8 | 93 | 93 |

### Example 10: Curable compositions comprising the self-crosslinkable urethane (meth)acrylate of Examples 5-8

The self-crosslinkable urethane (meth)acrylates of Examples 5-8 were blended in 1:1 weight ratio with SR355 (di(trimethylolpropane) tetraacrylate, Arkema). No additional discrete photoinitiator was added to the formulation. These 100% solids liquid formulations containing the self-crosslinkable urethane (meth)acrylates of Examples 5-8 were coated onto an aluminum substrate using a drawdown bar to produce nominally 50 µm thick films. The films were UV cured using either a Fusion H bulb (300 W/in, dose~1 J of UVA/cm²) or a Phoseon 8W/cm² 395 nm LED. The cured films were removed from the substrate, weighed, and soaked in THF for at least 24 hours to dissolve any non-crosslinked material. The remaining solid was isolated, dried at 65°C for four hours, and re-weighed to calculate gel content.

All the curable compositions which included the self-crosslinkable urethane (meth)acrylates of the invention exhibited high reactivity as shown by the high gel contents tabulated in Table 10 below. The high gel contents using the 395 nm LED exemplify the excellent activity of the resins at the longer wavelengths typical of common industrial LED light sources. This gel content study demonstrated that the self-crosslinkable urethane (meth)acrylates of the invention can efficiently serve as photoinitiators for other radically curable materials in addition to being inherently curable themselves. As noted in Example 9, the efficient cure obtained shows that no additional H-donor or synergist is needed to obtain excellent UV curability when the self-crosslinkable urethane (meth)acrylates of the invention are utilized.

**Table 10: Gel contents of the self-crosslinkable urethane (meth)acrylates of Example 5-8 in combination with SR355 in 1:1 weight ratio.**

| **Sample #** | **Gel Content H Bulb (% gel)** | **Gel Content 395 nm LED (% gel)** |
|---|---|---|
| Example 5 + SR355 | 77 | 92 |
| Example 6 + SR355 | 79 | 94 |
| Example 7 + SR355 | 92 | 92 |
| Example 8 + SR355 | 98 | 98 |

### Example 11: Curing kinetics of the self-crosslinkable urethane (meth)acrylate of Examples 5-8 as studied by photo DSC

The resins and compositions used for the gel content studies in Example 9 and Example 10 were analyzed by photo-differential scanning calorimetry (photo DSC) to study basic cure kinetics as shown by the onset-to-peak exotherm time. This onset-to-peak time value corresponds to the time it takes for the polymerization to reach its peak reaction rate once the sample is irradiated with the selected light source.

Samples of neat self-crosslinkable urethane (meth)acrylates of Examples 5-8 and of the curable compositions comprising the self-crosslinkable urethane (meth)acrylates of Examples 5-8 combined with SR355 (di(trimethylolpropane) tetraacrylate) in a 1:1 weight ratio were studied as in Examples 9 and 10. The samples were irradiated either with a Hg-arc light source (Intensity of about 280 mW/cm² UVA) or a 405 nm LED light source (Intensity of about 280 mW/cm²). For the blends with SR355, cure in both an air environment and a nitrogen environment were studied. The study results are summarized in Table 11, and plotted in Figure 1 below.

**Table 11: Photo DSC results for the self-crosslinkable urethane (meth)acrylates of Examples 5-8 neat and as blends with SR355 in a 1:1 weight ratio.**

| **Sample** | **Cured In** | **Light** | **Onset to Peak time (min)** |
|---|---|---|---|
| Example 5 | Nitrogen | H bulb | 0.12 |
| Example 6 | Nitrogen | H bulb | 0.13 |
| Example 8 | Nitrogen | H bulb | 0.14 |
| Example 7 | Nitrogen | H bulb | 0.39 |
| Example 5 | Nitrogen | 405nm LED | 0.23 |
| Example 6 | Nitrogen | 405nm LED | 0.21 |
| Example 8 | Nitrogen | 405nm LED | 0.2 |
| Example 7 | Nitrogen | 405nm LED | 0.21 |
| | | | |
| Example 5 + SR355 | Nitrogen | H bulb | 0.06 |
| Example 6 + SR355 | Nitrogen | H bulb | 0.06 |
| Example 8 + SR355 | Nitrogen | H bulb | 0.07 |
| Example 7 + SR355 | Nitrogen | H bulb | 0.12 |
| Example 5 + SR355 | Nitrogen | 405nm LED | 0.12 |
| Example 6 + SR355 | Nitrogen | 405nm LED | 0.1 |
| Example 8 + SR355 | Nitrogen | 405nm LED | 0.12 |
| Example 7 + SR355 | Nitrogen | 405nm LED | 0.14 |
| | | | |
| Example 5 + SR355 | Air | H bulb | 0.06 |
| Example 6 + SR355 | Air | H bulb | 0.07 |
| Example 8 + SR355 | Air | H bulb | 0.12 |
| Example 7 + SR355 | Air | H bulb | 0.14 |
| Example 5 + SR355 | Air | 405nm LED | 0.13 |
| Example 6 + SR355 | Air | 405nm LED | 0.14 |
| Example 8 + SR355 | Air | 405nm LED | 0.13 |
| Example 7 + SR355 | Air | 405nm LED | 0.11 |

The above results show that the self-crosslinkable urethane (meth)acrylates of the invention provide excellent cure speed both alone and when diluted with other photocurable species, without the addition of a separate photoinitiator. Notably, an adjunct cure agent, such as an amine synergist, was not needed either. This provides benefits in terms of reduced or no extractables due to adding a separate, low molecular weight photoinitiator or a separate small-molecule source of abstractable hydrogens.

### Example 12: Self-crosslinkable urethane (meth)acrylate according to the invention

A hydroxyl-functionalized photoinitiator of formula (XXe9) was prepared by the reaction of 2-carboxymethylthioxanthone with trimethylolpropane triglycidyl ether. More details of preparation of this hydroxyl-functionalized photoinitiator are disclosed in patent application EP23305849.4 filed on May 30, 2023, the entire contents of which is incorporated herein for all purposes.

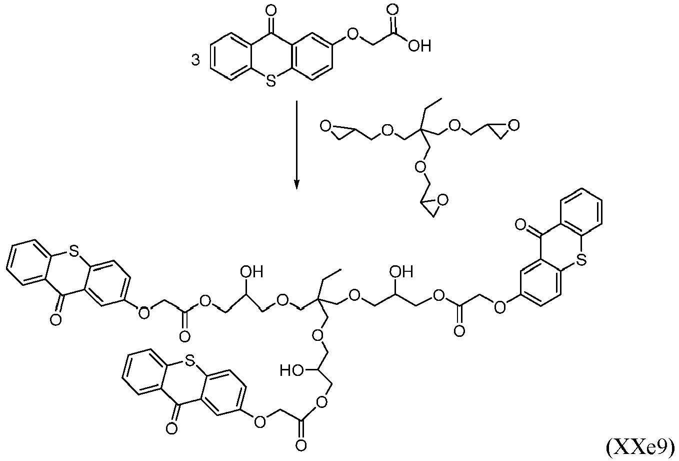

First, the hydroxyl-functionalized photoinitiator of formula (XXe9) was prepared by mixing 2-carboxymethylthioxanthone (CMTX from Arkema, 35.2 gram, 0.1167 mole), Trimethylolpropane triglycidyl ether (GE-30 from CVC, 18.7 gram, 0.136 mole), benzyltriethylammonium chloride (BTEAC from Aldrich, 0.14 g, 0.26 wt. %) as catalyst and cyclopentanone (54 g) as process solvent in a three-neck flask equipped with mechanical stirring under nitrogen, and heating to 130°C. The reaction was then run at 130°C until shut down criteria were reached at acid value (AV) < 2 mg KOH/gm and epoxy value (EV) < 2 mg KOH/gm (after 3 hours). The AV or EV was adjusted by adding more 2-carboxymethylthioxanthone or trimethylolpropane triglycidyl ether as the reaction proceeded as needed. The final product was a liquid exhibiting expected FT-IR and ¹H NMR spectral characteristics.

The self-crosslinkable urethane (meth)acrylate of Example 12 was then prepared as follows. A 1L resin kettle with lid equipped with air sparge, mechanical stirrer, thermocouple and internal temperature control and condenser was charged with SR495B (106.65 g, 0.3161 moles), the photoinitiator of formula (XXe9) (50% solids solution in cyclopentanone, 19.6 g of solution, 0.026 moles), BHT (0.19 g), and Desmodur W (66.7 g, 0.51 moles NCO). The mixture was stirred for 10 minutes, and then dibutyl tin dilaurate (DBTDL) was added (0.16 g) to the reactor. Stirring was continued and the reaction exothermed to an internal temperature of 75°C. The reaction was warmed to maintain an internal temperature of 75°C for 1 hour. An NCO titration as run and indicted 3.6% NCO. A second charge of SR495B was added (50.61 g, 0.15 moles) and the reaction was heated to 85°C until the NCO content of the reaction fell below 0.1 wt.% NCO. The contents of the reaction were discharged and exhibited FT-IR and GPC (Mₙ~1080, M_{w}~1458) properties consistent with the expected product.

### Example 13: Self-crosslinkable urethane (meth)acrylate according to the invention

An amino-functional self-crosslinkable urethane (meth)acrylate was prepared as follows using the amounts of reagents shown in Table 12 below. SR495B (part 1), the photoinitiator of formula (XXe1) pre-dissolved in cyclopentanone, N-methyldiethanolamine, BHT and Desmodur W were combined into a ½ liter resin kettle equipped with air sparge, agitator, thermocouple, temperature controller, heating mantle, side arm and condenser. This mixture was agitated for 10 minutes, and then DBTDL was added. The exotherm was allowed to reach 75°C, and heat was applied if needed. The reaction mixture was held at 75°C for 1 hour. The mixture was agitated at 75°C until NCO wt. % was below 0.1 wt. % as determined by FT-IR analysis. NCO wt.% was calculated from the 2260 cm⁻¹ NCO peak height based on an external standard calibration curve. SR495B was added if needed to reach the shutdown NCO criteria of <0.1 wt. % NCO.

**Table 12: Example 13 reagents loading:**

| **Component** | **MW** | **Grams** | **Mole** |
|---|---|---|---|
| (1) SR495B, part 1 | 337.4 | 68.83 | 0.204 |
| (2) Photoinitiator (XXe1)^{∗} (50 wt% in cyclopentanone) | 813 | 9.7 | 0.006 |
| (3) N-methyldiethanol amine (MDEA, Aldrich, > 99%) | 119.2 | 0.72 | 0.006 |
| (4) BHT (Aldrich) | | 0.16 | |
| (5) Desmodur W (Wanhua) | 262 | 35.0 | 0.1336 |
| (6) DBTDL | | 0.08 | |
| (7) SR495B, part 2 | | 20.58 | 0.061 |
| Total SR495B amount | | 89.41 | 0.265 |
| Total | | 135.07 | |

| | | | |
|---|---|---|---|
| * Photoinitiator (XXe1) with 50 wt% cyclopentanone and has a hydroxyl value of 69.1 mg KOH/gm by titration. | | | |

### Example 14: UV cured gel content study of the self-crosslinkable urethane (meth)acrylate of Example 13

Gel content studies were used to compare the reactivity of the amino-functional self-crosslinkable urethane (meth)acrylate of Example 13 with that of its analog of Example 5 without tertiary amine functionality. Samples of neat self-crosslinkable urethane (meth)acrylates of Examples 5 and 13 and of the curable compositions comprising the self-crosslinkable urethane (meth)acrylates of Examples 5 and 13 combined with SR355 (di(trimethylolpropane) tetraacrylate) in a 1:1 weight ratio were studied as in Examples 9 and 10. Gel content results are shown in Table 13 below. It can be seen that, while polymer bound amine synergist is not required to induce inherent reactivity, in some cases such as this one the presence of amine synergist functionality on the oligomer backbone can facilitate faster and more extensive UV cure.

**Table 13. Gel Content Comparison to Exemplify the Use of Polymer-Bound Tertiary Amine Synergist Effects on Reactivity.**

| **Sample #** | **Gel Content H Bulb (% gel)** | **Gel Content 395 nm LED (% gel)** |
|---|---|---|
| Example 13 | 93 | 94 |
| Example 13 + SR355 | 97 | 98 |
| Example 5 | 73 | 84 |
| Example 5 + SR355 | 77 | 92 |

### Example 15: Self-crosslinkable urethane (meth)acrylate according to the invention

A hydroxyl-functionalized photoinitiator of formula (XXe 15) was prepared by esterifying 2-carboxymethylthioxanthone and subsequently reacting the resulting methyl ester with diethanolamine.

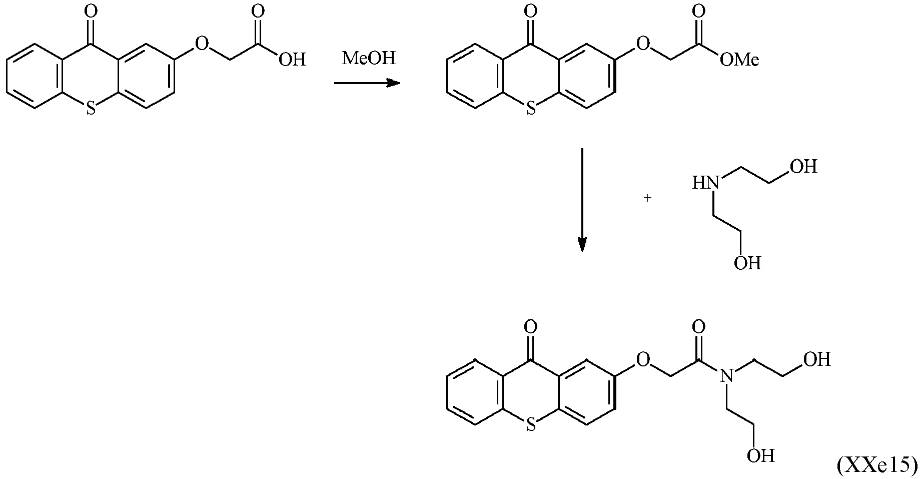

First, the hydroxyl-functionalized photoinitiator of formula (XXe15) was prepared by suspending 2-carboxymethylthioxanthone (130.4 g; 455.5 mmol) in methanol (1250 mL) and adding 98 wt% sulfuric acid (20 g) dropwise. The mixture was heated to reflux with stirring for 8 hours. The reaction was cooled to 20°C and analyzed by TLC (Petroleum Ether-AcOEt 1:1) to confirm near complete conversion to the methyl ester. Sodium acetate (30.0 g) was added and the mixture was stirred for 30 min. The resulting suspension was then partially evaporated until a thick mass was obtained (459.6 g). Water (1250 mL) was added and the suspension was stirred and then filtered. The solid crude product was then resuspended in water (750 mL) and a saturated aqueous NaHCO₃ solution (105 mL) was added. The mixture was stirred for 20 min, the solid product was filtered off washed with water (400 mL). The solid product was dried in vacuum at 50°C. This provided methyl [(9-oxo-9*H*-thioxanthen-2-yl)oxy]acetate (131.8 g; 96 % of theory) as a powder.

Methyl [(9-oxo-9*H*-thioxanthen-2-yl)oxy]acetate (131.2 g; 436.9 mmol), diethanolamine (161.5 g; 1.53 mol) and methanol (50 mL) were combined in a reaction flask purged with nitrogen. The reaction mixture was homogenised and heated to reflux (105-110°C) for 3 hours. The obtained dark orange mass was analysed by TLC (Dichloromethane-Methanol 10:1) to confirm complete conversion of the methyl ester. The reaction mass was poured into water (1000 mL) and the resulting suspension stirred vigorously for 30 min and filtered. The obtained crystalline product was resuspended in water (150mL), stirred vigorously for 1 hour and filtered. The isolated crystalline product was washed with water (100 mL) and dried in vacuum at 50°C. This provided the hydroxyl-functionalized photoinitiator of formula (XXe15) (146.5 g; 90 % of theory) as a crystalline solid having a melting point of 152-154°C with a purity (HPLC area) of 98.5%.

The self-crosslinkable urethane (meth)acrylate of Example 15 was then prepared as follows using the amounts of reagents detailed in Table 14. SR495B (part 1), the hydroxyl-functionalized photoinitiator of formula (XXe15), BHT and Desmodur W were combined in a 500 mL resin kettle equipped with air sparge, agitator, thermocouple, temperature controller, heating mantle, side arm and condenser. This mixture was agitated for 10 minutes, and then DBTDL was added. The reaction exothermed and was allowed to reach 110°C, and heat was applied when needed to maintain an internal temperature of about 110°C. The reaction mixture was held at 110°C for 1 hour after all the hydroxyl-functionalized photoinitiator of formula (XXe15) disappeared. The intermediate isocyanate (NCO) level was checked until it's below 3.2 wt. % or it stalled. SR495B (part 2) was added. The mixture was agitated at 110°C until NCO wt. % was below 0.1 wt. %.

**Table 14: Example 15 reagents loading**

| **Component** | **MW** | **Grams** | **Mole** |
|---|---|---|---|
| (1) SR495B, part 1 | 337.4 | 45.89 | 0.136 |
| (2) Photoinitiator (XXe15) | 373 | 4.0 | 0.011 |
| (3) BHT (Aldrich) | | 0.11 | |
| (4) Desmodur W (Covestro) | 262 | 28.7 | 0.110 |
| (5) DBTDL | | 0.09 | |
| (6) SR495B, part 2 | | 14.85 | 0.044 |
| Total SR495B amount | | 60.74 | 0.180 |
| Total | | 93.64 | |

### Example 16: Self-crosslinkable urethane (meth)acrylate according to the invention

The self-crosslinkable urethane (meth)acrylate of Example 16 was prepared in the same way as Example 15, except that isophorone diisocyanate (IPDI, Wanhua) was used as the diisocyanate component and the amounts of the reagents were adjusted as shown below in Table 15.

**Table 15: Example 16 reagent loading.**

| **Component** | **MW** | **Grams** | **Mole** |
|---|---|---|---|
| (1) SR495B, part 1 | 337.4 | 55.67 | 0.165 |
| (2) Photoinitiator (XXe15) | 373 | 4.1 | 0.011 |
| (3) BHT (Aldrich) | | 0.10 | |
| (4) IPDI (Wanhua) | 222.29 | 28.0 | 0.126 |
| (5) DBTDL | | 0.06 | |
| (6) SR495B, part 2 | | 16.87 | 0.050 |
| Total SR495B amount | | 72.54 | 0.215 |
| Total | | 104.8 | |

### Example 17: Self-crosslinkable urethane (meth)acrylate according to the invention

The self-crosslinkable urethane (meth)acrylate of Example 17 was prepared as follows using the amounts of reagents shown in Table 16 below. Pre-melted Fomrez^{®} 55-225 (a polyesterdiol from Chemtura), the hydroxyl-functionalized photoinitiator of formula (XXe 15) synthesized in Example 15 and IPDI were combined into a 500 mL resin kettle equipped with nitrogen sparge, agitator, thermocouple, temperature controller, heating mantle, side arm and condenser. This mixture was agitated for 10 minutes, and then Reaxis^{®} C716 (bismuth neodecanoate) catalyst was added. The reaction exothermed and was allowed to reach 130°C, and heat was applied if needed. The reaction mixture was held at 130°C for 1 hour after all the photoinitiator disappeared. The intermediate isocyanate (NCO) level was checked until it was below 12.8 wt. % or the value stopped changing over a period of 30 minutes. The mixture was cooled to 90°C. Irganox^{®} 1035 antioxidant was added and the nitrogen sparge was switched to air sparge. 2-hydroxyethyl acrylate (HEA) was fed into the resin kettle through an addition funnel over the course of one hour. The reaction mixture was allowed to exotherm to 95°C and held at 95°C until the NCO weight % was less than 0.1 %.

**Table 16: Example 17 reagent loading**

| **Component** | **MW** | **Grams** | **Mole** |
|---|---|---|---|
| (1) Fomrez^{®} 55-225 | 500 | 93.23 | 0.187 |
| (2) Photoinitiator (XXe15) | 373 | 12.7 | 0.034 |
| (3) IPDI (Evonik) | 222.29 | 129.0 | 0.580 |
| (4) Reaxis^{®} C716 | | 0.30 | |
| (5) Irganox^{®} 1035 | | 1.00 | |
| (6) HEA (Nippon Shokubai) | | 82.2 | 0.708 |
| Total | | 318.43 | |

### Example 18: Self-crosslinkable urethane (meth)acrylate according to the invention

The self-crosslinkable urethane (meth)acrylate of Example 18 was prepared as follows using the amounts of reagents shown in Table 17 below. Pre-melted poly(tetrahydrofuran) (PolyTHF 650, BASF, polyether diol), the hydroxyl-functionalized photoinitiator of formula (XXe 15) synthesized in Example 15 and IPDI were combined into a 500 mL resin kettle equipped with nitrogen sparge, agitator, thermocouple, temperature controller, heating mantle, side arm and condenser. This mixture was agitated for 10 minutes, and then Reaxis^{®} C716 (bismuth neodecanoate) catalyst was added. The exothermic temperature was allowed to reach 130°C, and heat was applied if needed. The reaction mixture was held at 130°C for 1 hour after all the photoinitiator disappeared. The intermediate isocyanate (NCO) level was monitored until it was below 8.1 wt. % or remained constant over a period of 30 minutes. The mixture was cooled to 90°C. Irganox^{®} 1035 antioxidant was added and the nitrogen sparge was switched to air sparge. 2-hydroxyethyl acrylate (HEA) was fed into the resin kettle through an addition funnel over the course of one hour. The reaction mixture was allowed to exotherm to 95°C and held at 95°C until the NCO weight % was less than 0.1 %.

**Table 17: Example 18 reagent loading**

| **Component** | **MW** | **Grams** | **Mole** |
|---|---|---|---|
| (1) Photoinitiator (XXe15) | 373 | 12.4 | 0.033 |
| (7) PolyTHF650 | 650 | 135.37 | 0.208 |
| (1) IPDI (Evonik) | 222.29 | 109.2 | 0.4913 |
| (4) Reaxis^{®} C716 | | 0.16 | |
| (2) Irganox^{®} 1035 (BASF) | | 1.34 | |
| (5) HEA (Nippon Shokubai) | | 56.09 | 0.483 |
| Total | | 314.56 | |

### Example 19: UV cured gel content study of the self-crosslinkable urethane (meth)acrylates of Examples 15-16.

The self-crosslinkable urethane (meth)acrylates of Examples 15-16 were coated onto an aluminum substrate using a drawdown bar to produce nominally 50 µm thick films. No discrete photoinitiator was added to the coated film material. The films were UV cured using either a Fusion H bulb (300 W/in, dose~1JUVA/cm²) or a Phoseon 8W/cm² 395 nm LED. Films were removed from the substrate, weighed, and soaked in THF for >24 h. The remaining solid was isolated, dried at 65°C/4h, and re-weighed to calculate gel content.

Both self-crosslinkable urethane (meth)acrylates exhibited inherent reactivity as judged by the gel contents tabulated in Table 18 below. This study exemplifies that the self-crosslinkable urethane (meth)acrylates of the invention can efficiently serve the role of both photoinitiator and curable oligomer. The examples also show that the hydrogen donor functionality needed for Type II photoinitiation can be supplied by the resin backbone itself.

**Table 18: Gel contents of self-crosslinkable urethane (meth)acrylates of Examples 15-16, neat.**

| **Sample #** | **Gel Content H Bulb (% gel)** | **Gel Content 395 nm LED (% gel)** |
|---|---|---|
| Example 15 | 65 | 50 |
| Example 16 | 73 | 59 |

### Example 20: Curable compositions comprising the self-crosslinkable urethane (meth)acrylates of Examples 15-16

The self-crosslinkable urethane (meth)acrylates of Examples 15-16 were blended in 1:1 weight ratio with SR355 (di(trimethylolpropane) tetraacrylate, Sartomer). No additional discrete photoinitiator was added to the formulation. These 100% solids liquid formulations containing the self-crosslinkable urethane (meth)acrylates of Examples 15-16 were coated onto an aluminum substrate using a drawdown bar to produce nominally 50 µm thick films. The films were UV cured using either a Fusion H bulb (300 W/in, dose~1 J of UVA/cm²) or a Phoseon 8W/cm² 395 nm LED. The cured films were removed from the substrate, weighed, and soaked in THF for at least 24 hours to dissolve any non-crosslinked material. The remaining solid was isolated, dried at 65°C for four hours, and re-weighed to calculate gel content.

All the curable formulations which included the self-crosslinkable urethane (meth)acrylates of the invention exhibited high reactivity as shown by the high gel contents tabulated in Table 19 below. The high gel contents using the 395 nm LED exemplify the excellent activity of the resins at the longer wavelengths typical of common industrial LED light sources. This gel content study demonstrated that the self-crosslinkable urethane (meth)acrylates of the invention can efficiently serve as photoinitiators for other radically curable materials in addition to being inherently curable themselves. As noted in Example 19, the efficient cure obtained shows that no additional H-donor or synergist is needed to obtain excellent UV curability when the self-crosslinkable urethane (meth)acrylates of the invention are utilized.

**Table 19: Gel contents of the self-crosslinkable urethane (meth)acrylates of Example 15-16 in combination with SR355 in 1:1 weight ratio.**

| **Sample #** | **Gel Content H Bulb (% gel)** | **Gel Content 395 nm LED (% gel)** |
|---|---|---|
| Example 15 + SR355 | 89 | 96 |
| Example 16 + SR355 | 80 | 97 |

Within this specification embodiments have been described in a way which enables a clear and concise specification to be written, but it is intended and will be appreciated that embodiments may be variously combined or separated without departing from the invention. For example, it will be appreciated that all preferred features described herein are applicable to all aspects of the invention described herein. The foregoing description of various forms of the invention has been presented for purposes of illustration and description. It is not intended to be exhaustive or to limit the invention to the precise forms disclosed. Numerous modifications or variations are possible in light of the above teachings. All such modifications and variations are within the scope of the invention as determined by the appended claims when interpreted in accordance with the breadth to which they are fairly, legally, and equitably entitled.

## Claims

1. A self-crosslinkable urethane (meth)acrylate comprising:
at least one (meth)acrylate-functionalized moiety ACR;
at least two polyurethane moieties UU;
at least one chromophore moiety Q;
optionally at least one chain-extending moiety EXT.

2. The self-crosslinkable urethane (meth)acrylate according to claim 1, wherein each (meth)acrylate-functionalized moiety ACR is independently according to the following formula (I): 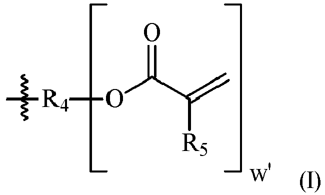 wherein
R₄ is a (w'+1)valent linker;
R₅ is H or methyl;
w' is 1 to 5; in particular 1 to 3, more particularly 1.

3. The self-crosslinkable urethane (meth)acrylate according to claim 1 or 2, wherein each polyurethane moiety UU is independently according to one of the following formula (VIIa) or (VIIb): 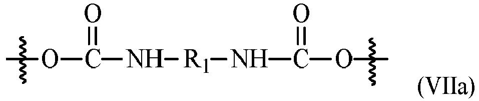 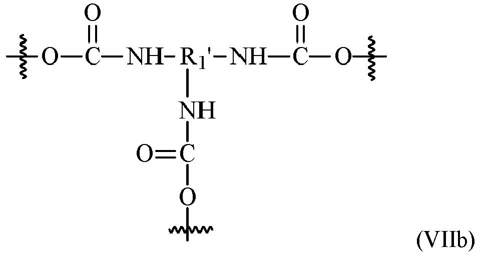 wherein
R₁ and R₁' are independently an aliphatic linker or an aromatic linker.

4. The self-crosslinkable urethane (meth)acrylate according to any one of claims 1 to 3, wherein each chromophore moiety Q independently comprises at least one monovalent photoinitiator moiety PI corresponding to one of the following formulae (VIII), (XI), (XIV), (XVII), (XVIII) or (XIX), or at least one divalent photoinitiator moiety PI corresponding to one of the following formulae (IX), (X), (XII), (XIII), (XV) or (XVI): 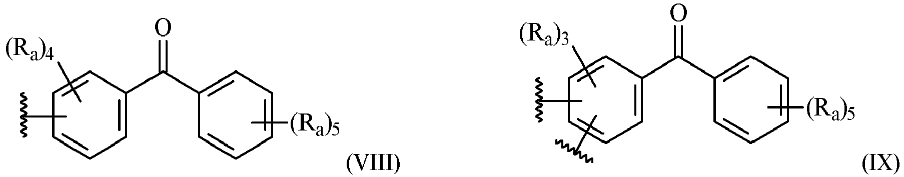 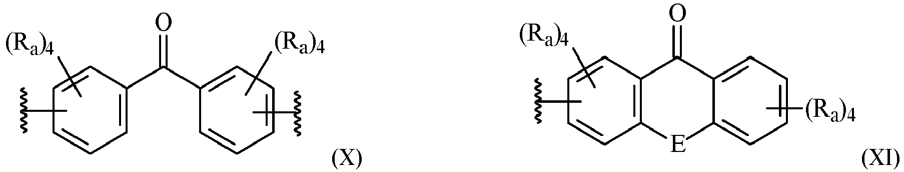 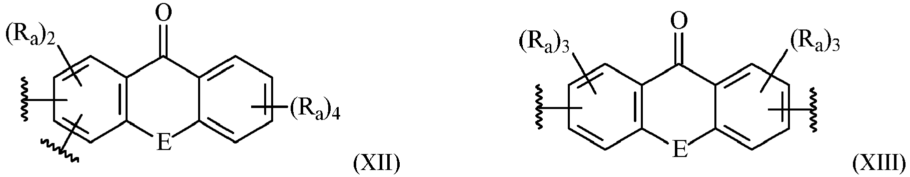 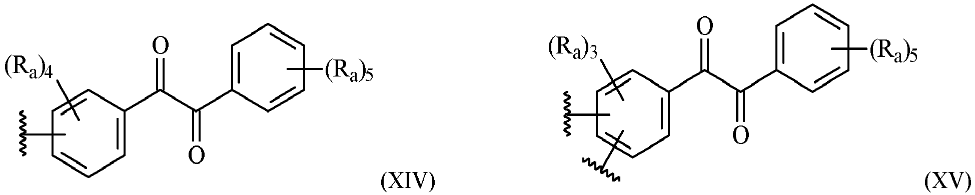 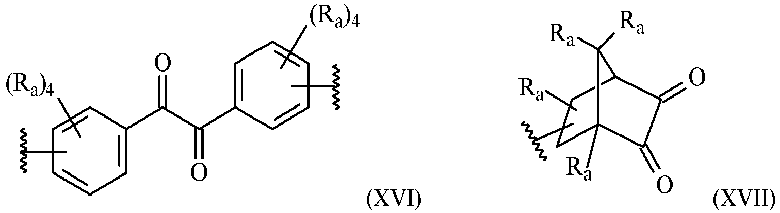 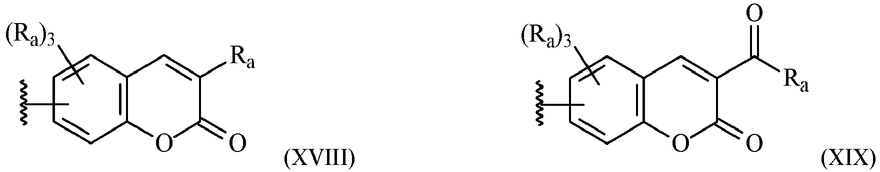 wherein
each E is independently S, O or NR, in particular S;
R is H, an optionally substituted alkyl or an optionally substituted aryl;
each Rₐ is independently H, F, Cl, Br, I, -OR_{b}, -SR_{b}, -N(R_{b})₂, -NO₂, -CN, -C(=O)R_{b}, -O-C(=O)R_{b}, -C(=O)OR_{b}, -C(=O)N(R_{b})₂, -NR_{b}-C(=O)-R_{b}, -SO₂-N(R_{b})₂, or an optionally substituted group selected from the group consisting of alkyl, heteroatom-containing alkyl, cycloalkyl, heterocycloalkyl, alkenyl, alkynyl, aryl, aralkyl, alkaryl and heteroaryl;
two adjacent Rₐ groups may form, with the carbon atoms to which they are attached, a 5 to 8 membered ring;
each R_{b} is independently H or an optionally substituted group selected from alkyl, heteroatom-containing alkyl, cycloalkyl, heterocycloalkyl, alkenyl, alkynyl, aryl, aralkyl, alkaryl and heteroaryl.

5. The self-crosslinkable urethane (meth)acrylate according to any one of claims 1 to 4, wherein each chromophore moiety Q is independently according to one of the following formulae (XXa), (XXb) or (XXc): 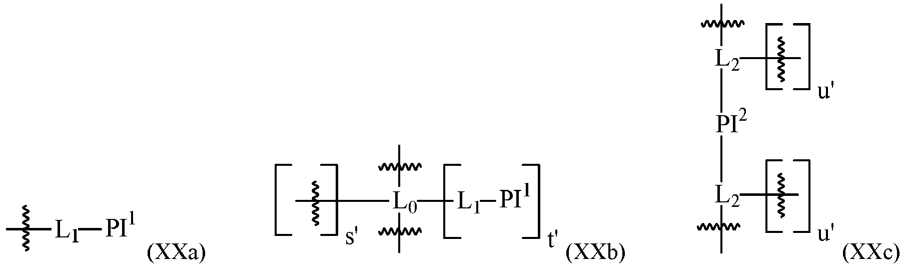 wherein
L₀ is a (s'+t'+2)valent linker;
each L₁ is independently a divalent linker;
each L₂ is independently a (u'+2)valent linker;
each PI¹ is independently a monovalent photoinitiator moiety PI as defined in claim 4;
each PI² is independently a divalent photoinitiator moiety PI as defined in claim 4;
s' is 0, 1 or 2; in particular 0 or 1;
t' is 1, 2, 3 or 4; in particular 2 or 3;
each u' is independently 0 or 1, in particular 0.

6. The self-crosslinkable urethane (meth)acrylate according to any one of claims 1 to 5, wherein the self-crosslinkable urethane (meth)acrylate comprises at least one chromophore moiety Q according to formula (XXa) as defined in claim 5, wherein
L₁ is preferably selected from direct bond, alkylene of formula (C-1), oxyalkylene of formula (C-2), thioalkylene of formula (C-3), ketoalkylene of formula (C-4), aminoalkylene of formula (C-5), carboxyalkylene of formula (C-6), amidoalkylene of formula (C-7), alkylene-aminoalkylene of formula (C-8), oxyalkylene-aminoalkylene of formula (C-9), oxyalkylene-amidoalkylene of formula (C-10): 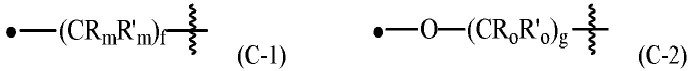 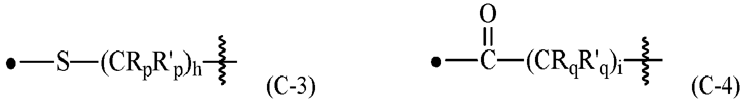 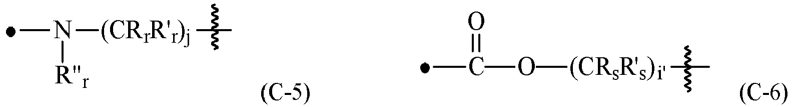 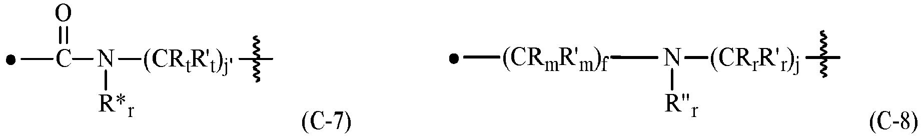 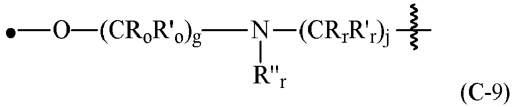 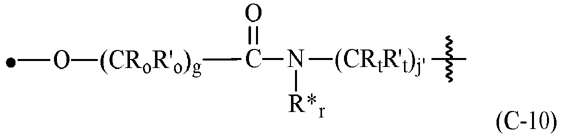
wherein
each Rₘ, R'ₘ, Rₒ, R'ₒ, Rₚ, R'ₚ, R_{q}, R'_{q}, Rᵣ, R'ᵣ, Rₛ, R'ₛ, Rₜ and R'ₜ is independently H or an optionally substituted alkyl, in particular H;
R"ᵣ and R^{∗}ᵣ are independently H or an optionally substituted alkyl;
f, g, h, j and j' are independently 1, 2, 3, 4, 5 or 6, in particular 1 or 2;
i and i' are independently 1, 2, 3, 4 or 5, in particular 1;
the symbol • represents a point of attachment to the PI moiety;
PI¹ is preferably according to formula (VIII) or (XI) as defined in claim 4 wherein E in formula (XI) is S.

7. The self-crosslinkable urethane (meth)acrylate according to any one of claims 1 to 6, wherein the self-crosslinkable urethane (meth)acrylate comprises at least one chromophore moiety Q according to formula (XXc) as defined in claim 5, wherein
each L₂ is preferably selected from direct bond, alkylene of formula (C-1) as defined in claim 6, oxyalkylene of formula (C-2) as defined in claim 6, thioalkylene of formula (C-3) as defined in claim 6, ketoalkylene of formula (C-4) as defined in claim 6, aminoalkylene of formula (C-5) as defined in claim 6, carboxyalkylene of formula (C-6) as defined in claim 6, amidoalkylene of formula (C-7) as defined in claim 6, alkylene-aminoalkylene of formula (C-8) as defined in claim 6, oxyalkylene-aminoalkylene of formula (C-9) as defined in claim 6, or oxyalkylene-amidoalkylene of formula (C-10) as defined in claim 6,
each u' is preferably 0;
PI² is preferably according to formula (IX), (X), (XII) or (XIII) as defined in claim 4 wherein E in formula (XII) and (XIII) is S.

8. The self-crosslinkable urethane (meth)acrylate according to any one of claims 1 to 7, wherein the self-crosslinkable urethane (meth)acrylate comprises at least one chromophore moiety Q according to the following formula (XXI): 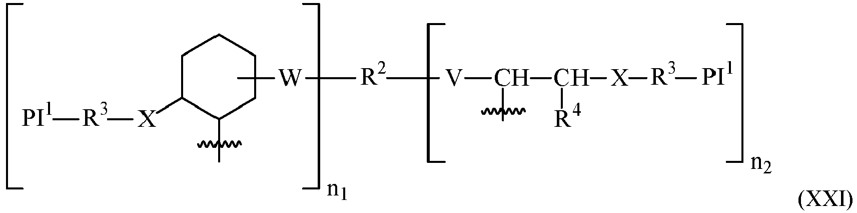 wherein
n₁ is 0, 1, 2, 3 or 4;
n₂ is 0, 1, 2, 3 or 4;
the sum n₁ + n₂ is equal to 1, 2, 3 or 4;
each PI¹ is independently a monovalent photoinitiator moiety PI as defined in claim 4;
each X is independently -NR¹-, -O-, -S-, ^{∗}-C(=O)-O-, or ^{∗}-C(=O)-NR¹-;
each V is independently direct bond, #-O-CH₂-, #-C(=O)-O-CH₂-, #-NR⁵-CH₂- or #-C(=O)-NR⁵-CH₂-;
each W is independently direct bond, -CH₂-O-C(=O))-# or -C(=O)-O-#;
each R¹ is independently H, alkyl or aryl;
R² is a direct bond or a linker;
each R³ is independently a direct bond or a linker;
each R⁴ is independently H, an optionally substituted alkyl or an optionally substituted alkenyl;
each R⁵ is independently H, alkyl, aryl or a group according to the following formula: 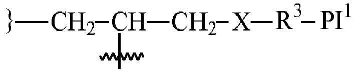 wherein X, R³ and PI¹ are as defined above and the symbol } represents the point of attachment to the nitrogen atom;
the symbol * represents a point of attachment to R³;
the symbol 4 represents a point of attachment to R².

9. The self-crosslinkable urethane (meth)acrylate according to any one of claims 1 to 8, wherein the self-crosslinkable urethane (meth)acrylate comprises at least one chromophore moiety Q according to one of the following formulae (XXXVI) to (XXXXI): 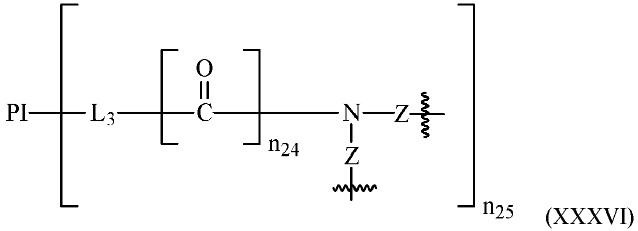 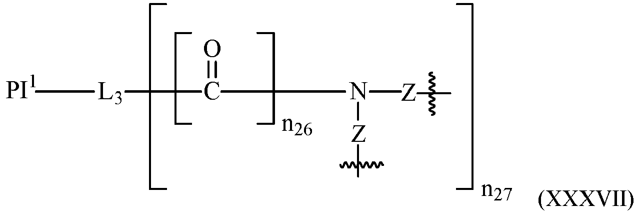 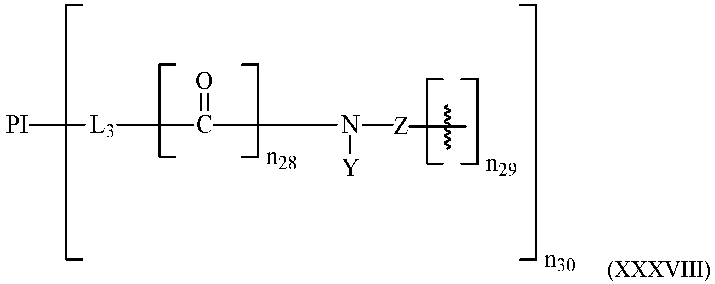 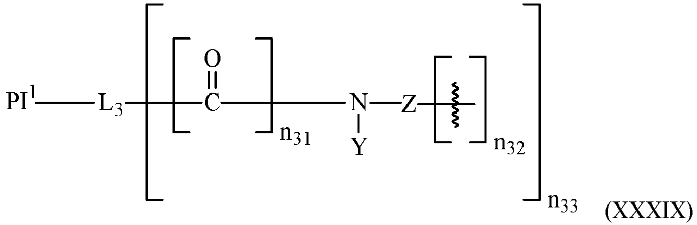 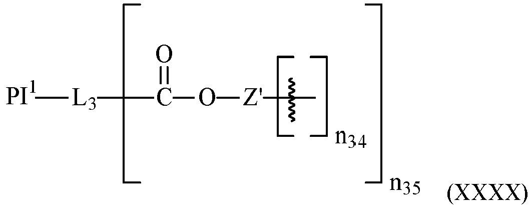 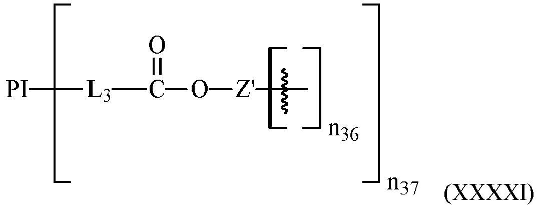 wherein
each PI is independently a monovalent or divalent photoinitiator moiety PI as defined in claim 4;
each PI¹ is independently a monovalent photoinitiator moiety PI as defined in claim 4;
each L₃ is independently selected from a direct bond or a linker comprising 1 to 20 carbon atoms;
each Y is independently H or alkyl;
each Z and Z' is independently a linker comprising 1 to 20 carbon atoms;
each n₂₄, n₂₆, n₂₈ and n₃₁ is independently 0 or 1, in particular 1;
each n₂₉, n₃₂, n₃₄ and n₃₆ is independently 1, 2 or 3, in particular 1;
each n₂₇ and n₃₃ is independently 1, 2 or 3; in particular 1 or 2;
each n₂₅ and n₃₀ is independently 1 or 2, in particular 1;
n₃₅ is 1, 2 or 3, in particular 2;
n₃₇ is 1 or 2, in particular 2.

10. The self-crosslinkable urethane (meth)acrylate according to any one of claims 1 to 9, wherein the self-crosslinkable urethane (meth)acrylate comprises at least one chain-extending moiety EXT which is the residue of a polyol selected from the residue of a polymeric polyol, the residue of a non-polymeric polyol or the residue of an amino-functional polyol.

11. The self-crosslinkable urethane (meth)acrylate according to any one of claims 1 to 10, wherein the self-crosslinkable urethane (meth)acrylate corresponds to the following structure (A) 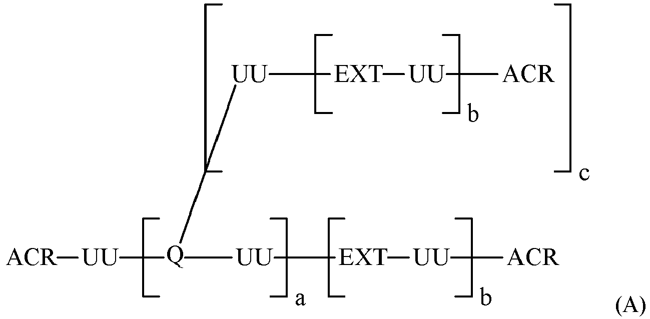
wherein ACR, UU, Q and EXT are as defined in any one of claims 1 to 10;
a is an integer from 1 to 100;
b is an integer from 0 to 100;
c is an integer equal to 0, 1 or 2, preferably 0 or 1.

12. The self-crosslinkable urethane (meth)acrylate according to any one of claims 1 to 10, wherein the self-crosslinkable urethane (meth)acrylate corresponds to the following structure (B): 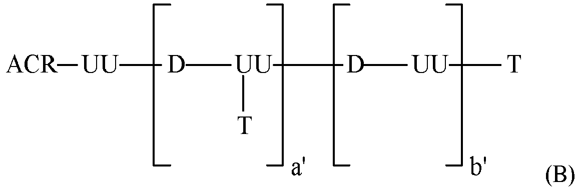 wherein
each D is independently Q or EXT;
each T is independently ACR or Q;
ACR, UU, Q and EXT are as defined in any one of claims 1 to 10;
a' is an integer from 0 to 100;
b' is an integer from 0 to 100;
wherein at least one of a' and b' is not 0; and
at least one of D and T is Q.

13. A process for the preparation of a self-crosslinkable urethane (meth)acrylate, wherein the process comprises reacting:
a) a hydroxyl-functionalized (meth)acrylate component;
b) a polyisocyanate component;
c) a hydroxyl-functionalized photoinitiator component; and
d) optionally, a polyol component.

14. A process for polymerizing one or more ethylenically unsaturated compounds comprising contacting one or more ethylenically unsaturated compounds with a self-crosslinkable urethane (meth)acrylate according to any one of claims 1 to 12 or prepared with the process according to claim 14, and irradiating the mixture, in particular with UV, near-UV, visible, infrared, near-infrared and/or electron beam radiation.

15. A curable composition comprising:
A) a self-crosslinkable urethane (meth)acrylate according to any one of claims 1 to 12 or prepared with the process according to claim 13; and
optionally B) a polymerizable component other than A).
